# EUROPEAN PATENT APPLICATION

(11) **EP 3 574 916 A1**
(43) Date of publication of application: **04.12.2019**
(21) Application number: 19172853.4
(22) Date of filing: 21.08.2014
(51) Int. Cl.: A61K 39/00, A61P 35/00

(54) **COMPOSITION AND VACCINE FOR TREATING LUNG CANCER**

(30) Priority: 21.08.2013 WO PCT/EP2013/002514
(62) Divisional of application: 14755334.1
(71) Applicant: CureVac AG, 72076 Tübingen (DE)
(72) Inventor: Kallen, Karl-Josef, 72076 Tübingen (DE); Fotin-Mleczek, Mariola, 72076 Tübingen (DE); Gnad-Vogt, Ulrike, 72076 Tübingen (DE)
(74) Representative: Graf von Stosch Patentanwaltsgesellschaft mbH

(57) **Abstract**

The present invention relates to a composition comprising at least one mRNA encoding a combination of antigens capable of eliciting an (adaptive) immune response in a mammal, wherein the antigens are selected from the group consisting of 5T4 (Trophoblast glycoprotein, TPBG), Survivin (Baculoviral IAP repeat-containing protein 5; BIRC5), NY-ESO-1 (New York esophageal squamous cell carcinoma 1, CTAG1B), MAGE-C1 (Melanoma antigen family C1), MAGE-C2 (Melanoma antigen family C2), and MUC1 (Mucin 1). The invention furthermore relates to a vaccine comprising at least one mRNA encoding such a combination of antigens, and to the use of said composition (for the preparation of a vaccine) and/or of the vaccine for eliciting an (adaptive) immune response for the treatment of lung cancer, preferably of non-small cell lung cancer (NSCLC), and diseases or disorders related thereto. Finally, the invention relates to kits, particularly to kits of parts, containing the composition and/or the vaccine.

SEQUENCE LISTING

<110>  CureVac AG

<120>  Composition and Vaccine for Treating Lung Cancer

<130>  CU01P148WO1EPT1

<160>  83

<170>  PatentIn version 3.5

<210>  1
<211>  1467
<212>  RNA
<213>  Artificial Sequence

<220>
<223>  5T4 (GC)-muag-A64-C30

<400>  1
   gggagaaagc uuaccaugcc cggcgggugc agccggggcc cggccgccgg ggacggccgc      60
   cugcggcucg cgcgccuggc ccuggugcuc cugggguggg ucuccagcuc cagccccacc      120
   uccagcgccu ccagcuucuc cagcuccgcc cccuuccugg ccagcgcggu guccgcccag      180
   cccccgcucc ccgaccagug ccccgcccug ugcgagugca gcgaggccgc gcggaccgug      240
   aagugcguca accgcaaccu gacggaggug cccaccgacc ucccggccua cgugcggaac      300
   cuguuccuga ccggcaacca gcucgccguc cugcccgccg gcgccuucgc gcgccggccg      360
   ccccuggccg agcucgccgc ccugaaccug uccgggagcc gccucgacga ggugcgggcc      420
   ggcgcguucg agcaccugcc gucccugcgc cagcucgacc ugagccacaa cccccuggcc      480
   gaccucuccc ccuucgccuu cagcgggagc aacgccuccg ugagcgcccc cuccccgcug      540
   gucgagcuga uccucaacca caucgugccc cccgaggacg agcggcagaa ccgcagcuuc      600
   gagggcaugg uggucgcggc ccugcuggcc gggcgggccc uccagggccu gcgccggcug      660
   gagcucgccu ccaaccacuu ccuguaccug ccccgcgacg ugcucgcgca gcugccgagc      720
   cugcggcacc ucgaccuguc caacaacagc cugguguccc ucaccuacgu cagcuuccgc      780
   aaccugacgc accuggaguc ccuccaccug gaggacaacg cccugaaggu gcugcacaac      840
   ggcacccucg ccgagcugca ggggcugccc cacauccggg uguuccucga caacaacccc      900
   ugggucugcg acugccacau ggccgacaug gugaccuggc ugaaggagac cgaggugguc      960
   cagggcaagg accgccugac gugcgcguac cccgagaaga ugcggaaccg ggugcuccug      1020
   gagcugaaca gcgccgaccu cgacugcgac ccgauccugc cccccucccu gcagaccagc      1080
   uacguguucc ucgggaucgu ccuggcccug aucggcgcca ucuuccuccu ggugcuguac      1140
   cucaaccgca agggcaucaa gaaguggaug cacaacaucc gggacgccug ccgcgaccac      1200
   auggaggggu accacuaccg guacgagauc aacgcggacc cccgccugac caaccugucc      1260
   agcaacuccg acgucugacc acuaguuaua agacugacua gcccgauggg ccucccaacg 1320
   ggcccuccuc cccuccuugc accgagauua auaaaaaaaa aaaaaaaaaa aaaaaaaaaa 1380
   aaaaaaaaaa aaaaaaaaaa aaaaaaaaaa aaaaaauauu cccccccccc cccccccccc 1440
   cccccccccc ucuagacaau uggaauu 1467

<210> 2
<211> 1263
<212> RNA
<213> Artificial Sequence

<220>
<223> 5T4 CDS wild type

<400> 2
augccugggg ggugcucccg gggccccgcc gccggggacg ggcgucugcg gcuggcgcga 60

cuagcgcugg uacuccuggg cugggucucc ucgucuucuc ccaccuccuc ggcauccucc 120

uucuccuccu cggcgccguu ccuggcuucc gccguguccg cccagccccc gcugccggac 180

cagugccccg cgcugugcga gugcuccgag gcagcgcgca cagucaagug cguuaaccgc 240

aaucugaccg aggugcccac ggaccugccc gccuacgugc gcaaccucuu ccuuaccggc 300

aaccagcugg ccgugcuccc ugccggcgcc uucgcccgcc ggccgccgcu ggcggagcug 360

gccgcgcuca accucagcgg cagccgccug gacgaggugc gcgcgggcgc cuucgagcau 420

cugcccagcc ugcgccagcu cgaccucagc cacaacccac uggccgaccu cagucccuuc 480

gcuuucucgg gcagcaaugc cagcgucucg gcccccaguc cccuugugga acugauccug 540

aaccacaucg ugcccccuga agaugagcgg cagaaccgga gcuucgaggg caugguggug 600

gcggcccugc uggcgggccg ugcacugcag gggcuccgcc gcuuggagcu ggccagcaac 660

cacuuccuuu accugccgcg ggaugugcug gcccaacugc ccagccucag gcaccuggac 720

uuaaguaaua auucgcuggu gagccugacc uacguguccu uccgcaaccu gacacaucua 780

gaaagccucc accuggagga caaugcccuc aagguccuuc acaauggcac ccuggcugag 840

uugcaagguc uaccccacau uaggguuuuc cuggacaaca aucccugggu cugcgacugc 900

cacauggcag acauggugac cuggcucaag gaaacagagg uagugcaggg caaagaccgg 960

cucaccugug cauauccgga aaaaaugagg aaucgggucc ucuuggaacu caacagugcu 1020

gaccuggacu gugacccgau ucuuccccca ucccugcaaa ccucuuaugu cuuccugggu 1080

auuguuuuag cccugauagg cgcuauuuuc cuccugguuu uguauuugaa ccgcaagggg 1140

auaaaaaagu ggaugcauaa caucagagau gccugcaggg aucacaugga aggguaucau 1200

uacagauaug aaaucaaugc ggaccccaga uuaacgaacc ucaguucuaa cucggauguc 1260

uga 1263

<210> 3
<211> 1263
<212> RNA
<213> Artificial Sequence

<220>
<223> 5T4 CDS GC-optimized

<400> 3
augcccggcg ggugcagccg gggcccggcc gccggggacg gccgccugcg gcucgcgcgc 60

cuggcccugg ugcuccuggg gugggucucc agcuccagcc ccaccuccag cgccuccagc 120

uucuccagcu ccgcccccuu ccuggccagc gcgguguccg cccagccccc gcuccccgac 180

cagugccccg cccugugcga gugcagcgag gccgcgcgga ccgugaagug cgucaaccgc 240

aaccugacgg aggugcccac cgaccucccg gccuacgugc ggaaccuguu ccugaccggc 300

aaccagcucg ccguccugcc cgccggcgcc uucgcgcgcc ggccgccccu ggccgagcuc 360

gccgcccuga accuguccgg gagccgccuc gacgaggugc gggccggcgc guucgagcac 420

cugccguccc ugcgccagcu cgaccugagc cacaaccccc uggccgaccu cucccccuuc 480

gccuucagcg ggagcaacgc cuccgugagc gcccccuccc cgcuggucga gcugauccuc 540

aaccacaucg ugccccccga ggacgagcgg cagaaccgca gcuucgaggg cauggugguc 600

gcggcccugc uggccgggcg ggcccuccag ggccugcgcc ggcuggagcu cgccuccaac 660

cacuuccugu accugccccg cgacgugcuc gcgcagcugc cgagccugcg gcaccucgac 720

cuguccaaca acagccuggu gucccucacc uacgucagcu uccgcaaccu gacgcaccug 780

gagucccucc accuggagga caacgcccug aaggugcugc acaacggcac ccucgccgag 840

cugcaggggc ugccccacau ccggguguuc cucgacaaca accccugggu cugcgacugc 900

cacauggccg acauggugac cuggcugaag gagaccgagg ugguccaggg caaggaccgc 960

cugacgugcg cguaccccga gaagaugcgg aaccgggugc uccuggagcu gaacagcgcc 1020

gaccucgacu gcgacccgau ccugcccccc ucccugcaga ccagcuacgu guuccucggg 1080

aucguccugg cccugaucgg cgccaucuuc cuccuggugc uguaccucaa ccgcaagggc 1140

aucaagaagu ggaugcacaa cauccgggac gccugccgcg accacaugga gggguaccac 1200

uaccgguacg agaucaacgc ggacccccgc cugaccaacc uguccagcaa cuccgacguc 1260

uga 1263

<210> 4
<211> 633
<212> RNA
<213> Artificial Sequence

<220>
<223> Survivin (GC)-muag-A64-C30

<400> 4

gggagaaagc uuaccauggg cgcccccacc cugccgccgg ccuggcagcc guuccucaag 60

gaccaccgca ucucgaccuu caagaacugg ccguuccugg agggcugcgc gugcaccccg 120

gagcggaugg ccgaggccgg cuucauccac ugccccaccg agaacgagcc ggaccuggcc 180

cagugcuucu ucugcuucaa ggagcuggag ggcugggagc cggacgacga cccgaucgag 240

gagcacaaga agcacagcag cggcugcgcc uuccugagcg ugaagaagca guucgaggag 300

cugacgcucg gggaguuccu gaagcuggac cgggagcggg ccaagaacaa gaucgcgaag 360

gagaccaaca acaagaagaa ggaguucgag gagaccgcca agaaggugcg gcgggccauc 420

gagcagcugg ccgccaugga cugaccacua guuauaagac ugacuagccc gaugggccuc 480

ccaacgggcc cuccuccccu ccuugcaccg agauuaauaa aaaaaaaaaa aaaaaaaaaa 540

aaaaaaaaaa aaaaaaaaaa aaaaaaaaaa aaaaaaaaaa aauauucccc cccccccccc 600

cccccccccc ccccccucua gacaauugga auu 633

<210> 5
<211> 429
<212> RNA
<213> Artificial Sequence

<220>
<223> Survivin CDS wild type

<400> 5
augggugccc cgacguugcc cccugccugg cagcccuuuc ucaaggacca ccgcaucucu 60

acauucaaga acuggcccuu cuuggagggc ugcgccugca ccccggagcg gauggccgag 120

gcuggcuuca uccacugccc cacugagaac gagccagacu uggcccagug uuucuucugc 180

uucaaggagc uggaaggcug ggagccagau gacgacccca uagaggaaca uaaaaagcau 240

ucguccgguu gcgcuuuccu uucugucaag aagcaguuug aagaauuaac ccuuggugaa 300

uuuuugaaac uggacagaga aagagccaag aacaaaauug caaaggaaac caacaauaag 360

aagaaagaau uugaggaaac ugcgaagaaa gugcgccgug ccaucgagca gcuggcugcc 420

auggauuga 429

<210> 6
<211> 429
<212> RNA
<213> Artificial Sequence

<220>
<223> Survivin CDS GC-optimized

<400> 6
augggcgccc ccacccugcc gccggccugg cagccguucc ucaaggacca ccgcaucucg 60

accuucaaga acuggccguu ccuggagggc ugcgcgugca ccccggagcg gauggccgag 120

gccggcuuca uccacugccc caccgagaac gagccggacc uggcccagug cuucuucugc 180

uucaaggagc uggagggcug ggagccggac gacgacccga ucgaggagca caagaagcac 240

agcagcggcu gcgccuuccu gagcgugaag aagcaguucg aggagcugac gcucggggag 300

uuccugaagc uggaccggga gcgggccaag aacaagaucg cgaaggagac caacaacaag 360

aagaaggagu ucgaggagac cgccaagaag gugcggcggg ccaucgagca gcuggccgcc 420

auggacuga 429

<210> 7
<211> 747
<212> RNA
<213> Artificial Sequence

<220>
<223> NY-ESO-1 (GC)-MUAG-A64-C30

<400> 7
   gggagaaagc uuaccaugca ggccgagggc cgcggcaccg gcggcucgac cggcgacgcc 60
   gacgggcccg gcggcccggg caucccggac ggcccgggcg ggaacgcggg cggcccgggc 120
   gaggccggcg ccaccggcgg gcggggcccg cggggcgccg gcgccgcccg ggcgagcggc 180
   cccggcgggg gcgccccgcg gggcccgcac ggcggcgccg ccagcggccu gaacgggugc 240
   ugccggugcg gcgcccgcgg cccggagagc cggcuccugg aguucuaccu ggccaugccg 300
   uucgcgaccc cgauggaggc cgagcuggcc cggcggagcc uggcccagga cgccccgccg 360
   cugcccgugc cgggcgugcu ccugaaggag uucacgguga gcggcaacau ccugaccauc 420
   cggcugaccg ccgcggacca ccggcagcug cagcugucga ucagcagcug ccuccagcag 480
   cugagccugc ugauguggau cacccagugc uuccugccgg uguuccuggc ccagccgccc 540
   agcggccagc gccggugacc acuaguuaua agacugacua gcccgauggg ccucccaacg 600
   ggcccuccuc cccuccuugc accgagauua auaaaaaaaa aaaaaaaaaa aaaaaaaaaa 660
   aaaaaaaaaa aaaaaaaaaa aaaaaaaaaa aaaaaauauu cccccccccc cccccccccc 720
   cccccccccc ucuagacaau uggaauu 747

<210> 8
<211> 543
<212> RNA
<213> Artificial Sequence

<220>
<223> NY-ESO-1 CDS wild type

<400> 8
augcaggccg aaggccgggg cacagggggu ucgacgggcg augcugaugg cccaggaggc 60

ccuggcauuc cugauggccc agggggcaau gcuggcggcc caggagaggc gggugccacg 120

ggcggcagag guccccgggg cgcaggggca gcaagggccu cggggccggg aggaggcgcc 180

ccgcgggguc cgcauggcgg cgcggcuuca gggcugaaug gaugcugcag augcggggcc 240

agggggccgg agagccgccu gcuugaguuc uaccucgcca ugccuuucgc gacacccaug 300

gaagcagagc uggcccgcag gagccuggcc caggaugccc caccgcuucc cgugccaggg 360

gugcuucuga aggaguucac uguguccggc aacauacuga cuauccgacu gacugcugca 420

gaccaccgcc aacugcagcu cuccaucagc uccugucucc agcagcuuuc ccuguugaug 480

uggaucacgc agugcuuucu gcccguguuu uuggcucagc cucccucagg gcagaggcgc 540

uaa 543

<210> 9
<211> 543
<212> RNA
<213> Artificial Sequence

<220>
<223> NY-ESO-1 CDS GC-optimized

<400> 9
augcaggccg agggccgcgg caccggcggc ucgaccggcg acgccgacgg gcccggcggc 60

ccgggcaucc cggacggccc gggcgggaac gcgggcggcc cgggcgaggc cggcgccacc 120

ggcgggcggg gcccgcgggg cgccggcgcc gcccgggcga gcggccccgg cgggggcgcc 180

ccgcggggcc cgcacggcgg cgccgccagc ggccugaacg ggugcugccg gugcggcgcc 240

cgcggcccgg agagccggcu ccuggaguuc uaccuggcca ugccguucgc gaccccgaug 300

gaggccgagc uggcccggcg gagccuggcc caggacgccc cgccgcugcc cgugccgggc 360

gugcuccuga aggaguucac ggugagcggc aacauccuga ccauccggcu gaccgccgcg 420

gaccaccggc agcugcagcu gucgaucagc agcugccucc agcagcugag ccugcugaug 480

uggaucaccc agugcuuccu gccgguguuc cuggcccagc cgcccagcgg ccagcgccgg 540

uga 543

<210> 10
<211> 1800
<212> RNA
<213> Artificial Sequence

<220>
<223> MAGE-C1 (aa 613-1142) (GC)-muag-A64-C30

<400> 10
gggagaaagc uuaccaugca guccccgcug cagggcgagg aguuccagag cucccugcag 60

agccccgugu ccaucugcag cuccagcacc cccuccagcc ucccgcagag cuuccccgag 120

uccagccagu ccccccccga gggcccgguc cagagccccc ugcacucccc gcagagcccc 180

ccggagggga ugcacuccca gagcccccug cagucccccg agagcgcccc cgagggcgag 240

gacucccuca gcccgcugca gaucccccag uccccgcugg agggggagga cagccucucc 300

agccugcacu ucccccaguc cccgcccgag ugggaggaca gccugagccc ccuccacuuc 360

ccccaguucc cgccccaggg cgaggacuuc caguccagcc ugcagucccc cgugagcauc 420

ugcuccagcu ccacgagccu gucccucccc cagagcuucc cggagucccc ccagagcccg 480

cccgaggggc cggcgcaguc cccccugcag cgccccguga gcuccuucuu cagcuacacc 540

cuggccuccc uccugcagag cucccacgag agcccgcaga gcccgcccga gggccccgcc 600

caguccccgc ugcagagccc cgucuccagc uuccccucca gcaccuccag cucccucagc 660

caguccagcc ccguguccag cuucccgucc agcaccucca gcucccugag caagagcucc 720

cccgagagcc cccugcaguc ccccgugauc agcuucucca gcuccacgag ccucuccccg 780

uucagcgagg aguccagcuc ccccgucgac gaguacacca gcuccagcga cacccugcug 840

gaguccgaca gccucaccga cuccgagagc cugaucgaga gcgagccccu guucaccuac 900

acgcucgacg agaaggugga cgagcuggcc cgguuccugc uccugaagua ccaggugaag 960

cagcccauca ccaaggccga gaugcugacc aacgucaucu cccgcuacac cggcuacuuc 1020

ccggugaucu uccggaaggc gcgcgaguuc aucgagaucc ucuucgggau cagccugcgg 1080

gagguggacc ccgacgacuc cuacgucuuc gugaacacgc uggaccucac cagcgagggc 1140

ugccuguccg acgagcaggg gaugagccag aaccgccugc ucauccugau ccuguccauc 1200

aucuucauca agggcaccua cgccagcgag gaggucaucu gggacgugcu cuccgggauc 1260

ggcgugcggg ccggccgcga gcacuucgcc uucggggagc cccgggagcu gcugaccaag 1320

gucugggugc aggagcacua ccucgaguac cgcgaggugc ccaacagcuc cccgccccgg 1380

uacgaguucc uguggggccc ccgcgcccac agcgagguca ucaagcggaa ggugguggag 1440

uuccuggcga ugcucaagaa cacggucccc aucaccuucc cguccagcua caaggacgcc 1500

cugaaggacg uggaggagcg ggcccaggcc aucaucgaca ccaccgacga cuccacggcc 1560

accgagagcg cguccagcuc cgugaugagc cccagcuucu ccagcgagug accacuaguu 1620

auaagacuga cuagcccgau gggccuccca acgggcccuc cuccccuccu ugcaccgaga 1680

uuaauaaaaa aaaaaaaaaa aaaaaaaaaa aaaaaaaaaa aaaaaaaaaa aaaaaaaaaa 1740

aaaaaaaaau auuccccccc cccccccccc cccccccccc cccucuagac aauuggaauu 1800

<210> 11
<211> 3429
<212> RNA
<213> Artificial Sequence

<220>
<223> MAGE-C1 full-length CDS wild type

<400> 11
augggggaca aggauaugcc uacugcuggg augccgaguc uucuccagag uuccucugag 60

aguccucaga guuguccuga gggggaggac ucccagucuc cucuccagau uccccagagu 120

ucuccugaga gcgacgacac ccuguauccu cuccagaguc cucagagucg uucugagggg 180

gaggacuccu cggauccucu ccagagaccu ccugagggga aggacuccca gucuccucuc 240

cagauucccc agaguucucc ugagggcgac gacacccagu cuccucucca gaauucucag 300

aguucuccug aggggaagga cucccugucu ccucuagaga uuucucagag cccuccugag 360

ggugaggaug uccagucucc ucugcagaau ccugcgaguu ccuucuucuc cucugcuuua 420

uugaguauuu uccagaguuc cccugagagu acucaaaguc cuuuugaggg uuuuccccag 480

ucuguucucc agauuccugu gagcgccgcc uccuccucca cuuuagugag uauuuuccag 540

aguuccccug agaguacuca aaguccuuuu gaggguuuuc cccagucucc acuccagauu 600

ccugugagcc gcuccuucuc cuccacuuua uugaguauuu uccagaguuc cccugagaga 660

acucagagua cuuuugaggg uuuugcccag ucuccucucc agauuccugu gagccccucc 720

uccuccucca cuuuacugag ucuuuuccag aguuucucug agagaacuca gaguacuuuu 780

gaggguuuug cccagucuuc ucuccagauu ccugugagcc ccuccuucuc cuccacuuua 840

gugagucuuu uccagaguuc cccugagaga acucagagua cuuuugaggg uuuuccccag 900

ucuccucucc agauuccugu gagcuccucc uccuccucca cuuuauugag ucuuuuccag 960

aguuccccug agagaacuca caguacuuuu gaggguuuuc cccagucucu ucuccagauu 1020

ccuaugaccu ccuccuucuc cucuacuuua uugaguauuu uccagaguuc uccugagagu 1080

gcucaaagua cuuuugaggg uuuuccccag ucuccucucc agauuccugg gagccccucc 1140

uucuccucca cuuuacugag ucuuuuccag aguuccccug agagaacuca caguacuuuu 1200

gaggguuuuc cccagucucc ucuccagauu ccuaugaccu ccuccuucuc cucuacuuua 1260

uugaguauuu uacagaguuc uccugagagu gcucaaagug cuuuugaggg uuuuccccag 1320

ucuccucucc agauuccugu gagcuccucu uucuccuaca cuuuauugag ucuuuuccag 1380

aguuccccug agagaacuca caguacuuuu gaggguuuuc cccagucucc ucuccagauu 1440

ccugugagcu ccuccuccuc cuccuccacu uuauugaguc uuuuccagag uuccccugag 1500

uguacucaaa guacuuuuga ggguuuuccc cagucuccuc uccagauucc ucagaguccu 1560

ccugaagggg agaauaccca uucuccucuc cagauuguuc caagucuucc ugagugggag 1620

gacucccugu cuccucacua cuuuccucag agcccuccuc agggggagga cucccuaucu 1680

ccucacuacu uuccucagag cccuccucag ggggaggacu cccugucucc ucacuacuuu 1740

ccucagagcc cucaggggga ggacucccug ucuccucacu acuuuccuca gagcccuccu 1800

cagggggagg acuccauguc uccucucuac uuuccucaga guccucuuca gggggaggaa 1860

uuccagucuu cucuccagag cccugugagc aucugcuccu ccuccacucc auccagucuu 1920

ccccagaguu ucccugagag uucucagagu ccuccugagg ggccugucca gucuccucuc 1980

cauaguccuc agagcccucc ugaggggaug cacucccaau cuccucucca gaguccugag 2040

agugcuccug agggggagga uucccugucu ccucuccaaa uuccucagag uccucuugag 2100

ggagaggacu cccugucuuc ucuccauuuu ccucagaguc cuccugagug ggaggacucc 2160

cucucuccuc uccacuuucc ucaguuuccu ccucaggggg aggacuucca gucuucucuc 2220

cagaguccug ugaguaucug cuccuccucc acuucuuuga gucuucccca gaguuucccu 2280

gagaguccuc agaguccucc ugagggccuu gcucagucuc cucuccagag accugucagc 2340

uccuucuucu ccuacacuuu agcgagucuu cuccaaaguu cccaugagag uccucagagu 2400

ccuccugagg ggccugccca gucuccucuc cagaguccug ugagcuccuu ccccuccucc 2460

acuucaucga gucuuuccca gaguucuccu gugagcuccu uccccuccuc cacuucaucg 2520

agucuuucca agaguucccc ugagaguccu cuccagaguc cugugaucuc cuucuccucc 2580

uccacuucau ugagcccauu cagugaagag uccagcagcc caguagauga auauacaagu 2640

uccucagaca ccuugcuaga gagugauucc uugacagaca gcgaguccuu gauagagagc 2700

gagcccuugu ucacuuauac acuggaugaa aagguggacg aguuggcgcg guuucuucuc 2760

cucaaauauc aagugaagca gccuaucaca aaggcagaga ugcugacgaa ugucaucagc 2820

agguacacgg gcuacuuucc ugugaucuuc aggaaagccc gugaguucau agagauacuu 2880

uuuggcauuu cccugagaga aguggacccu gaugacuccu augucuuugu aaacacauua 2940

gaccucaccu cugaggggug ucugagugau gagcagggca ugucccagaa ccgccuccug 3000

auucuuauuc ugaguaucau cuucauaaag ggcaccuaug ccucugagga ggucaucugg 3060

gaugugcuga guggaauagg ggugcgugcu gggagggagc acuuugccuu uggggagccc 3120

agggagcucc ucacuaaagu uugggugcag gaacauuacc uagaguaccg ggaggugccc 3180

aacucuucuc cuccucguua cgaauuccug ugggguccaa gagcucauuc agaagucauu 3240

aagaggaaag uaguagaguu uuuggccaug cuaaagaaua ccgucccuau uaccuuucca 3300

uccucuuaca aggaugcuuu gaaagaugug gaagagagag cccaggccau aauugacacc 3360

acagaugauu cgacugccac agaaagugca agcuccagug ucaugucccc cagcuucucu 3420

ucugaguga 3429

<210> 12
<211> 3429
<212> RNA
<213> Artificial Sequence

<220>
<223> MAGE-C1 full-length CDS GC-optimized

<400> 12
   augggcgaca aggacaugcc caccgccggg augccgagcc ugcuccaguc cagcuccgag 60
   agcccccagu ccugccccga gggcgaggac agccaguccc cccugcagau cccgcagagc 120
   ucccccgaga gcgacgacac ccuguacccc cuccaguccc cgcagagccg guccgagggg 180
   gaggacagcu ccgacccgcu gcagcgcccc cccgagggca aggacagcca guccccgcug 240
   cagaucccgc agagcucccc cgagggggac gacacgcaga gcccccucca gaacagccag 300
   uccagccccg agggcaagga cucccugagc ccgcuggaga ucucccagag cccccccgag 360
   ggcgaggacg ugcagucccc gcuccagaac ccggccagcu ccuucuucag cuccgcgcug 420
   cugagcaucu uccaguccag ccccgagucc acccagagcc ccuucgaggg guucccccag 480
   uccguccucc agaucccggu gagcgccgcc uccagcagca cccugguguc caucuuccag 540
   agcucccccg agagcaccca gucccccuuc gagggcuucc cccagagccc gcugcagauc 600
   cccguguccc ggagcuucuc cagcacgcuc cuguccaucu uccagagcuc ccccgagcgc 660
   acccagagca ccuucgaggg guucgcccag uccccgcugc agauccccgu gagccccucc 720
   agcagcucca cccuccugag ccuguuccag uccuucagcg agcggacgca guccaccuuc 780
   gagggcuucg cccagagcuc ccuccagauc cccgugagcc cguccuucag cuccacccug 840
   gucagccugu uccaguccag ccccgagcgc acccagucca cguucgaggg guucccccag 900
   agcccccucc agaucccggu guccagcucc agcagcucca cccugcugag ccucuuccag 960
   uccagccccg agcggaccca cuccaccuuc gagggcuucc cccagagccu gcugcagauc 1020
   cccaugacgu ccagcuucuc cagcacccuc cuguccaucu uccagagcuc cccggagagc 1080
   gcgcagucca ccuucgaggg cuucccccag agcccccugc agauccccgg guccccgagc 1140
   uucuccagca cccuccugag ccuguuccag uccagccccg agcgcacgca cuccaccuuc 1200
   gagggcuucc cccagagccc ccuccagauc ccgaugaccu ccagcuucuc cagcacccug 1260
   cuguccaucc uccagagcuc ccccgagagc gcccaguccg ccuucgaggg guucccccag 1320
   agcccccugc agaucccggu guccagcucc uucagcuaca cgcugcucuc ccuguuccag 1380
   agcagccccg agcggaccca cuccaccuuc gagggcuucc cccagagccc gcugcagauc 1440
   cccgugucca gcuccagcuc cagcuccacc cuccugagcc uguuccaguc cagccccgag 1500
   ugcacgcagu ccaccuucga gggcuucccc cagagcccgc ugcagauccc ccaguccccc 1560
   cccgaggggg agaacaccca cagcccgcuc cagaucgugc ccucccugcc cgagugggag 1620
   gacagccugu ccccgcacua cuucccgcag agccccccgc agggcgagga cagccucucc 1680
   ccccacuacu ucccgcagag cccgccccag ggggaggacu cccugagccc ccacuacuuc 1740
   ccgcaguccc cccagggcga ggacagccug uccccgcacu acuuccccca gagcccgccc 1800
   cagggggagg acuccaugag cccccucuac uucccccagu ccccgcugca gggcgaggag 1860
   uuccagagcu cccugcagag ccccgugucc aucugcagcu ccagcacccc cuccagccuc 1920
   ccgcagagcu uccccgaguc cagccagucc ccccccgagg gcccggucca gagcccccug 1980
   cacuccccgc agagcccccc ggaggggaug cacucccaga gcccccugca gucccccgag 2040
   agcgcccccg agggcgagga cucccucagc ccgcugcaga ucccccaguc cccgcuggag 2100
   ggggaggaca gccucuccag ccugcacuuc ccccaguccc cgcccgagug ggaggacagc 2160
   cugagccccc uccacuuccc ccaguucccg ccccagggcg aggacuucca guccagccug 2220
   cagucccccg ugagcaucug cuccagcucc acgagccugu cccuccccca gagcuucccg 2280
   gagucccccc agagcccgcc cgaggggccg gcgcaguccc cccugcagcg ccccgugagc 2340
   uccuucuuca gcuacacccu ggccucccuc cugcagagcu cccacgagag cccgcagagc 2400
   ccgcccgagg gccccgccca guccccgcug cagagccccg uguccagcuu ccccuccagc 2460
   accuccagcu cccucagcca guccagcccc guguccagcu ucccguccag caccuccagc 2520
   ucccugagca agagcucccc cgagagcccc cugcaguccc ccgugaucag cuucuccagc 2580
   uccacgagcc ucuccccguu cagcgaggag uccagcuccc ccgucgacga guacaccagc 2640
   uccagcgaca cccugcugga guccgacagc cucaccgacu ccgagagccu gaucgagagc 2700
   gagccccugu ucaccuacac gcucgacgag aagguggacg agcuggcccg guuccugcuc 2760
   cugaaguacc aggugaagca gcccaucacc aaggccgaga ugcugaccaa cgucaucucc 2820
   cgcuacaccg gcuacuuccc ggugaucuuc cggaaggcgc gcgaguucau cgagauccuc 2880
   uucgggauca gccugcggga gguggacccc gacgacuccu acgucuucgu gaacacgcug 2940
   gaccucacca gcgagggcug ccuguccgac gagcagggga ugagccagaa ccgccugcuc 3000
   auccugaucc uguccaucau cuucaucaag ggcaccuacg ccagcgagga ggucaucugg 3060
   gacgugcucu ccgggaucgg cgugcgggcc ggccgcgagc acuucgccuu cggggagccc 3120
   cgggagcugc ugaccaaggu cugggugcag gagcacuacc ucgaguaccg cgaggugccc 3180
   aacagcuccc cgccccggua cgaguuccug uggggccccc gcgcccacag cgaggucauc 3240
   aagcggaagg ugguggaguu ccuggcgaug cucaagaaca cgguccccau caccuucccg 3300
   uccagcuaca aggacgcccu gaaggacgug gaggagcggg cccaggccau caucgacacc 3360
   accgacgacu ccacggccac cgagagcgcg uccagcuccg ugaugagccc cagcuucucc 3420
   agcgaguga 3429

<210> 13
<211> 1326
<212> RNA
<213> Artificial Sequence

<220>
<223> MAGE-C2 (GC)-muag-A64-C30

<400> 13
gggagaaagc uuaccaugcc cccggugccc ggcguccccu uccggaacgu ggacaacgac 60

agccccaccu ccguggagcu ggaggacugg gucgacgccc agcacccgac cgacgaggag 120

gaggaggagg ccagcuccgc gagcuccacg cucuaccugg uguucagccc cuccagcuuc 180

uccaccagcu ccagccugau ccucgggggc cccgaggagg aggaggugcc cuccgggguc 240

aucccgaacc ugaccgagag cauccccucc agccccccgc agggcccgcc ccaggggccc 300

ucccagagcc cccuguccag cugcugcagc uccuucagcu gguccagcuu cuccgaggag 360

agcuccagcc agaagggcga ggacaccggc acgugccagg ggcucccgga cuccgagagc 420

uccuucaccu acacccugga cgagaaggug gccgagcugg uggaguuccu ccugcugaag 480

uacgaggccg aggagcccgu caccgaggcc gagaugcuca ugaucgugau caaguacaag 540

gacuacuucc ccgugauccu gaagcgcgcc cgggaguuca uggagcugcu cuucggccug 600

gcgcugaucg aggucgggcc cgaccacuuc ugcguguucg ccaacacggu gggccucacc 660

gacgagggga gcgacgacga gggcaugccg gagaacuccc ugcugaucau cauccucagc 720

gucaucuuca ucaagggcaa cugcgccucc gaggagguga ucugggaggu gcugaacgcc 780

gucggggugu acgcgggccg cgagcacuuc guguacgggg agccccggga gcugcucacc 840

aaggucuggg ugcagggcca cuaccuggag uaccgcgagg ugccgcacag cucccccccg 900

uacuacgagu uccugugggg cccccgggcc cacagcgagu ccaucaagaa gaagguccuc 960

gaguuccugg ccaagcugaa caacaccgug cccagcagcu uccccuccug guacaaggac 1020

gcccucaagg acgucgagga gcgcgugcag gccacgaucg acaccgcgga cgacgccacc 1080

gugauggcca gcgagucccu gagcgucaug uccagcaacg uguccuucag cgagugacca 1140

cuaguuauaa gacugacuag cccgaugggc cucccaacgg gcccuccucc ccuccuugca 1200

ccgagauuaa uaaaaaaaaa aaaaaaaaaa aaaaaaaaaa aaaaaaaaaa aaaaaaaaaa 1260

aaaaaaaaaa aaaaauauuc cccccccccc cccccccccc cccccccccu cuagacaauu 1320

ggaauu 1326

<210> 14
<211> 1339
<212> RNA
<213> Artificial Sequence

<220>
<223> MAGE-C2 (GC)-muag-A64-C30-histoneSL

<400> 14
gggagaaagc uuaccaugcc cccggugccc ggcguccccu uccggaacgu ggacaacgac 60

agccccaccu ccguggagcu ggaggacugg gucgacgccc agcacccgac cgacgaggag 120

gaggaggagg ccagcuccgc gagcuccacg cucuaccugg uguucagccc cuccagcuuc 180

uccaccagcu ccagccugau ccucgggggc cccgaggagg aggaggugcc cuccgggguc 240

aucccgaacc ugaccgagag cauccccucc agccccccgc agggcccgcc ccaggggccc 300

ucccagagcc cccuguccag cugcugcagc uccuucagcu gguccagcuu cuccgaggag 360

agcuccagcc agaagggcga ggacaccggc acgugccagg ggcucccgga cuccgagagc 420

uccuucaccu acacccugga cgagaaggug gccgagcugg uggaguuccu ccugcugaag 480

uacgaggccg aggagcccgu caccgaggcc gagaugcuca ugaucgugau caaguacaag 540

gacuacuucc ccgugauccu gaagcgcgcc cgggaguuca uggagcugcu cuucggccug 600

gcgcugaucg aggucggccc cgaccacuuc ugcguguucg ccaacacggu gggccucacc 660

gacgagggga gcgacgacga gggcaugccg gagaacuccc ugcugaucau cauccucagc 720

gucaucuuca ucaagggcaa cugcgccucc gaggagguga ucugggaggu gcugaacgcc 780

gucggggugu acgcgggccg cgagcacuuc guguacgggg agccccggga gcugcucacc 840

aaggucuggg ugcagggcca cuaccuggag uaccgcgagg ugccgcacag cucccccccg 900

uacuacgagu uccugugggg cccccgggcc cacagcgagu ccaucaagaa gaagguccuc 960

gaguuccugg ccaagcugaa caacaccgug cccagcagcu uccccuccug guacaaggac 1020

gcccucaagg acgucgagga gcgcgugcag gccacgaucg acaccgcgga cgacgccacc 1080

gugauggcca gcgagucccu gagcgucaug uccagcaacg uguccuucag cgagugacca 1140

cuaguuauaa gacugacuag cccgaugggc cucccaacgg gcccuccucc ccuccuugca 1200

ccgagauuaa uaaaaaaaaa aaaaaaaaaa aaaaaaaaaa aaaaaaaaaa aaaaaaaaaa 1260

aaaaaaaaaa aaaaaugcau cccccccccc cccccccccc cccccccccc caaaggcucu 1320

uuucagagcc accagaauu 1339

<210> 15
<211> 1122
<212> RNA
<213> Artificial Sequence

<220>
<223> MAGE-C2 CDS wild type

<400> 15
augccucccg uuccaggcgu uccauuccgc aacguugaca acgacucccc gaccucaguu 60

gaguuagaag acuggguaga ugcacagcau cccacagaug aggaagagga ggaagccucc 120

uccgccucuu ccacuuugua cuuaguauuu ucccccucuu cuuucuccac auccucuucu 180

cugauucuug gugguccuga ggaggaggag gugcccucug gugugauacc aaaucuuacc 240

gagagcauuc ccaguagucc uccacagggu ccuccacagg guccuuccca gaguccucug 300

agcuccugcu gcuccucuuu uucauggagc ucauucagug aggaguccag cagccagaaa 360

ggggaggaua caggcaccug ucagggccug ccagacagug aguccucuuu cacauauaca 420

cuagaugaaa agguggccga guuaguggag uuccugcucc ucaaauacga agcagaggag 480

ccuguaacag aggcagagau gcugaugauu gucaucaagu acaaagauua cuuuccugug 540

auacucaaga gagcccguga guucauggag cuucuuuuug gccuugcccu gauagaagug 600

ggcccugacc acuucugugu guuugcaaac acaguaggcc ucaccgauga ggguagugau 660

gaugagggca ugcccgagaa cagccuccug auuauuauuc ugagugugau cuucauaaag 720

ggcaacugug ccucugagga ggucaucugg gaagugcuga augcaguagg gguauaugcu 780

gggagggagc acuucgucua uggggagccu agggagcucc ucacuaaagu uugggugcag 840

ggacauuacc uggaguaucg ggaggugccc cacaguucuc cuccauauua ugaauuccug 900

uggggucca a gagcccauuc agaaagcauc aagaagaaag uacuagaguu uuuagccaag 960

cugaacaaca cuguuccuag uuccuuucca uccugguaca aggaugcuuu gaaagaugug 1020

gaagagagag uccaggccac aauugauacc gcagaugaug ccacugucau ggccagugaa 1080

agccucagug ucauguccag caacgucucc uuuucugagu ga 1122

<210> 16
<211> 1860
<212> RNA
<213> Artificial Sequence

<220>
<223> MUC1 5 VNTR (GC)-muag-A64-C30

<400> 16
gggagaaagc uuaccaugac ccccggcacc cagagcccgu ucuuccugcu ccugcugcuc 60

acggugcuga ccgucgugac cggguccggc cacgccagcu ccaccccggg gggcgagaag 120

gagacgagcg ccacccagcg guccagcgug cccuccagca ccgagaagaa cgcggucucc 180

augaccagcu ccgugcugag cucccacagc cccggguccg gcagcuccac gacccagggc 240

caggacguga cccucgcccc ggccaccgag cccgccagcg gguccgccgc gacguggggc 300

caggacguca ccagcgugcc cgugacccgc cccgcccugg ggagcaccac gccgcccgcc 360

cacgacguca ccuccgcccc cgacaacaag cccgcgccgg gcagcaccgc cccccccgcc 420

cacgggguga ccuccgcccc cgacacgcgg ccggcccccg gcagcaccgc gccccccgcc 480

cacggcguga ccuccgcccc ggacacccgc cccgcccccg ggagcacggc cccgccggcg 540

cacggcguca ccuccgcccc cgacacccgg cccgcccccg ggagcaccgc cccgcccgcc 600

cacggcguga cguccgcgcc cgacacccgc ccggcccccg gcagcaccgc cccccccgcc 660

cacgggguga ccuccgcccc ggacacgcgg cccgcgcccg gcagcaccgc cccgccggcc 720

cacgggguca ccuccgcgcc cgacaaccgc cccgcccugg ggagcaccgc cccgcccgug 780

cacaacguga ccuccgccag cggcuccgcg agcggguccg ccagcacccu cguccacaac 840

ggcacguccg cccgggccac caccaccccc gccagcaagu ccacgcccuu cagcaucccg 900

ucccaccaca gcgacacccc caccacccug gcgucccaca gcacgaagac cgacgccucc 960

agcacccacc acuccagcgu gcccccgcug accagcucca accacagcac guccccgcag 1020

cucagcaccg ggguguccuu cuucuuccug agcuuccaca ucuccaaccu gcaguucaac 1080

agcucccucg aggaccccag caccgacuac uaccaggagc ugcagcggga caucuccgag 1140

auguuccugc agaucuacaa gcagggcggc uuccucgggc ugagcaacau caaguuccgc 1200

cccggcuccg ucguggugca gcugacccuc gccuuccggg aggggacgau caacguccac 1260

gacguggaga cccaguucaa ccaguacaag accgaggccg ccagccgcua caaccugacc 1320

aucuccgacg ugagcgucuc cgacgugccc uucccguuca gcgcgcaguc cggcgccggc 1380

gugcccgggu ggggcaucgc ccugcucguc cuggugugcg ugcuggucgc ccucgccauc 1440

guguaccuga ucgcgcuggc cgugugccag ugccggcgca agaacuacgg gcagcucgac 1500

aucuuccccg cccgggacac guaccacccg augagcgagu acccgaccua ccacacccac 1560

ggccgcuacg ucccccccag cuccaccgac cggagccccu acgagaaggu guccgccggg 1620

aacggcggca gcucccugag cuacaccaac ccggcggugg ccgccgccuc cgccaaccug 1680

ugaccacuag uuauaagacu gacuagcccg augggccucc caacgggccc uccuccccuc 1740

cuugcaccga gauuaauaaa aaaaaaaaaa aaaaaaaaaa aaaaaaaaaa aaaaaaaaaa 1800

aaaaaaaaaa aaaaaaaaaa auauuccccc cccccccccc cccccccccc cccccucuag 1860

<210> 17
<211> 1885
<212> RNA
<213> Artificial Sequence

<220>
<223> MUC1 5 VNTR (GC)-muag-A64-C30-histoneSL

<400> 17
   gggagaaagc uuaccaugac ccccggcacc cagagcccgu ucuuccugcu ccugcugcuc 60
   acggugcuga ccgucgugac cggguccggc cacgccagcu ccacccccgg gggcgagaag 120
   gagacgagcg ccacccagcg guccagcgug cccuccagca ccgagaagaa cgcggucucc 180
   augaccagcu ccgugcugag cucccacagc cccggguccg gcagcuccac gacccagggc 240
   caggacguga cccucgcccc ggccaccgag cccgccagcg gguccgccgc gacguggggc 300
   caggacguca ccagcgugcc cgugacccgc cccgcccugg ggagcaccac gccgcccgcc 360
   cacgacguca ccuccgcccc cgacaacaag cccgcgccgg gcagcaccgc cccccccgcc 420
   cacgggguga ccuccgcccc cgacacgcgg ccggcccccg gcagcaccgc gccccccgcc 480
   cacggcguga ccuccgcccc ggacacccgc cccgcccccg ggagcacggc cccgccggcg 540
   cacggcguca ccuccgcccc cgacacccgg cccgcccccg ggagcaccgc cccgcccgcc 600
   cacggcguga cguccgcgcc cgacacccgc ccggcccccg gcagcaccgc cccccccgcc 660
   cacgggguga ccuccgcccc ggacacgcgg cccgcgcccg gcagcaccgc cccgccggcc 720
   cacgggguca ccuccgcgcc cgacaaccgc cccgcccugg ggagcaccgc cccgcccgug 780
   cacaacguga ccuccgccag cggcuccgcg agcggguccg ccagcacccu cguccacaac 840
   ggcacguccg cccgggccac caccaccccc gccagcaagu ccacgcccuu cagcaucccg 900
   ucccaccaca gcgacacccc caccacccug gcgucccaca gcacgaagac cgacgccucc 960
   agcacccacc acuccagcgu gcccccgcug accagcucca accacagcac guccccgcag 1020
   cucagcaccg ggguguccuu cuucuuccug agcuuccaca ucuccaaccu gcaguucaac 1080
   agcucccucg aggaccccag caccgacuac uaccaggagc ugcagcggga caucuccgag 1140
   auguuccugc agaucuacaa gcagggcggc uuccucgggc ugagcaacau caaguuccgc 1200
   cccggcuccg ucguggugca gcugacccuc gccuuccggg aggggacgau caacguccac 1260
   gacguggaga cccaguucaa ccaguacaag accgaggccg ccagccgcua caaccugacc 1320
   aucuccgacg ugagcgucuc cgacgugccc uucccguuca gcgcgcaguc cggcgccggc 1380
   gugcccgggu ggggcaucgc ccugcucguc cuggugugcg ugcuggucgc ccucgccauc 1440
   guguaccuga ucgcgcuggc cgugugccag ugccggcgca agaacuacgg gcagcucgac 1500
   aucuuccccg cccgggacac guaccacccg augagcgagu acccgaccua ccacacccac 1560
   ggccgcuacg ucccccccag cuccaccgac cggagccccu acgagaaggu guccgccggg 1620
   aacggcggca gcucccugag cuacaccaac ccggcggugg ccgccgccuc cgccaaccug 1680
   ugaccacuag uuauaagacu gacuagcccg augggccucc caacgggccc uccuccccuc 1740
   cuugcaccga gauuaauaaa aaaaaaaaaa aaaaaaaaaa aaaaaaaaaa aaaaaaaaaa 1800
   aaaaaaaaaa aaaaaaaaaa augcaucccc cccccccccc cccccccccc cccccccaaa 1860
   ggcucuuuuc agagccacca gaauu 1885

<210> 18
<211> 1668
<212> RNA
<213> Artificial Sequence

<220>
<223> Muc1-5 VNTR CDS wild type

<400> 18
augacaccgg gcacccaguc uccuuucuuc cugcugcugc uccucacagu gcuuacaguu 60

guuacagguu cuggucaugc aagcucuacc ccagguggag aaaaggagac uucggcuacc 120

cagagaaguu cagugcccag cucuacugag aagaaugcug ugaguaugac cagcagcgua 180

cucuccagcc acagccccgg uucaggcucc uccaccacuc agggacagga ugucacucug 240

gccccggcca cggaaccagc uucagguuca gcugccaccu ggggacagga ugucaccucg 300

gucccaguca ccaggccagc ccugggcucc accaccccgc cagcccacga ugucaccuca 360

gccccggaca acaagccagc cccgggcucc accgcccccc cagcccacgg ugucaccucg 420

gccccggaca ccaggccggc cccgggcucc accgcccccc cagcccacgg ugucaccucg 480

gccccggaca ccaggccggc cccgggcucc accgcccccc cagcccacgg ugucaccucg 540

gccccggaca ccaggccggc cccgggcucc accgcccccc cagcccacgg ugucaccucg 600

gccccggaca ccaggccggc cccgggcucc accgcccccc cagcccacgg ugucaccucg 660

gccccggaca ccaggccggc cccgggcucc accgcccccc cagcccacgg ugucaccucg 720

gccccggaca acaggcccgc cuugggcucc accgccccuc caguccacaa ugucaccucg 780

gccucaggcu cugcaucagg cucagcuucu acucuggugc acaacggcac cucugccagg 840

gcuaccacaa ccccagccag caagagcacu ccauucucaa uucccagcca ccacucugau 900

acuccuacca cccuugccag ccauagcacc aagacugaug ccaguagcac ucaccauagc 960

ucgguaccuc cucucaccuc cuccaaucac agcacuucuc cccaguuguc uacugggguc 1020

ucuuucuuuu uccugucuuu ucacauuuca aaccuccagu uuaauuccuc ucuggaagau 1080

cccagcaccg acuacuacca agagcugcag agagacauuu cugaaauguu uuugcagauu 1140

uauaaacaag gggguuuucu gggccucucc aauauuaagu ucaggccagg aucuguggug 1200

guacaauuga cucuggccuu ccgagaaggu accaucaaug uccacgacgu ggagacacag 1260

uucaaucagu auaaaacgga agcagccucu cgauauaacc ugacgaucuc agacgucagc 1320

gugagugaug ugccauuucc uuucucugcc cagucugggg cuggggugcc aggcuggggc 1380

aucgcgcugc uggugcuggu cuguguucug guugcgcugg ccauugucua ucucauugcc 1440

uuggcugucu gucagugccg ccgaaagaac uacgggcagc uggacaucuu uccagcccgg 1500

gauaccuacc auccuaugag cgaguacccc accuaccaca cccaugggcg cuaugugccc 1560

ccuagcagua ccgaucguag ccccuaugag aagguuucug cagguaacgg uggcagcagc 1620

cucucuuaca caaacccagc aguggcagcc gcuucugcca acuuguag 1668

<210> 19
<211> 1480
<212> RNA
<213> Artificial Sequence

<220>
<223> 5T4 (GC)-muag-A64-C30-HistoneSL

<400> 19
gggagaaagc uuaccaugcc cggcgggugc agccggggcc cggccgccgg ggacggccgc 60

cugcggcucg cgcgccuggc ccuggugcuc cugggguggg ucuccagcuc cagccccacc 120

uccagcgccu ccagcuucuc cagcuccgcc cccuuccugg ccagcgcggu guccgcccag 180

cccccgcucc ccgaccagug ccccgcccug ugcgagugca gcgaggccgc gcggaccgug 240

aagugcguca accgcaaccu gacggaggug cccaccgacc ucccggccua cgugcggaac 300

cuguuccuga ccggcaacca gcucgccguc cugcccgccg gcgccuucgc gcgccggccg 360

ccccuggccg agcucgccgc ccugaaccug uccgggagcc gccucgacga ggugcgggcc 420

ggcgcguucg agcaccugcc gucccugcgc cagcucgacc ugagccacaa cccccuggcc 480

gaccucuccc ccuucgccuu cagcgggagc aacgccuccg ugagcgcccc cuccccgcug 540

gucgagcuga uccucaacca caucgugccc cccgaggacg agcggcagaa ccgcagcuuc 600

gagggcaugg uggucgcggc ccugcuggcc gggcgggccc uccagggccu gcgccggcug 660

gagcucgccu ccaaccacuu ccuguaccug ccccgcgacg ugcucgcgca gcugccgagc 720

cugcggcacc ucgaccuguc caacaacagc cugguguccc ucaccuacgu cagcuuccgc 780

aaccugacgc accuggaguc ccuccaccug gaggacaacg cccugaaggu gcugcacaac 840

ggcacccucg ccgagcugca ggggcugccc cacauccggg uguuccucga caacaacccc 900

ugggucugcg acugccacau ggccgacaug gugaccuggc ugaaggagac cgaggugguc 960

cagggcaagg accgccugac gugcgcguac cccgagaaga ugcggaaccg ggugcuccug 1020

gagcugaaca gcgccgaccu cgacugcgac ccgauccugc cccccucccu gcagaccagc 1080

uacguguucc ucgggaucgu ccuggcccug aucggcgcca ucuuccuccu ggugcuguac 1140

cucaaccgca agggcaucaa gaaguggaug cacaacaucc gggacgccug ccgcgaccac 1200

auggaggggu accacuaccg guacgagauc aacgcggacc cccgccugac caaccugucc 1260

agcaacuccg acgucugacc acuaguuaua agacugacua gcccgauggg ccucccaacg 1320

ggcccuccuc cccuccuugc accgagauua auaaaaaaaa aaaaaaaaaa aaaaaaaaaa 1380

aaaaaaaaaa aaaaaaaaaa aaaaaaaaaa aaaaaaugca uccccccccc cccccccccc 1440

cccccccccc ccaaaggcuc uuuucagagc caccagaauu 1480

<210> 20
<211> 646
<212> RNA
<213> Artificial Sequence

<220>
<223> Survivin (GC)-muag-A64-C30-HistoneSL

<400> 20
gggagaaagc uuaccauggg cgcccccacc cugccgccgg ccuggcagcc guuccucaag 60

gaccaccgca ucucgaccuu caagaacugg ccguuccugg agggcugcgc gugcaccccg 120

gagcggaugg ccgaggccgg cuucauccac ugccccaccg agaacgagcc ggaccuggcc 180

cagugcuucu ucugcuucaa ggagcuggag ggcugggagc cggacgacga cccgaucgag 240

gagcacaaga agcacagcag cggcugcgcc uuccugagcg ugaagaagca guucgaggag 300

cugacgcucg gggaguuccu gaagcuggac cgggagcggg ccaagaacaa gaucgcgaag 360

gagaccaaca acaagaagaa ggaguucgag gagaccgcca agaaggugcg gcgggccauc 420

gagcagcugg ccgccaugga cugaccacua guuauaagac ugacuagccc gaugggccuc 480

ccaacgggcc cuccuccccu ccuugcaccg agauuaauaa aaaaaaaaaa aaaaaaaaaa 540

aaaaaaaaaa aaaaaaaaaa aaaaaaaaaa aaaaaaaaaa aaugcauccc cccccccccc 600

cccccccccc ccccccccaa aggcucuuuu cagagccacc agaauu 646

<210> 21
<211> 760
<212> RNA
<213> Artificial Sequence

<220>
<223> NY-ESO-1 (GC)-muag-A64-C30-histone SL

<400> 21
gggagaaagc uuaccaugca ggccgagggc cgcggcaccg gcggcucgac cggcgacgcc 60

gacgggcccg gcggcccggg caucccggac ggcccgggcg ggaacgcggg cggcccgggc 120

gaggccggcg ccaccggcgg gcggggcccg cggggcgccg gcgccgcccg ggcgagcggc 180

cccggcgggg gcgccccgcg gggcccgcac ggcggcgccg ccagcggccu gaacgggugc 240

ugccggugcg gcgcccgcgg cccggagagc cggcuccugg aguucuaccu ggccaugccg 300

uucgcgaccc cgauggaggc cgagcuggcc cggcggagcc uggcccagga cgccccgccg 360

cugcccgugc cgggcgugcu ccugaaggag uucacgguga gcggcaacau ccugaccauc 420

cggcugaccg ccgcggacca ccggcagcug cagcugucga ucagcagcug ccuccagcag 480

cugagccugc ugauguggau cacccagugc uuccugccgg uguuccuggc ccagccgccc 540

agcggccagc gccggugacc acuaguuaua agacugacua gcccgauggg ccucccaacg 600

ggcccuccuc cccuccuugc accgagauua auaaaaaaaa aaaaaaaaaa aaaaaaaaaa 660

aaaaaaaaaa aaaaaaaaaa aaaaaaaaaa aaaaaaugca uccccccccc cccccccccc 720

cccccccccc ccaaaggcuc uuuucagagc caccagaauu 760

<210> 22
<211> 1813
<212> RNA
<213> Artificial Sequence

<220>
<223> MAGE-C1 (aa 613-1142) (GC)-muag-A64-C30-histoneSL

<400> 22
gggagaaagc uuaccaugca guccccgcug cagggcgagg aguuccagag cucccugcag 60

agccccgugu ccaucugcag cuccagcacc cccuccagcc ucccgcagag cuuccccgag 120

uccagccagu ccccccccga gggcccgguc cagagccccc ugcacucccc gcagagcccc 180

ccggagggga ugcacuccca gagcccccug cagucccccg agagcgcccc cgagggcgag 240

gacucccuca gcccgcugca gaucccccag uccccgcugg aggggaggga cagccucucc 300

agccugcacu ucccccaguc cccgcccgag ugggaggaca gccugagccc ccuccacuuc 360

ccccaguucc cgccccaggg cgaggacuuc caguccagcc ugcagucccc cgugagcauc 420

ugcuccagcu ccacgagccu gucccucccc cagagcuucc cggagucccc ccagagcccg 480

cccgaggggc cggcgcaguc cccccugcag cgccccguga gcuccuucuu cagcuacacc 540

cuggccuccc uccugcagag cucccacgag agcccgcaga gcccgcccga gggccccgcc 600

caguccccgc ugcagagccc cgucuccagc uuccccucca gcaccuccag cucccucagc 660

caguccagcc ccguguccag cuucccgucc agcaccucca gcucccugag caagagcucc 720

cccgagagcc cccugcaguc ccccgugauc agcuucucca gcuccacgag ccucuccccg 780

uucagcgagg aguccagcuc ccccgucgac gaguacacca gcuccagcga cacccugcug 840

gaguccgaca gccucaccga cuccgagagc cugaucgaga gcgagccccu guucaccuac 900

acgcucgacg agaaggugga cgagcuggcc cgguuccugc uccugaagua ccaggugaag 960

cagcccauca ccaaggccga gaugcugacc aacgucaucu cccgcuacac cggcuacuuc 1020

ccggugaucu uccggaaggc gcgcgaguuc aucgagaucc ucuucgggau cagccugcgg 1080

gagguggacc ccgacgacuc cuacgucuuc gugaacacgc uggaccucac cagcgagggc 1140

ugccuguccg acgagcaggg gaugagccag aaccgccugc ucauccugau ccuguccauc 1200

aucuucauca agggcaccua cgccagcgag gaggucaucu gggacgugcu cuccgggauc 1260

ggcgugcggg ccggccgcga gcacuucgcc uucggggagc cccgggagcu gcugaccaag 1320

gucugggugc aggagcacua ccucgaguac cgcgaggugc ccaacagcuc cccgccccgg 1380

uacgaguucc ugugggcccc ccgcgcccac agcgagguca ucaagcggaa ggugguggag 1440

uuccuggcga ugcucaagaa cacggucccc aucaccuucc cguccagcua caaggacgcc 1500

cugaaggacg uggaggagcg ggcccaggcc aucaucgaca ccaccgacga cuccacggcc 1560

accgagagcg cguccagcuc cgugaugagc cccagcuucu ccagcgagug accacuaguu 1620

auaagacuga cuagcccgau gggccuccca acgggcccuc cuccccuccu ugcaccgaga 1680

uuaauaaaaa aaaaaaaaaa aaaaaaaaaa aaaaaaaaaa aaaaaaaaaa aaaaaaaaaa 1740

aaaaaaaaau gcaucccccc cccccccccc cccccccccc cccccaaagg cucuuuucag 1800

agccaccaga auu 1813

<210> 23
<211> 1339
<212> RNA
<213> Artificial Sequence

<220>

<223>  MAGE-C2 (GC)-muag-A64-C30-histoneSL

<400>  23
gggagaaagc uuaccaugcc cccggugccc ggcguccccu uccggaacgu ggacaacgac      60

agccccaccu ccguggagcu ggaggacugg gucgacgccc agcacccgac cgacgaggag     120

gaggaggagg ccagcuccgc gagcuccacg cucuaccugg uguucagccc cuccagcuuc     180

uccaccagcu ccagccugau ccucgggggc cccgaggagg aggaggugcc cuccgggguc     240

aucccgaacc ugaccgagag cauccccucc agccccccgc agggcccgcc ccaggggccc     300

ucccagagcc cccuguccag cugcugcagc uccuucagcu gguccagcuu cuccgaggag     360

agcuccagcc agaagggcga ggacaccggc acgugccagg ggcucccgga cuccgagagc     420

uccuucaccu acacccugga cgagaaggug gccgagcugg uggaguuccu ccugcugaag     480

uacgaggccg aggagcccgu caccgaggcc gagaugcuca ugaucgugau caaguacaag     540

gacuacuucc ccgugauccu gaagcgcgcc cgggaguuca uggagcugcu cuucggccug     600

gcgcugaucg aggucgggcc cgaccacuuc ugcguguucg ccaacacggu gggccucacc     660

gacgagggga gcgacgacga gggcaugccg gagaacuccc ugcugaucau cauccucagc     720

gucaucuuca ucaagggcaa cugcgccucc gaggagguga ucugggaggu gcugaacgcc     780

gucggggugu acgcgggccg cgagcacuuc guguacgggg agccccggga gcugcucacc     840

aaggucuggg ugcagggcca cuaccuggag uaccgcgagg ugccgcacag cucccccccg     900

uacuacgagu uccugugggg cccccgggcc cacagcgagu ccaucaagaa gaagguccuc     960

gaguuccugg ccaagcugaa caacaccgug cccagcagcu uccccuccug guacaaggac    1020

gcccucaagg acgucgagga gcgcgugcag gccacgaucg acaccgcgga cgacgccacc    1080

gugauggcca gcgagucccu gagcgucaug uccagcaacg uguccuucag cgagugacca    1140

cuaguuauaa gacugacuag cccgaugggc cucccaacgg gcccuccucc ccuccuugca    1200

ccgagauuaa uaaaaaaaaa aaaaaaaaaa aaaaaaaaaa aaaaaaaaaa aaaaaaaaaa    1260

aaaaaaaaaa aaaaaugcau cccccccccc cccccccccc cccccccccc caaaggcucu    1320

uuucagagcc accagaauu                                                 1339

<210>  24
<211>  1885
<212>  RNA
<213>  Artificial Sequence

<220>
<223>  MUC1 5 VNTR (GC)-muag-A64-C30-histoneSL

<400>  24
gggagaaagc uuaccaugac ccccggcacc cagagcccgu ucuuccugcu ccugcugcuc      60

acggugcuga ccgucgugac cggguccggc cacgccagcu ccacccccgg gggcgagaag     120

gagacgagcg ccacccagcg guccagcgug cccuccagca ccgagaagaa cgcggucucc 180

augaccagcu ccgugcugag cucccacagc cccggguccg gcagcuccac gacccagggc 240

caggacguga cccucgcccc ggccaccgag cccgccagcg gguccgccgc gacguggggc 300

caggacguca ccagcgugcc cgugacccgc cccgcccugg ggagcaccac gccgcccgcc 360

cacgacguca ccuccgcccc cgacaacaag cccgcgccgg gcagcaccgc cccccccgcc 420

cacgggguga ccuccgcccc cgacacgcgg ccggcccccg gcagcaccgc gccccccgcc 480

cacggcguga ccuccgcccc ggacacccgc cccgcccccg ggagcacggc cccgccggcg 540

cacggcguca ccuccgcccc cgacacccgg cccgcccccg ggagcaccgc cccgcccgcc 600

cacggcguga cguccgcgcc cgacacccgc ccggcccccg gcagcaccgc cccccccgcc 660

cacgggguga ccuccgcccc ggacacgcgg cccgcgcccg gcagcaccgc cccgccggcc 720

cacgggguca ccuccgcgcc cgacaaccgc cccgcccugg ggagcaccgc cccgcccgug 780

cacaacguga ccuccgccag cggcuccgcg agcggguccg ccagcacccu cguccacaac 840

ggcacguccg cccgggccac caccaccccc gccagcaagu ccacgcccuu cagcaucccg 900

ucccaccaca gcgacacccc caccacccug gcgucccaca gcacgaagac cgacgccucc 960

agcacccacc acuccagcgu gcccccgcug accagcucca accacagcac guccccgcag 1020

cucagcaccg ggguguccuu cuucuuccug agcuuccaca ucuccaaccu gcaguucaac 1080

agcucccucg aggaccccag caccgacuac uaccaggagc ugcagcggga caucuccgag 1140

auguuccugc agaucuacaa gcagggcggc uuccucgggc ugagcaacau caaguuccgc 1200

cccggcuccg ucguggugca gcugacccuc gccuuccggg aggggacgau caacguccac 1260

gacguggaga cccaguucaa ccaguacaag accgaggccg ccagccgcua caaccugacc 1320

aucuccgacg ugagcgucuc cgacgugccc uucccguuca gcgcgcaguc cggcgccggc 1380

gugcccgggu ggggcaucgc ccugcucguc cuggugugcg ugcuggucgc ccucgccauc 1440

guguaccuga ucgcgcuggc cgugugccag ugccggcgca agaacuacgg gcagcucgac 1500

aucuuccccg cccgggacac guaccacccg augagcgagu acccgaccua ccacacccac 1560

ggccgcuacg ucccccccag cuccaccgac cggagccccu acgagaaggu guccgccggg 1620

aacggcggca gcucccugag cuacaccaac ccggcggugg ccgccgccuc cgccaaccug 1680

ugaccacuag uuauaagacu gacuagcccg augggccucc caacgggccc uccuccccuc 1740

cuugcaccga gauuaauaaa aaaaaaaaaa aaaaaaaaaa aaaaaaaaaa aaaaaaaaaa 1800

aaaaaaaaaa aaaaaaaaaa augcaucccc cccccccccc cccccccccc cccccccaaa 1860

ggcucuuuuc agagccacca gaauu 1885

<210> 25
<211> 1596

<212>  RNA
<213>  Artificial Sequence

<220>
<223>  MAGE-C1 (aa 613-1142) CDS GC-optimized

<400>  25
augcaguccc cgcugcaggg cgaggaguuc cagagcuccc ugcagagccc cguguccauc      60

ugcagcucca gcacccccuc cagccucccg cagagcuucc ccgaguccag ccaguccccc     120

cccgagggcc cgguccagag cccccugcac uccccgcaga gccccccgga ggggaugcac     180

ucccagagcc cccugcaguc ccccgagagc gcccccgagg gcgaggacuc ccucagcccg     240

cugcagaucc cccagucccc gcuggagggg gaggacagcc ucuccagccu gcacuucccc     300

caguccccgc ccgaguggga ggacagccug agcccccucc acuuccccca guucccgccc     360

cagggcgagg acuuccaguc cagccugcag ucccccguga gcaucugcuc cagcuccacg     420

agccuguccc ucccccagag cuucccggag uccccccaga gcccgcccga ggggccggcg     480

cagucccccc ugcagcgccc cgugagcucc uucuucagcu acacccuggc cucccuccug     540

cagagcuccc acgagagccc gcagagcccg cccgagggcc ccgcccaguc cccgcugcag     600

agccccgugu ccagcuuccc cuccagcacc uccagcuccc ucagccaguc cagccccgug     660

uccagcuucc cguccagcac cuccagcucc cugagcaaga gcucccccga gagcccccug     720

cagucccccg ugaucagcuu cuccagcucc acgagccucu ccccguucag cgaggagucc     780

agcucccccg ucgacgagua caccagcucc agcgacaccc ugcuggaguc cgacagccuc     840

accgacuccg agagccugau cgagagcgag ccccuguuca ccuacacgcu cgacgagaag     900

guggacgagc uggcccgguu ccugcuccug aaguaccagg ugaagcagcc caucaccaag     960

gccgagaugc ugaccaacgu caucucccgc uacaccggcu acuucccggu gaucuuccgg    1020

aaggcgcgcg aguucaucga gauccucuuc gggaucagcc ugcgggaggu ggaccccgac    1080

gacuccuacg ucuucgugaa cacgcuggac cucaccagcg agggcugccu guccgacgag    1140

caggggauga gccagaaccg ccugcucauc cugauccugu ccaucaucuu caucaagggc    1200

accuacgcca gcgaggaggu caucugggac gugcucuccg ggaucggcgu gcgggccggc    1260

cgcgagcacu ucgccuucgg ggagccccgg gagcugcuga ccaaggucug ggugcaggag    1320

cacuaccucg aguaccgcga ggugcccaac agcuccccgc cccgguacga guuccugugg    1380

ggcccccgcg cccacagcga ggucaucaag cggaaggugg uggaguuccu ggcgaugcuc    1440

aagaacacgg uccccaucac cuucccgucc agcuacaagg acgcccugaa ggacguggag    1500

gagcgggccc aggccaucau cgacaccacc gacgacucca cggccaccga gagcgcgucc    1560

agcuccguga ugagccccag cuucuccagc gaguga                              1596

<210>  26

<211>  16
<212>  RNA
<213>  Artificial Sequence

<220>
<223>  Sequence according to formula (Ic)


<220>
<221>  misc_feature
<222>  (1)..(1)
<223>  n is a, u, t, g, and c, or a nucleotide analogue thereof

<220>
<221>  misc_feature
<222>  (3)..(8)
<223>  n is a, u, t, g, and c, or a nucleotide analogue thereof

<220>
<221>  misc_feature
<222>  (10)..(14)
<223>  n is a, u, t, g, and c, or a nucleotide analogue thereof

<220>
<221>  misc_feature
<222>  (16)..(16)
<223>  n is a, u, t, g, and c, or a nucleotide analogue thereof

<400>  26
ngnnnnnnun nnnncn                                                    16


<210>  27
<211>  26
<212>  RNA
<213>  Artificial Sequence

<220>
<223>  Sequence according to formula (IIc)


<220>
<221>  misc_feature
<222>  (1)..(2)
<223>  n is a, u, t, g, and c, or a nucleotide analogue thereof; may be
       present or not

<220>
<221>  misc_feature
<222>  (3)..(6)
<223>  n is a, u, t, g, and c, or a nucleotide analogue thereof

<220>
<221>  misc_feature
<222>  (8)..(13)
<223>  n is a, u, t, g, and c, or a nucleotide analogue thereof

<220>
<221>  misc_feature
<222>  (15)..(19)
<223>  n is a, u, t, g, and c, or a nucleotide analogue thereof

<220>

<221>  misc_feature
<222>  (21)..(24)
<223>  n is a, u, t, g, and c, or a nucleotide analogue thereof

<220>
<221>  misc_feature
<222>  (25)..(26)
<223>  n is a, u, t, g, and c, or a nucleotide analogue thereof; may be
       present or not.

<400>  27
nnnnnngnnn nnnunnnnnc nnnnnn                                          26


<210>  28
<211>  16
<212>  RNA
<213>  Artificial Sequence

<220>
<223>  Sequence according to formula (Id)


<220>
<221>  misc_feature
<222>  (1)..(1)
<223>  n is a, u, t, g, and c, or a nucleotide analogue thereof

<220>
<221>  misc_feature
<222>  (3)..(8)
<223>  n is a, u, t, g, and c, or a nucleotide analogue thereof

<220>
<221>  misc_feature
<222>  (10)..(14)
<223>  n is a, u, t, g, and c, or a nucleotide analogue thereof

<220>
<221>  misc_feature
<222>  (16)..(16)
<223>  n is a, u, t, g, and c, or a nucleotide analogue thereof

<400>  28
ncnnnnnnun nnnngn                                                     16


<210>  29
<211>  26
<212>  RNA
<213>  Artificial Sequence

<220>
<223>  Sequence according to formula (IId)


<220>
<221>  misc_feature
<222>  (1)..(2)
<223>  n is a, u, t, g, and c, or a nucleotide analogue thereof; may be
       present or not.

<220>

<221>  misc_feature
<222>  (3)..(6)
<223>  n is a, u, t, g, and c, or a nucleotide analogue thereof

<220>
<221>  misc_feature
<222>  (8)..(13)
<223>  n is a, u, t, g, and c, or a nucleotide analogue thereof

<220>
<221>  misc_feature
<222>  (15)..(19)
<223>  n is a, u, t, g, and c, or a nucleotide analogue thereof

<220>
<221>  misc_feature
<222>  (21)..(23)
<223>  n is a, u, t, g, and c, or a nucleotide analogue thereof

<220>
<221>  misc_feature
<222>  (24)..(26)
<223>  n is a, u, t, g, and c, or a nucleotide analogue thereof; may be
      present or not

<400>  29
nnnnnncnnn nnnunnnnng nnnnnn                                          26


<210>  30
<211>  16
<212>  RNA
<213>  Artificial Sequence

<220>
<223>  Sequence according to formula (Ie)


<220>
<221>  misc_feature
<222>  (3)..(8)
<223>  n is a, u, t, g, and c, or a nucleotide analogue thereof

<220>
<221>  misc_feature
<222>  (10)..(14)
<223>  n is a, u, t, g, and c, or a nucleotide analogue thereof

<400>  30
dgnnnnnnun nnnnch                                                     16


<210>  31
<211>  26
<212>  RNA
<213>  Artificial Sequence

<220>
<223>  Sequence according to formula (IIe)


<220>
<221>  misc_feature

<222>  (1)..(2)
<223>  n is a, u, t, g, and c, or a nucleotide analogue thereof; may be
       present or not

<220>
<221>  misc_feature
<222>  (3)..(5)
<223>  n is a, u, t, g, and c, or a nucleotide analogue thereof

<220>
<221>  misc_feature
<222>  (8)..(13)
<223>  n is a, u, t, g, and c, or a nucleotide analogue thereof

<220>
<221>  misc_feature
<222>  (15)..(19)
<223>  n is a, u, t, g, and c, or a nucleotide analogue thereof

<220>
<221>  misc_feature
<222>  (22)..(23)
<223>  n is a, u, t, g, and c, or a nucleotide analogue thereof

<220>
<221>  misc_feature
<222>  (24)..(26)
<223>  n is a, u, t, g, and c, or a nucleotide analogue thereof; may be
       present or not

<400>  31
nnnnndgnnn nnnunnnnnc hnnnnn                                          26


<210>  32
<211>  16
<212>  RNA
<213>  Artificial Sequence

<220>
<223>  Sequence according to formula (If)


<220>
<221>  misc_feature
<222>  (1)..(1)
<223>  n is a, u, t, g, and c, or a nucleotide analogue thereof

<220>
<221>  misc_feature
<222>  (3)..(3)
<223>  n is a, u, t, g, and c, or a nucleotide analogue thereof

<220>
<221>  misc_feature
<222>  (7)..(8)
<223>  n is a, u, t, g, and c, or a nucleotide analogue thereof

<220>
<221>  misc_feature
<222>  (10)..(10)
<223>  n is a, u, t, g, and c, or a nucleotide analogue thereof

<220>
<221>  misc_feature
<222>  (12)..(12)
<223>  n is a, u, t, g, and c, or a nucleotide analogue thereof

<220>
<221>  misc_feature
<222>  (14)..(14)
<223>  n is a, u, t, g, and c, or a nucleotide analogue thereof

<220>
<221>  misc_feature
<222>  (16)..(16)
<223>  n is a, u, t, g, and c, or a nucleotide analogue thereof

<400>  32
ngnbyynnun vndncn                                                       16


<210>  33
<211>  26
<212>  RNA
<213>  Artificial Sequence

<220>
<223>  Sequence according to formula (IIf)


<220>
<221>  misc_feature
<222>  (1)..(2)
<223>  n is a, u, t, g, and c, or a nucleotide analogue thereof; may be
       present or not

<220>
<221>  misc_feature
<222>  (3)..(6)
<223>  n is a, u, t, g, and c, or a nucleotide analogue thereof

<220>
<221>  misc_feature
<222>  (8)..(8)
<223>  n is a, u, t, g, and c, or a nucleotide analogue thereof

<220>
<221>  misc_feature
<222>  (12)..(13)
<223>  n is a, u, t, g, and c, or a nucleotide analogue thereof

<220>
<221>  misc_feature
<222>  (15)..(15)
<223>  n is a, u, t, g, and c, or a nucleotide analogue thereof

<220>
<221>  misc_feature
<222>  (17)..(17)
<223>  n is a, u, t, g, and c, or a nucleotide analogue thereof

<220>
<221>  misc_feature
<222>  (19)..(19)
<223>  n is a, u, t, g, and c, or a nucleotide analogue thereof

<220>
<221> misc_feature
<222> (21)..(23)
<223> n is a, u, t, g, and c, or a nucleotide analogue thereof

<220>
<221> misc_feature
<222> (24)..(26)
<223> n is a, u, t, g, and c, or a nucleotide analogue thereof; may be
      present or not

<400> 33
nnnnnngnby ynnunvndnc nnnnnn                                          26


<210> 34
<211> 16
<212> RNA
<213> Artificial Sequence

<220>
<223> Sequence according to formula (Ig)


<220>
<221> misc_feature
<222> (1)..(1)
<223> n is a, u, t, g, and c, or a nucleotide analogue thereof

<220>
<221> misc_feature
<222> (8)..(8)
<223> n is a, u, t, g, and c, or a nucleotide analogue thereof

<220>
<221> misc_feature
<222> (16)..(16)
<223> n is a, u, t, g, and c, or a nucleotide analogue thereof

<400> 34
nghyyydnuh abrdcn                                                     16


<210> 35
<211> 26
<212> RNA
<213> Artificial Sequence

<220>
<223> Sequence according to formula (IIg)


<220>
<221> misc_feature
<222> (1)..(2)
<223> n is a, u, t, g, and c, or a nucleotide analogue thereof; may be
      present or not

<220>
<221> misc_feature
<222> (4)..(6)
<223> n is a, u, t, g, and c, or a nucleotide analogue thereof

<220>
<221>  misc_feature
<222>  (13)..(13)
<223>  n is a, u, t, g, and c, or a nucleotide analogue thereof

<220>
<221>  misc_feature
<222>  (21)..(23)
<223>  n is a, u, t, g, and c, or a nucleotide analogue thereof

<220>
<221>  misc_feature
<222>  (24)..(25)
<223>  n is a, u, t, g, and c, or a nucleotide analogue thereof; may be
      present or not

<220>
<221>  misc_feature
<222>  (26)..(26)
<223>  may be present or not

<400>  35
nnhnnnghyy ydnuhabrdc nnnnnh                                          26


<210>  36
<211>  16
<212>  RNA
<213>  Artificial Sequence

<220>
<223>  Sequence according to formula (Ih)

<400>  36
dghycudyuh asrrcc                                                     16


<210>  37
<211>  26
<212>  RNA
<213>  Artificial Sequence

<220>
<223>  Sequence according to formula (IIh)


<220>
<221>  misc_feature
<222>  (1)..(1)
<223>  n is a, u, t, g, and c, or a nucleotide analogue thereof; may be
      present or not

<220>
<221>  misc_feature
<222>  (2)..(2)
<223>  may be present or not

<220>
<221>  misc_feature
<222>  (25)..(25)
<223>  n is a, u, t, g, and c, or a nucleotide analogue thereof; may be
      present or not

<220>
<221>  misc_feature
<222>  (26)..(26)
<223>  may be present or not

<400>  37
nhaahdghyc udyuhasrrc cvhbnh                                          26


<210>  38
<211>  16
<212>  DNA
<213>  Artificial Sequence

<220>
<223>  Sequence according to formula (Ic)

<400>  38
vgyyyyhhth rvvrcb                                                     16


<210>  39
<211>  16
<212>  DNA
<213>  Artificial Sequence

<220>
<223>  Sequence according to formula (Ic)

<400>  39
sgyyyttytm arrrcs                                                     16


<210>  40
<211>  16
<212>  DNA
<213>  Artificial Sequence

<220>
<223>  Sequence according to formula (Ic)

<400>  40
sgyycttttm agrrcs                                                     16


<210>  41
<211>  16
<212>  DNA
<213>  Artificial Sequence

<220>
<223>  Sequence according to formula (Ie)


<220>
<221>  misc_feature
<222>  (3)..(5)
<223>  n is a, u, t, g, and c, or a nucleotide analogue thereof

<220>
<221>  misc_feature
<222>  (7)..(8)

<223>  n is a, u, t, g, and c, or a nucleotide analogue thereof

<220>
<221>  misc_feature
<222>  (12)..(14)
<223>  n is a, u, t, g, and c, or a nucleotide analogue thereof

<400>  41
dgnnnbnnth vnnnch                                                        16


<210>  42
<211>  16
<212>  DNA
<213>  Artificial Sequence

<220>
<223>  Sequence according to formula (Ie)


<220>
<221>  misc_feature
<222>  (3)..(5)
<223>  n is a, u, t, g, and c, or a nucleotide analogue thereof

<220>
<221>  misc_feature
<222>  (13)..(14)
<223>  n is a, u, t, g, and c, or a nucleotide analogue thereof

<400>  42
rgnnnyhbth rdnncy                                                        16


<210>  43
<211>  16
<212>  DNA
<213>  Artificial Sequence

<220>
<223>  Sequence according to formula (Ie)


<220>
<221>  misc_feature
<222>  (3)..(3)
<223>  n is a, u, t, g, and c, or a nucleotide analogue thereof

<220>
<221>  misc_feature
<222>  (14)..(14)
<223>  n is a, u, t, g, and c, or a nucleotide analogue thereof

<400>  43
rgndbyhyth rdhncy                                                        16


<210>  44
<211>  16
<212>  DNA
<213>  Artificial Sequence

<220>

<223>  Sequence according to formula (If)

<400>  44
vgyyytyhth rvrrcb                                                        16


<210>  45
<211>  16
<212>  DNA
<213>  Artificial Sequence

<220>
<223>  Sequence according to formula (If)

<400>  45
sgyycttytm agrrcs                                                        16


<210>  46
<211>  16
<212>  DNA
<213>  Artificial Sequence

<220>
<223>  Sequence according to formula (If)

<400>  46
   sgyycttttm agrrcs                                                         16


<210>  47
<211>  16
<212>  DNA
<213>  Artificial Sequence

<220>
<223>  Sequence according to formula (Ig)

<400>  47
ggyycttyth agrrcc                                                        16


<210>  48
<211>  16
<212>  DNA
<213>  Artificial Sequence

<220>
<223>  Sequence according to formula (Ig)

<400>  48
ggcycttytm agrgcc                                                        16


<210>  49
<211>  16
<212>  DNA
<213>  Artificial Sequence

<220>
<223>  Sequence according to formula (Ig)

<400>  49
   ggctcttttm agrgcc                                                    16


<210>  50
<211>  16
<212>  DNA
<213>  Artificial Sequence

<220>
<223>  Sequence according to formula (Ih)

<400>  50
dghyctdyth asrrcc                                                    16


<210>  51
<211>  16
<212>  DNA
<213>  Artificial Sequence

<220>
<223>  Sequence according to formula (Ih)

<400>  51
ggcyctttth agrgcc                                                    16


<210>  52
<211>  16
<212>  DNA
<213>  Artificial Sequence

<220>
<223>  Sequence according to formula (Ih)

<400>  52
   ggcycttttm agrgcc                                                    16


<210>  53
<211>  26
<212>  DNA
<213>  Artificial Sequence

<220>
<223>  Sequence according to formula (IIc)


<220>
<221>  misc_feature
<222>  (1)..(2)
<223>  may be present or not

<220>
<221>  misc_feature
<222>  (25)..(26)
<223>  n is a, u, t, g, and c, or a nucleotide analogue thereof; may be
       present or not

<400>  53
hhhhvvgyyy yhhthrvvrc bvhhnn                                         26

<210>  54
<211>  26
<212>  DNA
<213>  Artificial Sequence

<220>
<223>  Sequence according to formula (IIc)


<220>
<221>  misc_feature
<222>  (1)..(2)
<223>  may be present or not

<220>
<221>  misc_feature
<222>  (25)..(26)
<223>  may be present or not

<400>  54
mhmhmsgyyy ttytmarrrc smchhh                                          26


<210>  55
<211>  26
<212>  DNA
<213>  Artificial Sequence

<220>
<223>  Sequence according to formula (IIc)


<220>
<221>  misc_feature
<222>  (1)..(2)
<223>  may be present or not

<220>
<221>  misc_feature
<222>  (25)..(26)
<223>  may be present or not

<400>  55
mmmmmsgyyc ttttmagrrc sachmh                                          26


<210>  56
<211>  26
<212>  DNA
<213>  Artificial Sequence

<220>
<223>  Sequence according to formula (IIe)


<220>
<221>  misc_feature
<222>  (1)..(2)
<223>  n is a, u, t, g, and c, or a nucleotide analogue thereof; may be
       present or not

<220>
<221>  misc_feature

<222>  (3)..(5)
<223>  n is a, u, t, g, and c, or a nucleotide analogue thereof

<220>
<221>  misc_feature
<222>  (8)..(10)
<223>  n is a, u, t, g, and c, or a nucleotide analogue thereof

<220>
<221>  misc_feature
<222>  (12)..(13)
<223>  n is a, u, t, g, and c, or a nucleotide analogue thereof

<220>
<221>  misc_feature
<222>  (17)..(19)
<223>  n is a, u, t, g, and c, or a nucleotide analogue thereof

<220>
<221>  misc_feature
<222>  (22)..(22)
<223>  n is a, u, t, g, and c, or a nucleotide analogue thereof

<220>
<221>  misc_feature
<222>  (24)..(26)
<223>  n is a, u, t, g, and c, or a nucleotide analogue thereof; may be
       present or not

<400>  56
nnnnndgnnn bnnthvnnnc hnhnnn                                          26


<210>  57
<211>  26
<212>  DNA
<213>  Artificial Sequence

<220>
<223>  Sequence according to formula (IIe)


<220>
<221>  misc_feature
<222>  (1)..(1)
<223>  n is a, u, t, g, and c, or a nucleotide analogue thereof; may be
       present or not

<220>
<221>  misc_feature
<222>  (2)..(2)
<223>  n is a, u, t, g, and c, or a nucleotide analogue thereof; may be
       present or not

<220>
<221>  misc_feature
<222>  (5)..(5)
<223>  n is a, u, t, g, and c, or a nucleotide analogue thereof

<220>
<221>  misc_feature
<222>  (8)..(10)
<223>  n is a, u, t, g, and c, or a nucleotide analogue thereof

<220>
<221>  misc_feature
<222>  (18)..(19)
<223>  n is a, u, t, g, and c, or a nucleotide analogue thereof

<220>
<221>  misc_feature
<222>  (24)..(24)
<223>  may be present or not

<220>
<221>  misc_feature
<222>  (25)..(26)
<223>  n is a, u, t, g, and c, or a nucleotide analogue thereof; may be
      present or not

<400>  57
nnhhnrgnnn yhbthrdnnc ydhhnn                                          26


<210>  58
<211>  26
<212>  DNA
<213>  Artificial Sequence

<220>
<223>  Sequence according to formula (IIe)


<220>
<221>  misc_feature
<222>  (1)..(1)
<223>  n is a, u, t, g, and c, or a nucleotide analogue thereof; may be
      present or not

<220>
<221>  misc_feature
<222>  (2)..(2)
<223>  may be present or not

<220>
<221>  misc_feature
<222>  (8)..(8)
<223>  n is a, u, t, g, and c, or a nucleotide analogue thereof

<220>
<221>  misc_feature
<222>  (19)..(19)
<223>  n is a, u, t, g, and c, or a nucleotide analogue thereof

<220>
<221>  misc_feature
<222>  (24)..(26)
<223>  may be present or not

<400>  58
nhhhvrgndb yhythrdhnc yrhhhh                                          26


<210>  59
<211>  26
<212>  DNA

<213>  Artificial Sequence

<220>
<223>  Sequence according to formula (IIf)


<220>
<221>  misc_feature
<222>  (1)..(2)
<223>  may be present or not

<220>
<221>  misc_feature
<222>  (25)..(25)
<223>  may be present or not

<220>
<221>  misc_feature
<222>  (26)..(26)
<223>  n is a, u, t, g, and c, or a nucleotide analogue thereof; may be
       present or not

<400>  59
hhmhmvgyyy tyhthrvrrc bvmhhn                                          26


<210>  60
<211>  26
<212>  DNA
<213>  Artificial Sequence

<220>
<223>  Sequence according to formula (IIf)


<220>
<221>  misc_feature
<222>  (1)..(2)
<223>  may be present or not

<220>
<221>  misc_feature
<222>  (25)..(26)
<223>  may be present or not

<400>  60
mmmmmsgyyc ttytmagrrc smchhh                                          26


<210>  61
<211>  26
<212>  DNA
<213>  Artificial Sequence

<220>
<223>  Sequence according to formula (IIf)


<220>
<221>  misc_feature
<222>  (1)..(2)
<223>  may be present or not

<220>
<221>  misc_feature
<222>  (25)..(26)
<223>  may be present or not

<400>  61
mmmmmsgyyc ttttmagrrc sachmh                                          26


<210>  62
<211>  26
<212>  DNA
<213>  Artificial Sequence

<220>
<223>  Sequence according to formula (IIg)


<220>
<221>  misc_feature
<222>  (1)..(2)
<223>  may be present or not

<220>
<221>  misc_feature
<222>  (24)..(25)
<223>  n is a, u, t, g, and c, or a nucleotide analogue thereof; may be
      present or not

<220>
<221>  misc_feature
<222>  (26)..(26)
<223>  may be present or not

<400>  62
hhmamggyyc ttythagrrc cvhnnm                                          26


<210>  63
<211>  26
<212>  DNA
<213>  Artificial Sequence

<220>
<223>  Sequence according to formula (IIg)


<220>
<221>  misc_feature
<222>  (1)..(2)
<223>  may be present or not

<220>
<221>  misc_feature
<222>  (25)..(26)
<223>  may be present or not

<400>  63
hhaamggcyc ttytmagrgc cvchhm                                          26


<210>  64
<211>  26

<212>  DNA
<213>  Artificial Sequence

<220>
<223>  Sequence according to formula (IIg)


<220>
<221>  misc_feature
<222>  (1)..(2)
<223>  may be present or not

<220>
<221>  misc_feature
<222>  (24)..(26)
<223>  may be present or not

<400>  64
mmaamggctc ttttmagrgc cmcymm                                          26


<210>  65
<211>  26
<212>  DNA
<213>  Artificial Sequence

<220>
<223>  Sequence according to formula (IIh)


<220>
<221>  misc_feature
<222>  (1)..(1)
<223>  n is a, u, t, g, and c, or a nucleotide analogue thereof; may be
       present or not

<220>
<221>  misc_feature
<222>  (2)..(2)
<223>  may be present or not

<220>
<221>  misc_feature
<222>  (24)..(24)
<223>  may be present or not

<220>
<221>  misc_feature
<222>  (25)..(25)
<223>  n is a, u, t, g, and c, or a nucleotide analogue thereof; may be
       present or not

<220>
<221>  misc_feature
<222>  (26)..(26)
<223>  may be present or not

<400>  65
nhaahdghyc tdythasrrc cvhbnh                                          26


<210>  66
<211>  26

<212>  DNA
<213>  Artificial Sequence

<220>
<223>  Sequence according to formula (IIh)


<220>
<221>  misc_feature
<222>  (1)..(2)
<223>  may be present or not

<220>
<221>  misc_feature
<222>  (24)..(24)
<223>  may be present or not

<220>
<221>  misc_feature
<222>  (25)..(25)
<223>  n is a, u, t, g, and c, or a nucleotide analogue thereof; may be
       present or not

<220>
<221>  misc_feature
<222>  (26)..(26)
<223>  may be present or not

<400>  66
hhaamggcyc ttttthagrgc cvmynm                                          26


<210>  67
<211>  26
<212>  DNA
<213>  Artificial Sequence

<220>
<223>  Sequence according to formula (IIh)


<220>
<221>  misc_feature
<222>  (1)..(2)
<223>  may be present or not

<220>
<221>  misc_feature
<222>  (24)..(26)
<223>  may be present or not

<400>  67
hmaaaggcyc ttttmagrgc crmyhm                                           26


<210>  68
<211>  13
<212>  RNA
<213>  Artificial Sequence

<220>
<223>  Kozak sequence

<400>  68
gccgccacca ugg                                                          13


<210>  69
<211>  15
<212>  RNA
<213>  Artificial Sequence

<220>
<223>  general formula of a 3-UTR stabilizing sequence


<220>
<221>  misc_feature
<222>  (1)..(1)
<223>  n is c or u

<220>
<221>  repeat_unit
<222>  (5)..(5)
<223>  n is a, u, t, g, or c; may be present or not

<220>
<221>  misc_feature
<222>  (5)..(5)
<223>  n is a, u, t, g, or c

<220>
<221>  misc_feature
<222>  (9)..(9)
<223>  n is a, or u

<220>
<221>  repeat_unit
<222>  (10)..(10)
<223>  n is c or u, may be present or not

<220>
<221>  misc_feature
<222>  (10)..(10)
<223>  n is c or u

<220>
<221>  misc_feature
<222>  (13)..(13)
<223>  n is c or u

<400>  69
nccancccnn ucncc                                                        15


<210>  70
<211>  44
<212>  RNA
<213>  Artificial Sequence

<220>
<223>  3'-UTR of an alpha-globin gene (muag)

<400>  70
gcccgauggg ccucccaacg ggcccuccuc cccuccuugc accg                        44

<210>  71
<211>  24
<212>  DNA
<213>  Artificial Sequence

<220>
<223>  Specific histone stem-loop sequence

<400>  71
caaaggctct tttcagagcc acca                                              24


<210>  72
<211>  24
<212>  RNA
<213>  Artificial Sequence

<220>
<223>  Specific histone stem-loop sequence

<400>  72
caaaggcucu uuucagagcc acca                                              24


<210>  73
<211>  9
<212>  PRT
<213>  Artificial Sequence

<220>
<223>  MUC1-derived peptide

<400>  73

Ser Ala Pro Asp Asn Arg Pro Ala Leu
1               5


<210>  74
<211>  8
<212>  PRT
<213>  Artificial Sequence

<220>
<223>  Connexin-derived peptide

<400>  74

Phe Glu Gln Asn Thr Ala Gln Pro
1               5


<210>  75
<211>  420
<212>  PRT
<213>  Artificial Sequence

<220>
<223>  5T4 Protein NP_001159864.1

<400>  75

Met Pro Gly Gly Cys Ser Arg Gly Pro Ala Ala Gly Asp Gly Arg Leu
1               5                   10                  15

Arg Leu Ala Arg Leu Ala Leu Val Leu Leu Gly Trp Val Ser Ser Ser
            20                  25                  30

Ser Pro Thr Ser Ser Ala Ser Ser Phe Ser Ser Ser Ala Pro Phe Leu
        35                  40                  45

Ala Ser Ala Val Ser Ala Gln Pro Pro Leu Pro Asp Gln Cys Pro Ala
    50                  55                  60

Leu Cys Glu Cys Ser Glu Ala Ala Arg Thr Val Lys Cys Val Asn Arg
65                  70                  75                  80

Asn Leu Thr Glu Val Pro Thr Asp Leu Pro Ala Tyr Val Arg Asn Leu
                85                  90                  95

Phe Leu Thr Gly Asn Gln Leu Ala Val Leu Pro Ala Gly Ala Phe Ala
            100                 105                 110

Arg Arg Pro Pro Leu Ala Glu Leu Ala Ala Leu Asn Leu Ser Gly Ser
        115                 120                 125

Arg Leu Asp Glu Val Arg Ala Gly Ala Phe Glu His Leu Pro Ser Leu
    130                 135                 140

Arg Gln Leu Asp Leu Ser His Asn Pro Leu Ala Asp Leu Ser Pro Phe
145                 150                 155                 160

Ala Phe Ser Gly Ser Asn Ala Ser Val Ser Ala Pro Ser Pro Leu Val
                165                 170                 175

Glu Leu Ile Leu Asn His Ile Val Pro Pro Glu Asp Glu Arg Gln Asn
            180                 185                 190

Arg Ser Phe Glu Gly Met Val Val Ala Ala Leu Leu Ala Gly Arg Ala
        195                 200                 205

Leu Gln Gly Leu Arg Arg Leu Glu Leu Ala Ser Asn His Phe Leu Tyr
    210                 215                 220

Leu Pro Arg Asp Val Leu Ala Gln Leu Pro Ser Leu Arg His Leu Asp
225                 230                 235                 240

Leu Ser Asn Asn Ser Leu Val Ser Leu Thr Tyr Val Ser Phe Arg Asn
                245                 250                 255

Leu Thr His Leu Glu Ser Leu His Leu Glu Asp Asn Ala Leu Lys Val
            260                 265                 270

Leu His Asn Gly Thr Leu Ala Glu Leu Gln Gly Leu Pro His Ile Arg
        275                 280                 285

Val Phe Leu Asp Asn Asn Pro Trp Val Cys Asp Cys His Met Ala Asp
    290                 295                 300

Met Val Thr Trp Leu Lys Glu Thr Glu Val Val Gln Gly Lys Asp Arg
305                 310                 315                 320

Leu Thr Cys Ala Tyr Pro Glu Lys Met Arg Asn Arg Val Leu Leu Glu
                325                 330                 335

Leu Asn Ser Ala Asp Leu Asp Cys Asp Pro Ile Leu Pro Pro Ser Leu
            340                 345                 350

Gln Thr Ser Tyr Val Phe Leu Gly Ile Val Leu Ala Leu Ile Gly Ala
        355                 360                 365

Ile Phe Leu Leu Val Leu Tyr Leu Asn Arg Lys Gly Ile Lys Lys Trp
    370                 375                 380

Met His Asn Ile Arg Asp Ala Cys Arg Asp His Met Glu Gly Tyr His
385                 390                 395                 400

Tyr Arg Tyr Glu Ile Asn Ala Asp Pro Arg Leu Thr Asn Leu Ser Ser
                405                 410                 415

Asn Ser Asp Val
            420

<210>  76
<211>  142
<212>  PRT
<213>  Artificial Sequence

<220>
<223>  Survivin (BIRC5) Protein O15392

<400>  76

Met Gly Ala Pro Thr Leu Pro Pro Ala Trp Gln Pro Phe Leu Lys Asp
1               5                   10                  15

His Arg Ile Ser Thr Phe Lys Asn Trp Pro Phe Leu Glu Gly Cys Ala
            20                  25                  30

Cys Thr Pro Glu Arg Met Ala Glu Ala Gly Phe Ile His Cys Pro Thr
        35                  40                  45

Glu Asn Glu Pro Asp Leu Ala Gln Cys Phe Phe Cys Phe Lys Glu Leu
    50                  55                  60

Glu Gly Trp Glu Pro Asp Asp Asp Pro Ile Glu Glu His Lys Lys His
65                  70                  75                  80

Ser Ser Gly Cys Ala Phe Leu Ser Val Lys Lys Gln Phe Glu Glu Leu
                85                  90                  95

Thr Leu Gly Glu Phe Leu Lys Leu Asp Arg Glu Arg Ala Lys Asn Lys
            100                 105                 110

Ile Ala Lys Glu Thr Asn Asn Lys Lys Lys Glu Phe Glu Glu Thr Ala
        115                 120                 125

Lys Lys Val Arg Arg Ala Ile Glu Gln Leu Ala Ala Met Asp
    130                 135                 140

<210> 77
<211> 142
<212> PRT
<213> Artificial Sequence

<220>
<223> Survivin (BIRC5) Protein NP_001159.2

<400> 77

Met Gly Ala Pro Thr Leu Pro Pro Ala Trp Gln Pro Phe Leu Lys Asp
1               5                   10                  15

His Arg Ile Ser Thr Phe Lys Asn Trp Pro Phe Leu Glu Gly Cys Ala
            20                  25                  30

Cys Thr Pro Glu Arg Met Ala Glu Ala Gly Phe Ile His Cys Pro Thr
        35                  40                  45

Glu Asn Glu Pro Asp Leu Ala Gln Cys Phe Phe Cys Phe Lys Glu Leu
    50                  55                  60

Glu Gly Trp Glu Pro Asp Asp Asp Pro Ile Glu Glu His Lys Lys His
65                  70                  75                  80

Ser Ser Gly Cys Ala Phe Leu Ser Val Lys Lys Gln Phe Glu Glu Leu
                85                  90                  95

Thr Leu Gly Glu Phe Leu Lys Leu Asp Arg Glu Arg Ala Lys Asn Lys

100                 105                 110

Ile Ala Lys Glu Thr Asn Asn Lys Lys Lys Glu Phe Glu Glu Thr Ala
        115                 120                 125

Glu Lys Val Arg Arg Ala Ile Glu Gln Leu Ala Ala Met Asp
    130                 135                 140

<210>  78
<211>  180
<212>  PRT
<213>  Artificial Sequence

<220>
<223>  NY-ESO-1 Protein NP_001318.1

<400>  78

Met Gln Ala Glu Gly Arg Gly Thr Gly Gly Ser Thr Gly Asp Ala Asp
1               5                   10                  15

Gly Pro Gly Gly Pro Gly Ile Pro Asp Gly Pro Gly Gly Asn Ala Gly
            20                  25                  30

Gly Pro Gly Glu Ala Gly Ala Thr Gly Gly Arg Gly Pro Arg Gly Ala
        35                  40                  45

Gly Ala Ala Arg Ala Ser Gly Pro Gly Gly Gly Ala Pro Arg Gly Pro
    50                  55                  60

His Gly Gly Ala Ala Ser Gly Leu Asn Gly Cys Cys Arg Cys Gly Ala
65                  70                  75                  80

Arg Gly Pro Glu Ser Arg Leu Leu Glu Phe Tyr Leu Ala Met Pro Phe
                85                  90                  95

Ala Thr Pro Met Glu Ala Glu Leu Ala Arg Arg Ser Leu Ala Gln Asp
            100                 105                 110

Ala Pro Pro Leu Pro Val Pro Gly Val Leu Leu Lys Glu Phe Thr Val
        115                 120                 125

Ser Gly Asn Ile Leu Thr Ile Arg Leu Thr Ala Ala Asp His Arg Gln
    130                 135                 140

Leu Gln Leu Ser Ile Ser Ser Cys Leu Gln Gln Leu Ser Leu Leu Met
145                 150                 155                 160

Trp Ile Thr Gln Cys Phe Leu Pro Val Phe Leu Ala Gln Pro Pro Ser
                165                 170                 175

Gly Gln Arg Arg
            180

<210>  79
<211>  1142
<212>  PRT
<213>  Artificial Sequence

<220>
<223>  MAGE-C1 Protein NP_005453.2

<400>  79

Met Gly Asp Lys Asp Met Pro Thr Ala Gly Met Pro Ser Leu Leu Gln
1               5                   10                  15

Ser Ser Ser Glu Ser Pro Gln Ser Cys Pro Glu Gly Glu Asp Ser Gln
            20                  25                  30

Ser Pro Leu Gln Ile Pro Gln Ser Ser Pro Glu Ser Asp Asp Thr Leu
        35                  40                  45

Tyr Pro Leu Gln Ser Pro Gln Ser Arg Ser Glu Gly Glu Asp Ser Ser
    50                  55                  60

Asp Pro Leu Gln Arg Pro Pro Glu Gly Lys Asp Ser Gln Ser Pro Leu
65                  70                  75                  80

Gln Ile Pro Gln Ser Ser Pro Glu Gly Asp Asp Thr Gln Ser Pro Leu
                85                  90                  95

Gln Asn Ser Gln Ser Ser Pro Glu Gly Lys Asp Ser Leu Ser Pro Leu
            100                 105                 110

Glu Ile Ser Gln Ser Pro Pro Glu Gly Glu Asp Val Gln Ser Pro Leu
        115                 120                 125

Gln Asn Pro Ala Ser Ser Phe Phe Ser Ser Ala Leu Leu Ser Ile Phe
    130                 135                 140

Gln Ser Ser Pro Glu Ser Thr Gln Ser Pro Phe Glu Gly Phe Pro Gln
145                 150                 155                 160

Ser Val Leu Gln Ile Pro Val Ser Ala Ala Ser Ser Ser Thr Leu Val
                165                 170                 175

Ser Ile Phe Gln Ser Ser Pro Glu Ser Thr Gln Ser Pro Phe Glu Gly
            180                 185                 190

Phe Pro Gln Ser Pro Leu Gln Ile Pro Val Ser Arg Ser Phe Ser Ser
        195                 200                 205

Thr Leu Leu Ser Ile Phe Gln Ser Ser Pro Glu Arg Thr Gln Ser Thr
    210                 215                 220

Phe Glu Gly Phe Ala Gln Ser Pro Leu Gln Ile Pro Val Ser Pro Ser
225                 230                 235                 240

Ser Ser Ser Thr Leu Leu Ser Leu Phe Gln Ser Phe Ser Glu Arg Thr
                245                 250                 255

Gln Ser Thr Phe Glu Gly Phe Ala Gln Ser Ser Leu Gln Ile Pro Val
            260                 265                 270

Ser Pro Ser Phe Ser Ser Thr Leu Val Ser Leu Phe Gln Ser Ser Pro
        275                 280                 285

Glu Arg Thr Gln Ser Thr Phe Glu Gly Phe Pro Gln Ser Pro Leu Gln
    290                 295                 300

Ile Pro Val Ser Ser Ser Ser Ser Ser Thr Leu Leu Ser Leu Phe Gln
305                 310                 315                 320

Ser Ser Pro Glu Arg Thr His Ser Thr Phe Glu Gly Phe Pro Gln Ser
                325                 330                 335

Leu Leu Gln Ile Pro Met Thr Ser Ser Phe Ser Ser Thr Leu Leu Ser
            340                 345                 350

Ile Phe Gln Ser Ser Pro Glu Ser Ala Gln Ser Thr Phe Glu Gly Phe
        355                 360                 365

Pro Gln Ser Pro Leu Gln Ile Pro Gly Ser Pro Ser Phe Ser Ser Thr
    370                 375                 380

Leu Leu Ser Leu Phe Gln Ser Ser Pro Glu Arg Thr His Ser Thr Phe
385                 390                 395                 400

Glu Gly Phe Pro Gln Ser Pro Leu Gln Ile Pro Met Thr Ser Ser Phe
                405                 410                 415

Ser Ser Thr Leu Leu Ser Ile Leu Gln Ser Ser Pro Glu Ser Ala Gln
            420                 425                 430

Ser Ala Phe Glu Gly Phe Pro Gln Ser Pro Leu Gln Ile Pro Val Ser
        435                 440                 445

Ser Ser Phe Ser Tyr Thr Leu Leu Ser Leu Phe Gln Ser Ser Pro Glu
    450                 455                 460

Arg Thr His Ser Thr Phe Glu Gly Phe Pro Gln Ser Pro Leu Gln Ile
465                 470                 475                 480

Pro Val Ser Ser Ser Ser Ser Ser Ser Thr Leu Leu Ser Leu Phe Gln
                485                 490                 495

Ser Ser Pro Glu Cys Thr Gln Ser Thr Phe Glu Gly Phe Pro Gln Ser
            500                 505                 510

Pro Leu Gln Ile Pro Gln Ser Pro Pro Glu Gly Glu Asn Thr His Ser
        515                 520                 525

Pro Leu Gln Ile Val Pro Ser Leu Pro Glu Trp Glu Asp Ser Leu Ser
    530                 535                 540

Pro His Tyr Phe Pro Gln Ser Pro Pro Gln Gly Glu Asp Ser Leu Ser
545                 550                 555                 560

Pro His Tyr Phe Pro Gln Ser Pro Pro Gln Gly Glu Asp Ser Leu Ser
                565                 570                 575

Pro His Tyr Phe Pro Gln Ser Pro Gln Gly Glu Asp Ser Leu Ser Pro
            580                 585                 590

His Tyr Phe Pro Gln Ser Pro Pro Gln Gly Glu Asp Ser Met Ser Pro
        595                 600                 605

Leu Tyr Phe Pro Gln Ser Pro Leu Gln Gly Glu Glu Phe Gln Ser Ser
    610                 615                 620

Leu Gln Ser Pro Val Ser Ile Cys Ser Ser Ser Thr Pro Ser Ser Leu
625                 630                 635                 640

Pro Gln Ser Phe Pro Glu Ser Ser Gln Ser Pro Pro Glu Gly Pro Val
                645                 650                 655

Gln Ser Pro Leu His Ser Pro Gln Ser Pro Pro Glu Gly Met His Ser
            660                 665                 670

Gln Ser Pro Leu Gln Ser Pro Glu Ser Ala Pro Glu Gly Glu Asp Ser
        675                 680                 685

Leu Ser Pro Leu Gln Ile Pro Gln Ser Pro Leu Glu Gly Glu Asp Ser

690                 695                 700

Leu Ser Ser Leu His Phe Pro Gln Ser Pro Pro Glu Trp Glu Asp Ser
705                 710                 715                 720

Leu Ser Pro Leu His Phe Pro Gln Phe Pro Pro Gln Gly Glu Asp Phe
                725                 730                 735

Gln Ser Ser Leu Gln Ser Pro Val Ser Ile Cys Ser Ser Ser Thr Ser
            740                 745                 750

Leu Ser Leu Pro Gln Ser Phe Pro Glu Ser Pro Gln Ser Pro Pro Glu
        755                 760                 765

Gly Pro Ala Gln Ser Pro Leu Gln Arg Pro Val Ser Ser Phe Phe Ser
    770                 775                 780

Tyr Thr Leu Ala Ser Leu Leu Gln Ser Ser His Glu Ser Pro Gln Ser
785                 790                 795                 800

Pro Pro Glu Gly Pro Ala Gln Ser Pro Leu Gln Ser Pro Val Ser Ser
                805                 810                 815

Phe Pro Ser Ser Thr Ser Ser Ser Leu Ser Gln Ser Ser Pro Val Ser
            820                 825                 830

Ser Phe Pro Ser Ser Thr Ser Ser Ser Leu Ser Lys Ser Ser Pro Glu
        835                 840                 845

Ser Pro Leu Gln Ser Pro Val Ile Ser Phe Ser Ser Ser Thr Ser Leu
    850                 855                 860

Ser Pro Phe Ser Glu Glu Ser Ser Ser Pro Val Asp Glu Tyr Thr Ser
865                 870                 875                 880

Ser Ser Asp Thr Leu Leu Glu Ser Asp Ser Leu Thr Asp Ser Glu Ser
                885                 890                 895

Leu Ile Glu Ser Glu Pro Leu Phe Thr Tyr Thr Leu Asp Glu Lys Val
            900                 905                 910

Asp Glu Leu Ala Arg Phe Leu Leu Leu Lys Tyr Gln Val Lys Gln Pro
        915                 920                 925

Ile Thr Lys Ala Glu Met Leu Thr Asn Val Ile Ser Arg Tyr Thr Gly
    930                 935                 940

Tyr Phe Pro Val Ile Phe Arg Lys Ala Arg Glu Phe Ile Glu Ile Leu
945                 950                 955                 960

Phe Gly Ile Ser Leu Arg Glu Val Asp Pro Asp Asp Ser Tyr Val Phe
                965                 970                 975

Val Asn Thr Leu Asp Leu Thr Ser Glu Gly Cys Leu Ser Asp Glu Gln
            980                 985                 990

Gly Met Ser Gln Asn Arg Leu Leu  Ile Leu Ile Leu Ser  Ile Ile Phe
        995                 1000                 1005

Ile Lys  Gly Thr Tyr Ala Ser  Glu Glu Val Ile Trp  Asp Val Leu
    1010                 1015                 1020

Ser Gly  Ile Gly Val Arg Ala  Gly Arg Glu His Phe  Ala Phe Gly
    1025                 1030                 1035

Glu Pro  Arg Glu Leu Leu Thr  Lys Val Trp Val Gln  Glu His Tyr
    1040                 1045                 1050

Leu Glu  Tyr Arg Glu Val Pro  Asn Ser Ser Pro Pro  Arg Tyr Glu
    1055                 1060                 1065

Phe Leu  Trp Gly Pro Arg Ala  His Ser Glu Val Ile  Lys Arg Lys
    1070                 1075                 1080

Val Val  Glu Phe Leu Ala Met  Leu Lys Asn Thr Val  Pro Ile Thr
    1085                 1090                 1095

Phe Pro  Ser Ser Tyr Lys Asp  Ala Leu Lys Asp Val  Glu Glu Arg
    1100                 1105                 1110

Ala Gln  Ala Ile Ile Asp Thr  Thr Asp Asp Ser Thr  Ala Thr Glu
    1115                 1120                 1125

Ser Ala  Ser Ser Ser Val Met  Ser Pro Ser Phe Ser  Ser Glu
    1130                 1135                 1140

<210>  80
<211>  373
<212>  PRT
<213>  Artificial Sequence

<220>
<223>  MAGE-C2 Protein NP_057333.1

<400>  80

Met Pro Pro Val Pro Gly Val Pro Phe Arg Asn Val Asp Asn Asp Ser

1               5                   10                  15

Pro Thr Ser Val Glu Leu Glu Asp Trp Val Asp Ala Gln His Pro Thr
            20                  25                  30

Asp Glu Glu Glu Glu Glu Ala Ser Ser Ala Ser Ser Thr Leu Tyr Leu
        35                  40                  45

Val Phe Ser Pro Ser Ser Phe Ser Thr Ser Ser Ser Leu Ile Leu Gly
    50                  55                  60

Gly Pro Glu Glu Glu Glu Val Pro Ser Gly Val Ile Pro Asn Leu Thr
65                  70                  75                  80

Glu Ser Ile Pro Ser Ser Pro Pro Gln Gly Pro Pro Gln Gly Pro Ser
                85                  90                  95

Gln Ser Pro Leu Ser Ser Cys Cys Ser Ser Phe Ser Trp Ser Ser Phe
            100                 105                 110

Ser Glu Glu Ser Ser Ser Gln Lys Gly Glu Asp Thr Gly Thr Cys Gln
        115                 120                 125

Gly Leu Pro Asp Ser Glu Ser Ser Phe Thr Tyr Thr Leu Asp Glu Lys
    130                 135                 140

Val Ala Glu Leu Val Glu Phe Leu Leu Leu Lys Tyr Glu Ala Glu Glu
145                 150                 155                 160

Pro Val Thr Glu Ala Glu Met Leu Met Ile Val Ile Lys Tyr Lys Asp
                165                 170                 175

Tyr Phe Pro Val Ile Leu Lys Arg Ala Arg Glu Phe Met Glu Leu Leu
            180                 185                 190

Phe Gly Leu Ala Leu Ile Glu Val Gly Pro Asp His Phe Cys Val Phe
        195                 200                 205

Ala Asn Thr Val Gly Leu Thr Asp Glu Gly Ser Asp Asp Glu Gly Met
    210                 215                 220

Pro Glu Asn Ser Leu Leu Ile Ile Ile Leu Ser Val Ile Phe Ile Lys
225                 230                 235                 240

Gly Asn Cys Ala Ser Glu Glu Val Ile Trp Glu Val Leu Asn Ala Val
                245                 250                 255

Gly Val Tyr Ala Gly Arg Glu His Phe Val Tyr Gly Glu Pro Arg Glu
            260                 265                 270

Leu Leu Thr Lys Val Trp Val Gln Gly His Tyr Leu Glu Tyr Arg Glu
        275                 280                 285

Val Pro His Ser Ser Pro Pro Tyr Tyr Glu Phe Leu Trp Gly Pro Arg
    290                 295                 300

Ala His Ser Glu Ser Ile Lys Lys Lys Val Leu Glu Phe Leu Ala Lys
305                 310                 315                 320

Leu Asn Asn Thr Val Pro Ser Ser Phe Pro Ser Trp Tyr Lys Asp Ala
                325                 330                 335

Leu Lys Asp Val Glu Glu Arg Val Gln Ala Thr Ile Asp Thr Ala Asp
            340                 345                 350

Asp Ala Thr Val Met Ala Ser Glu Ser Leu Ser Val Met Ser Ser Asn
        355                 360                 365

Val Ser Phe Ser Glu
    370

<210>  81
<211>  1255
<212>  PRT
<213>  Artificial Sequence

<220>
<223>  MUC1 Protein J05582.1

<400>  81

Met Thr Pro Gly Thr Gln Ser Pro Phe Phe Leu Leu Leu Leu Leu Thr
1               5                   10                  15

Val Leu Thr Val Val Thr Gly Ser Gly His Ala Ser Ser Thr Pro Gly
            20                  25                  30

Gly Glu Lys Glu Thr Ser Ala Thr Gln Arg Ser Ser Val Pro Ser Ser
        35                  40                  45

Thr Glu Lys Asn Ala Val Ser Met Thr Ser Ser Val Leu Ser Ser His
    50                  55                  60

Ser Pro Gly Ser Gly Ser Ser Thr Thr Gln Gly Gln Asp Val Thr Leu
65                  70                  75                  80

Ala Pro Ala Thr Glu Pro Ala Ser Gly Ser Ala Ala Thr Trp Gly Gln

                85                  90                  95

Asp Val Thr Ser Val Pro Val Thr Arg Pro Ala Leu Gly Ser Thr Thr
            100                 105                 110

Pro Pro Ala His Asp Val Thr Ser Ala Pro Asp Asn Lys Pro Ala Pro
        115                 120                 125

Gly Ser Thr Ala Pro Pro Ala His Gly Val Thr Ser Ala Pro Asp Thr
    130                 135                 140

Arg Pro Ala Pro Gly Ser Thr Ala Pro Pro Ala His Gly Val Thr Ser
145                 150                 155                 160

Ala Pro Asp Thr Arg Pro Ala Pro Gly Ser Thr Ala Pro Pro Ala His
                165                 170                 175

Gly Val Thr Ser Ala Pro Asp Thr Arg Pro Ala Pro Gly Ser Thr Ala
            180                 185                 190

Pro Pro Ala His Gly Val Thr Ser Ala Pro Asp Thr Arg Pro Ala Pro
        195                 200                 205

Gly Ser Thr Ala Pro Pro Ala His Gly Val Thr Ser Ala Pro Asp Thr
    210                 215                 220

Arg Pro Ala Pro Gly Ser Thr Ala Pro Pro Ala His Gly Val Thr Ser
225                 230                 235                 240

Ala Pro Asp Thr Arg Pro Ala Pro Gly Ser Thr Ala Pro Pro Ala His
                245                 250                 255

Gly Val Thr Ser Ala Pro Asp Thr Arg Pro Ala Pro Gly Ser Thr Ala
            260                 265                 270

Pro Pro Ala His Gly Val Thr Ser Ala Pro Asp Thr Arg Pro Ala Pro
        275                 280                 285

Gly Ser Thr Ala Pro Pro Ala His Gly Val Thr Ser Ala Pro Asp Thr
    290                 295                 300

Arg Pro Ala Pro Gly Ser Thr Ala Pro Pro Ala His Gly Val Thr Ser
305                 310                 315                 320

Ala Pro Asp Thr Arg Pro Ala Pro Gly Ser Thr Ala Pro Pro Ala His
                325                 330                 335

Gly Val Thr Ser Ala Pro Asp Thr Arg Pro Ala Pro Gly Ser Thr Ala
        340                 345                 350

Pro Pro Ala His Gly Val Thr Ser Ala Pro Asp Thr Arg Pro Ala Pro
    355                 360                 365

Gly Ser Thr Ala Pro Pro Ala His Gly Val Thr Ser Ala Pro Asp Thr
    370                 375                 380

Arg Pro Ala Pro Gly Ser Thr Ala Pro Pro Ala His Gly Val Thr Ser
385                 390                 395                 400

Ala Pro Asp Thr Arg Pro Ala Pro Gly Ser Thr Ala Pro Pro Ala His
                405                 410                 415

Gly Val Thr Ser Ala Pro Asp Thr Arg Pro Ala Pro Gly Ser Thr Ala
        420                 425                 430

Pro Pro Ala His Gly Val Thr Ser Ala Pro Asp Thr Arg Pro Ala Pro
    435                 440                 445

Gly Ser Thr Ala Pro Pro Ala His Gly Val Thr Ser Ala Pro Asp Thr
    450                 455                 460

Arg Pro Ala Pro Gly Ser Thr Ala Pro Pro Ala His Gly Val Thr Ser
465                 470                 475                 480

Ala Pro Asp Thr Arg Pro Ala Pro Gly Ser Thr Ala Pro Pro Ala His
                485                 490                 495

Gly Val Thr Ser Ala Pro Asp Thr Arg Pro Ala Pro Gly Ser Thr Ala
        500                 505                 510

Pro Pro Ala His Gly Val Thr Ser Ala Pro Asp Thr Arg Pro Ala Pro
    515                 520                 525

Gly Ser Thr Ala Pro Pro Ala His Gly Val Thr Ser Ala Pro Asp Thr
    530                 535                 540

Arg Pro Ala Pro Gly Ser Thr Ala Pro Pro Ala His Gly Val Thr Ser
545                 550                 555                 560

Ala Pro Asp Thr Arg Pro Ala Pro Gly Ser Thr Ala Pro Pro Ala His
                565                 570                 575

Gly Val Thr Ser Ala Pro Asp Thr Arg Pro Ala Pro Gly Ser Thr Ala
        580                 585                 590

Pro Pro Ala His Gly Val Thr Ser Ala Pro Asp Thr Arg Pro Ala Pro
        595                 600                 605

Gly Ser Thr Ala Pro Pro Ala His Gly Val Thr Ser Ala Pro Asp Thr
    610                 615                 620

Arg Pro Ala Pro Gly Ser Thr Ala Pro Pro Ala His Gly Val Thr Ser
625                 630                 635                 640

Ala Pro Asp Thr Arg Pro Ala Pro Gly Ser Thr Ala Pro Pro Ala His
                645                 650                 655

Gly Val Thr Ser Ala Pro Asp Thr Arg Pro Ala Pro Gly Ser Thr Ala
            660                 665                 670

Pro Pro Ala His Gly Val Thr Ser Ala Pro Asp Thr Arg Pro Ala Pro
        675                 680                 685

Gly Ser Thr Ala Pro Pro Ala His Gly Val Thr Ser Ala Pro Asp Thr
    690                 695                 700

Arg Pro Ala Pro Gly Ser Thr Ala Pro Pro Ala His Gly Val Thr Ser
705                 710                 715                 720

Ala Pro Asp Thr Arg Pro Ala Pro Gly Ser Thr Ala Pro Pro Ala His
                725                 730                 735

Gly Val Thr Ser Ala Pro Asp Thr Arg Pro Ala Pro Gly Ser Thr Ala
            740                 745                 750

Pro Pro Ala His Gly Val Thr Ser Ala Pro Asp Thr Arg Pro Ala Pro
        755                 760                 765

Gly Ser Thr Ala Pro Pro Ala His Gly Val Thr Ser Ala Pro Asp Thr
    770                 775                 780

Arg Pro Ala Pro Gly Ser Thr Ala Pro Pro Ala His Gly Val Thr Ser
785                 790                 795                 800

Ala Pro Asp Thr Arg Pro Ala Pro Gly Ser Thr Ala Pro Pro Ala His
                805                 810                 815

Gly Val Thr Ser Ala Pro Asp Thr Arg Pro Ala Pro Gly Ser Thr Ala
            820                 825                 830

Pro Pro Ala His Gly Val Thr Ser Ala Pro Asp Thr Arg Pro Ala Pro
        835                 840                 845

Gly Ser Thr Ala Pro Pro Ala His Gly Val Thr Ser Ala Pro Asp Thr
    850                 855                 860

Arg Pro Ala Pro Gly Ser Thr Ala Pro Pro Ala His Gly Val Thr Ser
865                 870                 875                 880

Ala Pro Asp Thr Arg Pro Ala Pro Gly Ser Thr Ala Pro Pro Ala His
                885                 890                 895

Gly Val Thr Ser Ala Pro Asp Thr Arg Pro Ala Pro Gly Ser Thr Ala
            900                 905                 910

Pro Pro Ala His Gly Val Thr Ser Ala Pro Asp Thr Arg Pro Ala Pro
        915                 920                 925

Gly Ser Thr Ala Pro Pro Ala His Gly Val Thr Ser Ala Pro Asp Asn
    930                 935                 940

Arg Pro Ala Leu Gly Ser Thr Ala Pro Pro Val His Asn Val Thr Ser
945                 950                 955                 960

Ala Ser Gly Ser Ala Ser Gly Ser Ala Ser Thr Leu Val His Asn Gly
                965                 970                 975

Thr Ser Ala Arg Ala Thr Thr Thr Pro Ala Ser Lys Ser Thr Pro Phe
            980                 985                 990

Ser Ile Pro Ser His His Ser Asp  Thr Pro Thr Thr Leu  Ala Ser His
        995                 1000                1005

Ser Thr  Lys Thr Asp Ala Ser  Ser Thr His His Ser  Ser Val Pro
    1010                1015                1020

Pro Leu  Thr Ser Ser Asn His  Ser Thr Ser Pro Gln  Leu Ser Thr
    1025                1030                1035

Gly Val  Ser Phe Phe Phe Leu  Ser Phe His Ile Ser  Asn Leu Gln
    1040                1045                1050

Phe Asn  Ser Ser Leu Glu Asp  Pro Ser Thr Asp Tyr  Tyr Gln Glu
    1055                1060                1065

Leu Gln  Arg Asp Ile Ser Glu  Met Phe Leu Gln Ile  Tyr Lys Gln
    1070                1075                1080

Gly Gly  Phe Leu Gly Leu Ser  Asn Ile Lys Phe Arg  Pro Gly Ser

1085                1090                1095

Val Val Val Gln Leu Thr Leu Ala Phe Arg Glu Gly Thr Ile Asn
    1100                1105                1110

Val His Asp Val Glu Thr Gln Phe Asn Gln Tyr Lys Thr Glu Ala
    1115                1120                1125

Ala Ser Arg Tyr Asn Leu Thr Ile Ser Asp Val Ser Val Ser Asp
    1130                1135                1140

Val Pro Phe Pro Phe Ser Ala Gln Ser Gly Ala Gly Val Pro Gly
    1145                1150                1155

Trp Gly Ile Ala Leu Leu Val Leu Val Cys Val Leu Val Ala Leu
    1160                1165                1170

Ala Ile Val Tyr Leu Ile Ala Leu Ala Val Cys Gln Cys Arg Arg
    1175                1180                1185

Lys Asn Tyr Gly Gln Leu Asp Ile Phe Pro Ala Arg Asp Thr Tyr
    1190                1195                1200

His Pro Met Ser Glu Tyr Pro Thr Tyr His Thr His Gly Arg Tyr
    1205                1210                1215

Val Pro Pro Ser Ser Thr Asp Arg Ser Pro Tyr Glu Lys Val Ser
    1220                1225                1230

Ala Gly Asn Gly Gly Ser Ser Leu Ser Tyr Thr Asn Pro Ala Val
    1235                1240                1245

Ala Ala Ala Ser Ala Asn Leu
    1250                1255

<210>  82
<211>  555
<212>  PRT
<213>  Artificial Sequence

<220>
<223>  MUC1 Protein 5xVNTR

<400>  82

Met Thr Pro Gly Thr Gln Ser Pro Phe Phe Leu Leu Leu Leu Leu Thr
1               5                   10                  15

Val Leu Thr Val Val Thr Gly Ser Gly His Ala Ser Ser Thr Pro Gly
            20                  25                  30

Gly Glu Lys Glu Thr Ser Ala Thr Gln Arg Ser Ser Val Pro Ser Ser
        35                  40                  45

Thr Glu Lys Asn Ala Val Ser Met Thr Ser Ser Val Leu Ser Ser His
    50                  55                  60

Ser Pro Gly Ser Gly Ser Ser Thr Thr Gln Gly Gln Asp Val Thr Leu
65                  70                  75                  80

Ala Pro Ala Thr Glu Pro Ala Ser Gly Ser Ala Ala Thr Trp Gly Gln
                85                  90                  95

Asp Val Thr Ser Val Pro Val Thr Arg Pro Ala Leu Gly Ser Thr Thr
            100                 105                 110

Pro Pro Ala His Asp Val Thr Ser Ala Pro Asp Asn Lys Pro Ala Pro
        115                 120                 125

Gly Ser Thr Ala Pro Pro Ala His Gly Val Thr Ser Ala Pro Asp Thr
    130                 135                 140

Arg Pro Ala Pro Gly Ser Thr Ala Pro Pro Ala His Gly Val Thr Ser
145                 150                 155                 160

Ala Pro Asp Thr Arg Pro Ala Pro Gly Ser Thr Ala Pro Pro Ala His
                165                 170                 175

Gly Val Thr Ser Ala Pro Asp Thr Arg Pro Ala Pro Gly Ser Thr Ala
            180                 185                 190

Pro Pro Ala His Gly Val Thr Ser Ala Pro Asp Thr Arg Pro Ala Pro
        195                 200                 205

Gly Ser Thr Ala Pro Pro Ala His Gly Val Thr Ser Ala Pro Asp Thr
    210                 215                 220

Arg Pro Ala Pro Gly Ser Thr Ala Pro Pro Ala His Gly Val Thr Ser
225                 230                 235                 240

Ala Pro Asp Asn Arg Pro Ala Leu Gly Ser Thr Ala Pro Pro Val His
                245                 250                 255

Asn Val Thr Ser Ala Ser Gly Ser Ala Ser Gly Ser Ala Ser Thr Leu
            260                 265                 270

Val His Asn Gly Thr Ser Ala Arg Ala Thr Thr Thr Pro Ala Ser Lys

275                 280                 285

Ser Thr Pro Phe Ser Ile Pro Ser His His Ser Asp Thr Pro Thr Thr
    290                 295                 300

Leu Ala Ser His Ser Thr Lys Thr Asp Ala Ser Ser Thr His His Ser
305                 310                 315                 320

Ser Val Pro Pro Leu Thr Ser Ser Asn His Ser Thr Ser Pro Gln Leu
                325                 330                 335

Ser Thr Gly Val Ser Phe Phe Phe Leu Ser Phe His Ile Ser Asn Leu
            340                 345                 350

Gln Phe Asn Ser Ser Leu Glu Asp Pro Ser Thr Asp Tyr Tyr Gln Glu
        355                 360                 365

Leu Gln Arg Asp Ile Ser Glu Met Phe Leu Gln Ile Tyr Lys Gln Gly
    370                 375                 380

Gly Phe Leu Gly Leu Ser Asn Ile Lys Phe Arg Pro Gly Ser Val Val
385                 390                 395                 400

Val Gln Leu Thr Leu Ala Phe Arg Glu Gly Thr Ile Asn Val His Asp
                405                 410                 415

Val Glu Thr Gln Phe Asn Gln Tyr Lys Thr Glu Ala Ala Ser Arg Tyr
            420                 425                 430

Asn Leu Thr Ile Ser Asp Val Ser Val Ser Asp Val Pro Phe Pro Phe
        435                 440                 445

Ser Ala Gln Ser Gly Ala Gly Val Pro Gly Trp Gly Ile Ala Leu Leu
    450                 455                 460

Val Leu Val Cys Val Leu Val Ala Leu Ala Ile Val Tyr Leu Ile Ala
465                 470                 475                 480

Leu Ala Val Cys Gln Cys Arg Arg Lys Asn Tyr Gly Gln Leu Asp Ile
                485                 490                 495

Phe Pro Ala Arg Asp Thr Tyr His Pro Met Ser Glu Tyr Pro Thr Tyr
            500                 505                 510

His Thr His Gly Arg Tyr Val Pro Pro Ser Ser Thr Asp Arg Ser Pro
        515                 520                 525

Tyr Glu Lys Val Ser Ala Gly Asn Gly Gly Ser Ser Leu Ser Tyr Thr
    530                 535                 540

Asn Pro Ala Val Ala Ala Ala Ser Ala Asn Leu
545                 550                 555

<210>  83
<211>  3768
<212>  PRT
<213>  Artificial Sequence

<220>
<223>  MUC1 CDS wild type

<400>  83
   Ala Thr Gly Ala Cys Ala Cys Cys Gly Gly Gly Cys Ala Cys Cys Cys
   1               5                   10                  15
   Ala Gly Thr Cys Thr Cys Cys Thr Thr Thr Cys Thr Thr Cys Cys Thr
               20                  25                  30
   Gly Cys Thr Gly Cys Thr Gly Cys Thr Cys Cys Thr Cys Ala Cys Ala
           35                  40                  45
   Gly Thr Gly Cys Thr Thr Ala Cys Ala Gly Thr Thr Gly Thr Thr Ala
       50                  55                  60
   Cys Ala Gly Gly Thr Thr Cys Thr Gly Gly Thr Cys Ala Thr Gly Cys
   65                  70                  75                  80
   Ala Ala Gly Cys Thr Cys Thr Ala Cys Cys Cys Cys Ala Gly Gly Thr
                   85                  90                  95
   Gly Gly Ala Gly Ala Ala Ala Ala Gly Gly Ala Gly Ala Cys Thr Thr
               100                 105                 110
   Cys Gly Gly Cys Thr Ala Cys Cys Cys Ala Gly Ala Gly Ala Ala Gly
           115                 120                 125
   Thr Thr Cys Ala Gly Thr Gly Cys Cys Cys Ala Gly Cys Thr Cys Thr
       130                 135                 140
   Ala Cys Thr Gly Ala Gly Ala Ala Gly Ala Ala Thr Gly Cys Thr Gly
   145                 150                 155                 160
   Thr Gly Ala Gly Thr Ala Thr Gly Ala Cys Cys Ala Gly Cys Ala Gly
                   165                 170                 175
   Cys Gly Thr Ala Cys Thr Cys Thr Cys Cys Ala Gly Cys Cys Ala Cys
   180 185 190
   Ala Gly Cys Cys Cys Cys Gly Gly Thr Thr Cys Ala Gly Gly Cys Thr
   195 200 205
   Cys Cys Thr Cys Cys Ala Cys Cys Ala Cys Thr Cys Ala Gly Gly Gly
   210 215 220
   Ala Cys Ala Gly Gly Ala Thr Gly Thr Cys Ala Cys Thr Cys Thr Gly
   225 230 235 240
   Gly Cys Cys Cys Cys Gly Gly Cys Cys Ala Cys Gly Gly Ala Ala Cys
   245 250 255
   Cys Ala Gly Cys Thr Thr Cys Ala Gly Gly Thr Thr Cys Ala Gly Cys
   260 265 270
   Thr Gly Cys Cys Ala Cys Cys Thr Gly Gly Gly Gly Ala Cys Ala Gly
   275 280 285
   Gly Ala Thr Gly Thr Cys Ala Cys Cys Thr Cys Gly Gly Thr Cys Cys
   290 295 300
   Cys Ala Gly Thr Cys Ala Cys Cys Ala Gly Gly Cys Cys Ala Gly Cys
   305 310 315 320
   Cys Cys Thr Gly Gly Gly Cys Thr Cys Cys Ala Cys Cys Ala Cys Cys
   325 330 335
   Cys Cys Gly Cys Cys Ala Gly Cys Cys Cys Ala Cys Gly Ala Thr Gly
   340 345 350
   Thr Cys Ala Cys Cys Thr Cys Ala Gly Cys Cys Cys Cys Gly Gly Ala
   355 360 365
   Cys Ala Ala Cys Ala Ala Gly Cys Cys Ala Gly Cys Cys Cys Cys Gly
   370 375 380
   Gly Gly Cys Thr Cys Cys Ala Cys Cys Gly Cys Cys Cys Cys Cys Cys
   385 390 395 400
   Cys Ala Gly Cys Cys Cys Ala Cys Gly Gly Thr Gly Thr Cys Ala Cys
   405 410 415
   Cys Thr Cys Gly Gly Cys Cys Cys Cys Gly Gly Ala Cys Ala Cys Cys
   420 425 430
   Ala Gly Gly Cys Cys Gly Gly Cys Cys Cys Cys Gly Gly Gly Cys Thr
           435                 440                 445
   Cys Cys Ala Cys Cys Gly Cys Cys Cys Cys Cys Cys Cys Ala Gly Cys
       450                 455                 460
   Cys Cys Ala Cys Gly Gly Thr Gly Thr Cys Ala Cys Cys Thr Cys Gly
   465                 470                 475                 480
   Gly Cys Cys Cys Cys Gly Gly Ala Cys Ala Cys Cys Ala Gly Gly Cys
                   485                 490                 495
   Cys Gly Gly Cys Cys Cys Cys Gly Gly Gly Cys Thr Cys Cys Ala Cys
               500                 505                 510
   Cys Gly Cys Cys Cys Cys Cys Cys Cys Ala Gly Cys Cys Cys Ala Cys
           515                 520                 525
   Gly Gly Thr Gly Thr Cys Ala Cys Cys Thr Cys Gly Gly Cys Cys Cys
       530                 535                 540
   Cys Gly Gly Ala Cys Ala Cys Cys Ala Gly Gly Cys Cys Gly Gly Cys
   545                 550                 555                 560
   Cys Cys Cys Gly Gly Gly Cys Thr Cys Cys Ala Cys Cys Gly Cys Cys
                   565                 570                 575
   Cys Cys Cys Cys Cys Ala Gly Cys Cys Cys Ala Cys Gly Gly Thr Gly
               580                 585                 590
   Thr Cys Ala Cys Cys Thr Cys Gly Gly Cys Cys Cys Cys Gly Gly Ala
           595                 600                 605
   Cys Ala Cys Cys Ala Gly Gly Cys Cys Gly Gly Cys Cys Cys Cys Gly
       610                 615                 620
   Gly Gly Cys Thr Cys Cys Ala Cys Cys Gly Cys Cys Cys Cys Cys Cys
   625                 630                 635                 640
   Cys Ala Gly Cys Cys Cys Ala Cys Gly Gly Thr Gly Thr Cys Ala Cys
                   645                 650                 655
   Cys Thr Cys Gly Gly Cys Cys Cys Cys Gly Gly Ala Cys Ala Cys Cys
               660                 665                 670
   Ala Gly Gly Cys Cys Gly Gly Cys Cys Cys Cys Gly Gly Gly Cys Thr
           675                 680                 685
   Cys Cys Ala Cys Cys Gly Cys Cys Cys Cys Cys Cys Cys Ala Gly Cys
       690                 695                 700
   Cys Cys Ala Cys Gly Gly Thr Gly Thr Cys Ala Cys Cys Thr Cys Gly
   705                 710                 715                 720
   Gly Cys Cys Cys Cys Gly Gly Ala Cys Ala Cys Cys Ala Gly Gly Cys
                   725                 730                 735
   Cys Gly Gly Cys Cys Cys Cys Gly Gly Gly Cys Thr Cys Cys Ala Cys
               740                 745                 750
   Cys Gly Cys Cys Cys Cys Cys Cys Cys Ala Gly Cys Cys Cys Ala Cys
           755                 760                 765
   Gly Gly Thr Gly Thr Cys Ala Cys Cys Thr Cys Gly Gly Cys Cys Cys
       770                 775                 780
   Cys Gly Gly Ala Cys Ala Cys Cys Ala Gly Gly Cys Cys Gly Gly Cys
   785                 790                 795                 800
   Cys Cys Cys Gly Gly Gly Cys Thr Cys Cys Ala Cys Cys Gly Cys Cys
                   805                 810                 815
   Cys Cys Cys Cys Cys Ala Gly Cys Cys Cys Ala Cys Gly Gly Thr Gly
               820                 825                 830
   Thr Cys Ala Cys Cys Thr Cys Gly Gly Cys Cys Cys Cys Gly Gly Ala
           835                 840                 845
   Cys Ala Cys Cys Ala Gly Gly Cys Cys Gly Gly Cys Cys Cys Cys Gly
       850                 855                 860
   Gly Gly Cys Thr Cys Cys Ala Cys Cys Gly Cys Cys Cys Cys Cys Cys
   865                 870                 875                 880
   Cys Ala Gly Cys Cys Cys Ala Cys Gly Gly Thr Gly Thr Cys Ala Cys
                   885                 890                 895
   Cys Thr Cys Gly Gly Cys Cys Cys Cys Gly Gly Ala Cys Ala Cys Cys
               900                 905                 910
   Ala Gly Gly Cys Cys Gly Gly Cys Cys Cys Cys Gly Gly Gly Cys Thr
           915                 920                 925
   Cys Cys Ala Cys Cys Gly Cys Cys Cys Cys Cys Cys Cys Ala Gly Cys
       930                 935                 940
   Cys Cys Ala Cys Gly Gly Thr Gly Thr Cys Ala Cys Cys Thr Cys Gly
   945                 950                 955                 960
   Gly Cys Cys Cys Cys Gly Gly Ala Cys Ala Cys Cys Ala Gly Gly Cys
                   965                 970                 975
   Cys Gly Gly Cys Cys Cys Cys Gly Gly Gly Cys Thr Cys Cys Ala Cys
               980                 985                 990
   Cys Gly Cys Cys Cys Cys Cys Cys Cys Ala Gly Cys Cys Cys Ala Cys
           995                 1000                1005
   Gly Gly Thr Gly Thr Cys Ala Cys Cys Thr Cys Gly Gly Cys Cys
       1010                1015                1020
   Cys Cys Gly Gly Ala Cys Ala Cys Cys Ala Gly Gly Cys Cys Gly
       1025                1030                1035
   Gly Cys Cys Cys Cys Gly Gly Gly Cys Thr Cys Cys Ala Cys Cys
       1040                1045                1050
   Gly Cys Cys Cys Cys Cys Cys Cys Ala Gly Cys Cys Cys Ala Cys
       1055                1060                1065
   Gly Gly Thr Gly Thr Cys Ala Cys Cys Thr Cys Gly Gly Cys Cys
       1070                1075                1080
   Cys Cys Gly Gly Ala Cys Ala Cys Cys Ala Gly Gly Cys Cys Gly
       1085                1090                1095
   Gly Cys Cys Cys Cys Gly Gly Gly Cys Thr Cys Cys Ala Cys Cys
       1100                1105                1110
   Gly Cys Cys Cys Cys Cys Cys Cys Ala Gly Cys Cys Cys Ala Cys
       1115                1120                1125
   Gly Gly Thr Gly Thr Cys Ala Cys Cys Thr Cys Gly Gly Cys Cys
       1130                1135                1140
   Cys Cys Gly Gly Ala Cys Ala Cys Cys Ala Gly Gly Cys Cys Gly
       1145                1150                1155
   Gly Cys Cys Cys Cys Gly Gly Gly Cys Thr Cys Cys Ala Cys Cys
       1160                1165                1170
   Gly Cys Cys Cys Cys Cys Cys Cys Ala Gly Cys Cys Cys Ala Cys
   1175                1180                1185
   Gly Gly  Thr Gly Thr Cys Ala  Cys Cys Thr Cys Gly  Gly Cys Cys
       1190                1195                1200
   Cys Cys  Gly Gly Ala Cys Ala  Cys Cys Ala Gly Gly  Cys Cys Gly
       1205                1210                1215
   Gly Cys  Cys Cys Cys Gly Gly  Gly Cys Thr Cys Cys  Ala Cys Cys
       1220                1225                1230
   Gly Cys  Cys Cys Cys Cys Cys  Cys Ala Gly Cys Cys  Cys Ala Cys
       1235                1240                1245
   Gly Gly  Thr Gly Thr Cys Ala  Cys Cys Thr Cys Gly  Gly Cys Cys
       1250                1255                1260
   Cys Cys  Gly Gly Ala Cys Ala  Cys Cys Ala Gly Gly  Cys Cys Gly
       1265                1270                1275
   Gly Cys  Cys Cys Cys Gly Gly  Gly Cys Thr Cys Cys  Ala Cys Cys
       1280                1285                1290
   Gly Cys  Cys Cys Cys Cys Cys  Cys Ala Gly Cys Cys  Cys Ala Cys
       1295                1300                1305
   Gly Gly  Thr Gly Thr Cys Ala  Cys Cys Thr Cys Gly  Gly Cys Cys
       1310                1315                1320
   Cys Cys  Gly Gly Ala Cys Ala  Cys Cys Ala Gly Gly  Cys Cys Gly
       1325                1330                1335
   Gly Cys  Cys Cys Cys Gly Gly  Gly Cys Thr Cys Cys  Ala Cys Cys
       1340                1345                1350
   Gly Cys  Cys Cys Cys Cys Cys  Cys Ala Gly Cys Cys  Cys Ala Cys
       1355                1360                1365
   Gly Gly  Thr Gly Thr Cys Ala  Cys Cys Thr Cys Gly  Gly Cys Cys
       1370                1375                1380
   Cys Cys  Gly Gly Ala Cys Ala  Cys Cys Ala Gly Gly  Cys Cys Gly
       1385                1390                1395
   Gly Cys  Cys Cys Cys Gly Gly  Gly Cys Thr Cys Cys  Ala Cys Cys
       1400                1405                1410
   Gly Cys Cys Cys Cys Cys Cys Cys Ala Gly Cys Cys Cys Ala Cys
       1415                1420                1425
   Gly Gly Thr Gly Thr Cys Ala Cys Cys Thr Cys Gly Gly Cys Cys
       1430                1435                1440
   Cys Cys Gly Gly Ala Cys Ala Cys Cys Ala Gly Gly Cys Cys Gly
       1445                1450                1455
   Gly Cys Cys Cys Cys Gly Gly Gly Cys Thr Cys Cys Ala Cys Cys
       1460                1465                1470
   Gly Cys Cys Cys Cys Cys Cys Cys Ala Gly Cys Cys Cys Ala Cys
       1475                1480                1485
   Gly Gly Thr Gly Thr Cys Ala Cys Cys Thr Cys Gly Gly Cys Cys
       1490                1495                1500
   Cys Cys Gly Gly Ala Cys Ala Cys Cys Ala Gly Gly Cys Cys Gly
       1505                1510                1515
   Gly Cys Cys Cys Cys Gly Gly Gly Cys Thr Cys Cys Ala Cys Cys
       1520                1525                1530
   Gly Cys Cys Cys Cys Cys Cys Cys Ala Gly Cys Cys Cys Ala Cys
       1535                1540                1545
   Gly Gly Thr Gly Thr Cys Ala Cys Cys Thr Cys Gly Gly Cys Cys
       1550                1555                1560
   Cys Cys Gly Gly Ala Cys Ala Cys Cys Ala Gly Gly Cys Cys Gly
       1565                1570                1575
   Gly Cys Cys Cys Cys Gly Gly Gly Cys Thr Cys Cys Ala Cys Cys
       1580                1585                1590
   Gly Cys Cys Cys Cys Cys Cys Cys Ala Gly Cys Cys Cys Ala Cys
       1595                1600                1605
   Gly Gly Thr Gly Thr Cys Ala Cys Cys Thr Cys Gly Gly Cys Cys
       1610                1615                1620
   Cys Cys Gly Gly Ala Cys Ala Cys Cys Ala Gly Gly Cys Cys Gly
       1625                1630                1635
   Gly Cys Cys Cys Cys Gly Gly Gly Cys Thr Cys Cys Ala Cys Cys
       1640                1645                1650
   Gly Cys Cys Cys Cys Cys Cys Cys Ala Gly Cys Cys Cys Ala Cys
       1655                1660                1665
   Gly Gly Thr Gly Thr Cys Ala Cys Cys Thr Cys Gly Gly Cys Cys
       1670                1675                1680
   Cys Cys Gly Gly Ala Cys Ala Cys Cys Ala Gly Gly Cys Cys Gly
       1685                1690                1695
   Gly Cys Cys Cys Cys Gly Gly Gly Cys Thr Cys Cys Ala Cys Cys
       1700                1705                1710
   Gly Cys Cys Cys Cys Cys Cys Cys Ala Gly Cys Cys Cys Ala Cys
       1715                1720                1725
   Gly Gly Thr Gly Thr Cys Ala Cys Cys Thr Cys Gly Gly Cys Cys
       1730                1735                1740
   Cys Cys Gly Gly Ala Cys Ala Cys Cys Ala Gly Gly Cys Cys Gly
       1745                1750                1755
   Gly Cys Cys Cys Cys Gly Gly Gly Cys Thr Cys Cys Ala Cys Cys
       1760                1765                1770
   Gly Cys Cys Cys Cys Cys Cys Cys Ala Gly Cys Cys Cys Ala Cys
       1775                1780                1785
   Gly Gly Thr Gly Thr Cys Ala Cys Cys Thr Cys Gly Gly Cys Cys
       1790                1795                1800
   Cys Cys Gly Gly Ala Cys Ala Cys Cys Ala Gly Gly Cys Cys Gly
       1805                1810                1815
   Gly Cys Cys Cys Cys Gly Gly Gly Cys Thr Cys Cys Ala Cys Cys
       1820                1825                1830
   Gly Cys Cys Cys Cys Cys Cys Cys Ala Gly Cys Cys Cys Ala Cys
       1835                1840                1845
   Gly Gly Thr Gly Thr Cys Ala Cys Cys Thr Cys Gly Gly Cys Cys
       1850                1855                1860
   Cys Cys Gly Gly Ala Cys Ala Cys Cys Ala Gly Gly Cys Cys Gly
       1865                1870                1875
   Gly Cys Cys Cys Cys Gly Gly Gly Cys Thr Cys Cys Ala Cys Cys
       1880                1885                1890
   Gly Cys Cys Cys Cys Cys Cys Cys Ala Gly Cys Cys Cys Ala Cys
       1895                1900                1905
   Gly Gly Thr Gly Thr Cys Ala Cys Cys Thr Cys Gly Gly Cys Cys
       1910                1915                1920
   Cys Cys Gly Gly Ala Cys Ala Cys Cys Ala Gly Gly Cys Cys Gly
       1925                1930                1935
   Gly Cys Cys Cys Cys Gly Gly Gly Cys Thr Cys Cys Ala Cys Cys
       1940                1945                1950
   Gly Cys Cys Cys Cys Cys Cys Cys Ala Gly Cys Cys Cys Ala Cys
       1955                1960                1965
   Gly Gly Thr Gly Thr Cys Ala Cys Cys Thr Cys Gly Gly Cys Cys
       1970                1975                1980
   Cys Cys Gly Gly Ala Cys Ala Cys Cys Ala Gly Gly Cys Cys Gly
       1985                1990                1995
   Gly Cys Cys Cys Cys Gly Gly Gly Cys Thr Cys Cys Ala Cys Cys
       2000                2005                2010
   Gly Cys Cys Cys Cys Cys Cys Cys Ala Gly Cys Cys Cys Ala Cys
       2015                2020                2025
   Gly Gly Thr Gly Thr Cys Ala Cys Cys Thr Cys Gly Gly Cys Cys
       2030                2035                2040
   Cys Cys Gly Gly Ala Cys Ala Cys Cys Ala Gly Gly Cys Cys Gly
       2045                2050                2055
   Gly Cys Cys Cys Cys Gly Gly Gly Cys Thr Cys Cys Ala Cys Cys
       2060                2065                2070
   Gly Cys Cys Cys Cys Cys Cys Cys Ala Gly Cys Cys Cys Ala Cys
       2075                2080                2085
   Gly Gly Thr Gly Thr Cys Ala Cys Cys Thr Cys Gly Gly Cys Cys
       2090                2095                2100
   Cys Cys Gly Gly Ala Cys Ala Cys Cys Ala Gly Gly Cys Cys Gly
       2105                2110                2115
   Gly Cys Cys Cys Cys Gly Gly Gly Cys Thr Cys Cys Ala Cys Cys
   2120                    2125                    2130
   Gly Cys  Cys Cys Cys Cys Cys  Cys Ala Gly Cys Cys  Cys Ala Cys
       2135                    2140                    2145
   Gly Gly  Thr Gly Thr Cys Ala  Cys Cys Thr Cys Gly  Gly Cys Cys
       2150                    2155                    2160
   Cys Cys  Gly Gly Ala Cys Ala  Cys Cys Ala Gly Gly  Cys Cys Gly
       2165                    2170                    2175
   Gly Cys  Cys Cys Cys Gly Gly  Gly Cys Thr Cys Cys  Ala Cys Cys
       2180                    2185                    2190
   Gly Cys  Cys Cys Cys Cys Cys  Cys Ala Gly Cys Cys  Cys Ala Cys
       2195                    2200                    2205
   Gly Gly  Thr Gly Thr Cys Ala  Cys Cys Thr Cys Gly  Gly Cys Cys
       2210                    2215                    2220
   Cys Cys  Gly Gly Ala Cys Ala  Cys Cys Ala Gly Gly  Cys Cys Gly
       2225                    2230                    2235
   Gly Cys  Cys Cys Cys Gly Gly  Gly Cys Thr Cys Cys  Ala Cys Cys
       2240                    2245                    2250
   Gly Cys  Cys Cys Cys Cys Cys  Cys Ala Gly Cys Cys  Cys Ala Cys
       2255                    2260                    2265
   Gly Gly  Thr Gly Thr Cys Ala  Cys Cys Thr Cys Gly  Gly Cys Cys
       2270                    2275                    2280
   Cys Cys  Gly Gly Ala Cys Ala  Cys Cys Ala Gly Gly  Cys Cys Gly
       2285                    2290                    2295
   Gly Cys  Cys Cys Cys Gly Gly  Gly Cys Thr Cys Cys  Ala Cys Cys
       2300                    2305                    2310
   Gly Cys  Cys Cys Cys Cys Cys  Cys Ala Gly Cys Cys  Cys Ala Cys
       2315                    2320                    2325
   Gly Gly  Thr Gly Thr Cys Ala  Cys Cys Thr Cys Gly  Gly Cys Cys
       2330                    2335                    2340
   Cys Cys  Gly Gly Ala Cys Ala  Cys Cys Ala Gly Gly  Cys Cys Gly
       2345                    2350                    2355
   Gly Cys Cys Cys Cys Gly Gly Gly Cys Thr Cys Cys Ala Cys Cys
       2360                2365                2370
   Gly Cys Cys Cys Cys Cys Cys Cys Ala Gly Cys Cys Cys Ala Cys
       2375                2380                2385
   Gly Gly Thr Gly Thr Cys Ala Cys Cys Thr Cys Gly Gly Cys Cys
       2390                2395                2400
   Cys Cys Gly Gly Ala Cys Ala Cys Cys Ala Gly Gly Cys Cys Gly
       2405                2410                2415
   Gly Cys Cys Cys Cys Gly Gly Gly Cys Thr Cys Cys Ala Cys Cys
       2420                2425                2430
   Gly Cys Cys Cys Cys Cys Cys Cys Ala Gly Cys Cys Cys Ala Cys
       2435                2440                2445
   Gly Gly Thr Gly Thr Cys Ala Cys Cys Thr Cys Gly Gly Cys Cys
       2450                2455                2460
   Cys Cys Gly Gly Ala Cys Ala Cys Cys Ala Gly Gly Cys Cys Gly
       2465                2470                2475
   Gly Cys Cys Cys Cys Gly Gly Gly Cys Thr Cys Cys Ala Cys Cys
       2480                2485                2490
   Gly Cys Cys Cys Cys Cys Cys Cys Ala Gly Cys Cys Cys Ala Cys
       2495                2500                2505
   Gly Gly Thr Gly Thr Cys Ala Cys Cys Thr Cys Gly Gly Cys Cys
       2510                2515                2520
   Cys Cys Gly Gly Ala Cys Ala Cys Cys Ala Gly Gly Cys Cys Gly
       2525                2530                2535
   Gly Cys Cys Cys Cys Gly Gly Gly Cys Thr Cys Cys Ala Cys Cys
       2540                2545                2550
   Gly Cys Cys Cys Cys Cys Cys Cys Ala Gly Cys Cys Cys Ala Cys
       2555                2560                2565
   Gly Gly Thr Gly Thr Cys Ala Cys Cys Thr Cys Gly Gly Cys Cys
       2570                2575                2580
   Cys Cys Gly Gly Ala Cys Ala Cys Cys Ala Gly Gly Cys Cys Gly
       2585                2590                2595
   Gly Cys Cys Cys Cys Gly Gly Gly Cys Thr Cys Cys Ala Cys Cys
       2600                2605                2610
   Gly Cys Cys Cys Cys Cys Cys Cys Ala Gly Cys Cys Cys Ala Cys
       2615                2620                2625
   Gly Gly Thr Gly Thr Cys Ala Cys Cys Thr Cys Gly Gly Cys Cys
       2630                2635                2640
   Cys Cys Gly Gly Ala Cys Ala Cys Cys Ala Gly Gly Cys Cys Gly
       2645                2650                2655
   Gly Cys Cys Cys Cys Gly Gly Gly Cys Thr Cys Cys Ala Cys Cys
       2660                2665                2670
   Gly Cys Cys Cys Cys Cys Cys Cys Ala Gly Cys Cys Cys Ala Cys
       2675                2680                2685
   Gly Gly Thr Gly Thr Cys Ala Cys Cys Thr Cys Gly Gly Cys Cys
       2690                2695                2700
   Cys Cys Gly Gly Ala Cys Ala Cys Cys Ala Gly Gly Cys Cys Gly
       2705                2710                2715
   Gly Cys Cys Cys Cys Gly Gly Gly Cys Thr Cys Cys Ala Cys Cys
       2720                2725                2730
   Gly Cys Cys Cys Cys Cys Cys Cys Ala Gly Cys Cys Cys Ala Cys
       2735                2740                2745
   Gly Gly Thr Gly Thr Cys Ala Cys Cys Thr Cys Gly Gly Cys Cys
       2750                2755                2760
   Cys Cys Gly Gly Ala Cys Ala Cys Cys Ala Gly Gly Cys Cys Gly
       2765                2770                2775
   Gly Cys Cys Cys Cys Gly Gly Gly Cys Thr Cys Cys Ala Cys Cys
       2780                2785                2790
   Gly Cys Cys Cys Cys Cys Cys Cys Ala Gly Cys Cys Cys Ala Thr
       2795                2800                2805
   Gly Gly Thr Gly Thr Cys Ala Cys Cys Thr Cys Gly Gly Cys Cys
       2810                2815                2820
   Cys Cys Gly Gly Ala Cys Ala Ala Cys Ala Gly Gly Cys Cys Cys
       2825                2830                2835
   Gly Cys Cys Thr Thr Gly Gly Gly Cys Thr Cys Cys Ala Cys Cys
       2840                2845                2850
   Gly Cys Cys Cys Cys Thr Cys Cys Ala Gly Thr Cys Cys Ala Cys
       2855                2860                2865
   Ala Ala Thr Gly Thr Cys Ala Cys Cys Thr Cys Gly Gly Cys Cys
       2870                2875                2880
   Thr Cys Ala Gly Gly Cys Thr Cys Thr Gly Cys Ala Thr Cys Ala
       2885                2890                2895
   Gly Gly Cys Thr Cys Ala Gly Cys Thr Thr Cys Thr Ala Cys Thr
       2900                2905                2910
   Cys Thr Gly Gly Thr Gly Cys Ala Cys Ala Ala Cys Gly Gly Cys
       2915                2920                2925
   Ala Cys Cys Thr Cys Thr Gly Cys Cys Ala Gly Gly Gly Cys Thr
       2930                2935                2940
   Ala Cys Cys Ala Cys Ala Ala Cys Cys Cys Cys Ala Gly Cys Cys
       2945                2950                2955
   Ala Gly Cys Ala Ala Gly Ala Gly Cys Ala Cys Thr Cys Cys Ala
       2960                2965                2970
   Thr Thr Cys Thr Cys Ala Ala Thr Thr Cys Cys Cys Ala Gly Cys
       2975                2980                2985
   Cys Ala Cys Cys Ala Cys Thr Cys Thr Gly Ala Thr Ala Cys Thr
       2990                2995                3000
   Cys Cys Thr Ala Cys Cys Ala Cys Cys Cys Thr Thr Gly Cys Cys
       3005                3010                3015
   Ala Gly Cys Cys Ala Thr Ala Gly Cys Ala Cys Cys Ala Ala Gly
       3020                3025                3030
   Ala Cys Thr Gly Ala Thr Gly Cys Cys Ala Gly Thr Ala Gly Cys
       3035                3040                3045
   Ala Cys Thr Cys Ala Cys Cys Ala Thr Ala Gly Cys Thr Cys Gly
       3050                3055                3060
   Gly Thr Ala Cys Cys Thr Cys Cys Thr Cys Thr Cys Ala Cys Cys
   3065                3070                3075
   Thr Cys Cys Thr Cys Cys Ala Ala Thr Cys Ala Cys Ala Gly Cys
       3080                3085                3090
   Ala Cys Thr Thr Cys Thr Cys Cys Cys Cys Ala Gly Thr Thr Gly
       3095                3100                3105
   Thr Cys Thr Ala Cys Thr Gly Gly Gly Gly Thr Cys Thr Cys Thr
       3110                3115                3120
   Thr Thr Cys Thr Thr Thr Thr Thr Cys Cys Thr Gly Thr Cys Thr
       3125                3130                3135
   Thr Thr Thr Cys Ala Cys Ala Thr Thr Thr Cys Ala Ala Ala Cys
       3140                3145                3150
   Cys Thr Cys Cys Ala Gly Thr Thr Thr Ala Ala Thr Thr Cys Cys
       3155                3160                3165
   Thr Cys Thr Cys Thr Gly Gly Ala Ala Gly Ala Thr Cys Cys Cys
       3170                3175                3180
   Ala Gly Cys Ala Cys Cys Gly Ala Cys Thr Ala Cys Thr Ala Cys
       3185                3190                3195
   Cys Ala Ala Gly Ala Gly Cys Thr Gly Cys Ala Gly Ala Gly Ala
       3200                3205                3210
   Gly Ala Cys Ala Thr Thr Thr Cys Thr Gly Ala Ala Ala Thr Gly
       3215                3220                3225
   Thr Thr Thr Thr Thr Gly Cys Ala Gly Ala Thr Thr Thr Ala Thr
       3230                3235                3240
   Ala Ala Ala Cys Ala Ala Gly Gly Gly Gly Gly Thr Thr Thr Thr
       3245                3250                3255
   Cys Thr Gly Gly Gly Cys Cys Thr Cys Thr Cys Cys Ala Ala Thr
       3260                3265                3270
   Ala Thr Thr Ala Ala Gly Thr Thr Cys Ala Gly Gly Cys Cys Ala
       3275                3280                3285
   Gly Gly Ala Thr Cys Thr Gly Thr Gly Gly Thr Gly Gly Thr Ala
       3290                3295                3300
   Cys Ala Ala Thr Thr Gly Ala Cys Thr Cys Thr Gly Gly Cys Cys
       3305                3310                3315
   Thr Thr Cys Cys Gly Ala Gly Ala Ala Gly Gly Thr Ala Cys Cys
       3320                3325                3330
   Ala Thr Cys Ala Ala Thr Gly Thr Cys Cys Ala Cys Gly Ala Cys
       3335                3340                3345
   Gly Thr Gly Gly Ala Gly Ala Cys Ala Cys Ala Gly Thr Thr Cys
       3350                3355                3360
   Ala Ala Thr Cys Ala Gly Thr Ala Thr Ala Ala Ala Ala Cys Gly
       3365                3370                3375
   Gly Ala Ala Gly Cys Ala Gly Cys Cys Thr Cys Thr Cys Gly Ala
       3380                3385                3390
   Thr Ala Thr Ala Ala Cys Cys Thr Gly Ala Cys Gly Ala Thr Cys
       3395                3400                3405
   Thr Cys Ala Gly Ala Cys Gly Thr Cys Ala Gly Cys Gly Thr Gly
       3410                3415                3420
   Ala Gly Thr Gly Ala Thr Gly Thr Gly Cys Cys Ala Thr Thr Thr
       3425                3430                3435
   Cys Cys Thr Thr Thr Cys Thr Cys Thr Gly Cys Cys Cys Ala Gly
       3440                3445                3450
   Thr Cys Thr Gly Gly Gly Gly Cys Thr Gly Gly Gly Gly Thr Gly
       3455                3460                3465
   Cys Cys Ala Gly Gly Cys Thr Gly Gly Gly Gly Cys Ala Thr Cys
       3470                3475                3480
   Gly Cys Gly Cys Thr Gly Cys Thr Gly Gly Thr Gly Cys Thr Gly
       3485                3490                3495
   Gly Thr Cys Thr Gly Thr Gly Thr Thr Cys Thr Gly Gly Thr Thr
       3500                3505                3510
   Gly Cys Gly Cys Thr Gly Gly Cys Cys Ala Thr Thr Gly Thr Cys
       3515                3520                3525
   Thr Ala Thr Cys Thr Cys Ala Thr Thr Gly Cys Cys Thr Thr Gly
       3530                3535                3540
   Gly Cys Thr Gly Thr Cys Thr Gly Thr Cys Ala Gly Thr Gly Cys
   3545 3550 3555
   Cys Gly Cys Cys Gly Ala Ala Ala Gly Ala Ala Cys Thr Ala Cys
   3560 3565 3570
   Gly Gly Gly Cys Ala Gly Cys Thr Gly Gly Ala Cys Ala Thr Cys
   3575 3580 3585
   Thr Thr Thr Cys Cys Ala Gly Cys Cys Cys Gly Gly Gly Ala Thr
   3590 3595 3600
   Ala Cys Cys Thr Ala Cys Cys Ala Thr Cys Cys Thr Ala Thr Gly
   3605 3610 3615
   Ala Gly Cys Gly Ala Gly Thr Ala Cys Cys Cys Cys Ala Cys Cys
   3620 3625 3630
   Thr Ala Cys Cys Ala Cys Ala Cys Cys Cys Ala Thr Gly Gly Gly
   3635 3640 3645
   Cys Gly Cys Thr Ala Thr Gly Thr Gly Cys Cys Cys Cys Cys Thr
   3650 3655 3660
   Ala Gly Cys Ala Gly Thr Ala Cys Cys Gly Ala Thr Cys Gly Thr
   3665 3670 3675
   Ala Gly Cys Cys Cys Cys Thr Ala Thr Gly Ala Gly Ala Ala Gly
   3680 3685 3690
   Gly Thr Thr Thr Cys Thr Gly Cys Ala Gly Gly Thr Ala Ala Cys
   3695 3700 3705
   Gly Gly Thr Gly Gly Cys Ala Gly Cys Ala Gly Cys Cys Thr Cys
   3710 3715 3720
   Thr Cys Thr Thr Ala Cys Ala Cys Ala Ala Ala Cys Cys Cys Ala
   3725 3730 3735
   Gly Cys Ala Gly Thr Gly Gly Cys Ala Gly Cys Cys Gly Cys Thr
   3740 3745 3750
   Thr Cys Thr Gly Cys Cys Ala Ala Cys Thr Thr Gly Thr Ala Gly
   3755 3760 3765

## Description

### Field of the Invention

The present invention relates to a composition comprising at least one mRNA encoding a combination of antigens capable of eliciting an (adaptive) immune response in a mammal, wherein the antigens are selected from the group consisting of 5T4 (Trophoblast glycoprotein, TPBG), Survivin (Baculoviral IAP repeat-containing protein 5; BIRC5), NY-ESO-1 (New York esophageal squamous cell carcinoma 1, CTAG1B), MAGE-C1 (Melanoma antigen family C1), MAGE-C2 (Melanoma antigen family C2), and MUC1 (Mucin 1). The invention furthermore relates to a vaccine comprising at least one mRNA encoding such a combination of antigens, and to the use of said composition (for the preparation of a vaccine) and/or of the vaccine for eliciting an (adaptive) immune response for the treatment of lung cancer, preferably of non-small cell lung cancer (NSCLC), and diseases or disorders related thereto. Finally, the invention relates to kits, particularly to kits of parts, containing the composition and/or the vaccine.

### Background of the invention

Of all malignant tumors 25 % are bronchial carcinoma (carcinoma of the lung). Worldwide, it is the most common cause of cancer-related death in men and the second most common in women. In Germany it is the third most abundant sort of carcinoma following carcinoma of the prostata and the colorectal carcinoma. It is responsible for 1.3 million deaths worldwide annually. In Central Europe the incidence is approximately 60 per 100.000 inhabitants and the number of newly people diagnosed with lung cancer is steadily on the rise (in Germany currently being at approximately 50.000 per year). When diagnosed with lung cancer, the avarage overall fife-year survival rate is a mere 5 percent. Nevertheless, the life expectancy of each single patient is wholly dependent on the disease stage (TMN classification) and the subtype of carcinoma (lung cancer) encountered (see below).

The main sub-types of lung cancer categorized by the size and appearance of the malignant cells identified under the microscope are small cell lung cancer (20%) and non-small cell lung cancer (NSCLC) (80%). This classification, although based on simple histological criteria, has very important implications for clinical management and prognosis of the disease, with small cell lung cancer usually being treated by chemotherapy, while non-small cell lung cancer is mostly subject to surgery as a first-line treatment.

The non-small cell lung cancers (NSCLC) are grouped together because their prognosis and management are roughly identical. There are three main sub-types: squamous cell lung carcinoma, adenocarcinoma and large cell lung carcinoma. Surgery is the mainstay of treatment; however, only a quarter of the patients undergo successful resection, with a recurrence rate of 50%. Therapeutic approaches in advanced disease involve - following surgery - both adjuvant chemotherapy and/or adjuvant radiotherapy, whereas chemotherapy as monotherapy (first-line therapy) seems to be an approach associated with relatively poor results. In a comparison of four commonly used combination chemotherapy regimens, none was superior. Response rates varied from 15% to 22%, with 1-year survival rates of 31% to 36% (see e.g. O'Mahony, D., S. Kummar, et al. (2005). "Non-small-cell lung cancer vaccine therapy: a concise review." J Clin Oncol 23(35): 9022-8). Thus, even though preoperative chemotherapy seems to have not resulted in a prolongation of life expectancy, adjuvant chemotherapy - also if combined with radiotherapy - did show a significant increase in life expectancy.

One of the chemotherapeutic approaches used today are combinations of platin-based substances with e.g. Gemcitabin even as first-line-therapy, wheras e.g. Pemetrexed is used as second-line therapy.

Another option used for the treatment of NSCLC is the so-called "Targeted Therapy" trying to enhance success of classical cytotoxic chemotherapy by influencing tumor specific target structures on a molecular level. Substances used include Bevacizumab (an angiogenesis inhibitor) or Erlotinib, which is aimed at the tyrosine kinases of the epidermal growth factor receptor (EGFR).

Even though doubtless there is some improvement in the current therapeutic approaches, treatment of lung cancer, especially of NSCLC, is still an uphill-struggle with - given the high mortality rates - a strong need for further, alternative or improved ways of treatment.

Thus, it is suggested here to use the immune system in an approach for the treatment of the NSCLC. The immune system plays an important role in the treatment and prevention of numerous diseases. According to the present stage of knowledge, various mechanisms are provided by mammalians to protect the organism by identifying and killing e.g. tumor cells. These tumor cells have to be detected and distinguished from the organism's normal cells and tissues.

The immune system of vertebrates such as humans consists of many types of proteins, cells, organs, and tissues, which interact in an elaborate and dynamic network. As part of this more complex immune response, the vertebrate system adapts over time to recognize particular pathogens or tumor cells more efficiently. The adaptation process creates immunological memories and allows even more effective protection during future encounters. This process of adaptive or acquired immunity forms the basis for vaccination strategies.

The adaptive immune system is antigen-specific and requires the recognition of specific "self" or "non-self" antigens during a process called antigen presentation. Antigen specificity allows for the generation of responses that are tailored to specific pathogens or pathogen-infected cells or tumor cells. The ability to mount these tailored responses is maintained in the body by so called "memory cells". Should a pathogen infect the body more than once, these specific memory cells are used to quickly eliminate it. The adaptive immune system thus allows for a stronger immune response as well as for an immunological memory, where each pathogen or tumor cell is "remembered" by one or more signature antigens.

The major components of the adaptive immune system in vertebrates predominantly include lymphocytes on the cellular level and antibodies on the molecular level. Lymphocytes as cellular components of the adaptive immune system include B cells and T cells which are derived from hematopoietic stem cells in the bone marrow. B cells are involved in the humoral response, whereas T cells are involved in cell mediated immune response. Both B cells and T cells carry receptor molecules that recognize specific targets.

T cells recognize a "non-self" target, such as a pathogenic target structure, only after antigens (e.g. small fragments of a pathogen) have been processed and presented in combination with a "self" receptor called a major histocompatibility complex (MHC) molecule. In contrast, the B cell antigen-specific receptor is an antibody molecule on the B cell surface, and recognizes pathogens as such when antibodies on its surface bind to a specific foreign antigen. This antigen/antibody complex is taken up by the B cell and processed by proteolysis into peptides. The B cell then displays these antigenic peptides on its surface MHC class II molecules. This combination of MHC and antigen attracts a matching helper T cell, which releases lymphokines and activates the B cell. As the activated B cell then begins to divide, its offspring secretes millions of copies of the antibody that recognizes this antigen. These antibodies circulate in blood plasma and lymph, bind to pathogens or tumor cells expressing the antigen and mark them for destruction by complement activation or for uptake and destruction by phagocytes.

As a cellular component of the adaptive immune system cytotoxic T cells (CD8⁺) may form a CTL-response. Cytotoxic T cells (CD8⁺) can recognize peptides from endogenous pathogens and self-antigens bound by MHC type I molecules. CD8⁺-T cells carry out their killing function by releasing cytotoxic proteins in the cell.

Mechanisms of the immune system form targets for curative treatments. Appropriate methods are typically based on the administration of adjuvants to elicit an innate immune response or on the administration of antigens or immunogens in order to evoke an adaptive immune response. As antigens are typically based on specific components of pathogens (e.g. surface proteins) or fragments thereof, administration of nucleic acids to the patient which is followed by the expression of desired polypeptides, proteins or antigens is envisaged as well.

Hitherto conventional methods for eliciting the immune response, immunization or vaccination are based on the use of DNA molecules in order to incorporate the required genetic information into the cell. Various methods have been developed for introducing DNA into cells, such as calcium phosphate transfection, polyprene transfection, protoplast fusion, electroporation, microinjection and lipofection, lipofection having in particular proven to be a suitable method. DNA viruses may likewise be used as a DNA vehicle. Because of their infectious properties, such viruses achieve a very high transfection rate. The viruses used are genetically modified in such a manner that no functional infectious particles are formed in the transfected cell. Despite these precautions, however, it is not possible to rule out the risk of uncontrolled propagation of the introduced gene and viral genes, for example due to potential recombination events. This also entails the risk of the DNA being inserted into an intact gene of the host cell's genome by e.g. recombination, with the consequence that this gene may be mutated and thus completely or partially inactivated or may give rise to misinformation. In other words, synthesis of a gene product which is vital to the cell may be completely suppressed or alternatively a modified or incorrect gene product is expressed. One particular risk occurs if the DNA is integrated into a gene which is involved in the regulation of cell growth. In this case, the host cell may become degenerate and lead to cancer or tumor formation. Furthermore, if the DNA introduced into the cell is to be expressed, it is necessary for the corresponding DNA vehicle to contain a strong promoter, such as the viral CMV promoter. The integration of such promoters into the genome of the treated cell may result in unwanted alterations of the regulation of gene expression in the cell. Another risk of using DNA as an agent to induce an immune response (e.g. as a vaccine) is the induction of pathogenic anti-DNA antibodies in the patient into whom the foreign DNA has been introduced, so bringing about a (possibly fatal) immune response.

Thus, in order to effectively stimulate the immune system to allow treatment of lung cancer while avoiding the problems of uncontrolled propagation of an introduced gene due to DNA based compositions, RNA based compositions have been developed. WO2009/046738 provides a composition comprising at least one RNA encoding at least one antigen selected from the group consisting of NY-ESO-1, MAGE-C1 and MAGE-C2 and further encoding at least one antigen selected from the group consisting of hTERT, WT1, MAGE-A2, 5T4, MAGE-A3, MUC1, Her-2/neu, NY-ESO-1, CEA, Survivin, MAGE-C1 and/or MAGE-C2. Even though the combination of at least two antigens in said composition represents an important step towards an active immunotherapy against lung cancer, the practitioner is still faced - when looking for treatment options for an individual subject - with the selection of a suitable antigen combination, which is both, effective and well-tolerated.

Thus overall, there is room and a need for an efficient system, which may be used to effectively stimulate the immune system to allow treatment of lung cancer, especially of non-small cell lung cancer (NSCLC), while avoiding the problems encountered when using compositions that are known in the art.

It is thus an object of the present invention to provide a composition, which a) allows treatment of lung cancer by stimulating the immune system, while b) avoiding the above mentioned disadvantages.

It is thus an object of the present invention to provide a lung cancer vaccine or a composition for treatment of lung cancer by stimulating the immune system.

The object underlying the present invention is solved by the claimed subject matter.

### Summary of the Invention

This object is solved by the subject matter of the present invention, particularly by a composition comprising at least one mRNA, wherein the at least one mRNA encodes the following antigens:
- 5T4 (Trophoblast glycoprotein, TPBG);
- Survivin (Baculoviral IAP repeat-containing protein 5; BIRC5),
- NY-ESO-1 (New York esophageal squamous cell carcinoma 1; CTAG1 B),
- MAGE-C1 (Melanoma antigen family C1);
- MAGE-C2 (Melanoma antigen family C2), and
- MUC1 (Mucin 1),
or fragments thereof and wherein the at least one mRNA is mono-, bi- or multicistronic.

Surprisingly, it has been found that the specific combination of the antigens, antigenic proteins or antigenic peptides of the afore mentioned group encoded by at least one mRNA of the composition according to the present invention, is capable of effectively stimulating the (adaptive) immune system to allow treatment of lung cancer, preferably of non-small cell lung cancers, and diseases or disorders related thereto. The advantageous effects on the treatment of the diseases and disorders mentioned above are achieved irrespective of whether the combination of antigens according to the invention is applied as one single composition or by separate administration of the individual antigens. Accordingly, any combination of antigens described herein, e.g. in the form of six separate mRNA formulations, may fulfil the very same purposes and achieves the desired effect. The number of responders to such vaccination strategy is expected to be significantly increased as compared to other approaches. Herein, the terms antigens, antigenic proteins or antigenic peptides may be used synomously. In the context of the present invention, an inventive composition shall be further understood as a composition, which is able to elicit an immune response, preferably an adaptive immune response as defined herein, due to at least one of the component(s) contained in the composition or, rather, due to at least one of the antigens encoded by the at least one component of the composition, i.e. by the at least one mRNA encoding the antigens as defined above. According to the invention, the combination of antigens, whether administered separately (e.g. concurrently) or as one single composition, is capable of eliciting the desired immune response. Separate administration may mean that the distinct mRNAs are either essentially simultaneously, e.g. within 10 minutes or time-staggered over an extended period of time, e.g. more than 30 minutes.

In the following, the combination of antigens according to the invention will be illustrated by the description of a composition comprising at least one mRNA encoding the combination of antigens. It is understood that the at least one mRNA according to the invention is characterized by the features as described herein, irrespective of whether it is administered as one single composition or in the form of separate formulations, e.g. formulated as six separate mRNAs, each of which encode one antigen and which are administered separately (e.g. concurrently).

Among the large amount of antigens expressed in lung cancer cells, according to the present invention the six antigens as defined herein, i.e. 5T4, Survivin, NY-ESO-1, MAGE-C1, MAGE-C2 and MUC1 have been selected. These antigens have been identified as potential targets in immunotherapy. According to the invention, one or more of the above antigens are encoded by the at least one ORF/coding region/coding sequence provided by the at least one mRNA. In this context, a messenger RNA is typically a single-stranded RNA, which is composed of (at least) several structural elements, e.g. an optional 5' UTR region, an upstream positioned ribosomal binding site followed by a coding region, an optional 3' UTR region, which may be followed by a poly-A tail (and/or a poly-C tail). According to the invention, the composition comprises at least one mRNA, which endodes at least the six antigens defined above. Therein, one mRNA may encode one or more antigens as long as the composition as such provides the at least six antigens as defined above. The at least one mRNA of the composition may thus comprise more than one ORF/coding region/coding sequence, wherein the composition as a whole comprises at least one coding region for each of the at least six antigens as defined above. Alternatively, a coding region for each of the at least six antigens may be located on separate mRNAs of the composition. More preferred embodiments for the at least one mRNA are provided below:
According to the present invention, the at least one mRNA of the composition encodes 5T4. "5T4" is trophoblast glycoprotein. Harrop, Connolly *et al.* (2006) reported that the human oncofetal antigen 5T4 is a 72-kDa leucine-rich membrane glycoprotein which is expressed at high levels on the placenta and also on a wide range of human carcinomas including colorectal, gastric, renal, and ovarian cancers but rarely on normal tissues (see Harrop, R., N. Connolly, et al. (2006). "Vaccination of colorectal cancer patients with modified Vaccinia Ankara delivering the tumor antigen 5T4 (TroVax) induces immune responses which correlate with disease control: a phase I/II trial." Clin Cancer Res 12(11 Pt 1): 3416-24). Overexpression of 5T4 is associated with poor prognosis in patients with colorectal, gastric, and ovarian carcinoma. Despite such compounding factors, 5T4-specific cellular and/or humoral immune responses were induced in the majority of patients (16 of 17; 94%) following TroVax immunization, which was considered encouraging compared with many other cancer immunotherapy trials. In summary, they showed safety and immunogenicity of TroVax delivered via i.m. and i.d. routes of administration. Zhao and Wang (2007) (Zhao, Y. and Y. Wang (2007). "5T4 oncotrophoblast glycoprotein: janus molecule in life and a novel potential target against tumors." Cell Mol Immunol 4(2): 99-104) reported that 5T4 oncotrophoblast glycoprotein is a transmembrane protein expressed on the embryonic tissue and various malignant tumor cell surfaces. It plays a vital role in the multiple biological and pathological processes including massive cellular migration during the embryogenesis, cell invasion associated with implantation, and neoplastic metastasis in the progression of tumorigenesis. According to Kopreski, Benko *et al.* (2001) 5T4 is a trophoblast glycoprotein frequently overexpressed in epithelial malignancies that provides a potential target for cancer therapeutics (see Kopreski, M. S., F. A. Benko, et al. (2001). "Circulating RNA as a tumor marker: detection of 5T4 mRNA in breast and lung cancer patient serum." Ann N Y Acad Sci 945: 172-8). Serum was collected from 19 patients with advanced breast cancer (5 patients) or non-small-cell lung cancer (14 patients), and from 25 normal control volunteers having amplifiable RNA. RNA extracted from the serum was RT-PCR amplified using heminested, two-stage reactions, with products detected by gel electrophoresis. 5T4 mRNA was reproducibly detected in 8/19 (42%) cancer patient sera, including 2/5 breast cancer patient sera and 6/14 lung cancer patient sera, but in only 3/25 (12%) normal control sera (p = 0.035).

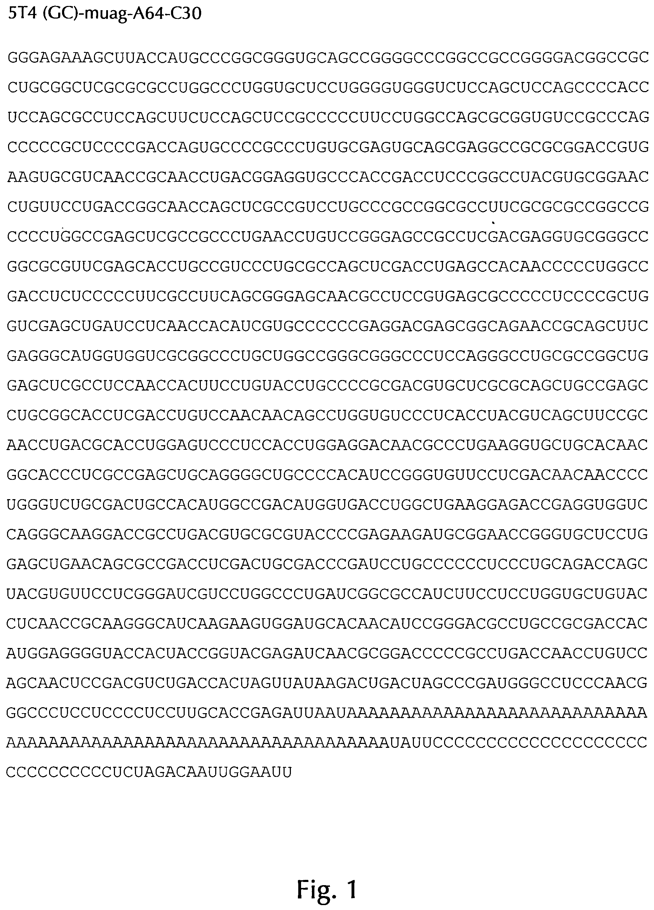

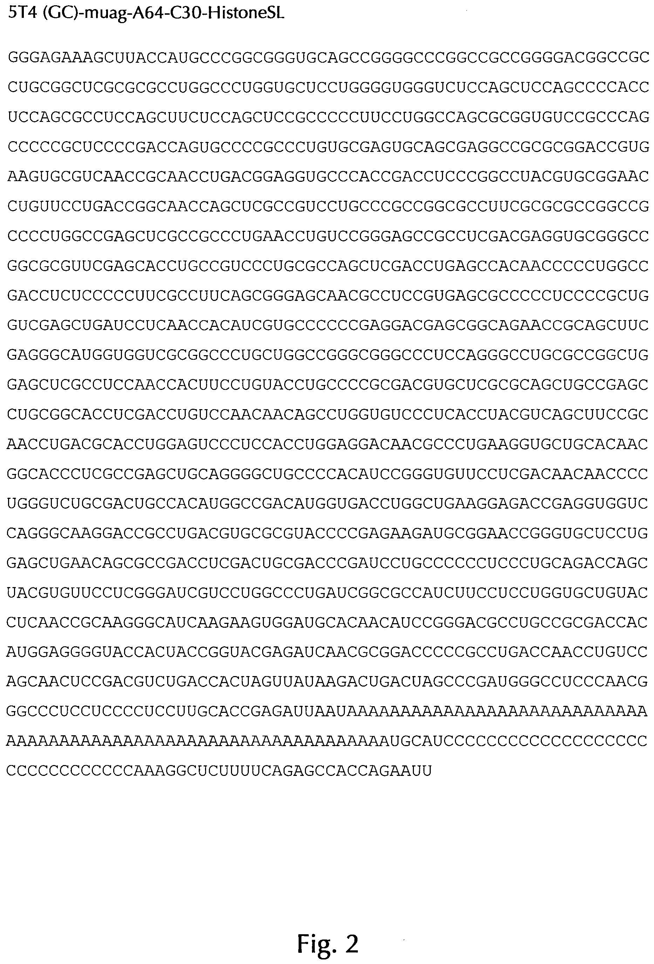

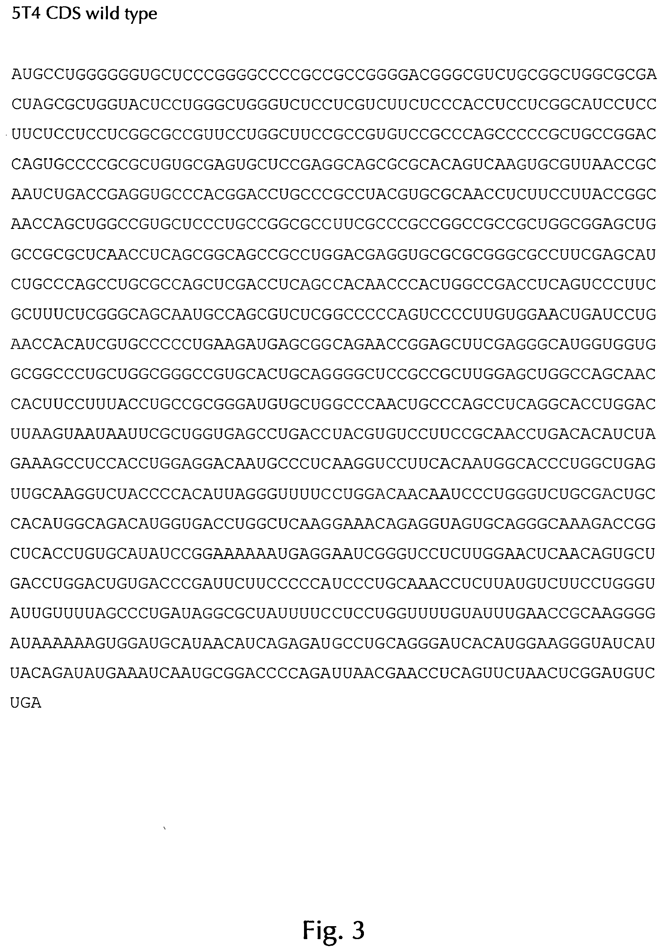

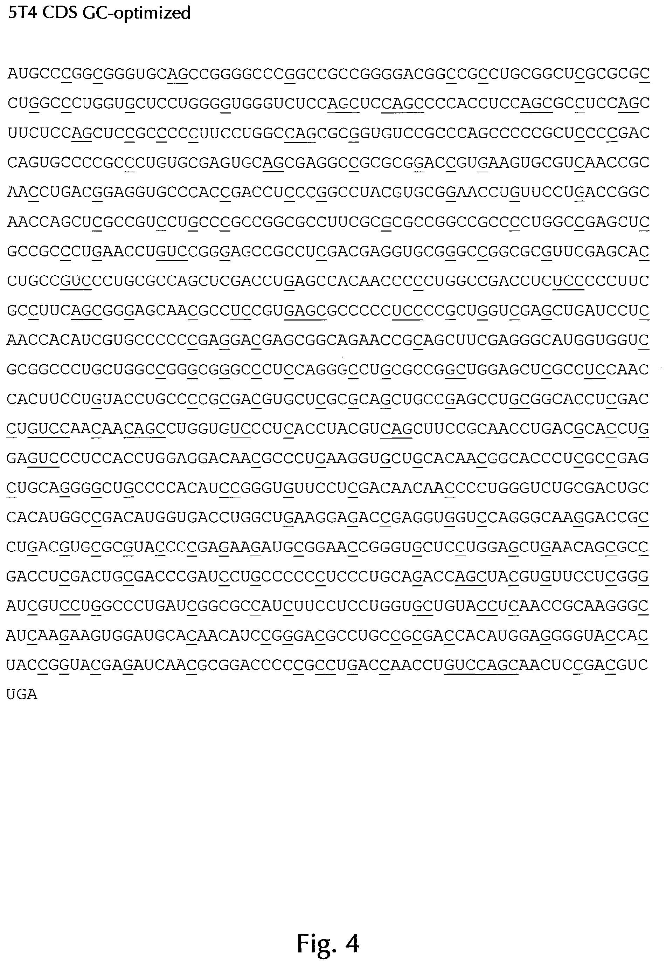

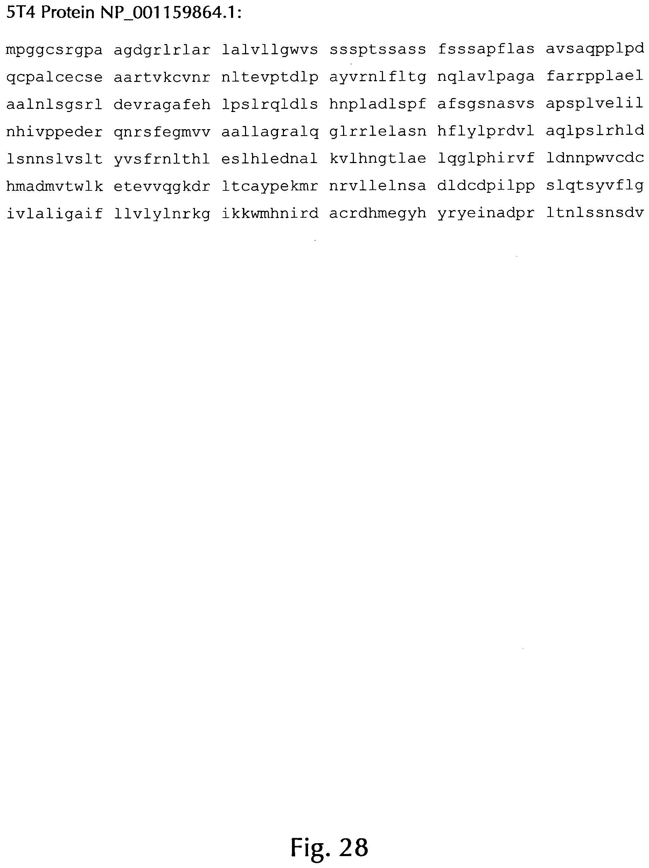

In the context of this invention, the preferred sequence of the at least one mRNA encoding 5T4 antigen may contain a coding sequence as shown in Fig. 3 (SEQ ID NO: 2), and - more preferably - as shown in Fig. 4 (SEQ ID NO: 3). Even more preferably, the at least one mRNA contains or consists of a sequence as shown in Fig. 1 or 2 (SEQ ID NO: 1 or 19). According to a further preferred embodiment, the at least one mRNA of the composition may alternatively encode a 5T4 antigen selected from a fragment, a variant or an epitope of 5T4 wherein the at least one mRNA comprises a fragment or variant of the sequence as shown in any of Figures 1, 2, 3 or 4 (SEQ ID NO: 1, 2, 3 or 19).
Furthermore the at least one mRNA preferably comprises a sequence coding for an amino acid sequence as shown in Fig. 28 (SEQ ID NO: 75), or a fragment, a variant or an epitope thereof.

The at least one mRNA of the composition furthermore encodes Survivin. "Survivin" is baculoviral IAP repeat-containing 5 (survivin). Grube, Moritz *et al.* (2007) described Survivin (see Grube, M., S. Moritz, et al. (2007). "CD8+ T cells reactive to survivin antigen in patients with multiple myeloma." Clin Cancer Res 13(3): 1053-60). Survivin is a member of the inhibitors of apoptosis family and is overexpressed in different types of malignancies. Cytotoxic T cells recognizing survivin epitopes can be elicited *in vitro* and by vaccination in patients with leukemia, breast cancer, and melanoma. It was investigated whether survivin-specific CD8+ T cells occur in patients with multiple myeloma and T cells recognizing HLA-A2.1-binding survivin peptide were detected in 9 of 23 patients and in 1 of 21 healthy volunteers. Survivin-reactive T cells were identified as terminally differentiated effector T cells (CD8+, CD45RA+, and CCR7-). Positive survivin expression of myeloma cells in bone marrow specimens was shown in 7 of 11 patients. Survivin is highly expressed in most human cancer cells of epithelial and hematopoietic origin, and overexpression is associated with cancer progression, poor prognosis, resistance, and short patient survival. Duffy, O'Donovan (2007) described that Survivin is a 16.5 kDa protein overexpressed in almost all malignancies but rarely detected in normal differentiated adult tissues (see Duffy, M. J., N. O'Donovan, et al. (2007). "Survivin: a promising tumor biomarker." Cancer Lett 249(1): 49-60). Functionally, survivin has been shown to inhibit apoptosis, promote cell proliferation and enhance angiogenesis. Consistent with its role in these processes, survivin was described as playing a key role in cancer progression. Because of the large difference in expression between normal and malignant tissue and its causal role in cancer progression, survivin is currently undergoing intensive investigation as a potential tumor marker. Emerging data suggests that measurement of survivin can aid the early diagnosis of bladder cancer, determine prognosis in multiple cancer types and predict response to diverse anticancer therapies. Zeis, Siegel *et al.* (2003) demonstrated that human survivin-specific CTLs generated from PBMC by stimulation with autologous dendritic cells transfected with survivin-RNA were cytotoxic for a range of hematopoietic malignant cell lines and primary tumor cells isolated from patients with acute myeloid leukemia (see Zeis, M., S. Siegel, et a/. (2003). "Generation of cytotoxic responses in mice and human individuals against hematological malignancies using survivin-RNA-transfected dendritic cells." J Immunol 170(11): 5391-7). It was also shown that vaccination of mice with survivin-RNA-transfected dendritic cells lead to long term resistance to challenge by a survivin-expressing lymphoma, demonstrating the potential of survivin as a tumor rejection Ag.

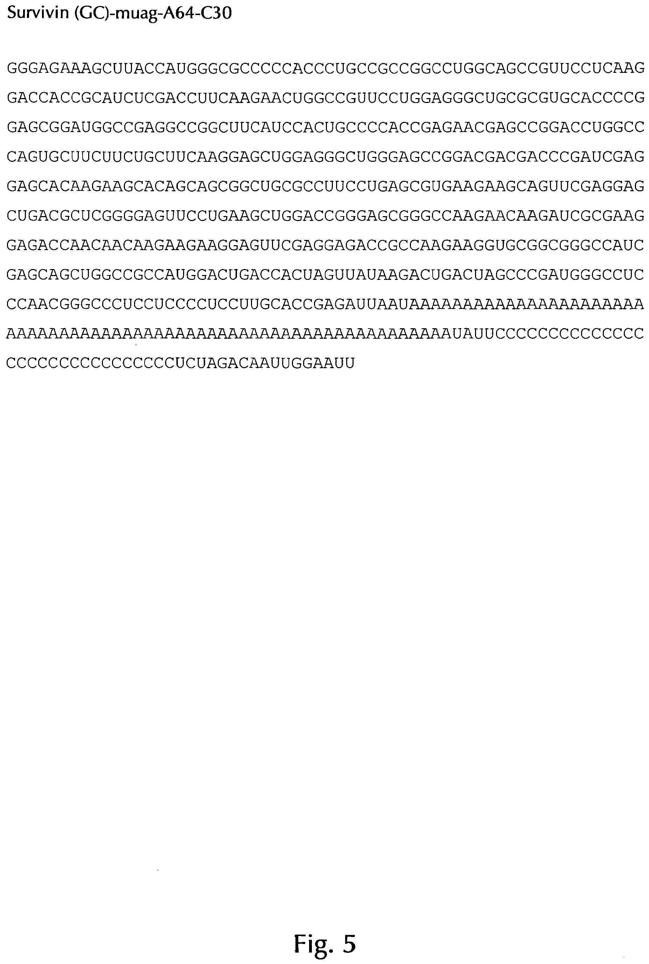

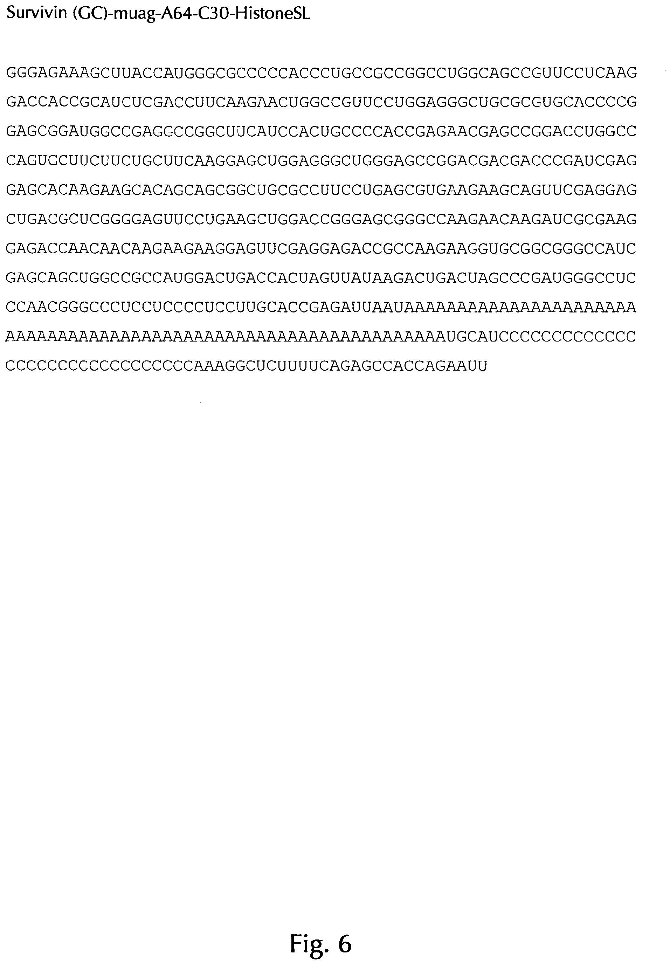

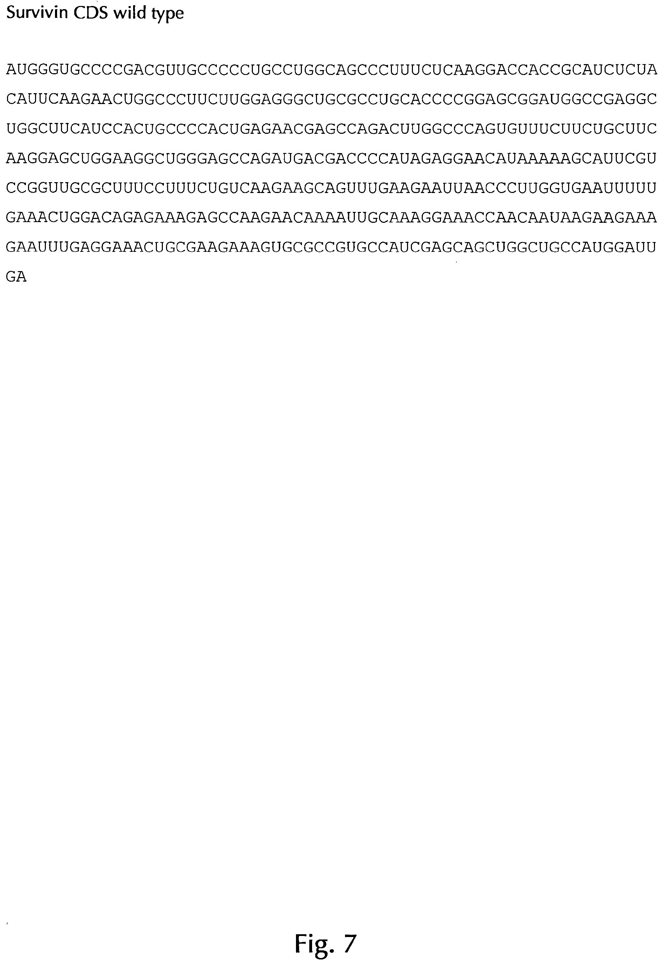

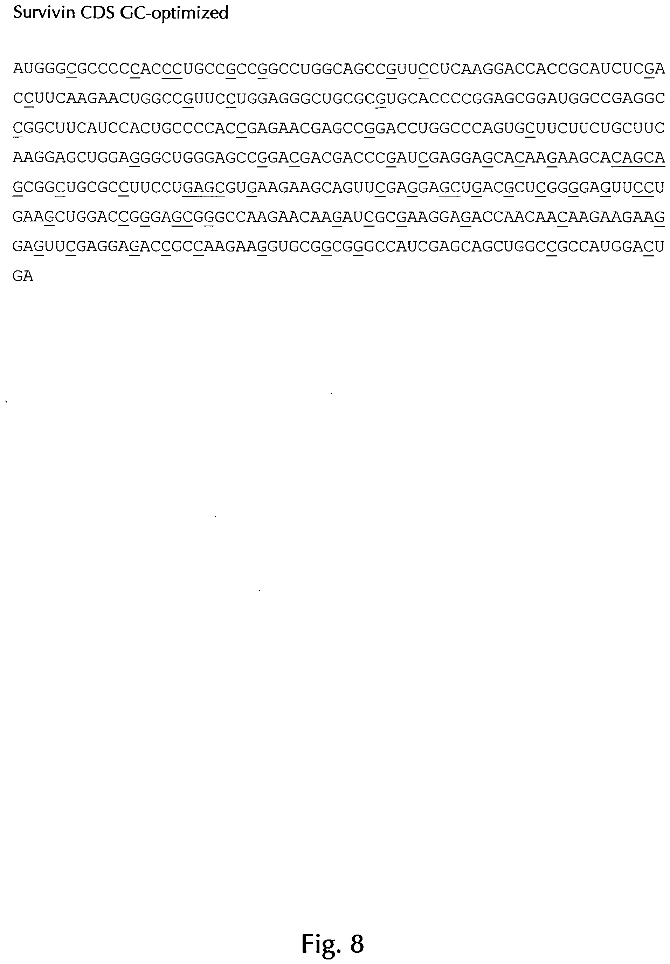

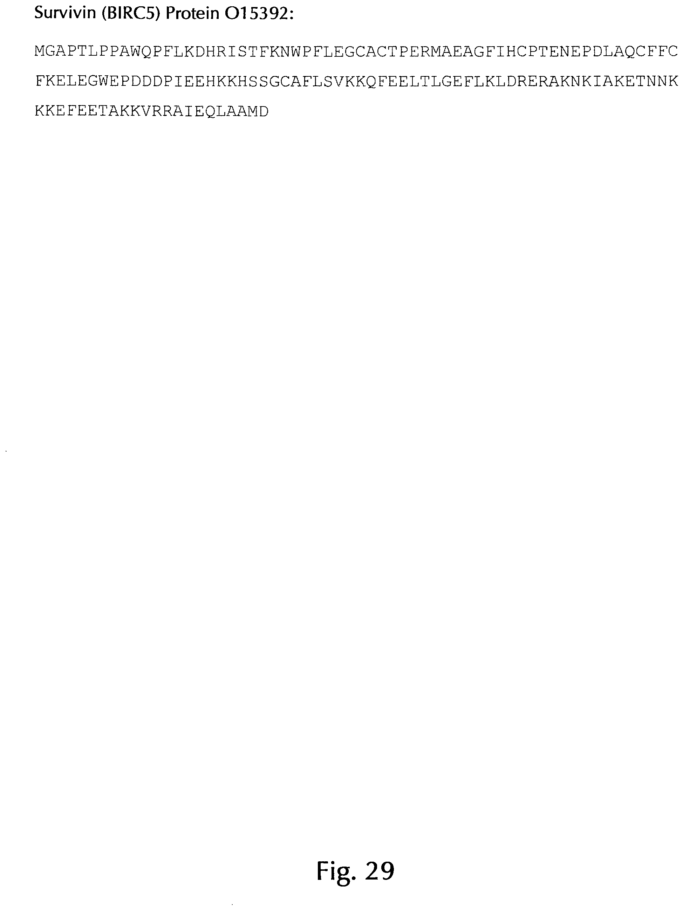

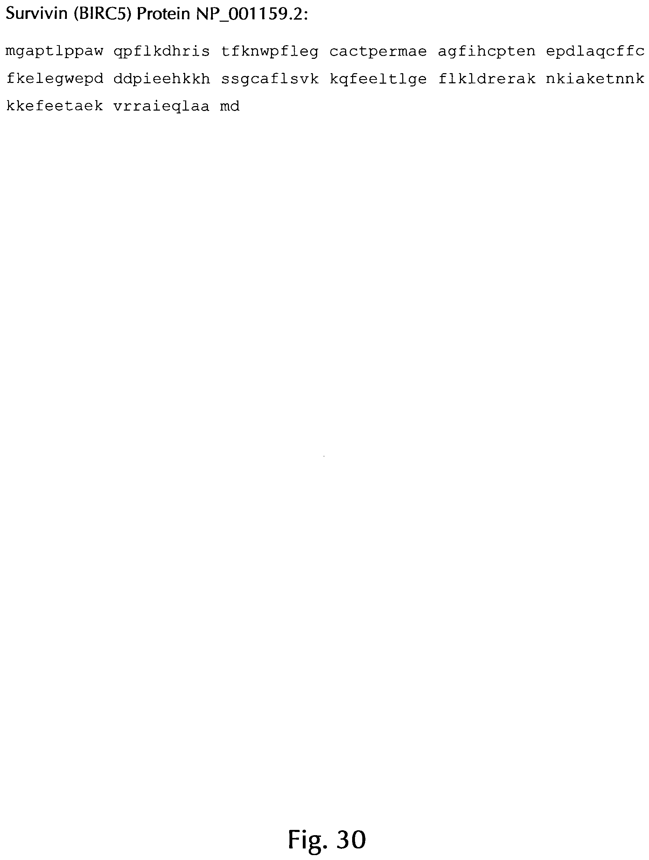

In the context of this invention, the preferred sequence of the at least one mRNA encoding Survivin may contain a coding sequence as shown in Fig. 7 (SEQ ID NO: 5), and - more preferably - as shown in Fig. 8 (SEQ ID NO: 6). Even more preferably, the at least one mRNA contains or consists of a sequence as shown in Fig. 5 or 6 (SEQ ID NO: 4 or 20). According to a further preferred embodiment, the at least one mRNA of the composition may alternatively encode a Survivin antigen selected from a fragment, a variant or an epitope of Survivin, wherein the at least one mRNA comprises a fragment or variant of the sequence as shown in any of Figures 5, 6, 7 or 8 (SEQ ID NO: 4, 5, 6 or 20).
Furthermore, the at least one mRNA preferably comprises a sequence coding for an amino acid sequence as shown in Fig. 29 or 30 (SEQ ID NO: 76 or 77), or a fragment, a variant or an epitope thereof.

The at least one mRNA of the composition furthermore encodes NY-ESO-1. "NY-ESO-1" is cancer/testis antigen 1B. Chen, Scanlan *et al.* (1997) reported the mRNA expression of NY-ESO-1 in various human tumors by RT-PCR finding Melanoma 23/67, Ovarian cancer 2/8, Breast cancer 10/33, Thyroid cancer 2/5, Prostate cancer 4/16, Bladder cancer 4/5, Colon cancer 0/16, Burkitt lymphoma 1/2, Glioma 0/15, Basal cell carcinoma 0/2, Gastric cancer 0/12, Leiomyosarcoma 0/2, Lung cancer 2/12, Other sarcomas 0/2, Renal cancer 0/10, Pancreatic cancer 0/2, Lymphoma 0/10, Seminoma 0/1, Hepatoma 2/7, Spinal cord tumor 0/1 (see Chen, Y. T., M. J. Scanlan, et al. (1997). "A testicular antigen aberrantly expressed in human cancers detected by autologous antibody screening." Proc Natl Acad Sci U S A 94(5): 1914-8). Jager, Karbach *et al.* (2006) reported that NY-ESO-1 is a cancer/testis antigen expressed in a range of human malignancies, and that a number of vaccine strategies targeting NY-ESO-1 were being developed (see Jager, E., J. Karbach, et al. (2006). "Recombinant vaccinia/fowlpox NY-ESO-1 vaccines induce both humoral and cellular NY-ESO-1-specific immune responses in cancer patients." Proc Natl Acad Sci U S A 103(39): 14453-8). In the presented study, the safety and immunogenicity of recombinant vaccinia-NY-ESO-1 and recombinant fowlpox-NY-ESO-1 were analyzed in a series of 36 patients with a range of different tumor types. Each construct was first tested individually at two different dose levels and then in a prime-boost setting with recombinant vaccinia-NY-ESO-1 followed by recombinant fowlpox-NY-ESO-1. The vaccines were well tolerated either individually or together. NY-ESO-1-specific antibody responses and/or specific CD8 and CD4 T cell responses directed against a broad range of NY-ESO-1 epitopes were induced by a course of at least four vaccinations at monthly intervals in a high proportion of patients. CD8 T cell clones derived from five vaccinated patients were shown to lyse NY-ESO-1-expressing melanoma target cells. In several patients with melanoma, there was a strong impression that the natural course of the disease was favorably influenced by vaccination. Davis, Chen *et al.* (2004) reported that HLA-A2-restricted NY-ESO-1 peptides injected intradermally were shown to be safe and immunogenic (Davis, I. D., W. Chen, et al. (2004). "Recombinant NY-ESO-1 protein with ISCOMATRIX adjuvant induces broad integrated antibody and CD4(+) and CD8(+) T cell responses in humans." Proc Natl Acad Sci U S A 101(29): 10697-702). Although these trials were designed only to determine safety and immunogenicity, some patients showed tumor regression or stabilization of disease. It was further expressed by Jager, Gnjatic *et al.* (2000) that a broad NY-ESO-1-specific immune response including antibody and CD4 and CD8 T cell responses was seen after immunization with recombinant NY-ESO-1 protein combined with ISCOMATRIX adjuvant (CSL Ltd., Parkville, Victoria, Australia) in patients with resected NY-ESO-1-expressing melanoma (see Jager, E., S. Gnjatic, et a/. (2000). "Induction of primary NY-ESO-1 immunity: CD8+ T lymphocyte and antibody responses in peptide-vaccinated patients with NY-ESO-1 + cancers." Proc Natl Acad Sci U S A 97(22): 12198-203). This immune response to the vaccine appeared to be associated with long disease-free survival. Furthermore Odunsi, Qian *et al.* (2007) reported that vaccination with an NY-ESO-1 peptide induces integrated humoral and T cell responses in ovarian cancer (see Odunsi, K., F. Qian, et al. (2007). "Vaccination with an NY-ESO-1 peptide of HLA class I/II specificities induces integrated humoral and T cell responses in ovarian cancer." Proc Natl Acad Sci U S A 104(31): 12837-42).

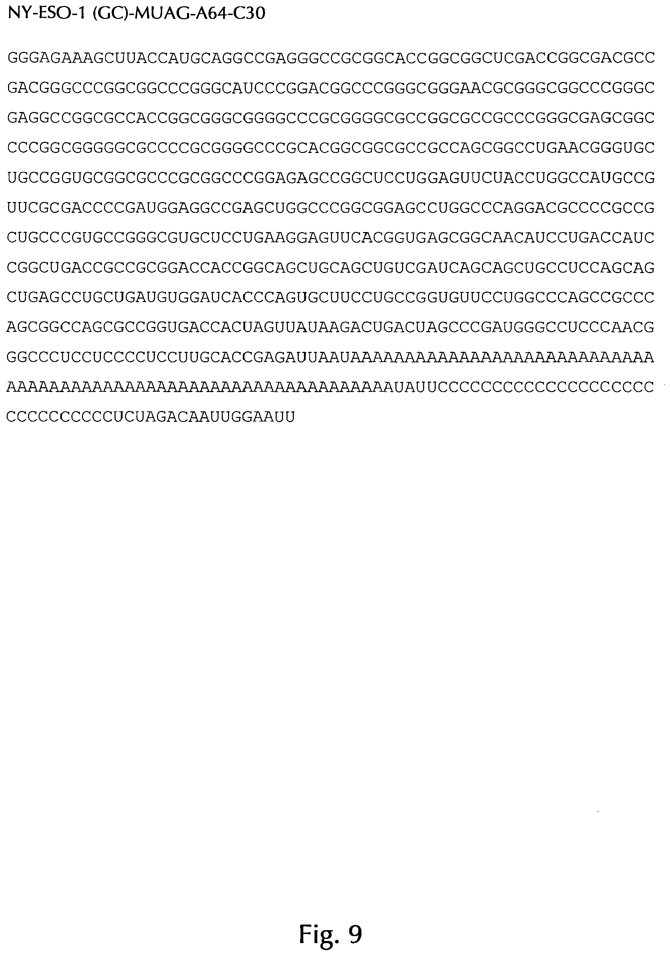

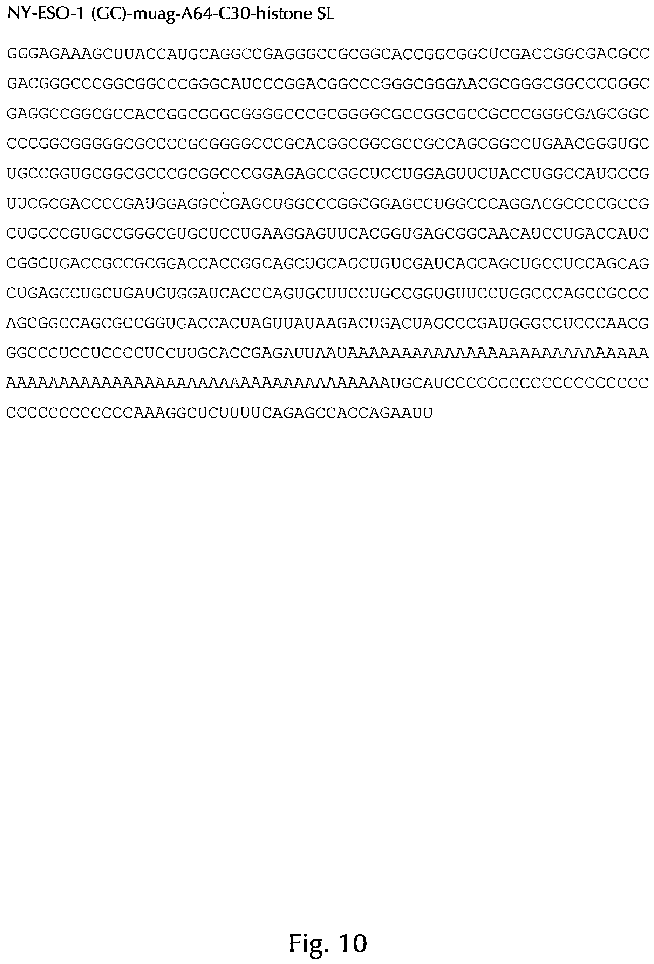

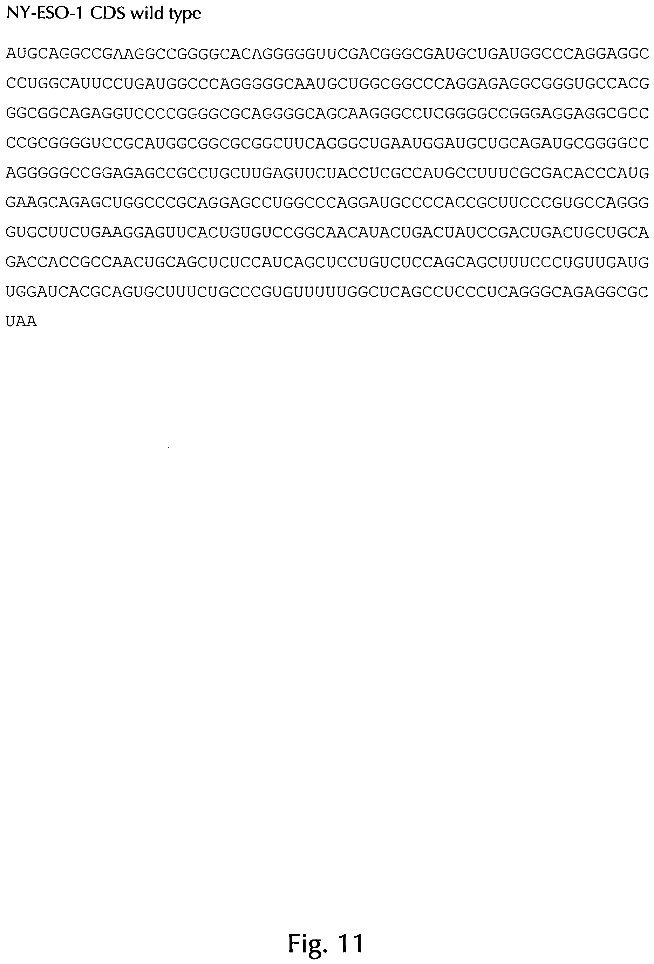

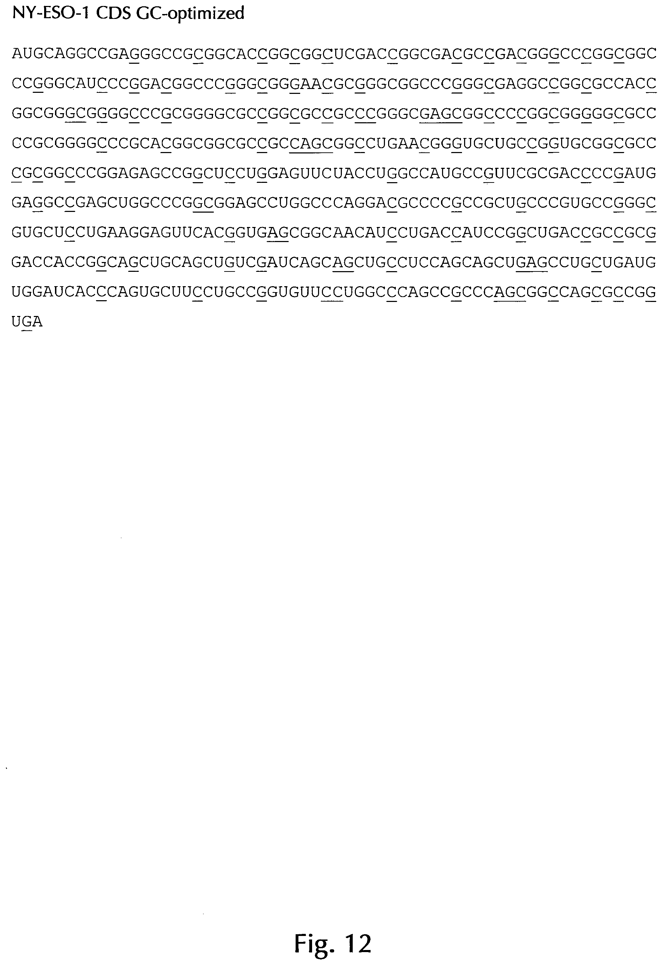

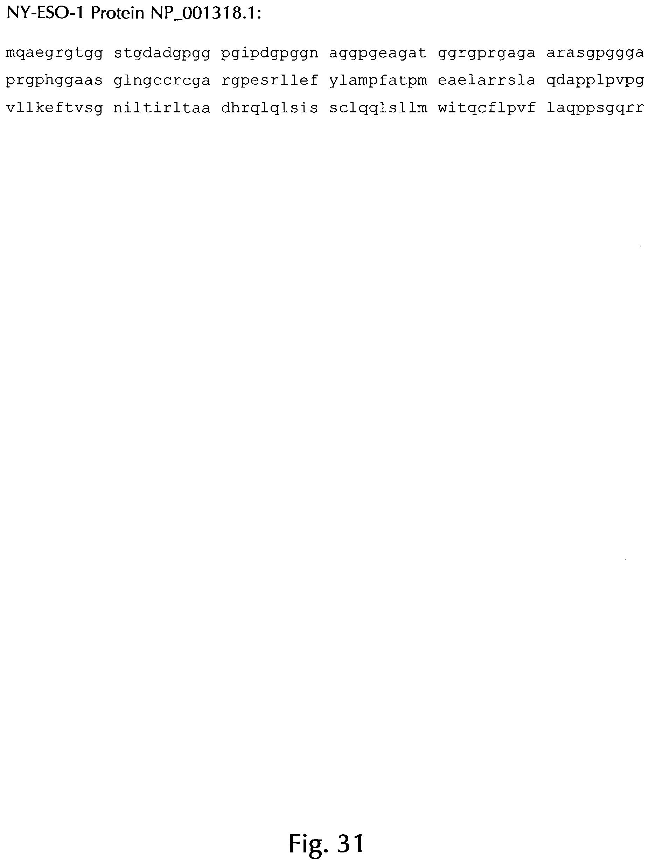

In the context of this invention, the preferred sequence of the at least one mRNA encoding NY-ESO-1 antigen may contain a coding sequence as shown in Fig. 11 (SEQ ID NO: 8), and - more preferably - as shown in Fig. 12 (SEQ ID NO: 9). Even more preferably, the at least one mRNA contains or consists of a sequence as shown in Fig. 9 or 10 (SEQ ID NO: 7 or 21). According to a further preferred embodiment, the at least one mRNA of the composition may alternatively encode a NY-ESO-1 antigen selected from a fragment, a variant or an epitope of NY-ESO-1, wherein the at least one mRNA comprises a fragment or variant of the sequence as shown in any of Figures 9, 10, 11, or 12 (SEQ ID NO: 7, 8, 9 or 21).
Furthermore, the at least one mRNA preferably comprises a sequence coding for an amino acid sequence as shown in Fig. 31 (SEQ ID NO: 78), or a fragment, a variant or an epitope thereof.

The at least one mRNA of the composition furthermore encodes MAGE-C1. "MAGE-C1" is the melanoma antigen family C, 1. Lucas, De Smet *et al.* (1998) recently identified MAGE-C1 by performing RDA (see Lucas, S., C. De Smet, et al. (1998). "Identification of a new MAGE gene with tumor-specific expression by representational difference analysis." Cancer Res 58(4): 743-52). MAGE-C1 was not expressed in a panel of normal tissues tested with the exception of testis. Among tumoral samples, MAGE-C1 was frequently expressed in seminomas, melanomas, and bladder carcinomas. It was also expressed in a significant fraction of head and neck carcinomas, breast carcinomas, non-small lung carcinomas, prostate adenocarcinomas and sarcomas. Jungbluth, Chen *et al.* (2002) described expression in breast cancer, ovary cancer, liver cancer, testis cancer, bladder cancer, melanoma and non-small cell lung cancer (39%) (see Jungbluth, A. A., Y. T. Chen, et al. (2002). "CT7 (MAGE-C1) antigen expression in normal and neoplastic tissues." Int J Cancer 99(6): 839-45). Gure, Chua *et al.* (2005) analyzed tumors from 523 non-small-cell lung cancer (NSCLC) patients for the expression of cancer-testis antigens (see Gure, A. O., R. Chua, et a/. (2005). "Cancer-testis genes are coordinately expressed and are markers of poor outcome in non-small cell lung cancer." Clin Cancer Res 11(22): 8055-62). MAGE-C1 was present in 18,8%. Scanlan, Altorki *et al.* (2000) furthermore reported expression of CT antigens in 33 non-small cell lung cancers: MAGE-C1: 30% (see Scanlan, M. J., N. K. Altorki, et al. (2000). "Expression of cancer-testis antigens in lung cancer: definition of bromodomain testis-specific gene (BRDT) as a new CT gene, CT9." Cancer Lett 150(2): 155-64).

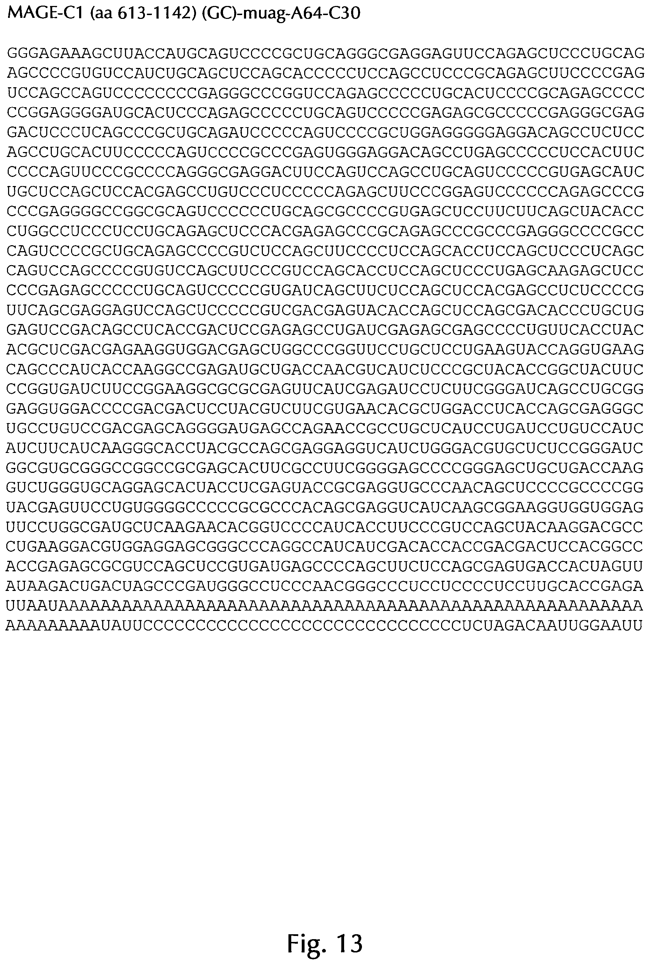

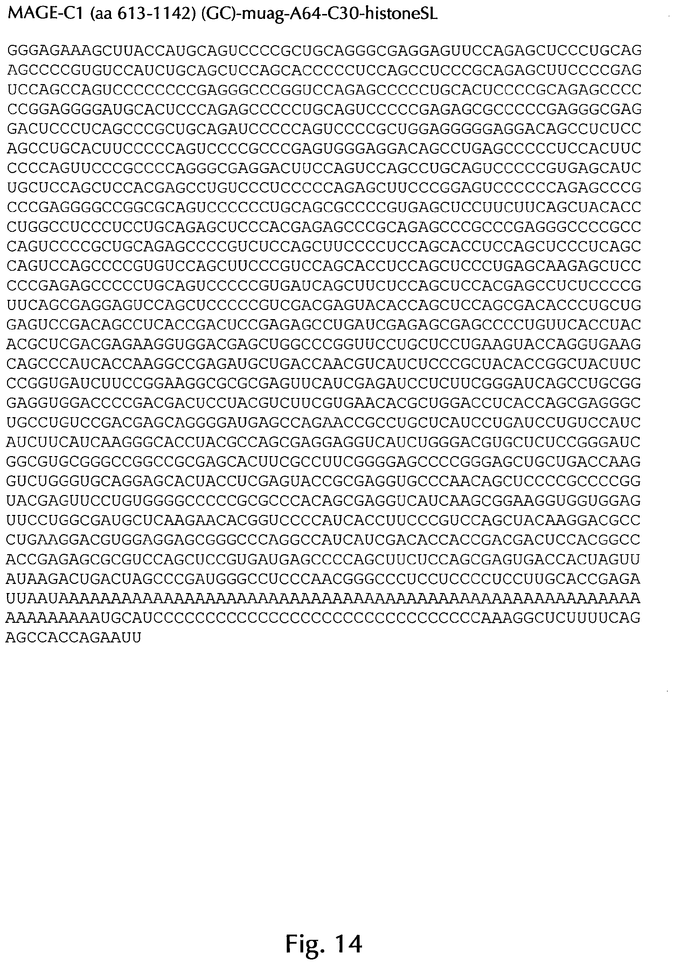

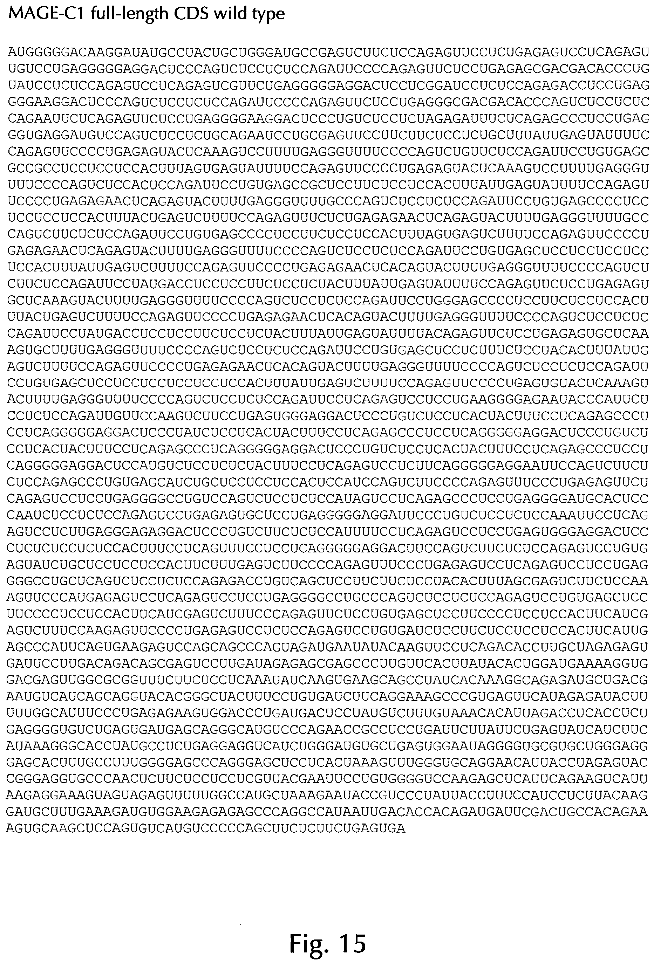

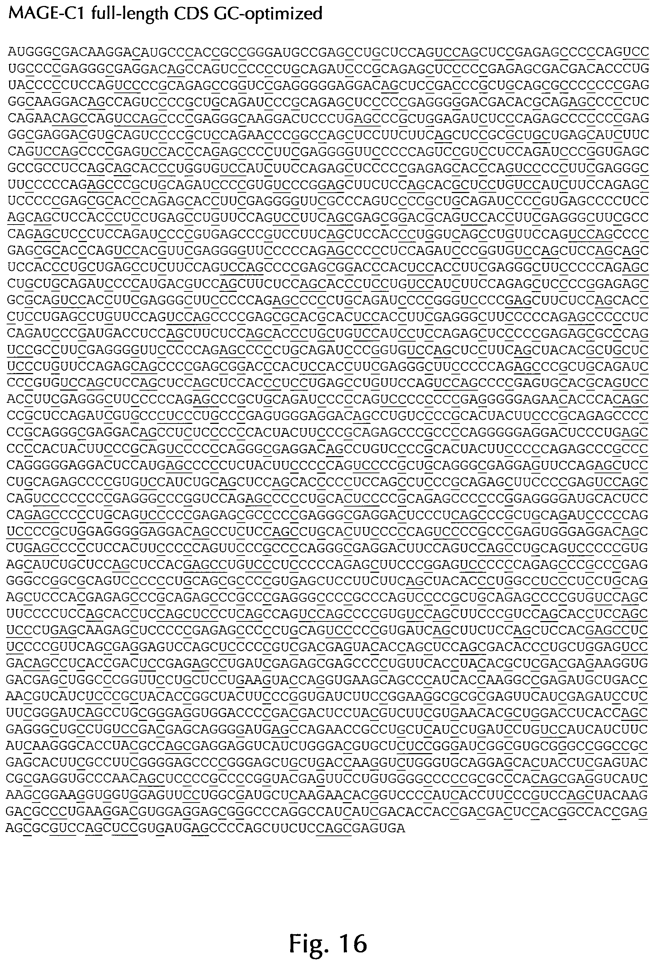

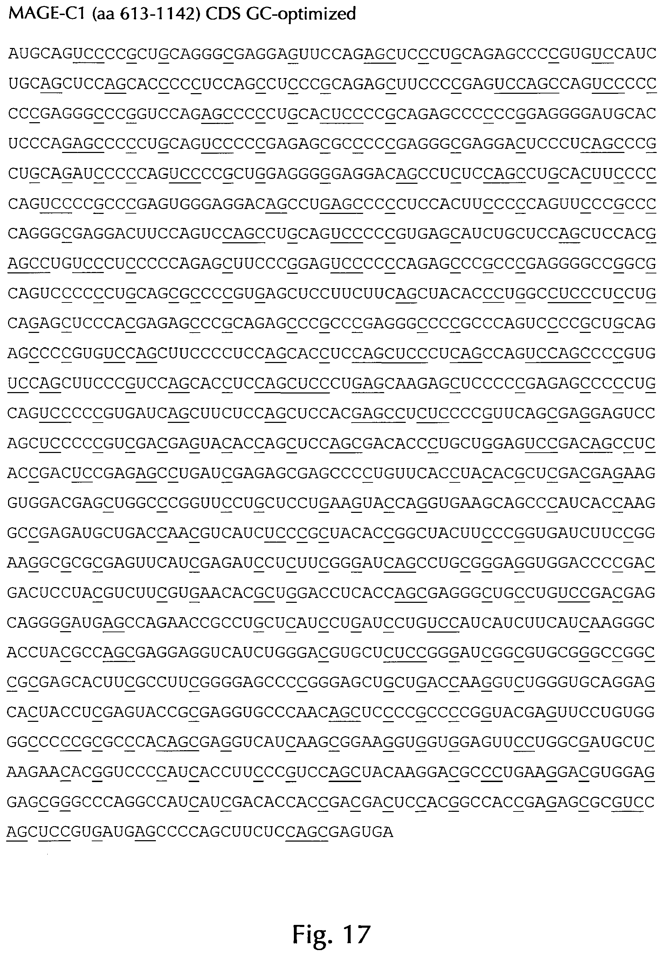

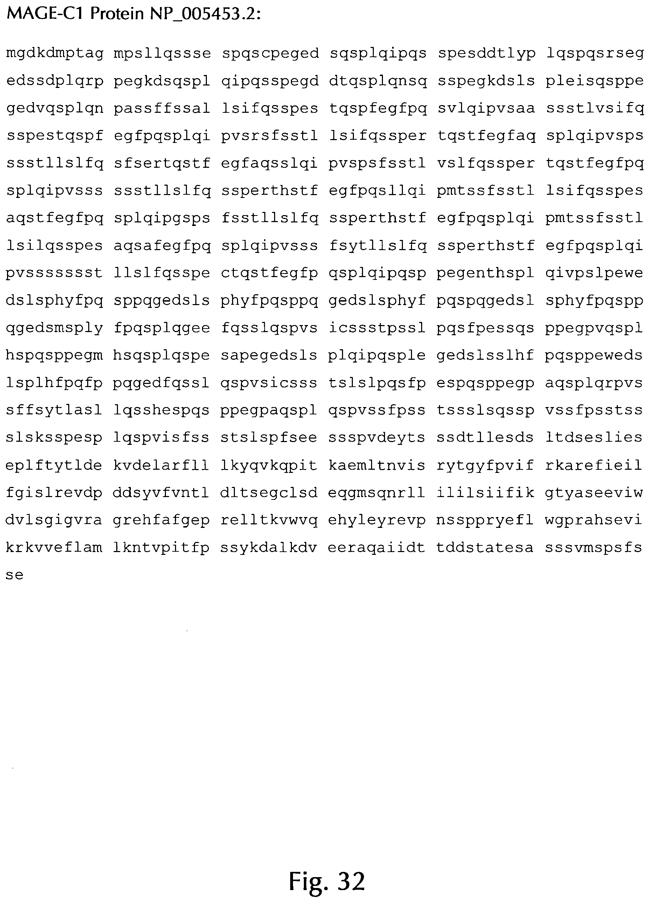

In the context of this invention, the preferred sequence of the at least one mRNA encoding MAGE-C1 antigen may contain a coding sequence as shown in Fig. 15 (SEQ ID NO: 11), and - more preferably - as shown in Fig. 16 (SEQ ID NO: 12) or even more preferably in Fig. 17 (SEQ ID NO: 25). Even more preferably, the at least one mRNA contains or consists of a sequence as shown in Fig. 13 or 14 (SEQ ID NO: 10 or 22). According to a further preferred embodiment, the at least one mRNA of the composition may alternatively encode MAGE-C1 antigen selected from a fragment, a variant or an epitope of a MAGE-C1, wherein the at least one mRNA comprises a fragment or variant of the sequence as shown in any of Figures 13, 14, 15, 16, or 17 (SEQ ID NO: 10, 11, 12, 22 or 25).
Furthermore, the at least one mRNA preferably comprises a sequence coding for an amino acid sequence as shown in Fig. 32 (SEQ ID NO: 79), or a fragment, a variant or an epitope thereof.

The at least one mRNA of the composition furthermore encodes MAGE-C2. "MAGE-C2" is the melanoma antigen family C2. Lucas, De Plaen *et al.* (2000) recently identified MAGE-C2 by performing RDA on a melanoma cell line (see Lucas, S., E. De Plaen, et al. (2000). "MAGE-B5, MAGE-B6, MAGE-C2, and MAGE-C3: four new members of the MAGE family with tumor-specific expression." Int J Cancer 87(1): 55-60). MAGE-C2 was not expressed in a panel of normal tissues tested with the exception of testis. Among tumoral samples, MAGE-C2 was frequently expressed in seminomas, melanomas, and bladder carcinomas. It was also expressed in a significant fraction of head and neck carcinomas, breast carcinomas, non-small lung carcinomas and sarcomas. Scanlan, Altorki *et a*/*.* (2000) reported expression of CT antigens in 33 non-small cell lung cancers: MAGE-C2: 30% (see Scanlan, M. J., N. K. Altorki, et al. (2000). "Expression of cancer-testis antigens in lung cancer: definition of bromodomain testis-specific gene (BRDT) as a new CT gene, CT9." Cancer Lett 150(2): 155-64).

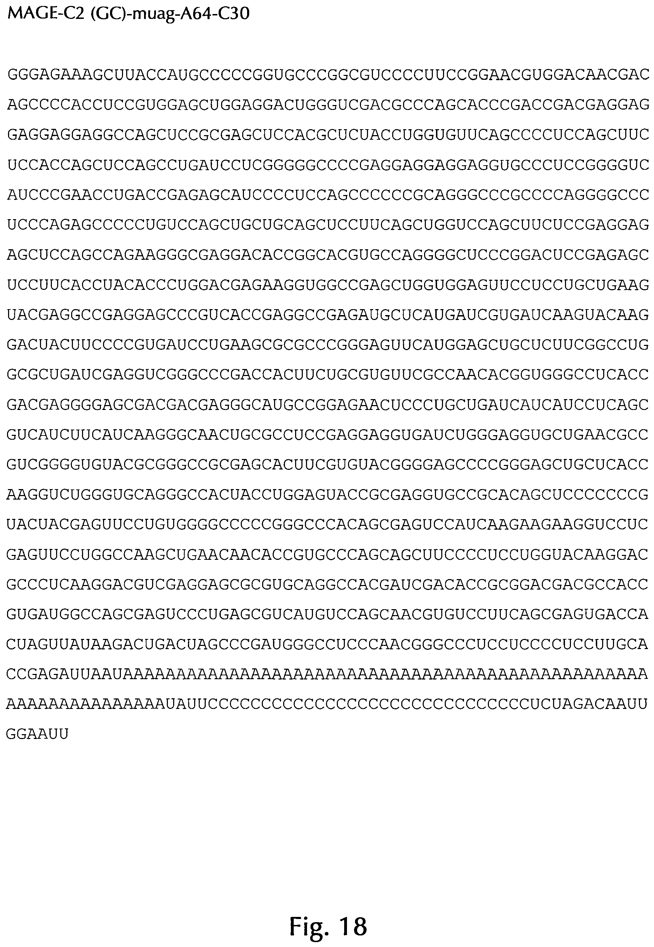

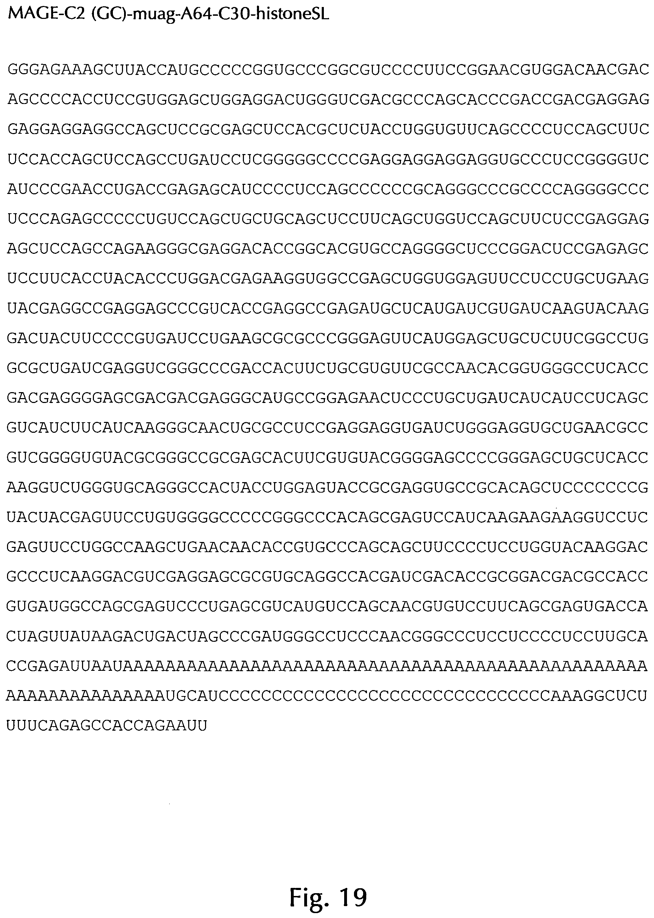

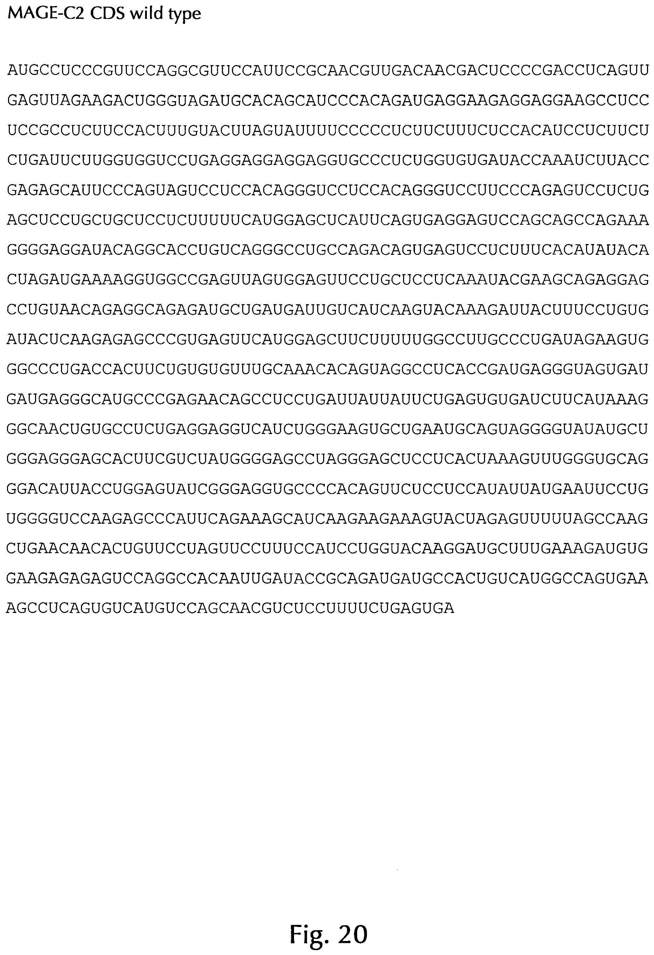

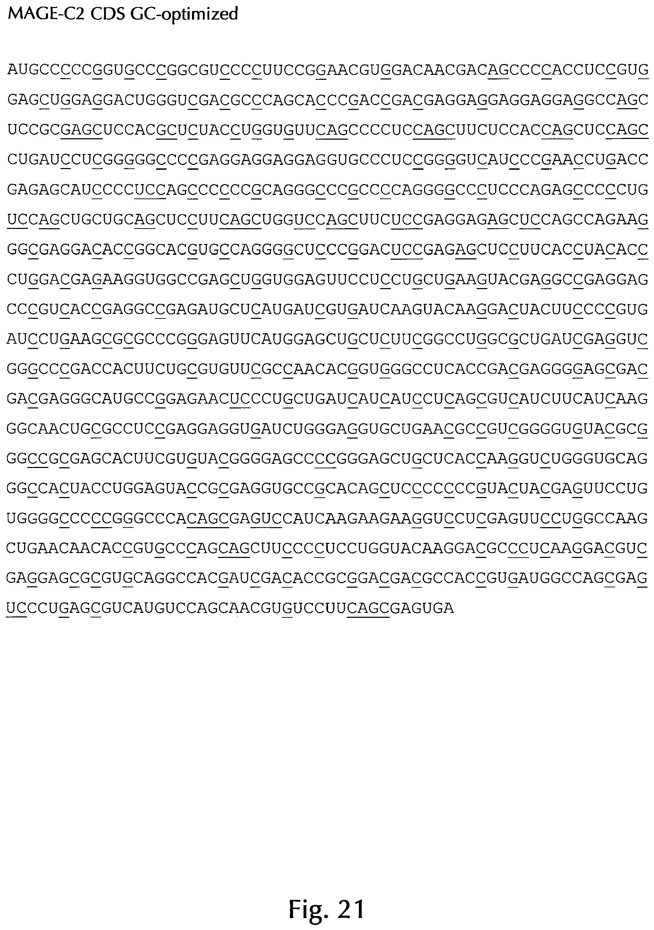

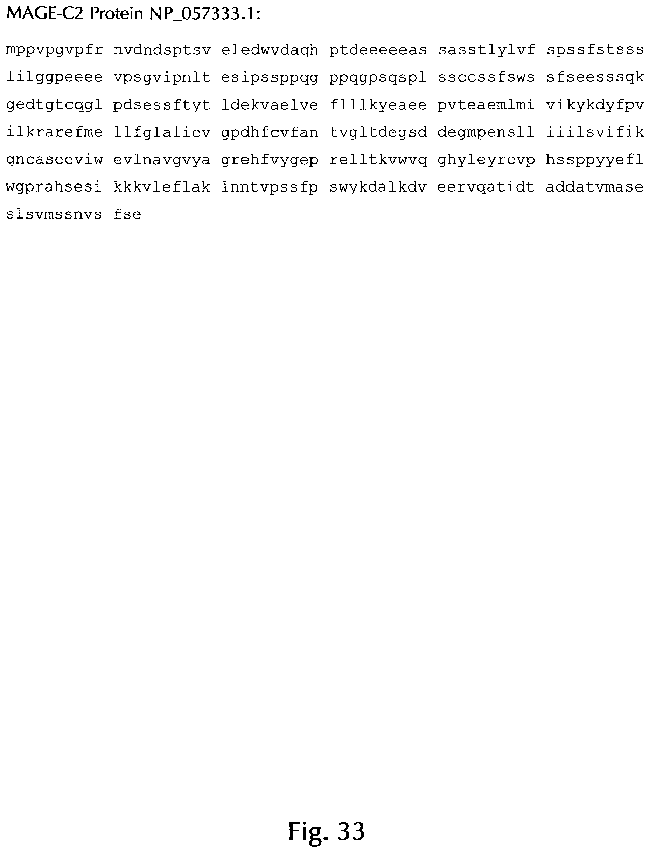

In the context of this invention, the preferred sequence of the at least one mRNA encoding MAGE-C2 antigen may contain a coding sequence as shown in Fig. 20 (SEQ ID NO: 14), and - more preferably - as shown in Fig. 21 (SEQ ID NO: 15). Even more preferably, the at least one mRNA contains or consists of a sequence as shown in Fig. 18 or 19 (SEQ ID NO: 13 or 23). According to a further preferred embodiment, the at least one mRNA of the composition may alternatively encode a MAGE-C2 antigen selected from a fragment, a variant or an epitope of MAGE-C2, wherein the at least one mRNA comprises a fragment or variant of the sequence as shown in any of Figures 18, 19, 20 or 21 (SEQ ID NO: 13, 14, 15 or 23).
Furthermore, the at least one mRNA preferably comprises a sequence coding for an amino acid sequence as shown in Fig. 33 (SEQ ID NO: 80), or a fragment, a variant or an epitope thereof.

Finally, the at least one mRNA of the composition furthermore encodes MUC1. "MUC1" is mucin 1. Cancer-associated mucins are thought to promote metastases by facilitating adhesion of malignant cells to the endothelial cell surface. According to Denda-Nagai and Irimura (2000) (Denda-Nagai, K. and T. Irimura (2000). "MUC1 in carcinoma-host interactions." Glycoconj J 17(7-9): 649-58) MUC-1 is overexpressed in 90% of all adenocarcinomas, including breast, lung, pancreas, prostate, stomach, colon and ovary. Kontani, Taguchi *et al.* (2001) found that MUC-1 has been found to be expressed in 60% of lung cancers (see Kontani, K., O. Taguchi, et al. (2001). "Modulation of MUC1 mucin as an escape mechanism of breast cancer cells from autologous cytotoxic T-lymphocytes." Br J Cancer 84(9): 1258-64), whereas Kontani, Taguchi *et al.* (2003) found in a study analyzing the use of pulsed DCs with MUC1 antigens to elicit cellular immunity in MUC1 positive cancers, that clinically seven of nine MUC-1 positive patients responded to the treatment with either a reduction in tumor marker levels or disappearance of malignant pleural effusion (see Kontani, K., O. Taguchi, et al. (2003). "Dendritic cell vaccine immunotherapy of cancer targeting MUC1 mucin." Int J Mol Med 12(4): 493-502). Three of these responding patients had NSCLC. Palmer, Parker *et al.* (2001) reported that in a phase I clinical trial using MUC1 peptide in stage III/IV NSCLC, safety and tolerability of this agent was established (see Palmer, M., J. Parker, et al. (2001). "Phase I study of the BLP25 (MUC1 peptide) liposomal vaccine for active specific immunotherapy in stage IIIB/IV non-small-cell lung cancer." Clin Lung Cancer 3(1): 49-57; discussion 58). Five of 12 patients (42%) had immunologic responses, and 4 of 12 patients (33%) achieved stable disease. Wierecky, Mueller *et al.* (2006) further identified two HLA-A2 binding novel 9-mer peptides of the TAA MUC1, which is overexpressed on various hematological and epithelial malignancies (see Wierecky, J., M. Mueller, et al. (2006). "Dendritic cell-based cancer immunotherapy targeting MUC-1." Cancer Immunol Immunother 55(1): 63-7). Cytotoxic T cells generated after pulsing DC with these peptides were able to induce lysis of tumor cells expressing MUC1 in an antigen-specific and HLA-restricted fashion. Within two clinical studies, it was demonstrated that vaccination of patients with advanced cancer using DCs pulsed with MUC1 derived peptides was well tolerated without serious side effects and was able to induce immunological responses. Of 20 patients with metastatic renal cell carcinoma, 6 patients showed regression of metastases with 3 objective responses (1 CR, 2 PR).

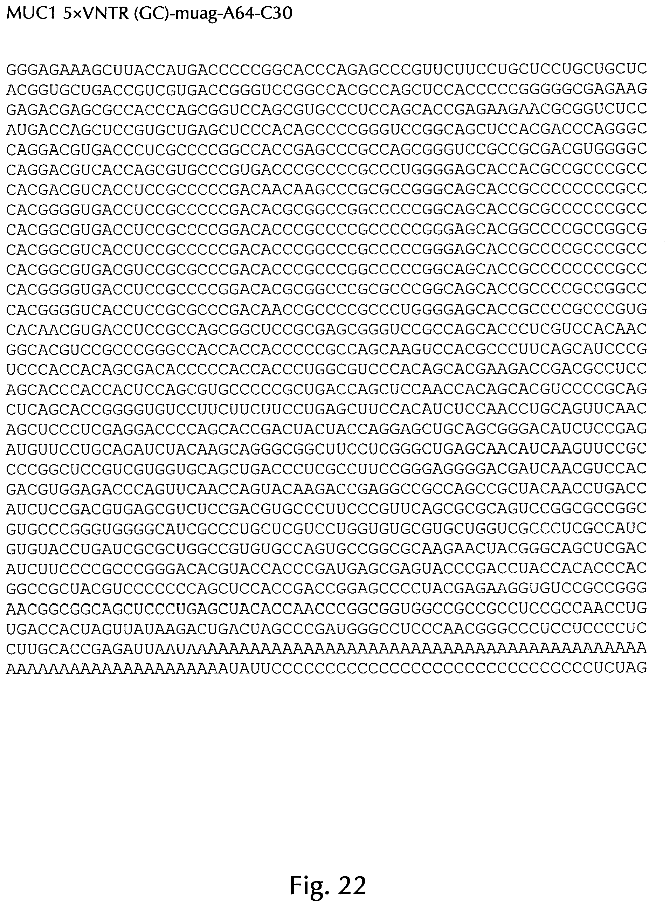

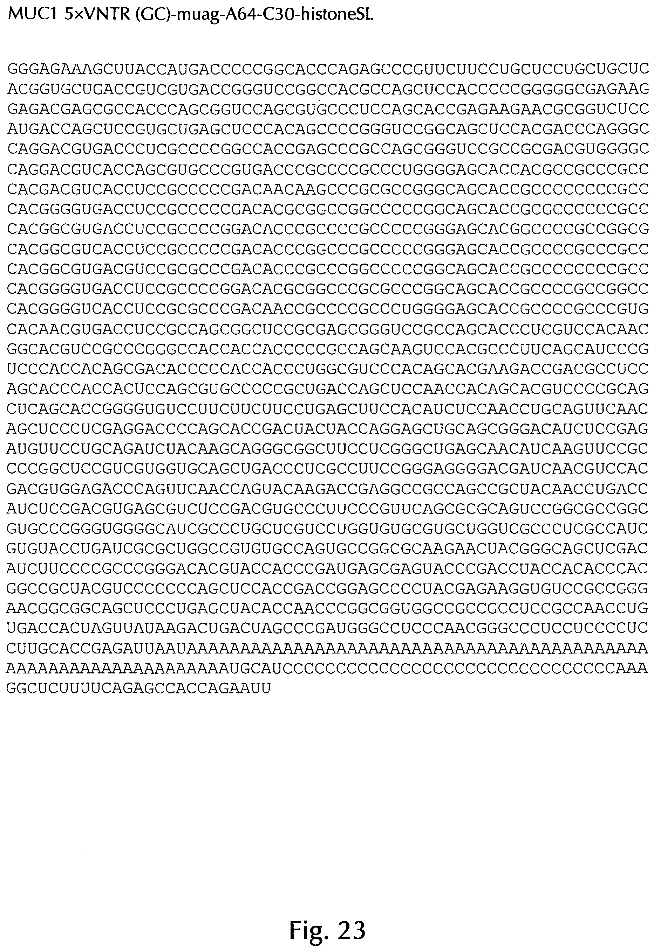

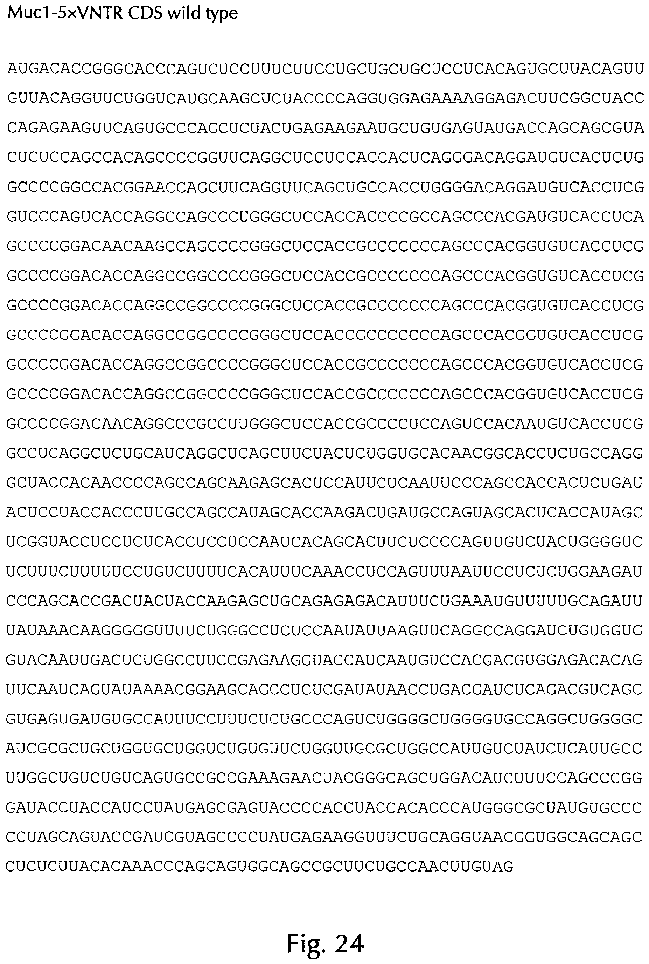

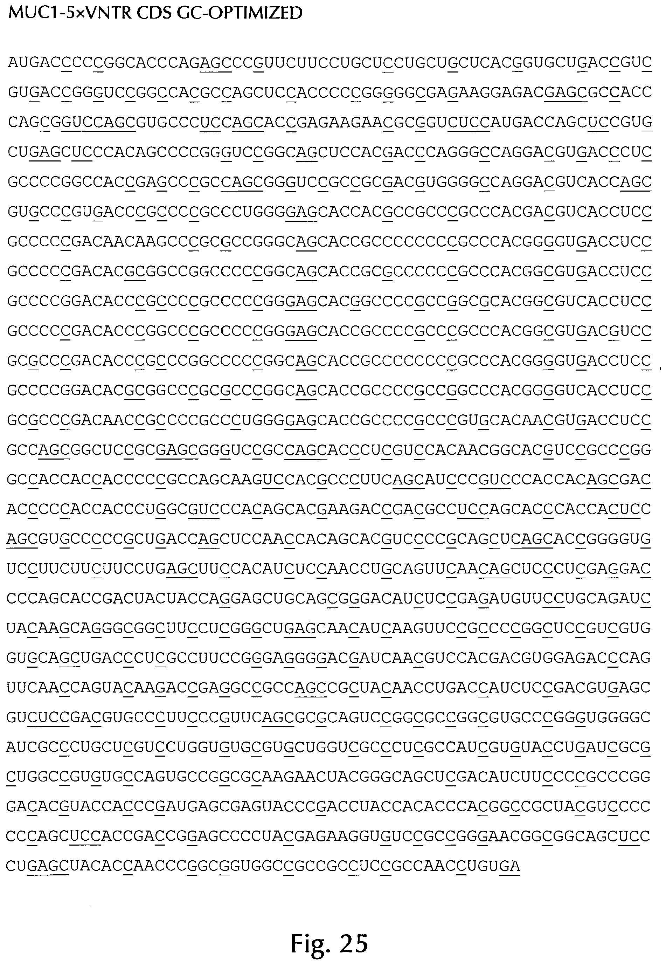

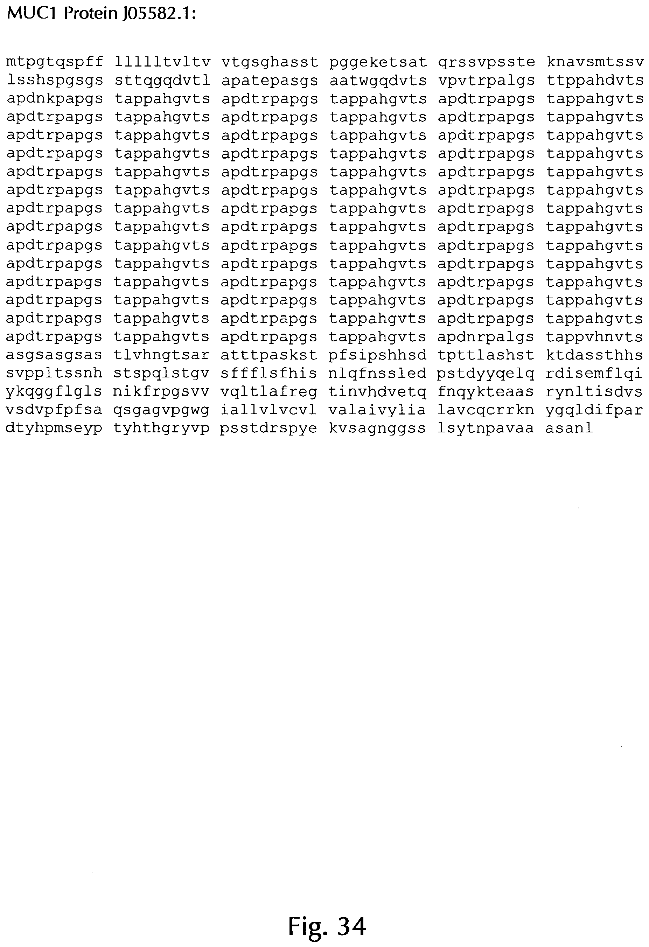

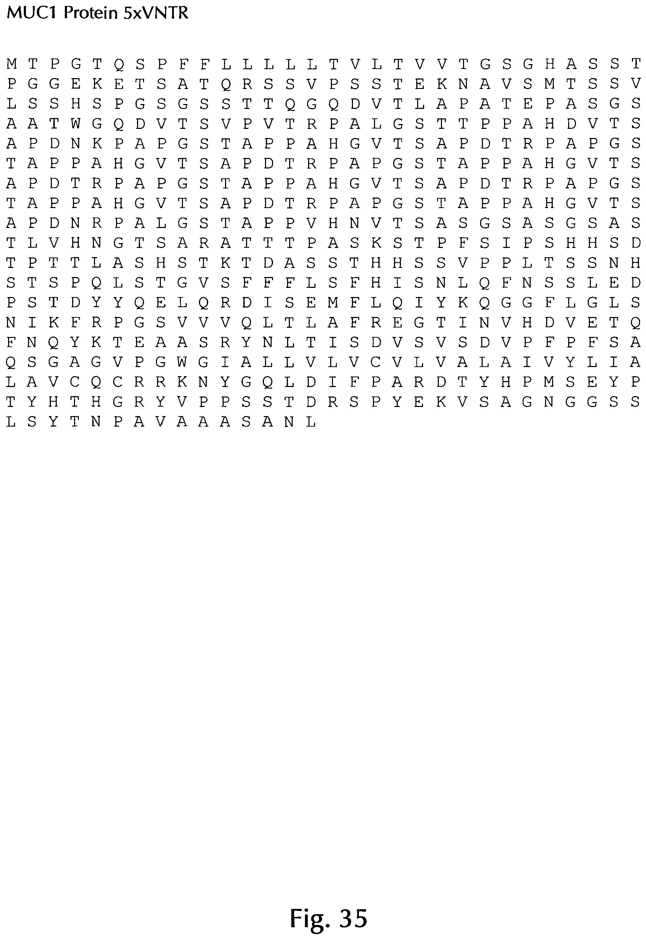

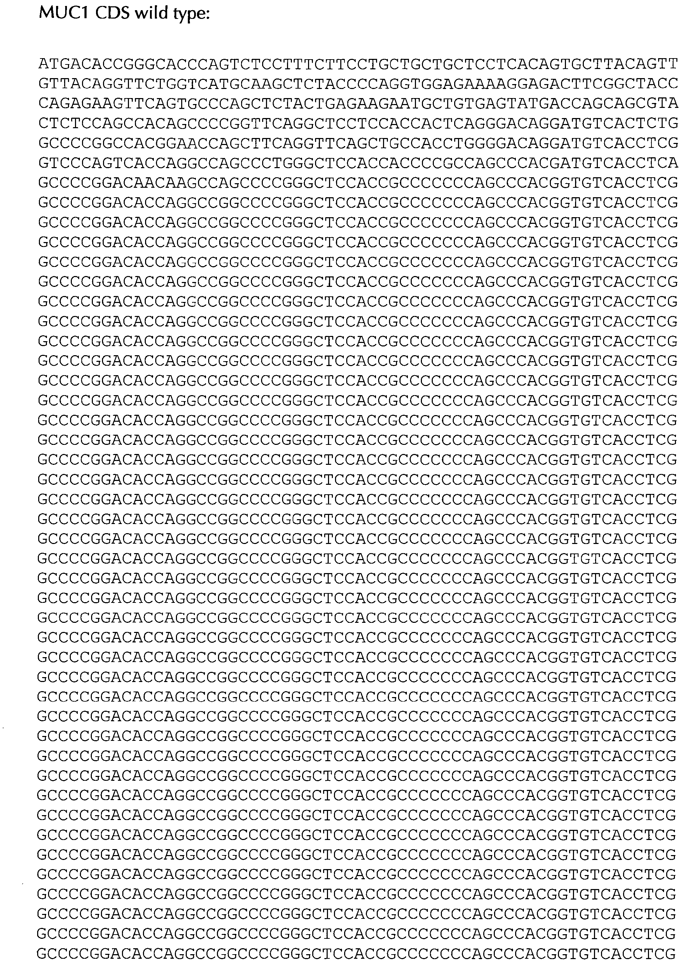

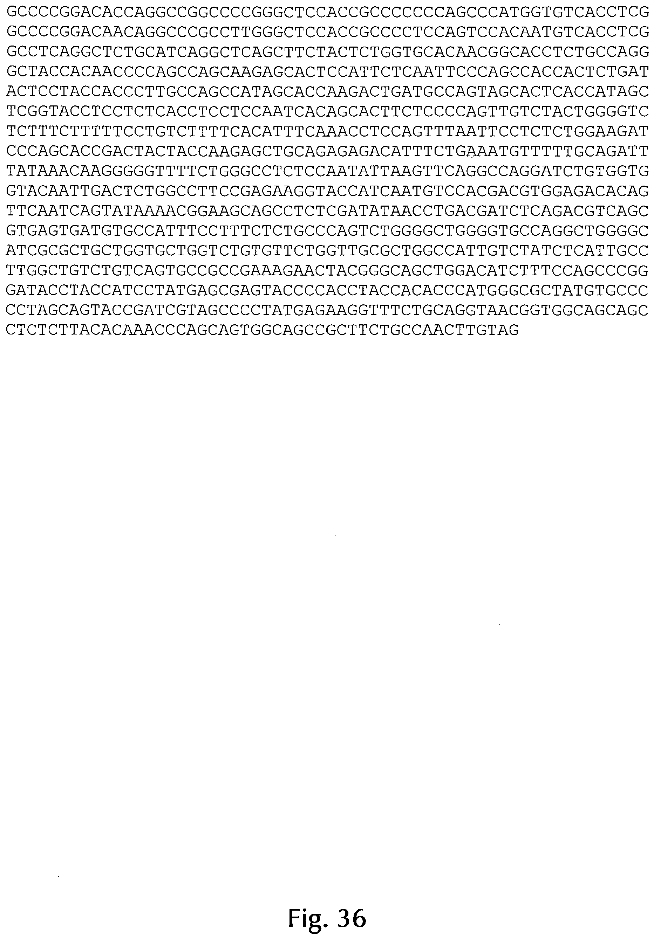

In the context of this invention, the preferred sequence of the at least one mRNA encoding MUC1 antigen may contain a coding sequence as shown in Fig. 24 or 36 (SEQ ID NO: 17 or 83), and - more preferably - as shown in Fig. 25 (SEQ ID NO: 18). Even more preferably, the at least one mRNA contains or consists of a sequence as shown in Fig. 22 or 23 (SEQ ID NO: 16 or 24). According to a further preferred embodiment, the at least one mRNA of the composition may alternatively encode a MUC1 antigen selected from a fragment, a variant or an epitope of MUC1, wherein the at least one mRNA comprises a fragment or variant of the sequence as shown in any of Figures 22, 24, 25, 23 or 36 (SEQ ID NO: 16, 17, 18, 24 or 83).
Furthermore, the at least one mRNA preferably comprises a sequence coding for an amino acid sequence as shown in Fig. 34 or 35 (SEQ ID NO: 81 or 82), or a fragment, a variant or an epitope thereof.

Where in the context of the present invention, reference is made to antigens or fragments, epitopes or variants thereof, it is understood that the reference concerns the antigen or peptide encoded by one or more of the mRNA sequences provided in the present invention. Further, antigens, antigenic proteins or antigenic peptides as defined above which are encoded by the at least one mRNA of the composition according to the present invention, may comprise fragments or variants of those sequences. Such fragments or variants may typically comprise a sequence having a sequence homology with one of the above mentioned antigens, antigenic proteins or antigenic peptides or sequences or their encoding nucleic acid sequences of at least 5%, 10%, 20%, 30%, 40%, 50%, 60%, preferably at least 70%, more preferably at least 80%, equally more preferably at least 85%, even more preferably at least 90% and most preferably at least 95% or even 97%, to the entire wild-type sequence, either on nucleic acid level or on amino acid level.

"Fragments" of antigens, antigenic proteins or antigenic peptides in the context of the present invention may comprise a sequence of an antigen, antigenic protein or antigenic peptide as defined above, which is, with regard to its amino acid sequence (or its encoded nucleic acid sequence), N-terminally, C-terminally and/or intrasequentially truncated compared to the amino acid sequence of the original (native) protein (or its encoded nucleic acid sequence). Such truncation may thus occur either on the amino acid level or correspondingly on the nucleic acid level. A sequence homology with respect to such a fragment as defined above may therefore preferably refer to the entire antigen, antigenic protein or antigenic peptide as defined above or to the entire (coding) nucleic acid sequence of such an antigen, antigenic protein or antigenic peptide.

Fragments of antigens, antigenic proteins or antigenic peptides in the context of the present invention may furthermore comprise a sequence of an antigen, antigenic protein or antigenic peptide as defined above, which has a length of about 6 to about 20 or even more amino acids, e.g. fragments as processed and presented by MHC class I molecules, preferably having a length of about 8 to about 10 amino acids, e.g. 8, 9, or 10, (or even 6, 7, 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T-cells in form of a complex consisting of the peptide fragment and an MHC molecule, i.e. the fragments are typically not recognized in their native form.

Fragments of antigens, antigenic proteins or antigenic peptides as defined herein may also comprise epitopes of those antigens, antigenic proteins or antigenic peptides. Epitopes (also called "antigen determinants") in the context of the present invention are typically fragments located on the outer surface of (native) antigens, antigenic proteins or antigenic peptides as defined herein, preferably having 5 to 15 amino acids, more preferably having 5 to 12 amino acids, even more preferably having 6 to 9 amino acids, which may be recognized by antibodies or B-cell receptors, i.e. in their native form. Such epitopes of antigens, antigenic proteins or antigenic peptides may furthermore be selected from any of the herein mentioned variants of such antigens, antigenic proteins or antigenic peptides. In this context, antigenic determinants can be conformational or discontinous epitopes, which are composed of segments of the antigens, antigenic proteins or antigenic peptides as defined herein that are discontinuous in the amino acid sequence of the antigens, antigenic proteins or antigenic peptides as defined herein but are brought together in the three-dimensional structure or continuous or linear epitopes which are composed of a single polypeptide chain.

"Variants" of antigens, antigenic proteins or antigenic peptides as defined above may be encoded by the at least one mRNA of the composition according to the present invention, wherein nucleic acids of the at least one mRNA, encoding the antigen, antigenic protein or antigenic peptide as defined above, are exchanged. Thereby, an antigen, antigenic protein or antigenic peptide may be generated having an amino acid sequence, which differs from the original sequence in one or more mutation(s), such as one or more substituted, inserted and/or deleted amino acid(s). Preferably, these fragments and/or variants have the same biological function or specific activity compared to the full-length native antigen or antigenic potein, e.g. its specific antigenic property.

The at least one mRNA of the composition according to the present invention may also encode an antigen or an antigenic protein as defined above, wherein the encoded amino acid sequence comprises conservative amino acid substitution(s) compared to its physiological sequence. Those encoded amino acid sequences as well as their encoding nucleotide sequences in particular fall under the term variants as defined above. Substitutions in which amino acids which originate from the same class are exchanged for one another are called conservative substitutions. In particular, these are amino acids having aliphatic side chains, positively or negatively charged side chains, aromatic groups in the side chains or amino acids, the side chains of which can enter into hydrogen bridges, e.g. side chains which have a hydroxyl function. This means that e.g. an amino acid having a polar side chain is replaced by another amino acid having a likewise polar side chain, or, for example, an amino acid characterized by a hydrophobic side chain is substituted by another amino acid having a likewise hydrophobic side chain (e.g. serine (threonine) by threonine (serine) or leucine (isoleucine) by isoleucine (leucine)). Insertions and substitutions are possible, in particular, at those sequence positions, which cause no modification to the three-dimensional structure or do not affect the binding region. Modifications to a three-dimensional structure by insertion(s) or deletion(s) can easily be determined e.g. using CD spectra (circular dichroism spectra) (Urry, 1985, Absorption, Circular Dichroism and ORD of Polypeptides, in: Modern Physical Methods in Biochemistry, Neuberger et al. (ed.), Elsevier, Amsterdam).

Furthermore, variants of antigens, antigenic proteins or antigenic peptides as defined above, which may be encoded by the at least one mRNA of the composition according to the present invention, may also comprise those sequences, wherein nucleic acids of the at least one mRNA are exchanged according to the degeneration of the genetic code, without leading to an alteration of respective amino acid sequence of the antigen, antigenic protein or antigenic peptide, i.e. the amino acid sequence or at least part thereof may not differ from the original sequence in one or more mutation(s) within the above meaning.

Furthermore, variants of antigens, antigenic proteins or antigenic peptides as defined above, which may be encoded by the at least one mRNA of the composition according to the present invention, may also comprise those DNA sequences, which correspond to an RNA sequence as defined herewithin and comprise further RNA sequences, which correspond to DNA sequences as defined herewithin. Those skilled in the art are familiar with the translation of an RNA sequence into a DNA sequence (or vice versa) or with the creation of the complementary strand sequence (i.e. by substitution of U residues with T residues and/or by constructing the complementary strand with respect to a given sequence).

In order to determine the percentage, to which two sequences (nucleic acid sequences, e.g. RNA or mRNA sequences as defined herein, or amino acid sequences, preferably their encoded amino acid sequences, e.g. the amino acid sequences of the antigens, antigenic proteins or antigenic peptides as defined above) are identical, the sequences can be aligned in order to be subsequently compared to one another. Therefore, e.g. gaps can be inserted into the sequence of the first sequence and the component at the corresponding position of the second sequence can be compared. If a position in the first sequence is occupied by the same component as is the case at a position in the second sequence, the two sequences are identical at this position. The percentage to which two sequences are identical is a function of the number of identical positions divided by the total number of positions. The percentage to which two sequences are identical can be determined using a mathematical algorithm. A preferred, but not limiting, example of a mathematical algorithm which can be used is the algorithm of Karlin et al. (1993), PNAS USA, 90:5873-5877 or Altschul et al. (1997), Nucleic Acids Res., 25:3389-3402. Such an algorithm is integrated in the BLAST program. Sequences which are identical to the sequences of the present invention to a certain extent can be identified by this program.

As used herein, the term "composition" refers to at least one mRNA and, optionally, further excipients. The term "composition" thus comprises any mixture of mRNAs (mRNA species) encoding the antigens as defined above, irrespective of whether the mRNAs are mono-, bi- or multicistronic. Within the meaning of the present invention, the term "composition" also refers to an embodiment consisting of a multicistronic mRNA, which encodes all six antigens as defined above. Preferably, the composition contains at least six distinct mRNA species, whereby each mRNA species encodes one of the above antigens. The term "composition" preferably relates to the at least one mRNA together with at least one other suitable substance. In general, the composition may be a pharmaceutical composition, which is designed for use in the medical field. Accordingly, the composition typically comprises at least one further excipient, which is pharmaceutically acceptable and which may be selected, for example, from carriers, vehicles and the like. The "composition" may be a liquid or a dry composition. If the composition is liquid, it will be preferably an aqueous solution or dispersion of the at least one mRNA. If the "composition" is a dry composition, it will typically be a lyophilized composition of at least one mRNA. The term "composition", as used herewithin, further refers to the at least one mRNA of the invention in combination with a further active ingredient. Preferably, the composition is an immunostimulatory composition, i.e. a composition comprising at least one component, which is able to induce an immune response or from which a component, which is able to induce an immune response, is derivable. In this context, the immune response may be the result of the adaptive and/or of the innate immune system.

The composition according to the present invention comprises at least one mRNA encoding at least six antigens as defined above, as it was found out that the specific combination of said antigens is capable of effectively stimulating the (adaptive) immune system, thus allowing treatment of lung cancer, preferably of non-small cell lung cancer (NSCLC).

In summary, the object of the present invention is solved by the provision of a composition comprising at least one mRNA coding for a novel combination of antigens as defined herein.

In a preferred embodiment, the composition comprises the six antigens (5T4 (Trophoblast glycoprotein, TPBG), Survivin (Baculoviral IAP repeat-containing protein 5; BIRC5), NY-ESO-1 (New York esophageal squamous cell carcinoma 1, CTAG1B), MAGE-C1 (Melanoma antigen family C1), MAGE-C2 (Melanoma antigen family C2), and MUC1 (Mucin 1)), which are encoded by six monocistronic mRNAs, each of these mRNAs encoding a different antigen selected from the defined group of antigens. Alternatively, the composition may comprise a combination of monocistronic, bi- and/or multicistronic mRNAs, wherein more than one of the six antigens is encoded by a bi- or multicistronic mRNA. According to the invention, any combination of mono-, bi- or multicistronic mRNAs is envisaged that encode all six antigens as defined herein, e.g. three bicistronic mRNAs, each of which encodes two of the above six antigens or two bicistronic and two monocistronic mRNAs.

According to a preferred embodiment, the composition comprises at least one mRNA, which comprises at least one coding sequence selected from RNA sequences being identical or at least 80% identical to the RNA sequence of SEQ ID NOs: 2, 5, 8, 11, 14 or 17. Even more preferably, the composition comprises six mRNAs, wherein the coding sequence in each mRNA is identical or at least 80% identical to one of the RNA sequences according to SEQ ID NOs: 2, 5, 8, 11, 14 and 17.
In a preferred embodiment, each of the at least six antigens of the composition of the present invention, may be encoded by one (monocistronic) mRNA. In other words, the composition of the present invention may contain six (monocistronic) mRNAs, wherein each of these six (monocistronic) mRNAs may encode just one antigen as defined above.

In a more preferred embodiment, the composition comprises six mRNAs, wherein one mRNA encodes 5T4 (according to SEQ ID NO: 75), one mRNA encodes Survivin (according to SEQ ID NO: 76 or 77), one mRNA encodes NY-ESO-1 (according to SEQ ID NO: 78), one mRNA encodes MAGE-C1 (according to SEQ ID NO: 79), one mRNA encodes MAGE-C2 (according to SEQ ID NO: 80) and one mRNA encodes MUC1 (according to SEQ ID NO: 81 or 82 or fragments or variants thereof, respectively.

In an even more preferred embodiment, the composition comprises six mRNAs, wherein one mRNA encodes 5T4 and comprises a coding sequence identical or at least 80% identical to SEQ ID NO: 2, one mRNA encodes Survivin and comprises a coding sequence identical or at least 80% identical to SEQ ID NO: 5, one mRNA encodes NY-ESO-1 and comprises a coding sequence identical or at least 80% identical to SEQ ID NO: 8, one mRNA encodes MAGE-C1 and comprises a coding sequence identical or at least 80% identical to SEQ ID NO: 11, one mRNA encodes MAGE-C2 and comprises a coding sequence identical or at least 80% identical to SEQ ID NO: 14 and one mRNA encodes MUC1 and comprises a coding sequence identical or at least 80% identical to SEQ ID NO:17 (or fragments or variants of each of these sequences) and optionally further excipients.

In an even more preferred embodiment, the composition comprises six mRNAs, wherein one mRNA encodes 5T4 and comprises the coding sequence according to SEQ ID NO: 2, one mRNA encodes Survivin and comprises the coding sequence according to SEQ ID NO: 5, one mRNA encodes NY-ESO-1 and comprises the coding sequence according to SEQ ID NO: 8, one mRNA encodes MAGE-C1 and comprises the coding sequence according to SEQ ID NO: 11, one mRNA encodes MAGE-C2 and comprises the coding sequence according to SEQ ID NO: 14 and one mRNA encodes MUC1 and comprises the coding sequence according to SEQ ID NO:17 or fragments thereof, respectively.

According to a particular preferred embodiment the invention, the at least one mRNA of the composition comprises a histone stem-loop in the 3' UTR region. Preferably, the composition comprises six mRNAs, wherein each mRNA comprises a histone stem-loop as defined above.

According to another particularly preferred embodiment, the composition of the present invention, may comprise (at least) one bi- or even multicistronic mRNA, i.e. (at least) one mRNA which carries the coding sequences of two or more of the six antigens according to the invention. Such coding sequences of two or more antigens of the (at least) one bi- or even multicistronic mRNA may be separated by at least one IRES (internal ribosomal entry site) sequence, as defined below. Thus, the term "encoding two or more antigens" may mean, without being limited thereto, that the (at least) one (bi- or even multicistronic) mRNA, may encode e.g. at least two, three, four, five or six (preferably different) antigens of the above mentioned antigens or their fragments or variants within the above definitions. More preferably, without being limited thereto, the (at least) one (bi- or even multicistronic) mRNA, may encode e.g. at least two, three, four, five or six (preferably different) antigens of the above mentioned antigens or their fragments or variants within the above definitions. In this context, a so-called IRES (internal ribosomal entry site) sequence as defined above can function as a sole ribosome binding site, but it can also serve to provide a bi- or even multicistronic mRNA as defined above which codes for several proteins, which are to be translated by the ribosomes independently of one another. Examples of IRES sequences which can be used according to the invention are those from picornaviruses (e.g. FMDV), pestiviruses (CFFV), polioviruses (PV), encephalomyocarditis viruses (ECMV), foot and mouth disease viruses (FMDV), hepatitis C viruses (HCV), classical swine fever viruses (CSFV), mouse leukoma virus (MLV), simian immunodeficiency viruses (SIV) or cricket paralysis viruses (CrPV).

According to a further particularly preferred embodiment, the composition of the present invention, may comprise a mixture of at least one monocistronic mRNA, as defined above, and at least one bi- or even multicistronic mRNA, as defined above. The at least one monocistronic mRNA and/or the at least one bi- or even multicistronic mRNA preferably encode different antigens or their fragments or variants within the above definitions. However, the at least one monocistronic mRNA and the at least one bi- or even multicistronic mRNA may preferably also encode (in part) identical antigens selected from the above mentioned antigens, provided that the composition of the present invention as a whole provides the six antigens as defined above. By providing multiple copies of one or more of the antigens, the relative protein amounts of said one or more antigens can be increased, i.e. the ratio between the amounts of each of the six antigens can be modulated. Such an embodiment may further be advantageous e.g. for a staggered, e.g. time dependent, administration of the composition of the present invention to a patient in need thereof. The components of such a composition of the present invention, particularly the different mRNAs encoding the at least six antigens, may be e.g. contained in (different parts of) a kit of parts composition or may be e.g. administered separately as components of different compositions according to the present invention.

Preferably, the at least one mRNA of the composition, encoding at least one of the six antigens typically comprises a length of about 50 to about 20000, or 100 to about 20000 nucleotides, preferably of about 250 to about 20000 nucleotides, more preferably of about 500 to about 10000, even more preferably of about 500 to about 5000.

According to one embodiment, the at least one mRNA of the composition, encoding at least one of the six antigens, may be in the form of a modified mRNA, wherein any modification, as defined herein, may be introduced into the at least one mRNA of the composition. Modifications as defined herein preferably lead to a stabilized at least one mRNA of the composition of the present invention.

According to one embodiment, the at least one mRNA of the composition of the present invention may thus be provided as a "stabilized mRNA", that is to say as an mRNA that is essentially resistant to in vivo degradation (e.g. by an exo- or endo-nuclease). Such stabilization can be effected, for example, by a modified phosphate backbone of the at least one mRNA of the composition of the present invention. A backbone modification in connection with the present invention is a modification in which phosphates of the backbone of the nucleotides contained in the mRNA are chemically modified. Nucleotides that may be preferably used in this connection contain e.g. a phosphorothioate-modified phosphate backbone, preferably at least one of the phosphate oxygens contained in the phosphate backbone being replaced by a sulfur atom. Stabilized mRNAs may further include, for example: non-ionic phosphate analogues, such as, for example, alkyl and aryl phosphonates, in which the charged phosphonate oxygen is replaced by an alkyl or aryl group, or phosphodiesters and alkylphosphotriesters, in which the charged oxygen residue is present in alkylated form. Such backbone modifications typically include, without implying any limitation, modifications from the group consisting of methylphosphonates, phosphoramidates and phosphorothioates (e.g. cytidine-5'-O-(1-thiophosphate)).

The at least one mRNA of the composition of the present invention may additionally or alternatively also contain sugar modifications. A sugar modification in connection with the present invention is a chemical modification of the sugar of the nucleotides of the at least one mRNA and typically includes, without implying any limitation, sugar modifications selected from the group consisting of 2'-deoxy-2'-fluoro-oligoribonucleotide (2'-fluoro-2'-deoxycytidine-5'-triphosphate, 2'-fluoro-2'-deoxyuridine-5'-triphosphate), 2'-deoxy-2'-deamine oligoribonucleotide (2'-amino-2'-deoxycytidine-5'-triphosphate, 2'-amino-2'-deoxyuridine-5'-triphosphate), 2'-O-alkyl oligoribonucleotide, 2'-deoxy-2'-C-alkyl oligoribonucleotide (2'-O-methylcytidine-5'-triphosphate, 2'-methyluridine-5'-triphosphate), 2'-C-alkyl oligoribonucleotide, and isomers thereof (2'-aracytidine-5'-triphosphate, 2'-arauridine-5'-triphosphate), or azidotriphosphate (2'-azido-2'-deoxycytidine-5'-triphosphate, 2'-azido-2'-deoxyuridine-5'-triphosphate).

The at least one mRNA of the composition of the present invention may additionally or alternatively also contain at least one base modification, which is preferably suitable for increasing the expression of the encoded protein as compared with the unaltered, i.e. natural (= native), mRNA sequence. Significant in this case means an increase in the expression of the protein compared with the expression of the native mRNA sequence by at least 20%, preferably at least 30%, 40%, 50% or 60%, more preferably by at least 70%, 80%, 90% or even 100% and most preferably by at least 150%, 200% or even 300% or more. In connection with the present invention, a nucleotide having such a base modification is preferably selected from the group of the base-modified nucleotides consisting of 2-amino-6-chloropurineriboside-5'-triphosphate, 2-aminoadenosine-5'-triphosphate, 2-thiocytidine-5'-triphosphate, 2-thiouridine-5'-triphosphate, 4-thiouridine-5'-triphosphate, 5-aminoallylcytidine-5'-triphosphate, 5-aminoallyluridine-5'-triphosphate, 5-bromocytidine-5'-triphosphate, 5-bromouridine-5'-triphosphate, 5-iodocytidine-5'-triphosphate, 5-iodouridine-5'-triphosphate, 5-methylcytidine-5'-triphosphate, 5-methyluridine-5'-triphosphate, 6-azacytidine-5'-triphosphate, 6-azauridine-5'-triphosphate, 6-chloropurineriboside-5'-triphosphate, 7-deazaadenosine-5'-triphosphate, 7-deazaguanosine-5'-triphosphate, 8-azaadenosine-5'-triphosphate, 8-azidoadenosine-5'-triphosphate, benzimidazole-riboside-5'-triphosphate, N1-methyladenosine-5'-triphosphate, N1-methylguanosine-5'-triphosphate, N6-methyladenosine-5'-triphosphate, O6-methylguanosine-5'-triphosphate, pseudouridine-5'-triphosphate, or puromycin-5'-triphosphate, xanthosine-5'-triphosphate. Particular preference is given to nucleotides for base modifications selected from the group of base-modified nucleotides consisting of 5-methylcytidine-5'-triphosphate, 7-deazaguanosine-5'-triphosphate, 5-bromocytidine-5'-triphosphate, and pseudouridine-5'-triphosphate.

According to another embodiment, the at least one mRNA of the composition of the present invention can likewise be modified (and preferably stabilized) by introducing further modified nucleotides containing modifications of their ribose or base moieties. Generally, the at least one mRNA of the composition of the present invention may contain any native (= naturally occurring) nucleotide, e.g. guanosine, uracil, adenosine, and/or cytosine or an analogue thereof. In this connection, nucleotide analogues are defined as non-natively occurring variants of naturally occurring nucleotides. Accordingly, analogues are chemically derivatized nucleotides with non-natively occurring functional groups, which are preferably added to or deleted from the naturally occurring nucleotide or which substitute the naturally occurring functional groups of a nucleotide. Accordingly, each component of the naturally occurring nucleotide may be modified, namely the base component, the sugar (ribose) component and/or the phosphate component forming the backbone (see above) of the RNA sequence. Analogues of guanosine, uracil, adenosine, and cytosine include, without implying any limitation, any naturally occurring or non-naturally occurring guanosine, uracil, adenosine, thymidine or cytosine that has been altered chemically, for example by acetylation, methylation, hydroxylation, etc., including 1-methyl-adenosine, 1-methyl-guanosine, 1-methyl-inosine, 2,2-dimethyl-guanosine, 2,6-diaminopurine, 2'-Amino-2'-deoxyadenosine, 2'-Amino-2'-deoxycytidine, 2'-Amino-2'-deoxyguanosine, 2'-Amino-2'-deoxyuridine, 2-Amino-6-chloropurineriboside, 2-Aminopurine-riboside, 2'-Araadenosine, 2'-Aracytidine, 2'-Arauridine, 2'-Azido-2'-deoxyadenosine, 2'-Azido-2'-deoxycytidine, 2'-Azido-2'-deoxyguanosine, 2'-Azido-2'-deoxyuridine, 2-Chloroadenosine, 2'-Fluoro-2'-deoxyadenosine, 2'-Fluoro-2'-deoxycytidine, 2'-Fluoro-2'-deoxyguanosine, 2'-Fluoro-2'-deoxyuridine, 2'-Fluorothymidine, 2-methyl-adenosine, 2-methyl-guanosine, 2-methyl-thio-N6-isopenenyladenosine, 2'-O-Methyl-2-aminoadenosine, 2'-O-Methyl-2'-deoxyadenosine, 2'-O-Methyl-2'-deoxycytidine, 2'-O-Methyl-2'-deoxyguanosine, 2'-O-Methyl-2'-deoxyuridine, 2'-O-Methyl-5-methyluridine, 2'-O-Methylinosine, 2'-O-Methylpseudouridine, 2-Thiocytidine, 2-thio-cytosine, 3-methyl-cytosine, 4-acetyl-cytosine, 4-Thiouridine, 5-(carboxyhydroxymethyl)-uracil, 5,6-Dihydrouridine, 5-Aminoallylcytidine, 5-Aminoallyldeoxy-uridine, 5-Bromouridine, 5-carboxymehtylaminomethyl-2-thio-uracil, 5-carboxymethylamonomethyl-uracil, 5-Chloro-Ara-cytosine, 5-Fluoro-uridine, 5-lodouridine, 5-methoxycarbonylmethyl-uridine, 5-methoxy-uridine, 5-methyl-2-thio-uridine, 6-Azacytidine, 6-Azauridine, 6-Chloro-7-deaza-guanosine, 6-Chloropurineriboside, 6-Mercapto-guanosine, 6-Methyl-mercaptopurine-riboside, 7-Deaza-2'-deoxy-guanosine, 7-Deazaadenosine, 7-methyl-guanosine, 8-Azaadenosine, 8-Bromo-adenosine, 8-Bromoguanosine, 8-Mercapto-guanosine, 8-Oxoguanosine, Benzimidazole-riboside, Beta-D-mannosyl-queosine, Dihydro-uracil, Inosine, N1-Methyladenosine, N6-([6-Aminohexyl]carbamoylmethyl)-adenosine, N6-isopentenyl-adenosine, N6-methyl-adenosine, N7-Methyl-xanthosine, N-uracil-5-oxyacetic acid methyl ester, Puromycin, Queosine, Uracil-5-oxyacetic acid, Uracil-5-oxyacetic acid methyl ester, Wybutoxosine, Xanthosine, and Xylo-adenosine. The preparation of such analogues is known to a person skilled in the art, for example from US Patents 4,373,071, US 4,401,796, US 4,415,732, US 4,458,066, US 4,500,707, US 4,668,777, US 4,973,679, US 5,047,524, US 5,132,418, US 5,153,319, US 5,262,530 and 5,700,642. In the case of an analogue as described above, particular preference may be given according to the invention to those analogues that increase the immunogenicity of the mRNA of the inventive composition and/or do not interfere with a further modification of the mRNA that has been introduced.

According to a particular embodiment, the at least one mRNA of the composition of the present invention can contain a lipid modification. Such a lipid-modified mRNA typically comprises an mRNA as defined herein, encoding at least one of the six antigens as defined above. Such a lipid-modified mRNA typically further comprises at least one linker covalently linked with that mRNA, and at least one lipid covalently linked with the respective linker. Alternatively, the lipid-modified mRNA comprises an (at least one) mRNA as defined herein and at least one (bifunctional) lipid covalently linked (without a linker) with that mRNA. According to a third alternative, the lipid-modified mRNA comprises an mRNA as defined herein, at least one linker covalently linked with that mRNA, and at least one lipid covalently linked with the respective linker, and also at least one (bifunctional) lipid covalently linked (without a linker) with that mRNA.

The lipid contained in the at least one mRNA of the inventive composition (complexed or covalently bound thereto) is typically a lipid or a lipophilic residue that preferably is itself biologically active. Such lipids preferably include natural substances or compounds such as, for example, vitamins, e.g. alpha-tocopherol (vitamin E), including RRR-alpha-tocopherol (formerly D-alpha-tocopherol), L-alpha-tocopherol, the racemate D,L-alpha-tocopherol, vitamin E succinate (VES), or vitamin A and its derivatives, e.g. retinoic acid, retinol, vitamin D and its derivatives, e.g. vitamin D and also the ergosterol precursors thereof, vitamin E and its derivatives, vitamin K and its derivatives, e.g. vitamin K and related quinone or phytol compounds, or steroids, such as bile acids, for example cholic acid, deoxycholic acid, dehydrocholic acid, cortisone, digoxygenin, testosterone, cholesterol or thiocholesterol. Further lipids or lipophilic residues within the scope of the present invention include, without implying any limitation, polyalkylene glycols (Oberhauser et al., Nucl. Acids Res., 1992, 20, 533), aliphatic groups such as, for example, C1-C20-alkanes, C1-C20-alkenes or C1-C20-alkanol compounds, etc., such as, for example, dodecanediol, hexadecanol or undecyl residues (Saison-Behmoaras et al., EMBO J, 1991, 10, 111; Kabanov et al., FEBS Lett., 1990, 259, 327; Svinarchuk et al., Biochimie, 1993, 75, 49), phospholipids such as, for example, phosphatidylglycerol, diacylphosphatidylglycerol, phosphatidylcholine, dipalmitoylphosphatidylcholine, distearoylphosphatidylcholine, phosphatidylserine, phosphatidylethanolamine, di-hexadecyl-rac-glycerol, sphingolipids, cerebrosides, gangliosides, or triethylammonium 1,2-di-O-hexadecyl-rac-glycero-3-H-phosphonate (Manoharan et al., Tetrahedron Lett., 1995, 36, 3651; Shea et al., Nucl. Acids Res., 1990, 18, 3777), polyamines or polyalkylene glycols, such as, for example, polyethylene glycol (PEG) (Manoharan et al., Nucleosides & Nucleotides, 1995, 14, 969), hexaethylene glycol (HEG), palmitin or palmityl residues (Mishra et al., Biochim. Biophys. Acta, 1995, 1264, 229), octadecylamines or hexylamino-carbonyl-oxycholesterol residues (Crooke et al., J. Pharmacol. Exp. Ther., 1996, 277, 923), and also waxes, terpenes, alicyclic hydrocarbons, saturated and mono- or poly-unsaturated fatty acid residues, etc..

The at least one mRNA of the composition of the present invention may likewise be stabilized in order to prevent degradation of the mRNA in vivo by various approaches. It is known in the art that instability and (fast) degradation of mRNA or of RNA in vivo in general may represent a serious problem in the application of RNA based compositions. This instability of RNA is typically due to RNA-degrading enzymes, "RNases" (ribonucleases), wherein contamination with such ribonucleases may sometimes completely degrade RNA in solution. Accordingly, the natural degradation of mRNA in the cytoplasm of cells is very finely regulated and RNase contaminations may be generally removed by special treatment prior to use of said sompositions, in particular with diethyl pyrocarbonate (DEPC). A number of mechanisms of natural degradation are known in this connection in the prior art, which may be utilized as well. E.g., the terminal structure is typically of critical importance for a mRNA in vivo. As an example, at the 5' end of naturally occurring mRNAs there is usually a so-called "cap structure" (a modified guanosine nucleotide), and at the 3' end is typically a sequence of up to 200 adenosine nucleotides (the so-called poly-A tail).

The at least one mRNA of the composition of the present invention can therefore be stabilized against degradation by RNases by the addition of a so-called "5' cap" structure. Particular preference is given in this connection to an m7G(5')ppp (5'(A,G(5')ppp(5')A or G(5')ppp(5')G as the 5' cap" structure. However, such a modification is introduced only if a modification, for example a lipid modification, has not already been introduced at the 5' end of the mRNA of the inventive composition or if the modification does not interfere with the immunogenic properties of the (unmodified or chemically modified) mRNA.

According to a further preferred embodiment, the at least one mRNA of the composition of the present invention may contain a poly-A tail on the 3' terminus of typically about 10 to 200 adenosine nucleotides, preferably about 10 to 100 adenosine nucleotides, more preferably about 40 to 80 adenosine nucleotides or even more preferably about 50 to 70 adenosine nucleotides.

According to a further preferred embodiment, the at least one mRNA of the composition of the present invention may contain a poly-C tail on the 3' terminus of typically about 10 to 200 cytosine nucleotides, preferably about 10 to 100 cytosine nucleotides, more preferably about 20 to 70 cytosine nucleotides or even more preferably about 20 to 60 or even 10 to 40 cytosine nucleotides.

The at least one mRNA according to the invention preferably comprises at least one histone stem-loop. In the context of the present invention, such a histone stem-loop, in general (irrespective of whether it is a histone stem loop or not), is typically derived from histone genes and comprises an intramolecular base pairing of two neighboring entirely or partially reverse complementary sequences, thereby forming a stem-loop. A stem-loop can occur in single-stranded DNA or, more commonly, in RNA.

In the context of the present application, a histone stem-loop sequence may be described by its DNA or by its corresponding RNA sequence. Thus, any reference - throughout the present application - to histone stem-loop sequences, which are represented herein by DNA sequences (e.g. SEQ ID NO: 38 to 67 and 71), also comprises the corresponding RNA sequence. This applies in particular to histone stem-loop sequences, which are comprised in the at least one mRNA according to the invention. Accordingly, by reference to a specific DNA sequence that defines a histone stem-loop, the corresponding RNA sequence is defined as well.

The structure is also known as a hairpin or hairpin loop and usually consists of a stem and a (terminal) loop within a consecutive sequence, wherein the stem is formed by two neighbored entirely or partially reverse complementary sequences separated by a short sequence as sort of spacer, which builds the loop of the stem-loop structure. The two neighbored entirely or partially reverse complementary sequences may be defined as e.g. stem loop elements stem1 and stem2. The stem loop is formed when these two neighbored entirely or partially reverse complementary sequences, e.g. stem loop elements stem1 and stem2, form base-pairs with each other, leading to a double stranded nucleic acid sequence stretch comprising an unpaired loop at its terminal ending formed by the short sequence located between stem loop elements stem1 and stem2 on the consecutive sequence. The unpaired loop thereby typically represents a region of the nucleic acid which is not capable of base pairing with either of these stem loop elements. The resulting lollipop-shaped structure is a key building block of many RNA secondary structures. The formation of a stem-loop structure is thus dependent on the stability of the resulting stem and loop regions, wherein the first prerequisite is typically the presence of a sequence that can fold back on itself to form a paired double strand. The stability of paired stem loop elements is determined by the length, the number of mismatches or bulges it contains (a small number of mismatches is typically tolerable, especially in a long double stranded stretch), and the base composition of the paired region. In the context of the present invention, a loop length of 3 to 15 bases is conceivable, while a more preferred loop length is 3-10 bases, more preferably 3 to 8, 3 to 7, 3 to 6 or even more preferably 4 to 5 bases, and most preferably 4 bases. The sequence forming the stem region in the histone stem-loop typically has a length of between 5 to 10 bases, more preferably, between 5 to 8 bases, wherein preferably at least one of the bases represents a mismatch, i.e. does not base pair.

In the context of the present invention, a histone stem-loop is typically derived from histone genes (e.g. genes from the histone families H1, H2A, H2B, H3, H4) and comprises an intramolecular base pairing of two neighbored entirely or partially reverse complementary sequences, thereby forming a stem-loop. Typically, a histone 3' UTR stem-loop is an RNA element involved in nucleocytoplasmic transport of the histone mRNAs, and in the regulation of stability and of translation efficiency in the cytoplasm. The mRNAs of metazoan histone genes lack polyadenylation and a poly-A tail, instead 3' end processing occurs at a site between this highly conserved stem-loop and a purine rich region around 20 nucleotides downstream (the histone downstream element, or HDE). The histone stem-loop is bound by a 31 kDa stem-loop binding protein (SLBP - also termed the histone hairpin binding protein, or HBP). Such histone stem-loop structures are preferably employed by the present invention in combination with other sequence elements and structures, which do not occur naturally (which means in untransformed living organisms/cells) in histone genes, but are combined - according to the invention - to provide an artificial, heterologous nucleic acid. Accordingly, the present invention provides an artificial (non-native) combination of a histone stem-loop structure with other heterologous sequence elements, which do not occur in histone genes or metazoan histone genes and are isolated from operational and/or regulatory sequence regions (influencing transcription and/or translation) of genes coding for proteins other than histones, provide advantageous effects. Accordingly, one embodiment of the invention provides the combination of a histone stem-loop structure with a poly(A) sequence or a sequence representing a polyadenylation signal (3'-terminal of a coding region), which does not occur in metazoan histone genes. According to another preferred aspect of the invention, a combination of a histone stem-loop structure with a coding region coding for at least one of the antigens according to the invention as defined above, which does, preferably not occur in metazoan histone genes, is provided herewith (coding region and histone stem loop sequence are heterologous).

A histone stem loop is, therefore, a stem-loop structure as described herein, which, if preferably functionally defined, exhibits/retains the property of binding to its natural binding partner, the stem-loop binding protein (SLBP - also termed the histone hairpin binding protein, or HBP).

In a preferred embodiment, the histone stem loop sequence is not derived from a mouse histone protein. More specifically, the histone stem loop sequence may not be derived from mouse histone gene H2A614. Also, the at least one mRNA according to the invention may neither contain a mouse histone stem loop sequence nor contain mouse histone gene H2A614. Further, the at least one mRNA according to the invention may not contain a stem-loop processing signal, more specifically, a mouse histone processing signal and, most specifically, may not contain mouse stem loop processing signal H2kA614, even if the at least one mRNA contains at least one mammalian histone gene. However, the at least one mammalian histone gene may not be Seq. ID No. 7 of WO 01/12824.

The at least one mRNA as define above, may preferably comprise a 5' UTR, a coding region encoding the antigens as defined above or a fragment, variant or derivative thereof; and/or a 3' UTR preferably containing at least one histone stem-loop. When in addition to the antigens defined above, a further peptide or protein is encoded by the at least one mRNA, then the encoded peptide or protein is preferably no histone protein, no reporter protein and/or no marker or selection protein, as defined above. The 3' UTR of the at least one mRNA preferably comprises also a poly(A) and/or a poly(C) sequence as defined herewithin. The single elements of the 3' UTR may occur therein in any order from 5' to 3' along the sequence of the at least one mRNA. In addition, further elements as described herein, may also be contained, such as a stabilizing sequence as defined herewithin (e.g. derived from the UTR of a globin gene), IRES sequences, etc. Each of the elements may also be repeated in the at least one mRNA according to the invention at least once (particularly in di- or multicistronic constructs), preferably twice or more. As an example, the single elements may be present in the at least one mRNA in the following order:
5' - coding region - histone stem-loop - poly(A)/(C) sequence - 3'; or
5' - coding region - poly(A)/(C) sequence - histone stem-loop - 3'; or
5' - coding region - histone stem-loop - polyadenylation signal - 3'; or
5' - coding region - polyadenylation signal- histone stem-loop - 3'; or

5' - coding region - histone stem-loop - histone stem-loop - poly(A)/(C) sequence - 3'; or
5' - coding region - histone stem-loop - histone stem-loop - polyadenylation signal- 3'; or
5' - coding region - stabilizing sequence - poly(A)/(C) sequence - histone stem-loop - 3'; or
5' - coding region - stabilizing sequence - poly(A)/(C) sequence - poly(A)/(C) sequence - histone stem-loop - 3'; etc.

In this context, it is particularly preferred that - if, in addition to the antigens defined above, a further peptide or protein is encoded by the at least one mRNA - the encoded peptide or protein is preferably no histone protein, no reporter protein (e.g. Luciferase, GFP, EGFP, β-Galactosidase, particularly EGFP) and/or no marker or selection protein (e.g. alpha-Globin, Galactokinase and Xanthine:Guanine phosphoribosyl transferase (GPT)).
In a preferred embodiment, the mRNA according to the invention does not comprise a reporter gene or a marker gene. Preferably, the mRNA according to the invention does not encode, for instance, luciferase; green fluorescent protein (GFP) and its variants (such as eGFP, RFP or BFP); α-globin; hypoxanthine-guanine phosphoribosyltransferase (HGPRT); β-galactosidase; galactokinase; alkaline phosphatase; secreted embryonic alkaline phosphatase (SEAP)) or a resistance gene (such as a resistance gene against neomycin, puromycin, hygromycin and zeocin). In a preferred embodiment, the mRNA according to the invention does not encode luciferase. In another embodiment, the mRNA according to the invention does not encode GFP or a variant thereof.

In a further preferred embodiment, the mRNA according to the invention does not encode a protein (or a fragment of a protein) derived from a virus, preferably from a virus belonging to the family of Orthomyxoviridae. Preferably the mRNA does not encode a protein that is derived from an influenza virus, more preferably an influenza A virus. Preferably, the mRNA according to the invention does not encode an influenza A protein selected from the group consisting of hemagglutinin (HA), neuraminidase (NA), nucleoprotein (NP), M1, M2, NS1, NS2 (NEP: nuclear export protein), PA, PB1 (polymerase basic 1), PB1-F2 and PB2. In another preferred embodiment, the mRNA according to the invention does not encode ovalbumin (OVA) or a fragment thereof. Preferably, the mRNA according to the invention does not encode an influenza A protein or ovalbumin.

According to one preferred embodiment, the at least one mRNA according to the invention comprises at least one histone stem-loop sequence, preferably according to at least one of the following formulae (I) or (II):
formula (I) (stem-loop sequence without stem bordering elements): 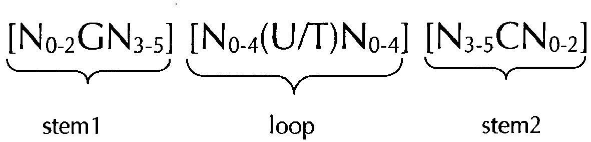
formula (II) (stem-loop sequence with stem bordering elements): 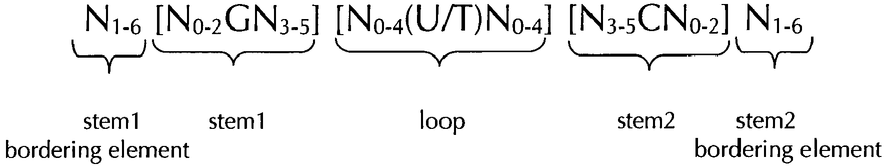 wherein:
   - stem1 or stem2 bordering elements N₁₋₆: is a consecutive sequence of 1 to 6, preferably of 2 to 6, more preferably of 2 to 5, even more preferably of 3 to 5, most preferably of 4 to 5 or 5 N, wherein each N is independently from another selected from a nucleotide selected from A, U, T, G and C, or a nucleotide analogue thereof;
   - stem1 [N₀₋₂GN₃₋₅]: is reverse complementary or partially reverse complementary with element stem2, and is a consecutive sequence between of 5 to 7 nucleotides;
   wherein N₀₋₂ is a consecutive sequence of 0 to 2, preferably of 0 to 1, more preferably of 1 N, wherein each N is independently from another selected from a nucleotide selected from A, U, T, G and C or a nucleotide analogue thereof;
   wherein N₃₋₅ is a consecutive sequence of 3 to 5, preferably of 4 to 5, more preferably of 4 N, wherein each N is independently from another selected from a nucleotide selected from A, U, T, G and C or a nucleotide analogue thereof, and
   wherein G is guanosine or an analogue thereof, and may be optionally replaced by a cytidine or an analogue thereof, provided that its complementary nucleotide cytidine in stem2 is replaced by guanosine;
   - loop sequence [N₀₋₄(U/T)N₀₋₄]: is located between elements stem1 and stem2, and is a consecutive sequence of 3 to 5 nucleotides, more preferably of 4 nucleotides;
   wherein each N₀₋₄ is independent from another a consecutive sequence of 0 to 4, preferably of 1 to 3, more preferably of 1 to 2 N, wherein each N is independently from another selected from a nucleotide selected from A, U, T, G and C or a nucleotide analogue thereof; and wherein U/T represents uridine, or optionally thymidine;
   - stem2 [N₃₋₅CN₀₋₂]: is reverse complementary or partially reverse complementary with element stem1, and is a consecutive sequence between of 5 to 7 nucleotides;
   wherein N₃₋₅ is a consecutive sequence of 3 to 5, preferably of 4 to 5, more preferably of 4 N, wherein each N is independently from another selected from a nucleotide selected from A, U, T, G and C or a nucleotide analogue thereof;
   wherein N₀₋₂ is a consecutive sequence of 0 to 2, preferably of 0 to 1, more preferably of 1 N, wherein each N is independently from another selected from a nucleotide selected from A, U, T, G or C or a nucleotide analogue thereof; and
   wherein C is cytidine or an analogue thereof, and may be optionally replaced by a guanosine or an analogue thereof provided that its complementary nucleotide guanosine in stem1 is replaced by cytidine;
wherein
stem1 and stem2 are capable of base pairing with each other forming a reverse complementary sequence, wherein base pairing may occur between stem1 and stem2, e.g. by Watson-Crick base pairing of nucleotides A and U/T or G and C or by non-Watson-Crick base pairing e.g. wobble base pairing, reverse Watson-Crick base pairing, Hoogsteen base pairing, reverse Hoogsteen base pairing or are capable of base pairing with each other forming a partially reverse complementary sequence, wherein an incomplete base pairing may occur between stem1 and stem2, on the basis that one or more bases in one stem do not have a complementary base in the reverse complementary sequence of the other stem.

In the above context, a wobble base pairing is typically a non-Watson-Crick base pairing between two nucleotides. The four main wobble base pairs in the present context, which may be used, are guanosine-uridine, inosine-uridine, inosine-adenosine, inosine-cytidine (G-U/T, I-U/T, I-A and I-C) and adenosine-cytidine (A-C).

Accordingly, in the context of the present invention, a wobble base is a base, which forms a wobble base pair with a further base as described above. Therefore non-Watson-Crick base pairing, e.g. wobble base pairing, may occur in the stem of the histone stem-loop structure in the at least one mRNA according to the present invention.

In the above context, a partially reverse complementary sequence comprises maximally 2, preferably only one mismatch in the stem-structure of the stem-loop sequence formed by base pairing of stem1 and stem2. In other words, stem1 and stem2 are preferably capable of (full) base pairing with each other throughout the entire sequence of stem1 and stem2 (100% of possible correct Watson-Crick or non-Watson-Crick base pairings), thereby forming a reverse complementary sequence, wherein each base has its correct Watson-Crick or non-Watson-Crick base pendant as a complementary binding partner. Alternatively, stem1 and stem2 are preferably capable of partial base pairing with each other throughout the entire sequence of stem1 and stem2, wherein at least about 70%, 75%, 80%, 85%, 90%, or 95% of the 100% possible correct Watson-Crick or non-Watson-Crick base pairings are occupied with the correct Watson-Crick or non-Watson-Crick base pairings and at most about 30%, 25%, 20%, 15%, 10%, or 5% of the remaining bases are unpaired.

According to a preferred embodiment, the at least one histone stem-loop sequence (with stem bordering elements) of the at least one mRNA as defined herein comprises a length of about 15 to about 45 nucleotides, preferably a length of about 15 to about 40 nucleotides, preferably a length of about 15 to about 35 nucleotides, preferably a length of about 15 to about 30 nucleotides and even more preferably a length of about 20 to about 30 and most preferably a length of about 24 to about 28 nucleotides.

According to a further preferred embodiment, the at least one histone stem-loop sequence (without stem bordering elements) of the the at least one mRNA as defined herein comprises a length of about 10 to about 30 nucleotides, preferably a length of about 10 to about 20 nucleotides, preferably a length of about 12 to about 20 nucleotides, preferably a length of about 14 to about 20 nucleotides and even more preferably a length of about 16 to about 17 and most preferably a length of about 16 nucleotides.

According to a further preferred embodiment, the at least one mRNA according to the present invention may comprise at least one histone stem-loop sequence according to at least one of the following specific formulae (Ia) or (IIa):
formula (Ia) (stem-loop sequence without stem bordering elements): 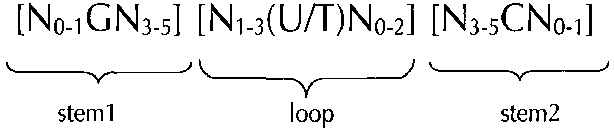
formula (IIa) (stem-loop sequence with stem bordering elements): 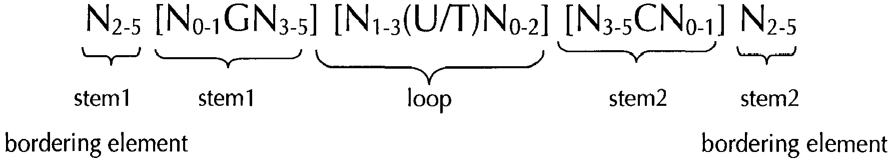 wherein:
   - N, C, G, T and U: are as defined above.

According to a further more particularly preferred embodiment of the first aspect, the at least one RNA may comprise or code for at least one histone stem-loop sequence according to at least one of the following specific formulae (Ib) or (IIb):
formula (Ib) (stem-loop sequence without stem bordering elements): 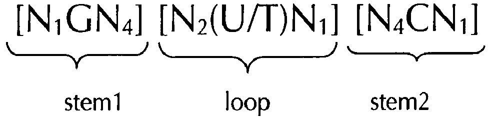
formula (IIb) (stem-loop sequence with stem bordering elements): 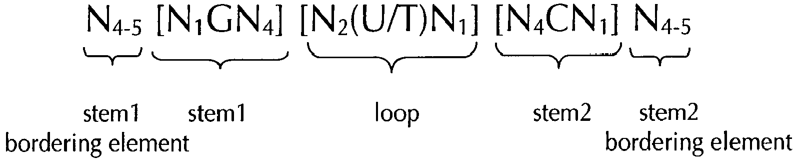 wherein:
   - N, C, G, T and U: are as defined above.

According to an even more preferred embodiment, the at least one mRNA according to the present invention may comprise at least one histone stem-loop sequence according to at least one of the following specific formulae (Ic) to (Ih) or (IIc) to (IIh), shown alternatively in its stem-loop structure and as a linear sequence representing histone stem-loop sequences:
formula (Ic): (metazoan and protozoan histone stem-loop consensus sequence without stem bordering elements): 
N U
N     N
N-N
N-N
N-N
N-N
G-C
N-N (stem-loop structure)
NGNNNNNNUNNNNNCN
(linear sequence) (SEQ ID NO: 26)
 formula (IIc): (metazoan and protozoan histone stem-loop consensus sequence with stem bordering elements): 
N U
N N
N-N
N-N
N-N
N-N
G-C
N*N*NNNN-NNNN*N*N* (stem-loop structure)
N*N*NNNNGNNNNNNUNNNNNCNNNN*N*N*
(linear sequence) (SEQ ID NO: 27)
 formula (Id): (without stem bordering elements) 
N U
N N
N-N
N-N
N-N
N-N
C-G
N-N (stem-loop structure)
NCNNNNNNUNNNNNGN
(linear sequence) (SEQ ID NO: 28)
 formula (IId): (with stem bordering elements) 
N U
N N
N-N
N-N
N-N
N-N
C-G
N*N*NNNN-NNNN*N*N* (stem-loop structure)
N*N*NNNNCNNNNNNUNNNNNGNNNN*N*N*
(linear sequence) (SEQ ID NO: 29)
 formula (Ie): (protozoan histone stem-loop consensus sequence without stem bordering elements) 
N U
N N
N-N
N-N
N-N
N-N
G-C
D-H (stem-loop structure)
DGNNNNNNUNNNNNCH
(linear sequence) (SEQ ID NO: 30)
 formula (IIe): (protozoan histone stem-loop consensus sequence with stem bordering elements) 
N U
N N
N-N
N-N
N-N
N-N
G-C
N*N*NNND-HNNN*N*N* (stem-loop structure)
N*N*NNNDGNNNNNNUNNNNNCHNNN*N*N*
(linear sequence) (SEQ ID NO: 31)
 formula (If): (metazoan histone stem-loop consensus sequence without stem bordering elements) 
N U
N N
Y-V
Y-N
B-D
N-N
G-C
N-N (stem-loop structure)
NGNBYYNNUNVNDNCN
(linear sequence) (SEQ ID NO: 32)
 formula (IIf): (metazoan histone stem-loop consensus sequence with stem bordering elements) 
N U
N N
Y-V
Y-N
B-D
N-N
G-C
N*N*NNNN-NNNN*N*N* (stem-loop structure)
N*N*NNNNGNBYYNNUNVDNCNNNN*N*N*
(linear sequence) (SEQ ID NO: 33)
 formula (Ig): (vertebrate histone stem-loop consensus sequence without stem bordering elements) 
N U
D H
Y-A
Y-B
Y-R
H-D
G-C
N-N (stem-loop structure)
NGHYYYDNUHABRDCN
(linear sequence) (SEQ ID NO: 34)
 formula (IIg): (vertebrate histone stem-loop consensus sequence with stem bordering elements) 
N U
D H
Y-A
Y-B
Y-R
H-D
G-C
N*N*HNNN-NNNN*N*H* (stem-loop structure)
N*N*HNNNGHYYYDNUHABRDCNNNN*N*H*
(linear sequence) (SEQ ID NO: 35)
 formula (Ih): (human histone stem-loop consensus sequence (Homo sapiens) without stem bordering elements) 
Y U
D H
U-A
C-S
Y-R
H-R
G-C
D-C (stem-loop structure)
DGHYCUDYUHASRRCC
(linear sequence) (SEQ ID NO: 36)
 formula (IIh): (human histone stem-loop consensus sequence (Homo sapiens) with stem bordering elements) 
Y U
D H
U-A
C-S
Y-R
H-R
G-C
N*H*AAHD-CVHB*N*H* (stem loop structure)
N*H*AAHDGHYCUDYUHASRRCCVHB*N*H*
(linear sequence) (SEQ ID NO: 37)
 wherein in each of above formulae (Ic) to (Ih) or (IIc) to (IIh):
N, C, G, A, T and U are as defined above;
each U may be replaced by T;
each (highly) conserved G or C in the stem elements 1 and 2 may be replaced by its complementary nucleotide base C or G, provided that its complementary nucleotide in the corresponding stem is replaced by its complementary nucleotide in parallel; and/or
G, A, T, U, C, R, Y, M, K, S, W, H, B, V, D, and N are nucleotide bases as defined in the following Table:

| **abbreviation** | **Nucleotide bases** | **remark** |
|---|---|---|
| G | G | Guanine |
| A | A | Adenine |
| T | T | Thymine |
| U | U | Uracile |
| C | C | Cytosine |
| R | G or A | Purine |
| Y | T/U or C | Pyrimidine |
| M | A or C | Amino |
| K | G or T/U | Keto |
| S | G or C | Strong (3H bonds) |
| W | A or T/U | Weak (2 H bonds) |
| H | A or C or T/U | Not G |
| B | G or T/U or C | Not A |
| V | G or C or A | Not T/U |
| D | G or A or T/U | Not C |
| N | G or C or T/U or A | Any base |
| * | Present or not | Base may be present or not |

In this context it is particularly preferred that the histone stem-loop sequence according to at least one of the formulae (I) or (Ia) to (Ih) or (II) or (IIa) to (IIh) above is selected from a naturally occurring histone stem loop sequence, more particularly preferred from protozoan or metazoan histone stem-loop sequences, and even more particularly preferred from vertebrate and mostly preferred from mammalian histone stem-loop sequences especially from human histone stem-loop sequences.

According to a particularly preferred embodiment, the histone stem-loop sequence according to at least one of the specific formulae (I) or (Ia) to (Ih) or (II) or (IIa) to (IIh) of the present invention is a histone stem-loop sequence comprising at each nucleotide position the most frequently occurring nucleotide, or either the most frequently or the second-most frequently occurring nucleotide of naturally occurring histone stem-loop sequences in metazoa and protozoa, protozoa, metazoa, vertebrates and humans. In this context it is particularly preferred that at least 80%, preferably at least 85%, or most preferably at least 90% of all nucleotides correspond to the most frequently occurring nucleotide of naturally occurring histone stem-loop sequences.

In a further particular embodiment, the histone stem-loop sequence according to at least one of the specific formulae (I) or (Ia) to (Ih) above is selected from following histone stem-loop sequences (without stem-bordering elements):
VGYYYYHHTHRVVRCB (SEQ ID NO: 38 according to formula (Ic))
SGYYYTTYTMARRRCS (SEQ ID NO: 39 according to formula (Ic))
SGYYCTTTTMAGRRCS (SEQ ID NO: 40 according to formula (Ic))

DGNNNBNNTHVNNNCH (SEQ ID NO: 41 according to formula (Ie))
RGNNNYHBTHRDNNCY (SEQ ID NO: 42 according to formula (Ie))
RGNDBYHYTHRDHNCY (SEQ ID NO: 43 according to formula (Ie))

VGYYYTYHTHRVRRCB (SEQ ID NO: 44 according to formula (If))
SGYYCTTYTMAGRRCS (SEQ ID NO: 45 according to formula (If))
SGYYCTTTTMAGRRCS (SEQ ID NO: 46 according to formula (If))

GGYYCTTYTHAGRRCC (SEQ ID NO: 47 according to formula (Ig))
GGCYCTTYTMAGRGCC (SEQ ID NO: 48 according to formula (Ig))
GGCTCTTTTMAGRGCC (SEQ ID NO: 49 according to formula (Ig))

DGHYCTDYTHASRRCC (SEQ ID NO: 50 according to formula (Ih))
GGCYCTTTTHAGRGCC (SEQ ID NO: 51 according to formula (Ih))
GGCYCTTTTMAGRGCC (SEQ ID NO: 52 according to formula (Ih))

Furthermore in this context, following histone stem-loop sequences (with stem bordering elements) according to one of specific formulae (II) or (IIa) to (IIh) are particularly preferred:
H*H*HHVVGYYYYHHTHRVVRCBVHH*N*N* (SEQ ID NO: 53 according to formula (IIc))
M*H*MHMSGYYYTTYTMARRRCSMCH*H*H* (SEQ ID NO: 54 according to formula (IIc))
M*M*MMMSGYYCTTTTMAGRRCSACH*M*H* (SEQ ID NO: 55 according to formula (IIc))

N*N*NNNDGNNNBNNTHVNNNCHNHN*N*N* (SEQ ID NO: 56 according to formula (IIe))
N*N*HHNRGNNNYHBTHRDNNCYDHH*N*N* (SEQ ID NO: 57 according to formula (IIe))
N*H*HHVRGNDBYHYTHRDHNCYRHH*H*H* (SEQ ID NO: 58 according to formula (IIe))
H*H*MHMVGYYYTYHTHRVRRCBVMH*H*N* (SEQ ID NO: 59 according to formula (IIf))
M*M*MMMSGYYCTTYTMAGRRCSMCH*H*H* (SEQ ID NO: 60 according to formula (IIf))
M*M*MMMSGYYCTTTTMAGRRCSACH*M*H* (SEQ ID NO: 61 according to formula (IIf))

H*H*MAMGGYYCTTYTHAGRRCCVHN*N*M* (SEQ ID NO: 62 according to formula (IIg))
H*H*AAMGGCYCTTYTMAGRGCCVCH*H*M* (SEQ ID NO: 63 according to formula (IIg))
M*M*AAMGGCTCTTTTMAGRGCCMCY*M*M* (SEQ ID NO: 64 according to formula (IIg))

N*H*AAHDGHYCTDYTHASRRCCVHB*N*H* (SEQ ID NO: 65 according to formula (IIh))
H*H*AAMGGCYCTTTTHAGRGCCVMY*N*M* (SEQ ID NO: 66 according to formula (IIh))
H*M*AAAGGCYCTTTTMAGRGCCRMY*H*M* (SEQ ID NO: 67 according to formula (IIh))

According to a further preferred embodiment, the at least one mRNA comprises at least one histone stem-loop sequence showing at least about 80%, preferably at least about 85%, more preferably at least about 90%, or even more preferably at least about 95%, sequence identity with the not to 100% conserved nucleotides in the histone stem-loop sequences according to at least one of specific formulae (I) or (Ia) to (Ih) or (II) or (IIa) to (IIh) or with a naturally occurring histone stem-loop sequence.
A particular preferred histone stem-loop sequence is the sequence according to SEQ ID NO: 71 CAAAGGCTCTTTTCAGAGCCACCA or more preferably the corresponding RNA sequence of the nucleic acid sequence according to SEQ ID NO: 71: CAAAGGCUCUUUUCAGAGCCACCA (SEQ ID NO: 72).

In a preferred embodiment, the histone stem loop sequence does not contain the loop sequence 5'-UUUC-3'. More specifically, the histone stem loop does not contain the stem1 sequence 5'-GGCUCU-3' and/or the stem2 sequence 5'-AGAGCC-3', respectively. In another preferred embodiment, the stem loop sequence does not contain the loop sequence 5'-CCUGCCC-3' or the loop sequence 5'-UGAAU-3'. More specifically, the stem loop does not contain the stem1 sequence 5'-CCUGAGC-3' or does not contain the stem1 sequence 5'-ACCUUUCUCCA-3' and/or the stem2 sequence 5'-GCUCAGG-3' or 5'-UGGAGAAAGGU-3', respectively. Also, stem loop sequences are preferably not derived from a mammalian insulin receptor 3'-untranslated region. Also, preferably, the at least one mRNA according to the invention may not contain histone stem loop processing signals, in particular not those derived from mouse histone gene H2A614 gene (H2kA614).

Preferably, the at least one mRNA of the composition according to the present invention does not contain one or two or at least one or all but one or all of the components of the group consisting of: a sequence encoding a ribozyme (preferably a self-splicing ribozyme), a viral nucleic acid sequence, a histone stem-loop processing signal, in particular a histone-stem loop processing sequence derived from mouse histone H2A614 gene, a Neo gene, an inactivated promoter sequence and an inactivated enhancer sequence. Even more preferably, the at least one mRNA according to the invention does not contain a ribozyme, preferably a self-splicing ribozyme, and one of the group consisting of: a Neo gene, an inactivated promoter sequence, an inactivated enhancer sequence, a histone stem-loop processing signal, in particular a histone-stem loop processing sequence derived from mouse histone H2A614 gene. Accordingly, the mRNA may in a preferred mode neither contain a ribozyme, preferably a self-splicing ribozyme, nor a Neo gene or, alternatively, neither a ribozyme, preferably a self-splicing ribozyme, nor any resistance gene (e.g. usually applied for selection). In another preferred mode, the at least one mRNA of the invention may neither contain a ribozyme, preferably a self-splicing ribozyme nor a histone stem-loop processing signal, in particular a histone-stem loop processing sequence derived from mouse histone H2A614 gene

Alternatively, the at least one mRNA of the composition according to the invention optionally comprises a polyadenylation signal which is defined herein as a signal which conveys polyadenylation to a (transcribed) mRNA by specific protein factors (e.g. cleavage and polyadenylation specificity factor (CPSF), cleavage stimulation factor (CstF), cleavage factors I and II (CF I and CF II), poly(A) polymerase (PAP)). In this context a consensus polyadenylation signal is preferred comprising the NN(U/T)ANA consensus sequence. In a particular preferred aspect the polyadenylation signal comprises one of the following sequences: AA(U/T)AAA or A(U/T)(U/T)AAA (wherein uridine is usually present in RNA and thymidine is usually present in DNA). In some embodiments, the polyadenylation signal used in the at least one mRNA according to the invention does not correspond to the U3 snRNA, U5, the polyadenylation processing signal from human gene G-CSF, or the SV40 polyadenylation signal sequences. In particular, the above polyadenylation signals are not combined with any antibiotics resistance gene (or any other reporter, marker or selection gene), in particular not with the resistance *neo* gene (neomycin phosphotransferase). And any of the above polyadenylation signals are preferably not combined with the histone stem loop or the histone stem loop processing signal from mouse histone gene H2A614 in the at least one mRNA according to the invention.

According to another embodiment, the at least one mRNA of the composition of the present invention may be modified, and thus stabilized, by modifying the G/C content of the mRNA, preferably of the coding region of the at least one mRNA.

In a particularly preferred embodiment of the present invention, the G/C content of the coding region of the at least one mRNA of the composition of the present invention is modified, particularly increased, compared to the G/C content of the coding region of its particular wild-type mRNA, i.e. the unmodified mRNA. The amino acid sequence encoded by the at least one mRNA is preferably not modified as compared to the amino acid sequence encoded by the particular wild-type mRNA. This modification of the at least one mRNA of the composition of the present invention is based on the fact that the sequence of any mRNA region to be translated is important for efficient translation of that mRNA. Thus, the composition and the sequence of various nucleotides are important. In particular, sequences having an increased G (guanosine)/C (cytosine) content are more stable than sequences having an increased A (adenosine)/U (uracil) content. According to the invention, the codons of the mRNA are therefore varied compared to the respective wild-type mRNA, while retaining the translated amino acid sequence, such that they include an increased amount of G/C nucleotides. In respect to the fact that several codons code for one and the same amino acid (so-called degeneration of the genetic code), the most favorable codons for the stability can be determined (so-called alternative codon usage). Depending on the amino acid to be encoded by the at least one mRNA, there are various possibilities for modification of the mRNA sequence, compared to its wild-type sequence. In the case of amino acids which are encoded by codons which contain exclusively G or C nucleotides, no modification of the codon is necessary. Thus, the codons for Pro (CCC or CCG), Arg (CGC or CGG), Ala (GCC or GCG) and Gly (GGC or GGG) require no modification, since no A or U is present. In contrast, codons which contain A and/or U nucleotides can be modified by substitution of other codons which code for the same amino acids but contain no A and/or U. Examples of these are: the codons for Pro can be modified from CCU or CCA to CCC or CCG; the codons for Arg can be modified from CGU or CGA or AGA or AGG to CGC or CGG; the codons for Ala can be modified from GCU or GCA to GCC or GCG; the codons for Gly can be modified from GGU or GGA to GGC or GGG. In other cases, although A or U nucleotides cannot be eliminated from the codons, it is however possible to decrease the A and U content by using codons which contain a lower content of A and/or U nucleotides. Examples of these are: the codons for Phe can be modified from UUU to UUC; the codons for Leu can be modified from UUA, UUG, CUU or CUA to CUC or CUG; the codons for Ser can be modified from UCU or UCA or AGU to UCC, UCG or AGC; the codon for Tyr can be modified from UAU to UAC; the codon for Cys can be modified from UGU to UGC; the codon for His can be modified from CAU to CAC; the codon for Gln can be modified from CAA to CAG; the codons for Ile can be modified from AUU or AUA to AUC; the codons for Thr can be modified from ACU or ACA to ACC or ACG; the codon for Asn can be modified from AAU to AAC; the codon for Lys can be modified from AAA to AAG; the codons for Val can be modified from GUU or GUA to GUC or GUG; the codon for Asp can be modified from GAU to GAC; the codon for Glu can be modified from GAA to GAG; the stop codon UAA can be modified to UAG or UGA. In the case of the codons for Met (AUG) and Trp (UGG), on the other hand, there is no possibility of sequence modification. The substitutions listed above can be used either individually or in all possible combinations to increase the G/C content of the at least one mRNA of the composition of the present invention compared to its particular wild-type mRNA (i.e. the original sequence). Thus, for example, all codons for Thr occurring in the wild-type sequence can be modified to ACC (or ACG). Preferably, however, for example, combinations of the above substitution possibilities are used:
substitution of all codons coding for Thr in the original sequence (wild-type mRNA) to ACC (or ACG) and
substitution of all codons originally coding for Ser to UCC (or UCG or AGC); substitution of all codons coding for Ile in the original sequence to AUC and
substitution of all codons originally coding for Lys to AAG and
substitution of all codons originally coding for Tyr to UAC; substitution of all codons coding for Val in the original sequence to GUC (or GUG) and
substitution of all codons originally coding for Glu to GAG and
substitution of all codons originally coding for Ala to GCC (or GCG) and
substitution of all codons originally coding for Arg to CGC (or CGG); substitution of all codons coding for Val in the original sequence to GUC (or GUG) and
substitution of all codons originally coding for Glu to GAG and
substitution of all codons originally coding for Ala to GCC (or GCG) and
substitution of all codons originally coding for Gly to GGC (or GGG) and
substitution of all codons originally coding for Asn to AAC; substitution of all codons coding for Val in the original sequence to GUC (or GUG) and
substitution of all codons originally coding for Phe to UUC and
substitution of all codons originally coding for Cys to UGC and
substitution of all codons originally coding for Leu to CUG (or CUC) and
substitution of all codons originally coding for Gln to CAG and
substitution of all codons originally coding for Pro to CCC (or CCG); etc. Preferably, the G/C content of the coding region of the at least one mRNA of the composition of the present invention is increased by at least 7%, more preferably by at least 15%, particularly preferably by at least 20%, compared to the G/C content of the coded region of the wild-type mRNA which codes for an antigen, antigenic protein or antigenic peptide as deinined herein or its fragment or variant thereof. According to a specific embodiment at least 5%, 10%, 20%, 30%, 40%, 50%, 60%, more preferably at least 70 %, even more preferably at least 80% and most preferably at least 90%, 95% or even 100% of the substitutable codons in the region coding for an antigen, antigenic protein or antigenic peptide as deinined herein or its fragment or variant thereof or the whole sequence of the wild type mRNA sequence are substituted, thereby increasing the GC/content of said sequence. In this context, it is particularly preferable to increase the G/C content of the at least one mRNA of the composition of the present invention to the maximum (i.e. 100% of the substitutable codons), in particular in the region coding for a protein, compared to the wild-type sequence. According to the invention, a further preferred modification of the at least one mRNA of the composition of the present invention is based on the finding that the translation efficiency is also determined by a different frequency in the occurrence of tRNAs in cells. Thus, if so-called "rare codons" are present in the at least one mRNA of the composition of the present invention to an increased extent, the corresponding modified at least one mRNA sequence is translated to a significantly poorer degree than in the case where codons coding for relatively "frequent" tRNAs are present. According to the invention, in the modified at least one mRNA of the composition of the present invention, the region which codes for one of the above defined antigens is modified compared to the corresponding region of the wild-type mRNA such that at least one codon of the wild-type sequence, which codes for a tRNA which is relatively rare in the cell, is exchanged for a codon, which codes for a tRNA which is relatively frequent in the cell and carries the same amino acid as the relatively rare tRNA. By this modification, the sequences of the at least one mRNA of the composition of the present invention is modified such that codons for which frequently occurring tRNAs are available are inserted. In other words, according to the invention, by this modification all codons of the wild-type sequence which code for a tRNA which is relatively rare in the cell can in each case be exchanged for a codon which codes for a tRNA which is relatively frequent in the cell and which, in each case, carries the same amino acid as the relatively rare tRNA. Which tRNAs occur relatively frequently in the cell and which, in contrast, occur relatively rarely is known to a person skilled in the art; cf. e.g. Akashi, Curr. Opin. Genet. Dev. 2001, 11(6): 660-666. The codons which use for the particular amino acid the tRNA which occurs the most frequently, e.g. the Gly codon, which uses the tRNA, which occurs the most frequently in the (human) cell, are particularly preferred. According to the invention, it is particularly preferable to link the sequential G/C content which is increased, in particular maximized, in the modified at least one mRNA of the composition of the present invention, with the "frequent" codons without modifying the amino acid sequence of the protein encoded by the coding region of the mRNA. This preferred embodiment allows provision of a particularly efficiently translated and stabilized (modified) at least one mRNA of the composition of the present invention. The determination of a modified at least one mRNA of the composition of the present invention as described above (increased G/C content; exchange of tRNAs) can be carried out using the computer program explained in WO 02/098443 - the disclosure content of which is included in its full scope in the present invention. Using this computer program, the nucleotide sequence of any desired mRNA can be modified with the aid of the genetic code or the degenerative nature thereof such that a maximum G/C content results, in combination with the use of codons which code for tRNAs occurring as frequently as possible in the cell, the amino acid sequence coded by the modified at least one mRNA preferably not being modified compared to the non-modified sequence. Alternatively, it is also possible to modify only the G/C content or only the codon usage compared to the original sequence. The source code in Visual Basic 6.0 (development environment used: Microsoft Visual Studio Enterprise 6.0 with Servicepack 3) is also described in WO 02/098443. In a further preferred embodiment of the present invention, the A/U content in the environment of the ribosome binding site of the at least one mRNA of the composition of the present invention is increased compared to the A/U content in the environment of the ribosome binding site of its particular wild-type mRNA. This modification (an increased A/U content around the ribosome binding site) increases the efficiency of ribosome binding to the at least one mRNA. An effective binding of the ribosomes to the ribosome binding site (Kozak sequence: GCCGCCACCAUGG (SEQ ID NO: 68), the AUG forms the start codon) in turn has the effect of an efficient translation of the at least one mRNA. According to a further embodiment of the present invention the at least one mRNA of the composition of the present invention may be modified with respect to potentially destabilizing sequence elements. Particularly, the coding region and/or the 5' and/or 3' untranslated region of this at least one mRNA may be modified compared to the particular wild-type mRNA such that it contains no destabilizing sequence elements, the coded amino acid sequence of the modified at least one mRNA preferably not being modified compared to its particular wild-type mRNA. It is known that, for example, in sequences of eukaryotic RNAs destabilizing sequence elements (DSE) occur, to which signal proteins bind and regulate enzymatic degradation of RNA in vivo. For further stabilization of the modified at least one mRNA, optionally in the region which encodes for an antigen, antigenic protein or antigenic peptide as defined herein, one or more such modifications compared to the corresponding region of the wild-type mRNA can therefore be carried out, so that no or substantially no destabilizing sequence elements are contained there. According to the invention, DSE present in the untranslated regions (3'- and/or 5'-UTR) can also be eliminated from the at least one mRNA of the composition of the present invention by such modifications. Such destabilizing sequences are e.g. AU-rich sequences (AURES), which occur in 3'-UTR sections of numerous unstable RNAs (Caput et al., Proc. Natl. Acad. Sci. USA 1986, 83: 1670 to 1674). The at least one mRNA of the composition of the present invention is therefore preferably modified compared to the wild-type mRNA such that the at least one mRNA contains no such destabilizing sequences. This also applies to those sequence motifs which are recognized by possible endonucleases, e.g. the sequence GAACAAG, which is contained in the 3'-UTR segment of the gene which codes for the transferrin receptor (Binder et al., EMBO J. 1994, 13: 1969 to 1980). These sequence motifs are also preferably removed in the at least one mRNA of the composition of the present invention. Also preferably according to the invention, the at least one mRNA of the composition of the present invention has, in a modified form, at least one IRES as defined above and/or at least one 5' and/or 3' stabilizing sequence, in a modified form, e.g. to enhance ribosome binding or to allow expression of different encoded antigens located on an at least one (bi- or even multicistronic) mRNA of the composition of the present invention.

According to the invention, the at least one mRNA of the composition furthermore preferably has at least one 5' and/or 3' stabilizing sequence. These stabilizing sequences in the 5' and/or 3' untranslated regions have the effect of increasing the half-life of the at least one mRNA in the cytosol. These stabilizing sequences can have 100% sequence homology to naturally occurring sequences, which occur in viruses, bacteria and eukaryotes, but can also be partly or completely synthetic. The untranslated sequences (UTR) of the β-globin gene, e.g. from Homo sapiens or Xenopus laevis may be mentioned as an example of stabilizing sequences, which can be used in the present invention for a stabilized mRNA. Another example of a stabilizing sequence has the general formula (C/U)CCANxCCC(U/A)PyxUC(C/U)CC (SEQ ID NO: 69), which is contained in the 3'UTR of the very stable mRNA which codes for α-globin, α(I)-collagen, 15-lipoxygenase or for tyrosine hydroxylase (cf. Holcik et al., Proc. Natl. Acad. Sci. USA 1997, 94: 2410 to 2414). Such stabilizing sequences can of course be used individually or in combination with one another and also in combination with other stabilizing sequences known to a person skilled in the art. The at least one mRNA of the composition of the present invention is therefore preferably present as globin UTR (untranslated regions)-stabilized mRNA, in particular as α-globin UTR-stabilized mRNA. Preferably the at least one mRNA of the composition comprises a stabilizing sequence in the 3'-UTR derived from the center, α-complex-binding portion of the 3'UTR of an α-globin gene, such as of a human α-globin gene, preferably according to SEQ ID No. 70:
Center, α-complex-binding portion of the 3'UTR of an α-globin gene (also named herein as "muag")
GCCCGAUGGGCCUCCCAACGGGCCCUCCUCCCCUCCUUGCACCG (SEQ ID NO. 70)

Nevertheless, substitutions, additions or eliminations of bases are preferably carried out with the at least one mRNA of the composition of the present invention, using a DNA matrix for preparation of the at least one mRNA of the composition of the present invention by techniques of the well known site directed mutagenesis or with an oligonucleotide ligation strategy (see e.g. Maniatis et al., Molecular Cloning: A Laboratory Manual, Cold Spring Harbor Laboratory Press, 3rd ed., Cold Spring Harbor, NY, 2001). In such a process, for preparation of the at least one mRNA, a corresponding DNA molecule may be transcribed in vitro. This DNA matrix preferably comprises a suitable promoter, e.g. a T7 or SP6 promoter, for in vitro transcription, which is followed by the desired nucleotide sequence for the at least one mRNA to be prepared and a termination signal for in vitro transcription. The DNA molecule, which forms the matrix of an at least one mRNA of interest, may be prepared by fermentative proliferation and subsequent isolation as part of a plasmid which can be replicated in bacteria. Plasmids which may be mentioned as suitable for the present invention are e.g. the plasmids pT7Ts (GenBank accession number U26404; Lai et al., Development 1995, 121: 2349 to 2360), pGEM® series, e.g. pGEM®-1 (GenBank accession number X65300; from Promega) and pSP64 (GenBank accession number X65327); cf. also Mezei and Storts, Purification of PCR Products, in: Griffin and Griffin (ed.), PCR Technology: Current Innovation, CRC Press, Boca Raton, FL, 2001.

The stabilization of the at least one mRNA of the composition of the present invention can likewise be carried out by associating or complexing the at least one mRNA with, or binding it to, a cationic compound, in particular a polycationic compound, for example a (poly)cationic peptide or protein. In particular, the use of protamine, nucleoline, spermin or spermidine as the polycationic, nucleic-acid-binding protein to the RNA is particularly effective. Furthermore, the use of other cationic peptides or proteins, such as poly-L-lysine or histones, is likewise possible. This procedure for stabilizing mRNA is described in EP-A-1083232, the disclosure of which is incorporated by reference into the present invention in its entirety. Further preferred cationic substances, which can be used for stabilizing the mRNA of the composition of the present invention, include cationic polysaccharides, for example chitosan, polybrene, polyethyleneimine (PEI) or poly-L-lysine (PLL), etc.. Association or complexing of the at least one mRNA of the inventive composition with cationic compounds, e.g. cationic proteins or cationic lipids, e.g. oligofectamine as a lipid based complexation reagent) preferably increases the transfer of the at least one mRNA present as a pharmaceutically active component into the cells to be treated or into the organism to be treated. It is also referred to the disclosure herein with regard to the stabilizing effect for the at least one mRNA of the composition of the present invention by complexation, which holds for the stabilization of mRNA as well.

According to another particularly preferred embodiment, the at least one mRNA of the composition may additionally or alternatively encode a secretory signal peptide. Such signal peptides are sequences, which typically exhibit a length of about 15 to 30 amino acids and are preferably located at the N-terminus of the encoded peptide, without being limited thereto. Signal peptides as defined herein preferably allow the transport of the antigen, antigenic protein or antigenic peptide as encoded by the at least one mRNA of the composition into a defined cellular compartiment, preferably the cell surface, the endoplasmic reticulum (ER) or the endosomal-lysosomal compartiment. Examples of secretory signal peptide sequences as defined herein include, without being limited thereto, signal sequences of classical or non-classical MHC-molecules (e.g. signal sequences of MHC I and II molecules, e.g. of the MHC class I molecule HLA-A*0201), signal sequences of cytokines or immunoglobulines as defined herein, signal sequences of the invariant chain of immunoglobulines or antibodies as defined herein, signal sequences of Lamp1, Tapasin, Erp57, Calretikulin, Calnexin, and further membrane associated proteins or of proteins associated with the endoplasmic reticulum (ER) or the endosomal-lysosomal compartiment. Particularly preferably, signal sequences of MHC class I molecule HLA-A*0201 may be used according to the present invention.

Any of the above modifications may be applied to the at least one mRNA of the composition of the present invention, and further to any RNA as used in the context of the present invention and may be, if suitable or necessary, be combined with each other in any combination, provided, these combinations of modifications do not interfere with each other in the respective at least one mRNA. A person skilled in the art will be able to take his choice accordingly.

According to a preferred embodiment, the composition comprises at least one mRNA that has been modified as described herewithin, which comprises at least one coding sequence selected from RNA sequences being identical or at least 80% identical to the RNA sequence of SEQ ID NOs: 3, 6, 9, 12, 15, 18 or 25. Even more preferably, the composition comprises six mRNAs, wherein the coding sequence in each mRNA is identical or at least 80% identical to one of the RNA sequences according to SEQ ID NOs: 3, 6, 9, 12, 15, 18 or 25.

In a preferred embodiment, each of the six antigens of the composition of the present invention, may be encoded by one (monocistronic) mRNA. In other words, the composition of the present invention may contain six (monocistronic) mRNAs, wherein each of these six (monocistronic) mRNAs, may encode just one antigen as defined above.

In an even more preferred embodiment, the composition comprises six mRNAs, each of which has been modified as described herewithin, wherein one mRNA encodes 5T4, one mRNA encodes Survivin, one mRNA encodes NY-ESO-1, one mRNA encodes MAGE-C1, one mRNA encodes MAGE-C2 and one mRNA encodes MUC1 or fragments or variants thereof, respectively.

In an even more preferred embodiment, the composition comprises six mRNAs, wherein one mRNA encodes 5T4 and comprises a coding sequence identical or at least 80% identical to SEQ ID NO: 3, one mRNA encodes Survivin and comprises a coding sequence identical or at least 80% identical to SEQ ID NO: 6, one mRNA encodes NY-ESO-1 and comprises a coding sequence identical or at least 80% identical to SEQ ID NO: 9, one mRNA encodes MAGE-C1 and comprises a coding sequence identical or at least 80% identical to SEQ ID NO: 12 or 25, one mRNA encodes MAGE-C2 and comprises a coding sequence identical or at least 80% identical to SEQ ID NO: 15 and one mRNA encodes MUC1 and comprises a coding sequence identical or at least 80% identical to SEQ ID NO:18 (or fragments or variants of each of these sequences) and optionally further excipients.

In one embodiment, the composition comprises at least one mRNA, which is identical or at least 80% identical to the RNA sequence of SEQ ID NOs: 1, 4, 7, 10, 13 or 16. Even more preferably, the composition comprises six mRNAs, wherein each mRNA is identical or at least 80% identical to one of the RNA sequences according to SEQ ID NOs: 1, 4, 7, 10, 13 or 16.

In an even more preferred embodiment, the composition comprises six mRNAs, wherein one mRNA encodes 5T4 and is identical or at least 80% identical to SEQ ID NO: 1, one mRNA encodes Survivin and is identical or at least 80% identical to SEQ ID NO: 4, one mRNA encodes NY-ESO-1 and is identical or at least 80% identical to SEQ ID NO: 7, one mRNA encodes MAGE-C1 and is identical or at least 80% identical to SEQ ID NO: 10, one mRNA encodes MAGE-C2 and is identical or at least 80% identical to SEQ ID NO: 13 and one mRNA encodes MUC1 and is identical or at least 80% identical to SEQ ID NO:16 (or fragments or variants of each of these sequences) and optionally further excipients.

According to the invention, the at least one mRNA of the compositions described above comprises a histone stem-loop in the 3' UTR region. Preferably, the composition comprises six mRNAs, wherein each of the mRNAs comprises a histone stem-loop as defined herewithin.

In a preferred embodiment, the composition comprises six mRNAs, wherein one mRNA encodes 5T4 and is identical or at least 80% identical to SEQ ID NO: 19, one mRNA encodes Survivin and is identical or at least 80% identical to SEQ ID NO: 20, one mRNA encodes NY-ESO-1 and is identical or at least 80% identical to SEQ ID NO: 21, one mRNA encodes MAGE-C1 and is identical or at least 80% identical to SEQ ID NO: 22, one mRNA encodes MAGE-C2 and is identical or at least 80% identical to SEQ ID NO: 23 and one mRNA encodes MUC1 and is identical or at least 80% identical to SEQ ID NO: 24 (or fragments or variants of each of these sequences) and optionally further excipients.

According to another embodiment, the composition according to the invention may comprise an adjuvant in order to enhance the immunostimulatory properties of the composition. In this context, an adjuvant may be understood as any compound, which is suitable to support administration and delivery of the composition according to the invention. Furthermore, such an adjuvant may, without being bound thereto, initiate or increase an immune response of the innate immune system, i.e. a non-specific immune response. With other words, when administered, the composition according to the invention typically initiates an adaptive immune response due to the at least six antigens encoded by the at least one mRNA contained in the inventive composition. Additionally, the composition according to the invention may generate an (supportive) innate immune response due to addition of an adjuvant as defined herein to the composition according to the invention.

Such an adjuvant may be selected from any adjuvant known to a skilled person and suitable for the present case, i.e. supporting the induction of an immune response in a mammal. Preferably, the adjuvant may be selected from the group consisting of, without being limited thereto, TDM, MDP, muramyl dipeptide, pluronics, alum solution, aluminium hydroxide, ADJUMERTM (polyphosphazene); aluminium phosphate gel; glucans from algae; algammulin; aluminium hydroxide gel (alum); highly protein-adsorbing aluminium hydroxide gel; low viscosity aluminium hydroxide gel; AF or SPT (emulsion of squalane (5%), Tween 80 (0.2%), Pluronic L121 (1.25%), phosphate-buffered saline, pH 7.4); AVRIDINETM (propanediamine); BAY R1005TM ((N-(2-deoxy-2-L-leucylamino-b-D-glucopyranosyl)-N-octadecyl-dodecanoyl-amide hydroacetate); CALCITRIOLTM (1-alpha,25-dihydroxy-vitamin D3); calcium phosphate gel; CAPTM (calcium phosphate nanoparticles); cholera holotoxin, cholera-toxin-A1-protein-A-D-fragment fusion protein, sub-unit B of the cholera toxin; CRL 1005 (block copolymer P1205); cytokine-containing liposomes; DDA (dimethyldioctadecylammonium bromide); DHEA (dehydroepiandrosterone); DMPC (dimyristoylphosphatidylcholine); DMPG (dimyristoylphosphatidylglycerol); DOC/alum complex (deoxycholic acid sodium salt); Freund's complete adjuvant; Freund's incomplete adjuvant; gamma inulin; Gerbu adjuvant (mixture of: i) N-acetylglucosaminyl-(P1-4)-N-acetylmuramyl-L-alanyl-D-glutamine (GMDP), ii) dimethyldioctadecylammonium chloride (DDA), iii) zinc-L-proline salt complex (ZnPro-8); GM-CSF); GMDP (N-acetylglucosaminyl-(b1-4)-N-acetylmuramyl-L-alanyl-D-isoglutamine); imiquimod (1-(2-methypropyl)-1H-imidazo[4,5-c]quinoline-4-amine); ImmTherTM (N-acetylglucosaminyl-N-acetylmuramyl-L-Ala-D-isoGlu-L-Ala-glycerol dipalmitate); DRVs (immunoliposomes prepared from dehydration-rehydration vesicles); interferon-gamma; interleukin-1beta; interleukin-2; interleukin-7; interleukin-12; ISCOMSTM; ISCOPREP 7.0.3. TM; liposomes; LOXORIBINETM (7-allyl-8-oxoguanosine); LT oral adjuvant (E.coli labile enterotoxin-protoxin); microspheres and microparticles of any composition; MF59TM; (squalene-water emulsion); MONTANIDE ISA 51TM (purified incomplete Freund's adjuvant); MONTANIDE ISA 720TM (metabolisable oil adjuvant); MPLTM (3-Q-desacyl-4'-monophosphoryl lipid A); MTP-PE and MTP-PE liposomes ((N-acetyl-L-alanyl-D-isoglutaminyl-L-alanine-2-(1,2-dipalmitoyl-sn-glycero-3-(hydroxyphosphoryloxy))-ethylamide, monosodium salt); MURAMETIDETM (Nac-Mur-L-Ala-D-Gln-OCH3); MURAPALMITINETM and D-MURAPALMITINETM (Nac-Mur-L-Thr-D-isoGln-sn-glyceroldipalmitoyl); NAGO (neuraminidase-galactose oxidase); nanospheres or nanoparticles of any composition; NISVs (non-ionic surfactant vesicles); PLEURANTM (β-glucan); PLGA, PGA and PLA (homo- and co-polymers of lactic acid and glycolic acid; microspheres/nanospheres); PLURONIC L121TM; PMMA (polymethyl methacrylate); PODDSTM (proteinoid microspheres); polyethylene carbamate derivatives; poly-rA: poly-rU (polyadenylic acid-polyuridylic acid complex); polysorbate 80 (Tween 80); protein cochleates (Avanti Polar Lipids, Inc., Alabaster, AL); STIMULONTM (QS-21); Quil-A (Quil-A saponin); S-28463 (4-amino-otec-dimethy)-2-ethoxymethy)-1H-imidazo[4,5 c]quinoline-1-ethanol); SAF-1TM ("Syntex adjuvant formulation"); Sendai proteoliposomes and Sendai-containing lipid matrices; Span-85 (sorbitan trioleate); Specol (emulsion of Marcol 52, Span 85 and Tween 85); squalene or Robane® (2,6,10,15,19,23-hexamethyltetracosan and 2,6,10,15,19,23-hexamethyl-2,6,10,14,18,22-tetracosahexane); stearyltyrosine (octadecyltyrosine hydrochloride); Theramid® (N-acetylglucosaminyl-N-acetylmuramyl-L-Ala-D-isoGlu-L-Ala-dipalmitoxypropylamide); Theronyl-MDP (TermurtideTM or [thr 1]-MDP; N-acetylmuramyl-L-threonyl-D-isoglutamine); Ty particles (Ty-VLPs or virus-like particles); Walter-Reed liposomes (liposomes containing lipid A adsorbed on aluminium hydroxide), and lipopeptides, including Pam3Cys, in particular aluminium salts, such as Adju-phos, Alhydrogel, Rehydragel; emulsions, including CFA, SAF, IFA, MF59, Provax, TiterMax, Montanide, Vaxfectin; copolymers, including Optivax (CRL1005), L121, Poloaxmer4010), etc.; liposomes, including Stealth, cochleates, including BIORAL; plant derived adjuvants, including QS21, Quil A, Iscomatrix, ISCOM; adjuvants suitable for costimulation including Tomatine, biopolymers, including PLG, PMM, Inulin,; microbe derived adjuvants, including Romurtide, DETOX, MPL, CWS, Mannose, CpG nucleic acid sequences, CpG7909, ligands of human TLR 1-10, ligands of murine TLR 1-13, ISS-1018, IC31, Imidazoquinolines, Ampligen, Ribi529, IMOxine, IRIVs, VLPs, cholera toxin, heatlabile toxin, Pam3Cys, Flagellin, GPI anchor, LNFPIII/Lewis X, antimicrobial peptides, UC-1V150, RSV fusion protein, cdiGMP; and adjuvants suitable as antagonists including CGRP neuropeptide.

Suitable adjuvants may also be selected from cationic or polycationic compounds wherein the adjuvant is preferably prepared upon complexing the at least one mRNA of the inventive composition with the cationic or polycationic compound. Association or complexing the mRNA of the composition with cationic or polycationic compounds as defined herein preferably provides adjuvant properties and confers a stabilizing effect to the at least one mRNA of the composition. Particularly such preferred, such cationic or polycationic compounds are selected from cationic or polycationic peptides or proteins, including protamine, nucleoline, spermin or spermidine, or other cationic peptides or proteins, such as poly-L-lysine (PLL), poly-arginine, basic polypeptides, cell penetrating peptides (CPPs), including HIV-binding peptides, Tat, HIV-1 Tat (HIV), Tat-derived peptides, Penetratin, VP22 derived or analog peptides, HSV VP22 (Herpes simplex), MAP, KALA or protein transduction domains (PTDs, PpT620, prolin-rich peptides, arginine-rich peptides, lysine-rich peptides, MPG-peptide(s), Pep-1, L-oligomers, Calcitonin peptide(s), Antennapedia-derived peptides (particularly from Drosophila antennapedia), pAntp, plsl, FGF, Lactoferrin, Transportan, Buforin-2, Bac715-24, SynB, SynB(1), pVEC, hCT-derived peptides, SAP, protamine, spermine, spermidine, or histones. Further preferred cationic or polycationic compounds may include cationic polysaccharides, for example chitosan, polybrene, cationic polymers, e.g. polyethyleneimine (PEI), cationic lipids, e.g. DOTMA: 1-(2,3-sioleyloxy)propyl)-N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-(-trimethylammonioacetyl)diethanolamine chloride, CLIP1: rac-(2,3-dioctadecyloxypropyl)(2-hydroxyethyl) -dimethylammonium chloride, CLIP6: rac- 2(2,3-dihexadecyloxypropyl-oxymethyloxy)ethyl trimethylammonium, CLIP9: rac- 2(2,3-dihexadecyloxypropyl-oxysuccinyloxy)ethyl -trimethylammonium, oligofectamine, or cationic or polycationic polymers, e.g. modified polyaminoacids, such as -aminoacidpolymers or reversed polyamides, etc., modified polyethylenes, such as PVP (poly(N-ethyl-4-vinylpyridinium bromide)), etc., modified acrylates, such as pDMAEMA (poly(dimethylaminoethyl methylacrylate)), etc., modified Amidoamines such as pAMAM (poly(amidoamine)), etc., modified polybetaaminoester (PBAE), such as diamine end modified 1,4 butanediol diacrylate-co-5-amino-1-pentanol polymers, etc., dendrimers, such as polypropylamine dendrimers or pAMAM based dendrimers, etc., polyimine(s), such as PEI: poly(ethyleneimine), poly(propyleneimine), etc., polyallylamine, sugar backbone based polymers, such as cyclodextrin based polymers, dextran based polymers, Chitosan, etc., silan backbone based polymers , such as PMOXA-PDMS copolymers, etc., Blockpolymers consisting of a combination of one or more cationic blocks (e.g. selected of a cationic polymer as mentioned above) and of one or more hydrophilic- or hydrophobic blocks (e.g polyethyleneglycole); etc.

Additionally, preferred cationic or polycationic proteins or peptides, which can be used as an adjuvant by complexing the at least one mRNA of the composition, may be selected from following proteins or peptides having the following total formula (III): (Arg)l;(Lys)m;(His)n;(Orn)o;(Xaa)x, wherein l + m + n +o + x = 8-15, and l, m, n or o independently of each other may be any number selected from 0, 1, 2, 3, 4, 5, 6, 7, 8, 9, 10, 11, 12, 13, 14 or 15, provided that the overall content of Arg, Lys, His and Orn represents at least 50% of all amino acids of the oligopeptide; and Xaa may be any amino acid selected from native (= naturally occurring) or non-native amino acids except of Arg, Lys, His or Orn; and x may be any number selected from 0, 1, 2, 3 or 4, provided, that the overall content of Xaa does not exceed 50 % of all amino acids of the oligopeptide. Particularly preferred oligoarginines in this context are e.g. Arg7, Arg8, Arg9, Arg7, H3R9, R9H3, H3R9H3, YSSR9SSY, (RKH)4, Y(RKH)2R, etc.

The ratio of the mRNA to the cationic or polycationic compound in the adjuvant component may be calculated on the basis of the nitrogen/phosphate ratio (N/P-ratio) of the entire mRNA complex, i.e. the ratio of positively charged (nitrogen) atoms of the cationic or polycationic compound to the negatively charged phosphate atoms of the nucleic acids. For example, 1 µg RNA typically contains about 3 nmol phosphate residues, provided the RNA exhibits a statistical distribution of bases. Additionally, 1 µg peptide typically contains about x nmol nitrogen residues, dependent on the molecular weight and the number of basic amino acids. When exemplarily calculated for (Arg)9 (molecular weight 1424 g/mol, 9 nitrogen atoms), 1 µg (Arg)9 contains about 700 pmol (Arg)9 and thus 700 x 9=6300 pmol basic amino acids = 6.3 nmol nitrogen atoms. For a mass ratio of about 1:1 RNA/(Arg)9 an N/P ratio of about 2 can be calculated. When exemplarily calculated for protamine (molecular weight about 4250 g/mol, 21 nitrogen atoms, when protamine from salmon is used) with a mass ratio of about 2:1 with 2 µg RNA, 6 nmol phosphate are to be calulated for the RNA; 1 µg protamine contains about 235 pmol protamine molecues and thus 235 x 21 = 4935 pmol basic nitrogen atoms = 4.9 nmol nitrogen atoms. For a mass ratio of about 2:1 RNA/protamine an N/P ratio of about 0.81 can be calculated. For a mass ratio of about 8:1 RNA/protamine an N/P ratio of about 0.2 can be calculated. In the context of the present invention, an N/P-ratio is preferably in the range of about 0.1-10, preferably in a range of about 0.3-4 and most preferably in a range of about 0.5-2 or 0.7-2 regarding the ratio of RNA:peptide in the complex, and most preferably in the range of about 0.7-1.5.

In a preferred embodiment, the composition is obtained in two separate steps in order to obtain both, an efficient immunostimulatory effect and efficient translation of the at least one mRNA according to the invention. Therein, a so called "adjuvant component" is prepared by complexing - in a first step - an at least one mRNA of the adjuvant component with a cationic or polycationic compound in a specific ratio to form a stable complex. In this context, it is important, that no free cationic or polycationic compound or only a neglibly small amount remains in the adjuvant component after complexing the mRNA. Accordingly, the ratio of the mRNA and the cationic or polycationic compound in the adjuvant component is typically selected in a range that the mRNA is entirely complexed and no free cationic or polycationic compound or only a neclectably small amount remains in the composition. Preferably the ratio of the adjuvant component, i.e. the ratio of the mRNA to the cationic or polycationic compound is selected from a range of about 6:1 (w/w) to about 0,25:1 (w/w), more preferably from about 5:1 (w/w) to about 0,5:1 (w/w), even more preferably of about 4:1 (w/w) to about 1:1 (w/w) or of about 3:1 (w/w) to about 1:1 (w/w), and most preferably a ratio of about 3:1 (w/w) to about 2:1 (w/w).

According to a preferred embodiment, the at least one mRNA encoding the antigens according to the invention is added in a second step to the complexed mRNA of the adjuvant component in order to form the (immunostimulatory) composition of the invention. Therein, the at least one mRNA of the invention is added as free mRNA, i.e. mRNA, which is not complexed by other compounds. Prior to addition, the at least one free mRNA is not complexed and will preferably not undergo any detectable or significant complexation reaction upon the addition of the adjuvant component. This is due to the strong binding of the cationic or polycationic compound to the above described at least one mRNA in the adjuvant component. In other words, when the at least one free mRNA, encoding at least one of the antigens according to the invention, is added to the "adjuvant component", preferably no free or substantially no free cationic or polycationic compound is present, which may form a complex with the at least one free mRNA. Accordingly, an efficient translation of the at least one free mRNA of the inventive composition is possible in vivo. Therein, the at least one free mRNA may occur as a mono-, di-, or multicistronic mRNA, i.e. an RNA which carries the coding sequences of one or more proteins. Such coding sequences in di-, or even multicistronic mRNA may be separated by at least one IRES sequence, e.g. as defined herein.

In a particularly preferred embodiment, the at least one free mRNA, which is comprised in the inventive composition, may be identical or different to the at least one mRNA of the adjuvant component of the inventive composition, depending on the specific requirements of therapy. Even more preferably, the at least one free mRNA, which is comprised in the inventive composition, is identical to the at least one RNA of the adjuvant component of the inventive composition.

In a particulary preferred embodiment, the composition comprises at least one mRNA, wherein at least one mRNA encodes the antigens as defined above and wherein said mRNA is present in the composition partially as free mRNA and partially as complexed mRNA.

Preferably, the at least one mRNA encoding one or more antigens as defined above is complexed as described above and the same at least one mRNA is then added as free RNA, wherein preferably the compound, which is used for complexing the mRNA is not present in free form in the composition at the moment of addition of the free mRNA component.

The ratio of the first component (i.e. the adjuvant component comprising or consisting of at least one mRNA complexed with a cationic or polycationic compound) and the second component (i.e. the at least one free mRNA) may be selected in the inventive composition according to the specific requirements of a particular therapy. Typically, the ratio of the mRNA in the adjuvant component and the at least one free mRNA (mRNA in the adjuvant component : free RNA) of the inventive composition is selected such that a significant stimulation of the innate immune system is elicited due to the adjuvant component. In parallel, the ratio is selected such that a significant amount of the at least one free mRNA can be provided *in vivo* leading to an efficient translation and concentration of the expressed protein *in vivo,* e.g. the atsix antigens, etc. as defined above. Preferably the ratio of the mRNA in the adjuvant component : free mRNA in the inventive composition is selected from a range of about 5:1 (w/w) to about 1:10 (w/w), more preferably from a range of about 4:1 (w/w) to about 1:8 (w/w), even more preferably from a range of about 3:1 (w/w) to about 1:5 (w/w) or 1:3 (w/w), and most preferably the ratio of mRNA in the adjuvant component : free mRNA in the inventive composition is selected from a ratio of about 1:1 (w/w).

Additionally or alternatively, the ratio of the first component (i.e. the adjuvant component comprising or consisting of at least one mRNA complexed with a cationic or polycationic compound) and the second component (i.e. the at least one free mRNA) may be calculated on the basis of the nitrogen/phosphate ratio (N/P-ratio) of the entire mRNA complex. In the context of the present invention, an N/P-ratio is preferably in the range of about 0.1-10, preferably in a range of about 0.3-4 and most preferably in a range of about 0.5-2 or 0.7-2 regarding the ratio of mRNA:peptide in the complex, and most preferably in the range of about 0.7-1.5.

Additionally or alternatively, the ratio of the first component (i.e. the adjuvant component comprising or consisting of at least one mRNA complexed with a cationic or polycationic compound) and the second component (i.e. the at least one free mRNA) may also be selected in the inventive composition on the basis of the molar ratio of both mRNAs to each other, i.e. the mRNA of the adjuvant component, being complexed with a cationic or polycationic compound and the at least one free mRNA of the second component. Typically, the molar ratio of the mRNA of the adjuvant component to the at least one free mRNA of the second component may be selected such, that the molar ratio suffices the above (w/w) and/or N/P-definitions. More preferably, the molar ratio of the mRNA of the adjuvant component to the at least one free mRNA of the second component may be selected e.g. from a molar ratio of about 0.001:1, 0.01:1, 0.1:1, 0.2:1, 0.3:1, 0.4:1, 0.5:1, 0.6:1, 0.7:1, 0.8:1, 0.9:1, 1:1, 1:0.9, 1:0.8, 1:0.7, 1:0.6, 1:0.5, 1:0.4, 1:0.3, 1:0.2, 1:0.1, 1:0.01, 1:0.001, etc. or from any range formed by any two of the above values, e.g. a range selected from about 0.001:1 to 1:0.001, including a range of about 0.01:1 to 1:0.001, 0.1:1 to 1:0.001, 0.2:1 to 1:0.001, 0.3:1 to 1:0.001, 0.4:1 to 1:0.001, 0.5:1 to 1:0.001, 0.6:1 to 1:0.001, 0.7:1 to 1:0.001, 0.8:1 to 1:0.001, 0.9:1 to 1:0.001, 1:1 to 1:0.001, 1:0.9 to 1:0.001, 1:0.8 to 1:0.001, 1:0.7 to 1:0.001, 1:0.6 to 1:0.001, 1:0.5 to 1:0.001, 1:0.4 to 1:0.001, 1:0.3 to 1:0.001, 1:0.2 to 1:0.001, 1:0.1 to 1:0.001, 1:0.01 to 1:0.001, or a range of about 0.01:1 to 1:0.01, 0.1:1 to 1:0.01, 0.2:1 to 1:0.01, 0.3:1 to 1:0.01, 0.4:1 to 1:0.01, 0.5:1 to 1:0.01, 0.6:1 to 1:0.01, 0.7:1 to 1:0.01, 0.8:1 to 1:0.01, 0.9:1 to 1:0.01, 1:1 to 1:0.01, 1:0.9 to 1:0.01, 1:0.8 to 1:0.01, 1:0.7 to 1:0.01, 1:0.6 to 1:0.01, 1:0.5 to 1:0.01, 1:0.4 to 1:0.01, 1:0.3 to 1:0.01, 1:0.2 to 1:0.01, 1:0.1 to 1:0.01, 1:0.01 to 1:0.01, or including a range of about 0.001:1 to 1:0.01, 0.001:1 to 1:0.1, 0.001:1 to 1:0.2, 0.001:1 to 1:0.3, 0.001:1 to 1:0.4, 0.001:1 to 1:0.5, 0.001:1 to 1:0.6, 0.001:1 to 1:0.7, 0.001:1 to 1:0.8, 0.001:1 to 1:0.9, 0.001:1 to 1:1, 0.001 to 0.9:1, 0.001 to 0.8:1, 0.001 to 0.7:1, 0.001 to 0.6:1, 0.001 to 0.5:1, 0.001 to 0.4:1, 0.001 to 0.3:1, 0.001 to 0.2:1, 0.001 to 0.1:1, or a range of about 0.01:1 to 1:0.01, 0.01:1 to 1:0.1, 0.01:1 to 1:0.2, 0.01:1 to 1:0.3, 0.01:1 to 1:0.4, 0.01:1 to 1:0.5, 0.01:1 to 1:0.6, 0.01:1 to 1:0.7, 0.01:1 to 1:0.8, 0.01:1 to 1:0.9, 0.01:1 to 1:1, 0.001 to 0.9:1, 0.001 to 0.8:1, 0.001 to 0.7:1, 0.001 to 0.6:1, 0.001 to 0.5:1, 0.001 to 0.4:1, 0.001 to 0.3:1, 0.001 to 0.2:1, 0.001 to 0.1:1, etc.

Even more preferably, the molar ratio of the mRNA of the adjuvant component to the at least one free mRNA of the second component may be selected e.g. from a range of about 0.01:1 to 1:0.01. Most preferably, the molar ratio of the mRNA of the adjuvant component to the at least one free mRNA of the second component may be selected e.g. from a molar ratio of about 1:1. Any of the above definitions with regard to (w/w) and/or N/P ratio may also apply.

Suitable adjuvants may furthermore be selected from nucleic acids having the formula (IV): GlXmGn, wherein: G is guanosine, uracil or an analogue of guanosine or uracil; X is guanosine, uracil, adenosine, thymidine, cytosine or an analogue of the above-mentioned nucleotides; I is an integer from 1 to 40, wherein when I = 1 G is guanosine or an analogue thereof, when I > 1 at least 50% of the nucleotides are guanosine or an analogue thereof; m is an integer and is at least 3; wherein when m = 3 X is uracil or an analogue thereof, when m > 3 at least 3 successive uracils or analogues of uracil occur; n is an integer from 1 to 40, wherein when n = 1 G is guanosine or an analogue thereof, when n > 1 at least 50% of the nucleotides are guanosine or an analogue thereof.

Other suitable adjuvants may furthermore be selected from nucleic acids having the formula (V): ClXmCn, wherein: C is cytosine, uracil or an analogue of cytosine or uracil; X is guanosine, uracil, adenosine, thymidine, cytosine or an analogue of the above-mentioned nucleotides; I is an integer from 1 to 40, wherein when I = 1 C is cytosine or an analogue thereof, when I > 1 at least 50% of the nucleotides are cytosine or an analogue thereof; m is an integer and is at least 3; wherein when m = 3 X is uracil or an analogue thereof, when m > 3 at least 3 successive uracils or analogues of uracil occur; n is an integer from 1 to 40, wherein when n = 1 C is cytosine or an analogue thereof, when n > 1 at least 50% of the nucleotides are cytosine or an analogue thereof.

According to one further aspect, the present invention may provide a vaccine which is based on at least one mRNA, preferably at least six distinct mRNA species, encoding at least the above defined antigens 5T4, Suvivin, NY-ESO-1, MAGE-C1, MAGE-C2 and MUC-1. Accordingly, the inventive vaccine is based on the same components as the composition as defined above. Insofar, it may be referred to the above disclosure defining the inventive composition. The inventive vaccine may, however, be provided in physically separate form and may be administered by separate administration steps. The inventive vaccine may correspond to the inventive composition, if the mRNA components are provided by one single composition. However, the inventive vaccine may e.g. be provided physically separated. E.g., the mRNA species may be provided such that two separate compositions, which may contain at least one mRNA species each (e.g. three distinct mRNA species) encoding for three distinct antigens, are provided, which may or may not be combined. Also, the inventive vaccine may be a combination of three distinct compositions, each composition comprising at least one mRNA encoding two of the above six antigens. Or, the vaccine may be provided as a combination of at least one mRNA, preferably six mRNAs, each encoding for one of the above defined six antigens. The vaccine may be combined to provide one single composition prior to its use or it may be used such that more than one administration is required to administer the distinct mRNA species coding for the above defined six distinct antigens. If the vaccine contains at least one mRNA molecule, typically at least two mRNA molecules, encoding for the above defined six antigens, it may e.g. be administered by one single administration (combining all mRNA species), by two separate administrations (e.g. each administration administering mRNA molecules encoding for three of the above six antigens), by three, four, five or six administrations (in case all of the mRNA species encode one of the above defined six antigens and are provided physically separate). Accordingly; any combination of mono-, bi- or multicirstronic mRNAs encoding for the above defined six antigens (and optionally further antigens), provided as separate entitities (containing one mRNA species) or as combined entity (containing more than one mRNA species), is understood as a vaccine according to the present invention. According to a particularly preferred embodiment of the inventive vaccine, each of the antigens according to the invention is provided as an individual (monocistronic) mRNA, which is administered separately.

As with the composition according to the present invention, the entities of the vaccine may be provided in liquid and or in dry (e.g. lyophylized) form. They may contain further components, in particular further components allowing for its pharmaceutical use. The inventive vaccine or the inventive composition may, e.g., additionally contain a pharmaceutically acceptable carrier and/or further auxiliary substances and additives and/or adjuvants.

The inventive vaccine or composition typically comprises a safe and effective amount of the at least one mRNA of the composition as defined above encoding the antigens as defined above. As used herein, "safe and effective amount" means an amount of the at least one mRNA of the composition or the vaccine as defined above, that is sufficient to significantly induce a positive modification of lung cancer, preferably of a non-small cell lung cancer (NSCLC) related condition to be treated, more preferably conditions related to the three main sub-types of NSCLC including, without being restricted thereto, squamous cell lung carcinoma, adenocarcinoma and large cell lung carcinoma. At the same time, however, a "safe and effective amount" is small enough to avoid serious side-effects, that is to say to permit a sensible relationship between advantage and risk. The determination of these limits typically lies within the scope of sensible medical judgment. In relation to the inventive vaccine or composition, the expression "safe and effective amount" preferably means an amount of the mRNA (and thus of the encoded antigens) that is suitable for stimulating the adaptive immune system in such a manner that no excessive or damaging immune reactions are achieved but, preferably, also no such immune reactions below a measurable level. Such a "safe and effective amount" of the at least one mRNA of the composition or vaccine as defined above may furthermore be selected in dependence of the type of mRNA, e.g. monocistronic, bi- or even multicistronic mRNA, since a bi- or even multicistronic mRNA may lead to a significantly higher expression of the encoded antigen(s) than use of an equal amount of a monocistronic mRNA. A "safe and effective amount" of the at least one mRNA of the composition or vaccine as defined above will furthermore vary in connection with the particular condition to be treated and also with the age and physical condition of the patient to be treated, the severity of the condition, the duration of the treatment, the nature of the accompanying therapy, of the particular pharmaceutically acceptable carrier used, and similar factors, within the knowledge and experience of the accompanying doctor. The vaccine or composition according to the invention can be used according to the invention for human and also for veterinary medical purposes, as a pharmaceutical composition or as a vaccine.

In a preferred embodiment, the at least one mRNA of the composition, vaccine or kit of parts according to the invention is provided in lyophilized form. Preferably, the at least one lyophilized mRNA is reconstituted in a suitable buffer, advantageously based on an aqueous carrier, prior to administration, e.g. Ringer-Lactate solution, which is preferred, Ringer solution, a phosphate buffer solution. In a preferred embodiment, the composition, the vaccine or the kit of parts according to the invention contains six mRNAs, which are provided separately in lyophilized form (optionally together with at least one further additive) and which are preferably reconstituted separately in a suitable buffer (such as Ringer-Lactate solution) prior to its use so as to allow individual administration of each of the six (monocistronic) mRNAs.

The vaccine or composition according to the invention may typically contain a pharmaceutically acceptable carrier. The expression "pharmaceutically acceptable carrier" as used herein preferably includes the liquid or non-liquid basis of the inventive vaccine. If the inventive vaccine is provided in liquid form, the carrier will be water, typically pyrogenfree water; isotonic saline or buffered (aqueous) solutions, e.g phosphate, citrate etc. buffered solutions. Particularly for injection of the inventive vaccine, water or preferably a buffer, more preferably an aqueous buffer, may be used, containing a sodium salt, preferably at least 50 mM of a sodium salt, a calcium salt, preferably at least 0,01 mM of a calcium salt, and optionally a potassium salt, preferably at least 3 mM of a potassium salt. According to a preferred embodiment, the sodium, calcium and, optionally, potassium salts may occur in the form of their halogenides, e.g. chlorides, iodides, or bromides, in the form of their hydroxides, carbonates, hydrogen carbonates, or sulfates, etc.. Without being limited thereto, examples of sodium salts include e.g. NaCl, Nal, NaBr, Na2CO3, NaHCO3, Na2SO4, examples of the optional potassium salts include e.g. KCl, KI, KBr, K2CO3, KHCO3, K2SO4, and examples of calcium salts include e.g. CaCl2, Cal2, CaBr2, CaCO3, CaSO4, Ca(OH)2. Furthermore, organic anions of the aforementioned cations may be contained in the buffer. According to a more preferred embodiment, the buffer suitable for injection purposes as defined above, may contain salts selected from sodium chloride (NaCl), calcium chloride (CaCl2) and optionally potassium chloride (KCI), wherein further anions may be present additional to the chlorides. CaCl2 can also be replaced by another salt like KCI. Typically, the salts in the injection buffer are present in a concentration of at least 50 mM sodium chloride (NaCl), at least 3 mM potassium chloride (KCl) and at least 0,01 mM calcium chloride (CaCl2). The injection buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of cells due to osmosis or other concentration effects. Reference media are e.g. in "in vivo" methods occurring liquids such as blood, lymph, cytosolic liquids, or other body liquids, or e.g. liquids, which may be used as reference media in "in vitro" methods, such as common buffers or liquids. Such common buffers or liquids are known to a skilled person. Ringer-Lactate solution is particularly preferred as a liquid basis.

However, one or more compatible solid or liquid fillers or diluents or encapsulating compounds may be used as well, which are suitable for administration to a person. The term "compatible" as used herein means that the constituents of the inventive vaccine are capable of being mixed with the the at least one mRNA of the composition, encoding at least six antigens as defined above, in such a manner that no interaction occurs which would substantially reduce the pharmaceutical effectiveness of the inventive vaccine under typical use conditions. Pharmaceutically acceptable carriers, fillers and diluents must, of course, have sufficiently high purity and sufficiently low toxicity to make them suitable for administration to a person to be treated. Some examples of compounds which can be used as pharmaceutically acceptable carriers, fillers or constituents thereof are sugars, such as, for example, lactose, glucose, trehalose and sucrose; starches, such as, for example, corn starch or potato starch; dextrose; cellulose and its derivatives, such as, for example, sodium carboxymethylcellulose, ethylcellulose, cellulose acetate; powdered tragacanth; malt; gelatin; tallow; solid glidants, such as, for example, stearic acid, magnesium stearate; calcium sulfate; vegetable oils, such as, for example, groundnut oil, cottonseed oil, sesame oil, olive oil, corn oil and oil from theobroma; polyols, such as, for example, polypropylene glycol, glycerol, sorbitol, mannitol and polyethylene glycol; alginic acid.

The choice of a pharmaceutically acceptable carrier is determined, in principle, by the manner in which the inventive vaccine is administered. The inventive vaccine can be administered, for example, systemically or locally. Routes for systemic administration in general include, for example, transdermal, oral, parenteral routes, including subcutaneous, intravenous, intramuscular, intraarterial, intradermal and intraperitoneal injections and/or intranasal administration routes. Routes for local administration in general include, for example, topical administration routes but also intradermal, transdermal, subcutaneous, or intramuscular injections or intralesional, intracranial, intrapulmonal, intracardial, and sublingual injections. More preferably, vaccines may be administered by an intradermal, subcutaneous, or intramuscular route, preferably by injection, which may be needle-free and/or needle injection. Compositions/vaccines are therefore preferably formulated in liquid or solid form. The suitable amount of the inventive vaccine to be administered can be determined by routine experiments with animal models. Such models include, without implying any limitation, rabbit, sheep, mouse, rat, dog and non-human primate models. Preferred unit dose forms for injection include sterile solutions of water, physiological saline or mixtures thereof. The pH of such solutions should be adjusted to about 7.4. Suitable carriers for injection include hydrogels, devices for controlled or delayed release, polylactic acid and collagen matrices. Suitable pharmaceutically acceptable carriers for topical application include those which are suitable for use in lotions, creams, gels and the like. If the inventive vaccine is to be administered perorally, tablets, capsules and the like are the preferred unit dose form. The pharmaceutically acceptable carriers for the preparation of unit dose forms which can be used for oral administration are well known in the prior art. The choice thereof will depend on secondary considerations such as taste, costs and storability, which are not critical for the purposes of the present invention, and can be made without difficulty by a person skilled in the art.

The inventive vaccine or composition can additionally contain one or more auxiliary substances in order to further increase the immunogenicity. A synergistic action of the at least one mRNA of the composition or vaccine as defined above and of an auxiliary substance, which may be optionally be co-formulated (or separately formulated) with the inventive vaccine or composition as described above, is preferably achieved thereby. Depending on the various types of auxiliary substances, various mechanisms can come into consideration in this respect. For example, compounds that permit the maturation of dendritic cells (DCs), for example lipopolysaccharides, TNF-alpha or CD40 ligand, form a first class of suitable auxiliary substances. In general, it is possible to use as auxiliary substance any agent that influences the immune system in the manner of a "danger signal" (LPS, GP96, etc.) or cytokines, such as GM-CFS, which allow an immune response produced by the immune-stimulating adjuvant according to the invention to be enhanced and/or influenced in a targeted manner. Particularly preferred auxiliary substances are cytokines, such as monokines, lymphokines, interleukins or chemokines, that - additional to induction of the adaptive immune response by the encoded at least six antigens - promote the innate immune response, such as IL-1, IL-2, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, INF-alpha, IFN-beta, INF-gamma, GM-CSF, G-CSF, M-CSF, LT-beta or TNF-alpha, growth factors, such as hGH. Preferably, such immunogenicity increasing agents or compounds are provided separately (not co-formulated with the inventive vaccine or composition) and administered individually.

Further additives which may be included in the inventive vaccine or composition are emulsifiers, such as, for example, Tween; wetting agents, such as, for example, sodium lauryl sulfate; colouring agents; taste-imparting agents, pharmaceutical carriers; tablet-forming agents; stabilizers; antioxidants; preservatives.

The inventive vaccine or composition can also additionally contain any further compound, which is known to be immune-stimulating due to its binding affinity (as ligands) to human Toll-like receptors TLR1, TLR2, TLR3, TLR4, TLR5, TLR6, TLR7, TLR8, TLR9, TLR10, or due to its binding affinity (as ligands) to murine Toll-like receptors TLR1, TLR2, TLR3, TLR4, TLR5, TLR6, TLR7, TLR8, TLR9, TLR10, TLR11, TLR12 or TLR13.

Another class of compounds, which may be added to an inventive vaccine or composition in this context, may be CpG nucleic acids, in particular CpG-RNA or CpG-DNA. A CpG-RNA or CpG-DNA can be a single-stranded CpG-DNA (ss CpG-DNA), a double-stranded CpG-DNA (dsDNA), a single-stranded CpG-RNA (ss CpG-RNA) or a double-stranded CpG-RNA (ds CpG-RNA). The CpG nucleic acid is preferably in the form of CpG-RNA, more preferably in the form of single-stranded CpG-RNA (ss CpG-RNA). The CpG nucleic acid preferably contains at least one or more (mitogenic) cytosine/guanine dinucleotide sequence(s) (CpG motif(s)). According to a first preferred alternative, at least one CpG motif contained in these sequences, that is to say the C (cytosine) and the G (guanine) of the CpG motif, is unmethylated. All further cytosines or guanines optionally contained in these sequences can be either methylated or unmethylated. According to a further preferred alternative, however, the C (cytosine) and the G (guanine) of the CpG motif can also be present in methylated form.

Preferably, the above compounds are formulated and administered separately from the above composition or vaccine (of the invention) containing the at least one mRNA encoding at least the above defined six antigens.

According to a further preferred object of the present invention, the inventive composition or vaccine may be used according to the invention (for the preparation of a medicament) for the treatment of lung cancer and diseases or disorders related thereto, preferably of a non-small cell lung cancer (NSCLC) related condition to be treated, more preferably conditions related to the three main sub-types of NSCLC including, without being restricted thereto, squamous cell lung carcinoma, adenocarcinoma and large cell lung carcinoma.

According to a further preferred object of the present invention, the inventive vaccine or the inventive composition containing the at least one mRNA encoding the antigens as defined herein may be used for the treatment of lung cancer and diseases or disorders related thereto, preferably of a non-small cell lung cancer (NSCLC) related condition to be treated, more preferably conditions related to the three main sub-types of NSCLC including, without being restricted thereto, squamous cell lung carcinoma, adenocarcinoma and large cell lung carcinoma.

In this context, also included in the present invention are methods of treating lung cancer, preferably of non-small cell lung cancer, and diseases or disorders related thereto, preferably of a non-small cell lung cancer (NSCLC) related condition to be treated, more preferably conditions related to the three main sub-types of NSCLC including, without being restricted thereto, squamous cell lung carcinoma, adenocarcinoma and large cell lung carcinoma, by administering to a subject in need thereof a pharmaceutically effective amount of an inventive vaccine, or a pharmaceutically effective amount of an inventive composition. Such a method typically comprises an optional first step of preparing the inventive composition, or the inventive vaccine, and a second step, comprising administering (a pharmaceutically effective amount of) said inventive composition or said inventive vaccine to a patient in need thereof. A subject in need thereof will typically be a mammal. In the context of the present invention, the mammal is preferably selected from the group comprising, without being limited thereto, e.g. goat, cattle, swine, dog, cat, donkey, monkey, ape, a rodent such as a mouse, hamster, rabbit and, particularly, human, wherein the mammal typically suffers from lung cancer, preferably of non-small cell lung cancer, and diseases or disorders related thereto, preferably a non-small cell lung cancer (NSCLC) related condition to be treated, more preferably conditions related to the three main sub-types of NSCLC including, without being restricted thereto, squamous cell lung carcinoma, adenocarcinoma and large cell lung carcinoma.

The invention relates also to the use of the inventive composition or the at least one mRNA encoding the antigens as defined herein (for the preparation of an inventive vaccine), preferably for eliciting an immune response in a mammal, preferably for the treatment of lung cancer, more preferably for the treatment of a non-small cell lung cancer (NSCLC) related condition as defined herein.

Preferably, the subject receiving the inventive composition or vaccine is a patient with stage 0, I (IA and/or IB), II (IIA and/or IIB), III (IIIA and/or IIIB) or stage IV non-small cell lung cancer.

In a particular embodiment, the subject receiving the inventive composition or vaccine is a patient with stage III or stage IV non-small cell lung cancer.

Additionally, the subject receiving the inventive composition or vaccine may be a patient with non-small cell lung cancer receiving chemotherapy (e.g. first-line or second-line chemotherapy), radiotherapy, chemoradiation (combination of chemotherapy and radiotherapy), tyrosine kinase inhibitors (e.g. EGFR tyrosine kinase inhibitors), antibody therapy and/or PD1 pathway inhibitors, or a patient, who has achieved partial response or stable disease after having received one or more of the treatments specified above.

In this context, a PD-1 pathway inhibitor is preferably defined herein as a compound capable of impairing the PD-1 pathway signaling, preferably signaling mediated by the PD-1 receptor. Therefore, the PD-1 pathway inhibitor may be any inhibitor directed against any member of the PD-1 pathway capable of antagonizing PD-1 pathway signaling. In this context, the inhibitor may be an antagonistic antibody, targeting any member of the PD-1 pathway, preferably directed against PD-1 receptor, PD-L1 or PD-L2. This antagonistic antibody may also be encoded by a nucleic acid. Also, the PD-1 pathway inhibitor may be a fragment of the PD-1 receptor or the PD1-receptor blocking the activity of PD1 ligands. B7-1 or fragments thereof may act as PD1-inhibiting ligands as well. Furthermore, the PD-1 pathway inhibitor may be siRNA (small interfering RNA) or antisense RNA directed against a member of the PD-1 pathway, preferably PD-1, PD-L1 or PD-L2. Additionally, a PD-1 pathway inhibitor may be a protein comprising (or a nucleic acid coding for) an amino acid sequence capable of binding to PD-1 but preventing PD-1 signaling, e.g. by inhibiting PD-1 and B7-H1 or B7-DL interaction. Additionally, a PD-1 pathway inhibitor may be a small molecule inhibitor capable of inhibiting PD-1 pathway signaling, e.g. a PD-1 binding peptide or a small organic molecule.

Furthermore in this context, a tyrosine kinase inhibitor is preferably defined herein as a compound capable of impairing signalling of one or more tyrosine kinases, preferably of one or more growth factor tyrosine kinases. Preferably, a tyrosine kinase inhibitor, as used in this context, is an inhibitor of epidermal growth factor receptor (EGFR) tyrosine kinase. In a preferred embodiment, a tyrosine kinase inhibitor, as used herein, is an oral EGFR tyrosine kinase inhibitor. In a further preferred embodiment, a tyrosine kinase inhibitor is selected from the group of erlotinib, gefitinib or afatinib. In another embodiment, a tyrosine kinase inhibitor, as used in this context, is an ALK inhibitor, preferably crizotinib or ceritinib.

As used herein, an antibody used in antibody therapy is preferably directed against a growth factor or a growth factor recetor, which is preferably functionally linked with a tyrosine kinase. Preferably, an antibody, in this context, is directed against vascular endothelial growth factor (VEGF) or against epidermal growth factor receptor (EGFR). In a preferred embodiment, bevacizumab is used in an antibody therapy. In another embodiment, cetuximab is used in an antibody therapy.

In a preferred embodiment, the subject receiving the inventive composition or vaccine is a patient before or after surgery (e.g. lobectomy), wherein the patient preferably has NSCLC in stage I or II.

In a further preferred embodiment, the subject receiving the inventive composition or vaccine is a patient receiving radiotherapy, or a patient, who has achieved partial response (PR) or stable disease (SD) after radiotherapy wherein the patient preferably has NSCLC in stage I or II.

According to a particular preferred embodiment, the subject receiving the inventive composition or vaccine is a patient receiving chemotherapy, preferably a platinum-based chemotherapy or a platinum-based combination chemotherapy (e.g. cisplatin, carboplatin, cisplatin in combination with vinorelbine, cisplatin in combination with etoposide, cisplatin in combination with gemcitabine, cisplatin in combination with taxanes, cisplatin or carboplatin in combination with premetrexed, or carboplatin in combination with paclitaxel), or a patient, who has achieved partial response (PR) or stable disease (SD) after chemotherapy, wherein the patient preferably has NSCLC in stage III or IV.

In another preferred embodiment, the subject receiving the inventive composition or vaccine is a patient receiving chemotherapy, preferably a platinum-based chemotherapy or a platinum-based combination chemotherapy (e.g. cisplatin, carboplatin, cisplatin in combination with vinorelbine, cisplatin in combination with etoposide, cisplatin in combination with gemcitabine, cisplatin in combination with taxanes, cisplatin or carboplatin in combination with premetrexed, or carboplatin in combination with paclitaxel) in combination with radiotherapy (chemoradiation), or a patient, who has achieved partial response (PR) or stable disease (SD) after chemoradiation, wherein the patient preferably has NSCLC in stage III (preferably locally advanced) or IV.

According to a further preferred embodiment, the subject receiving the inventive composition or vaccine is a patient receiving only one chemotherapy, preferably gemcitabine, taxanes, premetrexed, paclitaxel, vinorelbine, or etoposide, preferably as second-line or third line treatment, or a patient, who has achieved partial response (PR) or stable disease (SD) after such second-line or third-line treatment.

In a preferred embodiment, the subject receiving the inventive composition or vaccine is a patient with stage III or stage IV non-small cell lung cancer (NSCLC) after first-line and optional second-line chemotherapy (e.g. platinum-based chemotherapy or platinum-based combination chemotherapy (combination of platinum-based chemotherapy with at least one further chemotherapeutic agent)) or first-line and optional second-line chemoradiation, wherein the patient has preferably achieved partial response (PR) or stable disease (SD) after first-line and optional second-line chemotherapy.

According to a preferred embodiment, the subject receiving the inventive composition or vaccine is a patient with stage III or IV non-small cell lung cancer (NSCLC) and non-squamous histology, with or without activating epidermal growth factor receptor (EGFR) mutations.

According to another preferred embodiment, the subject receiving the inventive composition or vaccine is a patient with stage III or IV non-small cell lung cancer (NSCLC) and squamous histology, with or without activating epidermal growth factor receptor (EGFR) mutations.

In a particularly preferred embodiment, the subject receiving the inventive composition or vaccine is a patient with stage III or IV non-small cell lung cancer (NSCLC) and non-squamous histology, preferably without activating epidermal growth factor receptor (EGFR) mutations, who has preferably achieved partial response (PR) or stable disease (SD) after first-line chemotherapy using platinum or platinum-based combination chemotherapy (e.g. platinum and pemetrexed).

In another embodiment, the subject receiving the inventive composition or vaccine is a patient with stage III or IV NSCLC and squamous cell histology, who has preferably achieved partial response (PR) or stable disease (SD) after first-line chemotherapy using platinum or platinum-based combination chemotherapy (e.g. platinum and pemetrexed).

Even more preferably, the subject receiving the inventive composition or vaccine is a patient suffering from metastatic lung cancer, preferably metastatic non-small cell lung cancer.

In a further preferred embodiment, the subject receiving the inventive composition or vaccine is a patient with stage III locoregionally advanced NSCLC, preferably receiving treatment with concomitant chemoradiation (e.g. as defined above), or has achieved a response or stable disease (non-progression) after chemoradiation (as defined herein).

In a further preferred embodiment, the subject receiving the inventive composition or vaccine is a patient with stage III or IV NSCLC, irrespective of histological or molecular subtype and is preferably receiving concomitant treatment with a PD-1 pathway inhibitor or has achieved a response or stable disease (non-progression) after treatment with a PD-1 pathway inhibitor.

Furthermore, according to a specific embodiment, the subject receiving the inventive composition or vaccine is a patient receiving an antibody or a combination of chemotherapy and an antibody, preferably bevacizumab or a combination of bevacizumab with chemotherapy, preferably a platinum-based chemotherapy, or a patient, who has achieved a response or stable disease after this treatment.

According to another particularly preferred embodiment, the subject receiving the inventive composition or vaccine is a patient receiving a tyrosine kinase inhibitor, preferably an EGFR tyrosine kinase inhibitor (e.g. erlotinib, gefitinib or afatinib), and preferably as second-line treatment after first-line chemotherapy, or a patient, who has achieved a response or stable disease after EGFR tyrosine kinase inhibitor therapy.

According to another particularly preferred embodiment, the subject receiving the inventive composition or vaccine is a patient harboring an activating EGFR mutation and receiving a tyrosine kinase inhibitor, preferably an EGFR tyrosine kinase inhibitor (e.g. erlotinib, gefitinib or afatinib), or a patient, who has achieved a response or stable disease after EGFR tyrosine kinase inhibitor therapy.

In another preferred embodiment, the subject receiving the inventive composition or vaccine is a patient with stage III or IV NSCLC and preferably with a non-squamous histology, preferably harboring an activating EGFR mutation and is preferably receiving concomitant treatment with an EGFR tyrosine kinase inhibitor, or a patient, who has achieved a response or stable disease after receiving treatment with an EGFR tyrosine kinase inhibitor (e.g. erlotinib, gefitinib or afatinib or other EGFR tyrosine kinase inhibitors).

In another embodiment, the subject receiving the inventive composition or vaccine is a patient receiving the tyrosine kinase inhibitor crizotinib or ceritinib or other ALK inhibitors.

Similarly, the invention also relates to the use of the inventive vaccine per se or the at least one mRNA encoding the antigens as defined herein for eliciting an adaptive immune response in a mammal, preferably for the treatment of lung cancer, more preferably for the treatment of a non-small cell lung cancer (NSCLC) related condition as defined herein.

Prevention or treatment of lung cancer in a patient in need thereof, preferably of a non-small cell lung cancer, and diseases or disorders related thereto, may be carried out by administering the inventive composition and/or the inventive vaccine at once or in a time staggered manner, e.g. as a kit of parts, each part containing at least one preferably different antigen. Preferably, each of the antigens is administered separately, i.e. each antigen is administered to a different part or region of the body of the subject to be treated, preferably simultaneously or within the same short time-frame, respectively. In a preferred embodiment, the individual mRNAs are administered distributed over the subject's four limbs (i.e. left/right arm and leg). Preferably, the administration (of all at least one mRNAs) occurs within an hour, more preferably within 30 minutes, even more preferably within 15, 10, 5, 4, 3, or 2 minutes or even within 1 minute.

For administration, preferably any of the administration routes may be used as defined above. In particular, an administration route is used, which is suitable for treating lung cancer, preferably non-small cell lung cancer, and diseases or disorders related thereto, by inducing or enhancing an adaptive immune response on the basis of the antigens encoded by the at least one mRNA of the inventive composition. Administering of the inventive composition and/or the inventive vaccine may then occur prior, concurrent and/or subsequent to administering another inventive composition and/or inventive vaccine as defined herein which may - in addition - contain another combination of mRNAs encoding different antigens, wherein each antigen encoded by the at least one mRNA of the inventive composition may preferably be suitable for the therapy of lung cancer, preferably of non-small cell lung cancer, and diseases or disorders related thereto. In this context, a therapy as defined herein may also comprise the modulation of a disease associated to lung cancer, preferably of non-small cell lung cancer, and of diseases or disorders related thereto.

According to one further aspect, the present invention furthermore comprises the use of the inventive composition or the at least one mRNA encoding the antigens as defined herein (for the preparation of an (inventive) vaccine) for modulating, preferably to induce or enhance, an immune response in a mammal as defined above, more preferably to treat and/or to support the treatment of lung cancer, preferably of non-small cell lung cancer, or of diseases or disorders related thereto. In this context, support of the treatment of lung cancer, may be any combination of a conventional lung cancer therapy method of such as surgery, radiation therapy, chemotherapy (e.g. first-line or second-line chemotherapy), chemoradiation, treatment with tyrosine kinase inhibitors, treatment with PD-1 pathway inhibitors , antibody therapy or some combination of these, and a therapy using the inventive composition as defined herein. Support of the treatment of lung cancer may be also envisaged in any of the other embodiments defined herein. Accordingly, any use of the inventive composition or vaccine in co-therapy with any of the above therapeutic approaches, in particular in combination with surgery, radiation therapy, chemotherapy, chemoradiation, and/or treatment with kinase inhibitors or antibodies is within the scope of the present invention.

In a preferred embodiment, the composition or vaccine according to the invention is used in a treatment of lung cancer, which further comprises chemotherapy (e.g. first-line or second-line chemotherapy), radiotherapy, chemoradiation (combination of chemotherapy and radiotherapy), tyrosine kinase inhibitors (e.g. EGFR tyrosine kinase inhibitors), antibody therapy and/or PD1 pathway inhibitors, or a patient, who has achieved partial response or stable disease after having received one or more of the treatments specified above.

In this context, a PD-1 pathway inhibitor is preferably defined herein as a compound capable of impairing the PD-1 pathway signaling, preferably signaling mediated by the PD-1 receptor. Therefore, the PD-1 pathway inhibitor may be any inhibitor directed against any member of the PD-1 pathway capable of antagonizing PD-1 pathway signaling. In this context, the inhibitor may be an antagonistic antibody, targeting any member of the PD-1 pathway, preferably directed against PD-1 receptor, PD-L1 or PD-L2. This antagonistic antibody may also be encoded by a nucleic acid. Also, the PD-1 pathway inhibitor may be a fragment of the PD-1 receptor or the PD1-receptor blocking the activity of PD1 ligands. B7-1 or fragments thereof may act as PD1-inhibiting ligands as well. Furthermore, the PD-1 pathway inhibitor may be siRNA (small interfering RNA) or antisense RNA directed against a member of the PD-1 pathway, preferably PD-1, PD-L1 or PD-L2. Additionally, a PD-1 pathway inhibitor may be a protein comprising (or a nucleic acid coding for) an amino acid sequence capable of binding to PD-1 but preventing PD-1 signaling, e.g. by inhibiting PD-1 and B7-H1 or B7-DL interaction. Additionally, a PD-1 pathway inhibitor may be a small molecule inhibitor capable of inhibiting PD-1 pathway signaling, e.g. a PD-1 binding peptide or a small organic molecule.

As used herein, an antibody used in antibody therapy is preferably directed against a growth factor or a growth factor recetor, which is preferably functionally linked with a tyrosine kinase. Preferably, an antibody, in this context, is directed against vascular endothelial growth factor (VEGF) or against epidermal growth factor receptor (EGFR). In a preferred embodiment, bevacizumab is used in an antibody therapy. In another embodiment, cetuximab is used in an antibody therapy.

Preferably, the composition or vaccine according to the invention is used in a treatment of lung cancer, which further comprises radiation therapy, wherein at least one tumor lesion is eligible for radiation. According to a preferred embodiment, a tumor lesion eligible for radiation is selected from the group consisting of bone metastases, lymph nodes in the paraclavicular, axillary or cervical regions, skin or subcutaneous metastases and thoracic lesions (centrally located lung tumor, lymph nodes in the lung hilus or mediastinum). Preferably, radiation is administered in 4 daily fractions of 5 GY each to be administered within one week, preferably from Day 9-12.

In a further preferred embodiment, the composition or vaccine according to the invention is used in a treatment of lung cancer, which further comprises administration of a kinase inhibitor. Therein, a kinase inhibitor is preferably a tyrosine kinase inhibitor, even more preferably a growth factor tyrosine kinase inhibitor, most preferably an oral EGFR tyrosine kinase inhibitor, such as, for instance, gefitinib, erlotinib or afatinib. Furthermore in this context, a tyrosine kinase inhibitor is preferably defined herein as a compound capable of impairing signalling of one or more tyrosine kinases, preferably of one or more growth factor tyrosine kinases. In another embodiment, a tyrosine kinase inhibitor, as used in this context, is an ALK inhibitor, preferably crizotinib or ceritinib.
In another preferred embodiment, the composition or vaccine according to the invention is used in a treatment of lung cancer, which further comprises administration of a chemotherapeutic agent, such as a platinum-based compound (e.g carboplatin, cisplatin), pemetrexed, gemcitabine, taxanes, vinorelbine, etoposide docetaxel or paclitaxel. Even more preferably, the composition or vaccine according to the invention is used in a subject receiving chemotherapy, preferably platinum-based combination chemotherapy preferably as defined above, which is preferably further combined with radiation therapy (chemoradiation).

Preferably, the composition or vaccine according to the invention is used in a combination treatment of lung cancer, wherein a patient has a stage 0, I (IA and/or IB), II (IIA and/or IIB), III (IIIA and/or IIIB) or stage IV non-small cell lung cancer.

In a preferred embodiment, the composition or vaccine according to the invention is used in a treatment of lung cancer in a patient before or after surgery (e.g. lobectomy), wherein the patient preferably has NSCLC in stage I or II.

In a further preferred embodiment, the composition or vaccine according to the invention is used in a treatment of lung cancer in a patient receiving radiotherapy, or in a patient, who has achieved partial response (PR) or stable disease (SD) after radiotherapy, wherein the patient preferably has NSCLC in stage I or II.

According to a particular preferred embodiment, the composition or vaccine according to the invention is used in a treatment of lung cancer in a patient receiving chemotherapy, preferably a platinum-based chemotherapy or a platinum-based combination chemotherapy (e.g. cisplatin, carboplatin, cisplatin in combination with vinorelbine, cisplatin in combination with etoposide, cisplatin in combination with gemcitabine, cisplatin in combination with taxanes, cisplatin or carboplatin in combination with premetrexed, or carboplatin in combination with paclitaxel), or in a patient, who has achieved partial response (PR) or stable disease (SD) after chemotherapy, wherein the patient preferably has NSCLC in stage III or IV.

In another preferred embodiment, the composition or vaccine according to the invention is used in a treatment of lung cancer in a patient receiving chemotherapy, preferably a platinum-based chemotherapy or a platinum-based combination chemotherapy (e.g. cisplatin, carboplatin, cisplatin in combination with vinorelbine, cisplatin in combination with etoposide, cisplatin in combination with gemcitabine, cisplatin in combination with taxanes, cisplatin or carboplatin in combination with premetrexed, or carboplatin in combination with paclitaxel) in combination with radiotherapy (chemoradiation), or in a patient, who has achieved partial response (PR) or stable disease (SD) after chemoradiation, wherein the patient preferably has NSCLC in stage III (preferably locally advanced) or IV.

According to a further preferred embodiment, the composition or vaccine according to the invention is used in a treatment of lung cancer in a patient receiving only one chemotherapy, preferably gemcitabine, taxanes, premetrexed, paclitaxel, vinorelbine, or etoposide, preferably as second-line or third line treatment, or in a patient, who has achieved partial response (PR) or stable disease (SD) after such second-line or third-line treatment.

In a preferred embodiment, the composition or vaccine according to the invention is used in a treatment of lung cancer in a patient with stage III or stage IV non-small cell lung cancer (NSCLC) after first-line and optional second-line chemotherapy (e.g. platinum-based chemotherapy or platinum-based combination chemotherapy (combination of platinum-based chemotherapy with at least one further chemotherapeutic agent)) or first-line and optional second-line chemoradiation, wherein the patient has preferably achieved partial response (PR) or stable disease (SD) after first-line and optional second-line chemotherapy.

According to a preferred embodiment, the composition or vaccine according to the invention is used in a treatment of lung cancer in a patient with stage III or IV non-small cell lung cancer (NSCLC) and non-squamous histology, with or without activating epidermal growth factor receptor (EGFR) mutations.

According to another preferred embodiment, the composition or vaccine according to the invention is used in a treatment of lung cancer in a patient with stage III or IV non-small cell lung cancer (NSCLC) and squamous histology, with or without activating epidermal growth factor receptor (EGFR) mutations.

In a particularly preferred embodiment the composition or vaccine according to the invention is used in a treatment of lung cancer in a patient with stage III or IV non-small cell lung cancer (NSCLC) and non-squamous histology, preferably without activating epidermal growth factor receptor (EGFR) mutations, wherein the patient has preferably achieved partial response (PR) or stable disease (SD) after first-line chemotherapy using platinum or platinum-based combination chemotherapy (e.g. platinum and pemetrexed).

In another embodiment, the composition or vaccine according to the invention is used in a treatment of lung cancer in a patient with stage III or IV NSCLC and squamous cell histology, wherein the patient has preferably achieved partial response (PR) or stable disease (SD) after first-line chemotherapy using platinum or platinum-based combination chemotherapy (e.g. platinum and pemetrexed).

Even more preferably, the composition or vaccine according to the invention is used in a treatment of lung cancer in a patient suffering from metastatic lung cancer, preferably metastatic non-small cell lung cancer.

In a further preferred embodiment, the composition or vaccine according to the invention is used in a treatment of lung cancer in a patient with stage III locoregionally advanced NSCLC, wherein the patient preferably receives concomitant treatment with chemoradiation (e.g. as defined above), or wherein the patient has achieved a response or stable disease (non-progression) after chemoradiation (as defined herein).

In a further preferred embodiment, the composition or vaccine according to the invention is used in a treatment of lung cancer in a patient with stage III or IV NSCLC, irrespective of histological or molecular subtype, wherein the patient is preferably receiving concomitant treatment with a PD-1 pathway inhibitor or has achieved a response or stable disease (non-progression) after treatment with a PD-1 pathway inhibitor.

Furthermore, according to a specific embodiment, the composition or vaccine according to the invention is used in a treatment of lung cancer in a patient receiving an antibody or a combination of chemotherapy and an antibody, preferably in a patient receiving bevacizumab or a combination of bevacizumab with chemotherapy, preferably a platinum-based chemotherapy, or in a patient, who has achieved a response or stable disease after this treatment.

According to another particularly preferred embodiment, the composition or vaccine according to the invention is used in a treatment of lung cancer in a patient receiving a tyrosine kinase inhibitor, preferably an EGFR tyrosine kinase inhibitor (e.g. erlotinib, gefitinib or afatinib), and preferably as second-line treatment after first-line chemotherapy, or in a patient, who has achieved a response or stable disease after EGFR tyrosine kinase inhibitor therapy.

According to another particularly preferred embodiment, the composition or vaccine according to the invention is used in a treatment of lung cancer in a patient harboring an activating EGFR mutation and receiving a tyrosine kinase inhibitor, preferably an EGFR tyrosine kinase inhibitor (e.g. erlotinib, gefitinib or afatinib), or in a patient, who has achieved a response or stable disease after EGFR tyrosine kinase inhibitor therapy.

In another preferred embodiment, the composition or vaccine according to the invention is used in a treatment of lung cancer in a patient with stage III or IV NSCLC, preferably with a non-squamous histology, preferably harboring an activating EGFR mutation and wherein the patient is preferably receiving concomitant treatment with an EGFR tyrosine kinase inhibitor, or in a patient, who has achieved a response or stable disease after receiving treatment with an EGFR tyrosine kinase inhibitor (e.g. erlotinib, gefitinib or afatinib or other EGFR tyrosine kinase inhibitors).

In another embodiment, the composition or vaccine according to the invention is used in a treatment of lung cancer in a patient receiving the tyrosine kinase inhibitor crizotinib or ceritinib or other ALK inhibitors.

The immunization protocol for the immunization of a subject against the combination of at least six antigens as defined herein typically comprises a series of single doses or dosages of the inventive composition or the inventive vaccine. A single dosage, as used herin, refers to the initial/first dose, a second dose or any futher doses, respectively, which are preferably administered in order to "boost" the immune reaction. In this context, each single dosage comprises the administration of all of the at least six antigens according to the invention, wherein the interval between the administration of two single dosages can vary from at least one day, preferably 2, 3, 4, 5, 6 or 7 days, to at least one week, preferably 2, 3, 4, 5, 6, 7 or 8 weeks. The intervals between single dosages may be constant or vary over the course of the immunization protocol, e.g. the intervals may be shorter in the beginning and longer towards the end of the protocol. Depending on the total number of single dosages and the interval between single dosages, the immunization protocol may extend over a period of time, which preferably lasts at least one week, more preferably several weeks (e.g. 2, 3, 4, 5, 6, 7, 8, 9, 10, 11 or 12 weeks), even more preferably several months (e.g. 3, 4, 5, 6, 7, 8, 9, 10, 11, 12, 18 or 24 months). Each single dosage encompasses the administration of all of the at least six antigens as defined herein and may therefore involve at least one, preferably 1, 2, 3, 4, 5, 6, 7, 8, 9, 10, 11 or 12 injections. In the case, where the composition according to the invention is administered, a single dosage typically comprises one injection. In the case, where the vaccine comprises separate mRNA formulations encoding the respective antigens according to the invention, the minimum number of injections carried out during the administration of a single dosage corresponds to the number of separate components of the vaccine. In certain embodiments, the administration of a single dosage may encompass more than one injection for each component of the vaccine (e.g. a specific mRNA formulation comprising a mRNA encoding, for instance, one of the six antigens according to the invention). For example, parts of the total volume of an individual component of the vaccine may be injected into different body parts, thus involving more than one injection. In a more specific example, a single dosage of a vaccine comprising six separate mRNA formulations, each of which is administered in two different body parts, comprises twelve injections. Typically, a single dosage comprises all injections required to administer all components of the vaccine, wherein a single component may be involve more than one injection as outlined above. In the case, where the administration of a single dosage of the vaccine according to the invention encompasses more than one injection, the injection are carried out essentially simultaneously or concurrently, i.e. typically in a time-staggered fashion within the time-frame that is required for the practitioner to carry out the single injection steps, one after the other. The administration of a single dosage therefore preferably extends over a time period of several minutes, e.g. 2, 3, 4, 5, 10, 15, 30 or 60 minutes.

Administration of the inventive composition or the at least one mRNA encoding the antigens as defined herein or the inventive vaccine may be carried out in a time staggered treatment. A time staggered treatment may be e.g. administration of the inventive composition or the at least one mRNA encoding the antigens as defined herein or the inventive vaccine prior, concurrent and/or subsequent to a conventional therapy of lung cancer, preferably of non-small cell lung cancer, and diseases or disorders related thereto, e.g. by administration of the inventive medicament or the inventive composition or vaccine prior, concurrent and/or subsequent to a therapy or an administration of a therapeutic suitable for the treatment of lung cancer, preferably of non-small cell lung cancer, and diseases or disorders related thereto. Such time staggered treatment may be carried out using e.g. a kit, preferably a kit of parts as defined below.

Time staggered treatment may additionally or alternatively also comprise an administration of the inventive composition or vaccine, preferably of the at least one mRNA encoding the antigens as defined above, in a form, wherein the at least one mRNA encoding the antigens as defined above, preferably forming part of the inventive composition or vaccine, is administered parallel, prior or subsequent to another at least one mRNA encoding the antigens as defined above, preferably forming part of the same inventive composition or vaccine. Preferably, the administration (of all at least one mRNAs) occurs within an hour, more preferably within 30 minutes, even more preferably within 15, 10, 5, 4, 3, or 2 minutes or even within 1 minute. Such time staggered treatment may be carried out using e.g. a kit, preferably a kit of parts as defined below.

In a preferred embodiment, the composition or vaccine is administered repeatedly, wherein each administration preferably comprises individual administration of the at least one mRNA according to the invention. At each time point of administration, the at least one mRNA may be administered more than once (e.g. 2 or 3 times). In a particularly preferred embodiment of the invention, six mRNAs (each encoding one of the antigens as defined above) are administered at each time point, wherein each mRNA is administered twice by injection, thus resulting in twelve injections distributed over the four limbs.

According to a final embodiment, the present invention also provides kits, particularly kits of parts, comprising the active inventive (immunostimulatory) composition, and/or the inventive vaccine, optionally a liquid vehicle for solubilising and optionally technical instructions with information on the administration and dosage of the inventive composition and/or the inventive vaccine. The technical instructions may contain information about administration and dosage of the inventive composition, and/or the inventive vaccine. Such kits, preferably kits of parts, may be applied e.g. for any of the above mentioned applications or uses, preferably for the use of at least one inventive composition (for the preparation of an inventive medicament, preferably a vaccine) for the treatment of lung cancer, preferably of non-small cell lung cancer, and diseases or disorders related thereto. The kits may also be applied for the use of at least one inventive composition (for the preparation of an inventive vaccine) for the treatment of lung cancer, preferably of non-small cell lung cancer, and diseases or disorders related thereto, wherein the inventive composition) and/or the vaccine due to the encoded at least six antigens may be capable to induce or enhance an immune response in a mammal as defined above. Such kits may further be applied for the use of at least one inventive composition, (for the preparation of an inventive medicament, preferably a vaccine) for modulating, preferably for eliciting, e.g. to induce or enhance, an immune response in a mammal as defined above, and preferably to support treatment of lung cancer, preferably of non-small cell lung cancer, and diseases or disorders related thereto. Kits of parts, as a special form of kits, may contain one or more identical or different active inventive (immunostimulatory) compositions and/or one or more identical or different inventive vaccines in different parts of the kit. Kits of parts may also contain an (e.g. one) active inventive composition, an (e.g. one) inventive vaccine and/or the at least one mRNA encoding at least one antigen as defined above in different parts of the kit, e.g. each part of the kit containing at least one mRNA encoding a preferably different antigen. Additionally, a combination of both types of kits of parts is possible. Kits of parts may be used, e.g. when a time staggered treatment is envisaged, e.g. when using different formulations and/or increasing concentrations of the inventive composition, the inventive vaccine and/or the at least one mRNA encoding at least one antigen as defined above during the same treatment in vivo. Kits of parts may also be used when a separated formulation or administration of at least one of the antigens of the inventive composition (i.e. in parts) is envisaged or necessary (e.g. for technical reasons), but e.g. a combined presence of the different antigens in vivo is still to be achieved. Particularly kits of parts as a special form of kits are envisaged, wherein each part of the kit contains at least one preferably different antigen as defined above, all parts of the kit of parts forming the inventive composition or the inventive vaccine as defined herein. Such specific kits of parts may particularly be suitable, e.g. if different antigens are formulated separately as different parts of the kits, but are then administered at once together or in a time staggered manner to the mammal in need thereof. In the latter case administration of all of the different parts of such a kit typically occurs within a short time limit, such that all antigens are present in the mammal at about the same time subsequent to administration of the last part of the kit. In a preferred embodiment, the kit contains at least two parts containing the six mRNAs according to the invention. Preferably, all six mRNAs are provided in separate parts of the kit, wherein the mRNAs are preferably lyophilised. More preferably, the kit further contains as a part a vehicle for solubilising the at least one mRNA, the vehicle preferably being Ringer-lactate solution. Any of the above kits may be used in a treatment as defined above.

### Advantages of the present invention

The present invention provides a composition for the treatment of lung cancer, wherein the composition comprises at least one mRNA, encoding at least six antigens capable of eliciting an (adaptive) immune response in a mammal wherein the antigens are selected from the group consisting of 5T4 (Trophoblast glycoprotein, TPBG), Survivin (Baculoviral IAP repeat-containing protein 5; BIRC5), NY-ESO-1 (New York esophageal squamous cell carcinoma 1, CTAG1 B), MAGE-C1 (Melanoma antigen family C1), MAGE-C2 (Melanoma antigen family C2), and MUC1 (Mucin 1). Such a composition allows efficient treatment of lung cancer or supplementary treatment when using conventional therapies. It furthermore avoids the problem of uncontrolled propagation of the introduced DNA sequences by the use of RNA as an approach for curative methods. mRNA as used in the inventive composition has additional considerable advantages over DNA expression systems e.g. in immune response, immunization or vaccination. These advantages include, inter alia, that RNA introduced into a cell is not integrated into the genome. This avoids the risk of mutation of this gene, which otherwise may be completely or partially inactivated or give rise to misinformation. It further avoids other risks of using DNA as an agent to induce an immune response (e.g. as a vaccine) such as the induction of pathogenic anti-DNA antibodies in the patient into whom the foreign DNA has been introduced, so bringing about a (possibly fatal) immune response. In contrast, no anti-RNA antibodies have yet been detected.

### Figures

The following Figures are intended to illustrate the invention further. They are not intended to limit the subject matter of the invention thereto.
- Figure 1:: depicts the mRNA sequence 5T4 (GC)-muag-A64-C30 (SEQ ID NO: 1), encoding 5T4 (Trophoblast glycoprotein, TPBG). The mRNA contains following sequence elements:
A 5'-CAP, a GC-optimized coding sequence for stabilization and a better codon usage encoding 5T4 according to SEQ ID No. 3, the stabilizing sequence "muag" in the 3'-UTR according to SEQ ID No.70, ∼ 64 Adenosin at the 3'-terminal end (poly-A-tail), ∼ 30 Cytosin at the 3'-terminal end (poly-C-tail).
- Figure 2:: depicts the mRNA sequence 5T4 (GC)-muag-A64-C30-HistoneSL (SEQ ID NO: 19), encoding 5T4 (Trophoblast glycoprotein, TPBG). The mRNA contains following sequence elements:
A 5'-CAP, a GC-optimized coding sequence for stabilization and a better codon usage encoding 5T4 according to SEQ ID No. 3, the stabilizing sequence "muag" in the 3'-UTR according to SEQ ID No.70, ∼ 64 Adenosin at the 3'-terminal end (poly-A-tail), ∼ 30 Cytosin at the 3'-terminal end (poly-C-tail); and a histone stem-loop sequence according to SEQ ID No. 72.
- Figure 3:: depicts the wildtype coding sequence, encoding 5T4 (Trophoblast glycoprotein, TPBG), according to SEQ ID NO: 2, i.e. the coding sequence (CDS) encoding 5T4 (Trophoblast glycoprotein, TPBG) without GC-optimized coding sequence.
- Figure 4:: depicts the GC-optimized coding sequence encoding 5T4 (Trophoblast glycoprotein, TPBG) according to SEQ ID NO: 3.
- Figure 5:: depicts the mRNA sequence Survivin (GC)-muag-A64-C30 (SEQ ID NO: 4), encoding Survivin (Baculoviral IAP repeat-containing protein 5; BIRC5). The mRNA contains following sequence elements:
A 5'-CAP, a GC-optimized coding sequence for stabilization and a better codon usage encoding Survivin according to SEQ ID No. 6, the stabilizing sequence "muag" in the 3'-UTR according to SEQ ID No.70, ∼ 64 Adenosin at the 3'-terminal end (poly-A-tail), ∼ 30 Cytosin at the 3'-terminal end (poly-C-tail).
- Figure 6:: depicts the mRNA sequence Survivin (GC)-muag-A64-C30-HistoneSL (SEQ ID NO: 20), encoding Survivin (Baculoviral IAP repeat-containing protein 5; BIRC5). The mRNA contains following sequence elements:
A 5'-CAP, a GC-optimized coding sequence for stabilization and a better codon usage encoding Survivin according to SEQ ID No. 6, the stabilizing sequence "muag" in the 3'-UTR according to SEQ ID No.70, ∼ 64 Adenosin at the 3'-terminal end (poly-A-tail), ∼ 30 Cytosin at the 3'-terminal end (poly-C-tail); and a histone stem-loop sequence according to SEQ ID No. 72.
- Figure 7:: depicts the wildtype coding sequence, encoding Survivin, according to SEQ ID NO: 5, i.e. the coding sequence (CDS) encoding Survivin without GC-optimized coding sequence.
- Figure 8:: depicts the GC-optimized coding sequence encoding Survivin according to SEQ ID NO: 6.
- Figure 9:: depicts the mRNA sequence NY-ESO-1 (GC)-muag-A64-C30 (SEQ ID NO: 7), encoding NY-ESO-1 (New York esophageal squamous cell carcinoma 1, CTAG1 B). The mRNA contains following sequence elements:
A 5'-CAP, a GC-optimized coding sequence for stabilization and a better codon usage encoding NY-ESO-1 according to SEQ ID No. 9, the stabilizing sequence "muag" in the 3'-UTR according to SEQ ID No.70, ∼ 64 Adenosin at the 3'-terminal end (poly-A-tail), ∼ 30 Cytosin at the 3'-terminal end (poly-C-tail).
- Figure 10:: depicts the mRNA sequence NY-ESO-1 (GC)-muag-A64-C30-HistoneSL (SEQ ID NO: 21), encoding NY-ESO-1 (New York esophageal squamous cell carcinoma 1, CTAG1B). The mRNA contains following sequence elements:
A 5'-CAP, a GC-optimized coding sequence for stabilization and a better codon usage encoding NY-ESO-1 according to SEQ ID No. 9, the stabilizing sequence "muag" in the 3'-UTR according to SEQ ID No.70, ∼ 64 Adenosin at the 3'-terminal end (poly-A-tail), ∼ 30 Cytosin at the 3'-terminal end (poly-C-tail); and a histone stem-loop sequence according to SEQ ID No. 72.
- Figure 11:: depicts the wildtype coding sequence, encoding NY-ESO-1 (New York esophageal squamous cell carcinoma 1, CTAG1B), according to SEQ ID NO: 8, i.e. the coding sequence (CDS) encoding NY-ESO-1 without GC-optimized coding sequence.
- Figure 12:: depicts the GC-optimized coding sequence encoding NY-ESO-1 (New York esophageal squamous cell carcinoma 1, CTAG1B) according to SEQ ID NO: 9.
- Figure 13:: depicts the mRNA sequence MAGE-C1 (aa 613-1142) (GC)-muag-A64-C30 (SEQ ID NO: 10), encoding MAGE-C1 comprising the amino acid sequence aa 613-1142 of the wild type protein MAGE-C1. The mRNA contains following sequence elements:
A 5'-CAP, a GC-optimized coding sequence for stabilization and a better codon usage encoding MAGE-C1 (aa 613-1142) according to SEQ ID No. 25, the stabilizing sequence "muag" in the 3'-UTR according to SEQ ID No.70, ∼ 64 Adenosin at the 3'-terminal end (poly-A-tail), ∼ 30 Cytosin at the 3'- terminal end (poly-C-tail).
- Figure 14:: depicts the mRNA sequence MAGE-C1 (aa 613-1142) (GC)-muag-A64-C30-HistoneSL (SEQ ID NO: 22), encoding MAGE-C1 (aa 613-1142). The mRNA contains following sequence elements:
A 5'-CAP, a GC-optimized coding sequence for stabilization and a better codon usage encoding MAGE-C1 (aa 613-1142) according to SEQ ID No. 25, the stabilizing sequence "muag" in the 3'-UTR according to SEQ ID No.70, ∼ 64 Adenosin at the 3'-terminal end (poly-A-tail), ∼ 30 Cytosin at the 3'- terminal end (poly-C-tail); and a histone stem-loop sequence according to SEQ ID No. 72.
- Figure 15:: depicts the wildtype coding sequence, encoding the full-length protein of MAGE-C1 according to SEQ ID NO: 11, i.e. the coding sequence (CDS) encoding MAGE-C1 without GC-optimized coding sequence.
- Figure 16:: depicts the GC-optimized coding sequence encoding the full-length protein of MAGE-C1 according to SEQ ID NO: 12.
- Figure 17:: depicts the GC-optimized coding sequence encoding MAGE-C1 (aa 613-1142) according to SEQ ID NO: 25.
- Figure 18:: depicts the mRNA sequence MAGE-C2 (GC)-muag-A64-C30 (SEQ ID NO: 13), encoding MAGE-C2. The mRNA contains following sequence elements: A 5'-CAP, a GC-optimized coding sequence for stabilization and a better codon usage encoding MAGE-C2 according to SEQ ID No. 15, the stabilizing sequence "muag" in the 3'-UTR according to SEQ ID No.70, ∼ 64 Adenosin at the 3'-terminal end (poly-A-tail), ∼ 30 Cytosin at the 3'-terminal end (poly-C-tail).
- Figure 19:: depicts the mRNA sequence MAGE-C2 (GC)-muag-A64-C30-HistoneSL (SEQ ID NO: 21), encoding MAGE-C2. The mRNA contains following sequence elements:
A 5'-CAP, a GC-optimized coding sequence for stabilization and a better codon usage encoding MAGE-C2 according to SEQ ID No. 9, the stabilizing sequence "muag" in the 3'-UTR according to SEQ ID No.70, ∼ 64 Adenosin at the 3'-terminal end (poly-A-tail), ∼ 30 Cytosin at the 3'-terminal end (poly-C-tail); and a histone stem-loop sequence according to SEQ ID No. 72.
- Figure 20:: depicts the wildtype coding sequence, encoding MAGE-C2, according to SEQ ID NO: 14, i.e. the coding sequence (CDS) encoding MAGE-C2 without GC-optimized coding sequence.
- Figure 21:: depicts the GC-optimized coding sequence encoding MAGE-C2 according to SEQ ID NO: 15.
- Figure 22:: depicts the mRNA sequence MUC1 5xVNTR (GC)-muag-A64-C30 (SEQ ID NO: 16), encoding MUC1 (Mucin 1) comprising 5 tandem repeats. The mRNA contains following sequence elements:
A 5'-CAP, a GC-optimized coding sequence for stabilization and a better codon usage encoding MUC1 5xVNTR according to SEQ ID No. 18, the stabilizing sequence "muag" in the 3'-UTR according to SEQ ID No.70, ∼ 64 Adenosin at the 3'-terminal end (poly-A-tail), ∼ 30 Cytosin at the 3'-terminal end (poly-C-tail).
- Figure 23:: depicts the mRNA sequence MUC1 5xVNTR (GC)-muag-A64-C30-HistoneSL (SEQ ID NO: 24), encoding MUC1 (Mucin 1) comprising 5 tandem repeats. The mRNA contains following sequence elements:
A 5'-CAP, a GC-optimized coding sequence for stabilization and a better codon usage encoding MUC1 5xVNTR according to SEQ ID No. 18, the stabilizing sequence "muag" in the 3'-UTR according to SEQ ID No.70, ∼ 64 Adenosin at the 3'-terminal end (poly-A-tail), ∼ 30 Cytosin at the 3'-terminal end (poly-C-tail); and a histone stem-loop sequence according to SEQ ID No. 72.
- Figure 24:: depicts the wildtype coding sequence, encoding MUC1 5xVNTR, according to SEQ ID NO: 17, i.e. the coding sequence (CDS) encoding MUC1 without GC-optimized coding sequence.
- Figure 25:: depicts the GC-optimized coding sequence encoding MUC1 5xVNTR according to SEQ ID NO: 18.

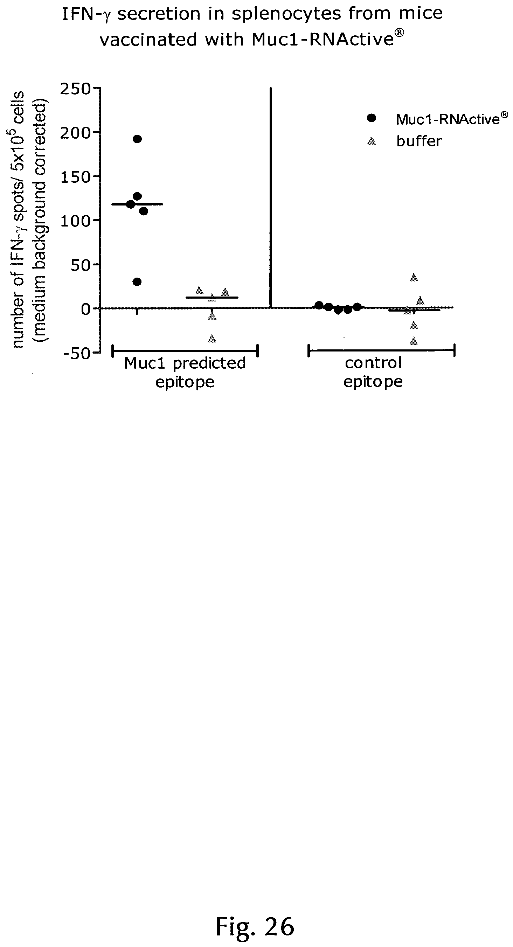

- Figure 26:: shows detection of a MUC1-specific cellular immune response by ELISPOT. C57BL/6 mice were vaccinated with 32µg MUC1-RNActive® (SEQ ID NO: 16) on days 1, 5, 8, 12 and 15. Ex vivo ELISpot analysis of the secretion of IFN-gamma in splenocytes from vaccinated and control mice was performed on day 6 after last vaccination. Cells were stimulated on the plate either with MUC1-derived peptide (predicted MHC-class I epitope) or with control peptide. The graph shows single data points for individual mice.

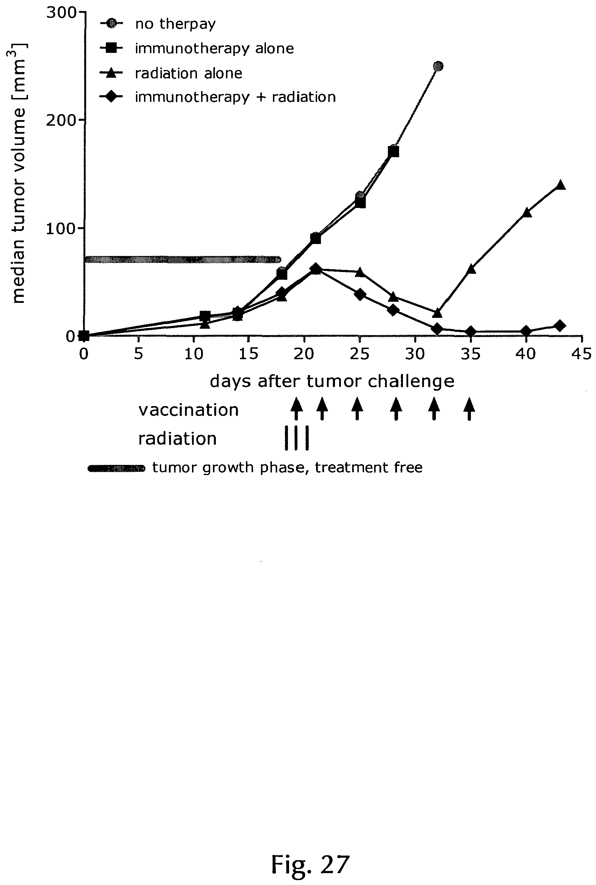

- Figure 27:: shows the effect of the combination of mRNA immunotherapy with radiation therapy in the treatment of low immunogenic and radioresistant Lewis Lung Carcinoma (LLC).
- Figure 28:: shows the protein sequence of 5T4 NP_001159864.1 according to SEQ ID NO: 75.
- Figure 29:: shows the protein sequence of Survivin (BIRC5) 015392 according to SEQ ID NO: 76.
- Figure 30:: shows the protein sequence of Survivin (BIRC5) NP_001159.2 according to SEQ ID NO: 77.
- Figure 31:: shows the protein sequence of NY-ESO-1 NP_001318.1 according to SEQ ID NO: 78.
- Figure 32:: shows the protein sequence of MAGE-C1 NP_005453.2 according to SEQ ID NO: 79.
- Figure 33:: shows the protein sequence of MAGE-C2 NP_057333.1 according to SEQ ID NO: 80.
- Figure 34:: shows the protein sequence of MUC1 Protein J05582.1 according to SEQ ID NO: 81.
- Figure 35:: shows the protein sequence of MUC1 Protein 5xVNTR according to SEQ ID NO: 82.
- Figure 36:: depicts the wildtype coding sequence, encoding MUC1, according to SEQ ID NO: 83, i.e. the full-length coding sequence (CDS) encoding MUC1 without GC-optimized coding sequence.

### Examples:

The following examples are intended to illustrate the invention further. They are not intended to limit the subject matter of the invention thereto.

### 1. Preparation of an mRNA vaccine based on MUC1 and induction of antigen-specific cytotoxic T-cells:

### 1.1 Preparation of an mRNA vaccine based on MUC1:

The mRNA vaccine consists of GC-optimized mRNAs coding for MUC1 (SEQ ID NO: 16). The mRNA was complexed with protamine by addition of protamine to the mRNA in the ratio (1:2) (w/w) (adjuvant component). After incubation for 10 min, the same amount of free mRNA used as antigen-providing mRNA was added.

The resulting composition was dissolved in 80% (v/v) Ringer-lactate solution.

### 1.2 Vaccination

C57BL/6 mice were vaccinated intradermally with 32 µg of one of the mRNA vaccines as described under 1.1 above. Control mice were treated injected intradermally with buffer (Ringer-lactate). Vaccination comprised five immunizations with 2 immunizations per week. The immune response was analysed 5 or 6 days after completion of the vaccination cycle.

### 1.3 ELISPOT - Detection of CTL (cytotoxic T cell) responses

For the detection of CTL (cytotoxic T cell) responses the analysis of IFN-gamma secretion in response to a specific stimulus can be visualized at a single cell level using the ELISPOT technique.

Splenocytes from mice vaccinated with the mRNA vaccine as described under 1.1 above and control mice were isolated 5 or 6 days after the last vaccination and then transferred into 96-well ELISPOT plates coated with an IFN-gamma capture antibody. The cells were then stimulated for 24 hours at 37°C using the following peptides:

| MUC1-derived peptide (SEQ ID NO:73) | Connexin-derived peptide (control) (SEQ ID NO: 74) |
|---|---|
| SAPDNRPAL | FEQNTAQP |

After the incubation period the cells were washed out of the plate and the IFN-gamma secreted by the cells was detected using a biotinylated secondary antibody against murine IFN-gamma, followed by streptavidin-AKP. Spots were visualized using BCIP/NBT substrate and counted using an automated ELISPOT reader (Immunospot Analyzer, CTL Analyzers LLC).

Intradermal vaccination with the MUC1-encoding mRNA led to the activation of antigen-specific CD8⁺ T-cells as demonstrated by the secretion of IFN-gamma in the ELISpot.

### 2. Combination of mRNA vaccination and irradiation in mice bearing low immunogenic (LLC) tumors

This study examined the efficacy of a vaccination and irradiation combination in C57BL/6 mice bearing low immunogenic (LLC) tumors.

On day 0 C57BL/6 mice (n = 10 per group) were challenged subcutaneously with syngeneic 3LL cells (LLC cells expressing tumor antigens EGFR and Connexin) in the right hind limb.

Treatment was started when tumors reached 50 mm³. Mice were either vaccinated with mRNAs encoding EGFR and Connexin (twice a week) or tumors were radiated with 36 Gy distributed in 3 equal doses for 3 consecutive days, or mice were treated with combination therapy (with first vaccination given 6h before second radiation) as indicated in Fig. 28. Untreated mice served as control. Tumor growth was monitored by measuring the tumor size in 3 dimensions using calipers.

Due to the lack of MHC class I expression, immunotherapy alone was ineffective in inhibiting tumor growth and also radiation therapy resulted in only transient tumor control. On the other hand, mRNA vaccines combined with radiotherapy resulted in a strong synergistic anti-tumor effect.

### 3. Clinical study: mRNA-derived cancer vaccine and local radiation as treatment of NSCLC

### Study Drug:

A composition comprising an mRNA coding for 5T4 (Trophoblast glycoprotein, TPBG) according to SEQ ID No. 19, an mRNA coding for Survivin (Baculoviral IAP repeat-containing protein 5; BIRC5) according to SEQ ID No. 20, an mRNA coding for NY-ESO-1 (New York esophageal squamous cell carcinoma 1; CTAG1B) according to SEQ ID No. 21, an mRNA coding for MAGE-C1 (Melanoma antigen family C1) according to SEQ ID No. 22, an mRNA coding for MAGE-C2 (Melanoma antigen family C2) according to SEQ ID No. 23, and an mRNA coding for MUC1 (Mucin 1) according to SEQ ID No. 24 complexed with protamine according to example 1.1. is used as vaccine. Each antigen-providing mRNA is provided as a sterile lyophilizate. Reconstitution in Ringer-Lactate provides a solution for intradermal injection. The drug product used in the clinical trial is denominated CV9202. It is a vaccine comprising six drug product components each comprising a different antigen-providing mRNA.

### Study Population:

- Stratum 1: Patients with stage IV non-small cell lung cancer (NSCLC) and non-squamous histology, without activating epidermal growth factor receptor (EGFR) mutations, who have achieved partial response (PR) or stable disease (SD) after at least 4 cycles of platinum- and pemetrexed-based first-line chemotherapy, and an indication for maintenance therapy with pemetrexed.
- Stratum 2: Patients with stage IV NSCLC and squamous cell histology, who have achieved PR or SD after at least 4 cycles of platinum-based and non-platinum compound first-line chemotherapy (platinum-based combination chemotherapy).
- Stratum 3: Patients with stage IV NSCLC and non-squamous histology, harboring an activating EGFR mutation, who have achieved PR after up to 6 months of treatment with an EGFR tyrosine kinase inhibitor (TKI).

### Study population: Inclusion criteria

1. Patients: > 18 years of age with histologically or cytologically-confirmed stage IV NSCLC, and a confirmed EGFR mutation status in case of non-squamous cell histology
   - Stratum 1: Non-squamous NSCLC without activating EGFR mutation
   - Stratum 2: Squamous NSCLC
   - Stratum 3: Non-squamous NSCLC harboring an activating EGFR mutation
2. PR or SD according to RECIST Version 1.1 after first-line therapy which should have consisted of:
   Stratum 1: PR or SD after cisplatin or carboplatin and pemetrexed treatment (at least 4 cycles)
   Stratum 2: PR or SD after cisplatin or carboplatin and a non-platinum compound treatment (at least 4 cycles)
   Stratum 3: PR after gefitinib, erlotinib or afatinib treatment of up to 6 months
3. For patients in stratum 1, maintenance therapy with pemetrexed should be indicated as to the investigator's opinion
4. Presence of at least one tumor lesion that is eligible for radiation with 4 x 5 GY, and at least one additional measurable tumor lesion according to RECIST Version 1.1.
   Tumor lesions eligible for radiation are:
   Bone metastases
   Lymph nodes in the paraclavicular, axillary or cervical regions
   Skin or subcutaneous metastases
   For patients in strata 1 and 2 only: Thoracic lesions (centrally located lung tumor, lymph nodes in the lung hilus or mediastinum)
5. Performance Status: Eastern Cooperative Oncology Group (ECOG) 0 to 1
6. Adequate organ function:
   Bone marrow function: Hematologic values at the start of vaccination: hemoglobin ≥ 95 g/L, platelet count ≥ 75000/µL, white blood cell count ≥ 2000/µL, absolute neutrophil count ≥ 1000/µL, lymphocyte count ≥ 0.8 x 109/L
   Hepatic: ALT and AST ≤ 2.5 times ULN in patients without liver metastases and ≤ 5 times ULN in patients with liver metastases
   Renal: Serum creatinine ≤ 2 mg/dL, and creatinine clearance ≥ 45 mL/min according to MDRD formula
7. Use of highly effective contraception in patients of child-producing potential while enrolled in the study, and for 1 month after the last immunization
8. Written informed consent prior to conducting any study related procedure
   - Treatment duration: In each patient, the vaccine will be administered until disease progression and the need to start a subsequent systemic second-line treatment, or occurrence of unacceptable toxicity, whichever comes first.
   - Timing of screening: Patients will start screening 2 weeks after day 1 of the last cycle of their first-line chemotherapy (strata 1 and 2), or within 6 months after start of treatment with an EGFR TKI (erlotinib or gefitinib) (stratum 3).

### Method of administration

Every antigen-providing mRNA is applied individually on the same day as two intradermal injections of 200 µl each giving a total of 12 injections.

The first three vaccinations will be in a weekly interval (days 1, 8, 15), followed by intervals of 2 or 3 weeks until day 57, 3-weekly intervals until month 6 and 6-weekly thereafter as specified in the study protocol as specified in the study protocol.. Vaccinations will be administered until occurrence of unacceptable toxicity or tumor progression requiring the initiation of a systemic second line therapy.

In strata 1 and 3, CV9202 will be administered initially on Day 1, 8, 15, 36, and 57. During treatment with pemetrexed (stratum 1), vaccination will be administered 4-7 days before the next scheduled dose of pemetrexed. This schedule is chosen to avoid vaccination during the neutrophil nadir and interference with anti-inflammatory steroids.
In stratum 2, CV9202 will be administered initially on Day 1, 8, 15, 29, 43 and 57. This intensified schedule is chosen since patients have an expected shorter time to disease progression because they do not receive concomitant anticancer treatment.
In all strata, patients will continue vaccination after Day 57 in case no criterion for treatment discontinuation is met. After Day 57 vaccinations will be administered every 3 weeks until 6 months from the day of first vaccination. After the 6-month period, vaccination will be performed every 6 weeks until criteria for discontinuation are met:

### Criteria for treatment discontinuation:

1. Occurrence of toxicity requiring permanent discontinuation of treatment
2. Disease progression requiring the start of a subsequent systemic treatment

### Administration of

Vaccines: At every single vaccination time point, each of the 6 drug components will be administered separately on the same day. Two intradermal (i.d.) injections of 200 µL each will be performed per component, resulting in a total of 12 injections.

Radiation: Radiotherapy will be administered in 4 daily fractions of 5 GY each to be administered within one week, preferably from Day 9-12.

### Selection of tumor lesions for radiation

Lesions selected for radiation should measure at least 2 cm in the longest diameter, and the radiation oncologist at the trial site must confirm that the respective lesion is eligible for radiation in accordance with this protocol before the patient is enrolled.

The following lesions are potentially eligible and should be selected according to the following hierarchy:
First Preference: Bone metastases
Second Preference: Lymph nodes in the paraclavicular, axillary or cervical regions
Third Preference: Skin or subcutaneous metastases
Fourth preference (for patients in strata 1 and 2 only): Thoracic lesions (centrally located lung tumor, lymph nodes in the lung hilus or mediastinum)
In patients in stratum 3, no thoracic lesion should be selected for radiation since patients are at higher risk for radiation pneumonitis after thoracic radiation due to concomitant treatment with EGFR TKIs.

Irradiation of more than one lesion with the same schedule of 4 x 5 GY (e.g. multiple bone metastases) is permitted if clinically indicated as long as a measurable lesion remains for the evaluation of an abscopal response. Irradiation of more than one lesion should be performed within the same time window.

### Items

1. Composition comprising at least one mRNA, wherein the at least one mRNA encodes the following antigens:
   - 5T4 (Trophoblast glycoprotein, TPBG);
   - Survivin (Baculoviral IAP repeat-containing protein 5; BIRC5),
   - NY-ESO-1 (New York esophageal squamous cell carcinoma 1; CTAG1B),
   - MAGE-C1 (Melanoma antigen family C1);
   - MAGE-C2 (Melanoma antigen family C2), and
   - MUC1 (Mucin 1),
   or fragments thereof and wherein the at least one mRNA is mono-, bi- or multicistronic.
2. The composition according to item 1, wherein each of the antigens or fragments thereof is encoded by a separate mRNA.
3. The composition according to item 1, wherein 5T4, Survivin, NY-ESO-1, MAGE-C1, MAGE-C2 and MUC1 or fragments thereof are encoded by one mRNA.
4. The composition according to item 1, wherein the antigens or fragments thereof are encoded by at least one bicistronic and/or multicistronic mRNA.
5. The composition according to any of items 1 to 4, wherein at least one mRNA, preferably at least two mRNAs, more preferably at least three mRNAs, even more preferably at least four mRNAs, even more preferably at least five mRNAs, or even more preferably at least six mRNAs, each comprise at least one coding sequence selected from RNA sequences being identical or at least 80% identical to the RNA sequence of SEQ ID NOs: 2, 5, 8, 11, 14 or 17.
6. The composition according to any of items 1 to 5, wherein at least one mRNA comprises a coding sequence, which contains or consists of an RNA sequence that is identical or at least 80% identical to the RNA sequences of SEQ ID NOs: 2, 5, 8, 11, 14 or 17.
7. The composition according to any of items 1 to 6, wherein the at least one mRNA is a modified mRNA, in particular a stabilized mRNA.
8. The composition according to any of items 1 to 7, wherein the G/C content of the coding region of the at least one mRNA is increased compared to the G/C content of the coding region of the wild-type mRNA, the coded amino acid sequence of the at least one mRNA preferably not being modified compared to the coded amino acid sequence of the wild-type mRNA.
9. The composition according to any of items 1 to 8, wherein at least one mRNA comprises a coding sequence, which contains or consists of an RNA sequence that is identical or at least 80% identical to the RNA sequences of SEQ ID NOs: 3, 6, 9, 12, 15, 18 or 25.
10. The composition according to any of items 1 to 9, wherein the at least one mRNA contains a 5' cap structure and/or the 3' UTR contains a poly(A) tail, preferably of 10 to 200, 10 to 100, 40 to 80 or 50 to 70 adenosine nucleotides, and/or the 3' UTR contains a poly(C) tail, preferably of 10 to 200, 10 to 100, 20 to 70, 20 to 60 or 10 to 40 cytosine nucleotides.
11. The composition according to any of items 1 to 10, wherein the at least one mRNA comprises a 3' UTR, which comprises (in 5' to 3' direction) the following elements:
   a) a poly(A) tail, preferably consisting of 10 to 200, 10 to 100, 40 to 80 or 50 to 70 adenosine nucleotides,
   b) a poly(C) tail, preferably consisting of 10 to 200, 10 to 100, 20 to 70, 20 to 60 or 10 to 40 cytosine nucleotides, and
   c) a histone stem-loop.
12. The composition according to any of items 1 to 11 comprising six mRNAs, wherein each mRNA encodes a different antigen selected from the group consisting of 5T4 (Trophoblast glycoprotein, TPBG), Survivin (Baculoviral IAP repeat-containing protein 5; BIRC5), NY-ESO-1 (New York esophageal squamous cell carcinoma 1, CTAG1B), MAGE-C1 (Melanoma antigen family C1), MAGE-C2 (Melanoma antigen family C2), and MUC1 (Mucin 1) and wherein preferably each mRNA comprises an RNA sequence, which is identical or at least 80% identical to a different RNA sequence selected from the RNA sequences according to SEQ ID NOs: 1, 4, 7, 10, 13 and 16.
13. The composition according to any of items 1 to 12, wherein the histone stem-loop is formed by intramolecular base pairing of two neighbouring sequences, which are entirely or partially reverse complementary.
14. The composition according to any of items 1 to 13, wherein a paired stem-loop element comprises at least one mismatched base.
15. The composition according to any of items 1 to 14, wherein a loop in a histone stem-loop has a length of 3 to 15 bases, preferably of 3 to 10, 3 to 8, 3 to 7, 3 to 6, 4 to 5 or 4 bases.
16. The composition according to any of items 1 to 15, wherein a sequence forming a stem region in a histone stem-loop has a length of 5 to 10 bases, preferably 5 to 8 bases.
17. The composition according to any of items 1 to 16, wherein the 3' UTR of the at least one mRNA contains at least one histone stem-loop that is selected from the following formulae (I) or (II):
   formula (I) (stem-loop sequence without stem bordering elements): 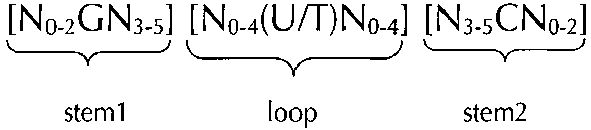
   formula (II) (stem-loop sequence with stem bordering elements): 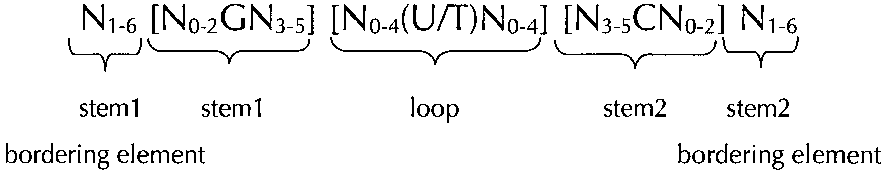 wherein:
      - stem1 or stem2 bordering elements N₁₋₆: is a consecutive sequence of 1 to 6, preferably of 2 to 6, more preferably of 2 to 5, even more preferably of 3 to 5, most preferably of 4 to 5 or 5 N, wherein each N is independently from another selected from a nucleotide selected from A, U, T, G and C, or a nucleotide analogue thereof;
      - stem1 [N₀₋₂GN₃₋₅]: is reverse complementary or partially reverse complementary with element stem2, and is a consecutive sequence between of 5 to 7 nucleotides;
      wherein N₀₋₂ is a consecutive sequence of 0 to 2, preferably of 0 to 1, more preferably of 1 N, wherein each N is independently from another selected from a nucleotide selected from A, U, T, G and C or a nucleotide analogue thereof;
      wherein N₃₋₅ is a consecutive sequence of 3 to 5, preferably of 4 to 5, more preferably of 4 N, wherein each N is independently from another selected from a nucleotide selected from A, U, T, G and C or a nucleotide analogue thereof, and
      wherein G is guanosine or an analogue thereof, and may be optionally replaced by a cytidine or an analogue thereof, provided that its complementary nucleotide cytidine in stem2 is replaced by guanosine;
      - loop sequence [N₀₋₄(U/T)N₀₋₄]: is located between elements stem1 and stem2, and is a consecutive sequence of 3 to 5 nucleotides, more preferably of 4 nucleotides;
      wherein each N₀₋₄ is independent from another a consecutive sequence of 0 to 4, preferably of 1 to 3, more preferably of 1 to 2 N, wherein each N is independently from another selected from a nucleotide selected from A, U, T, G and C or a nucleotide analogue thereof; and
      wherein U/T represents uridine, or optionally thymidine;
      - stem2 [N₃₋₅CN₀₋₂]: is reverse complementary or partially reverse complementary with element stem1, and is a consecutive sequence between of 5 to 7 nucleotides;
      wherein N₃₋₅ is a consecutive sequence of 3 to 5, preferably of 4 to 5, more preferably of 4 N, wherein each N is independently from another selected from a nucleotide selected from A, U, T, G and C or a nucleotide analogue thereof;
      wherein N₀₋₂ is a consecutive sequence of 0 to 2, preferably of 0 to 1, more preferably of 1 N, wherein each N is independently from another selected from a nucleotide selected from A, U, T, G and C or a nucleotide analogue thereof; and
      wherein C is cytidine or an analogue thereof, and may be optionally replaced by a guanosine or an analogue thereof provided that its complementary nucleotide guanosine in stem1 is replaced by cytidine;
      wherein
      stem1 and stem2 are capable of base pairing with each other
      forming a reverse complementary sequence, wherein base pairing may occur between stem1 and stem2, or
      forming a partially reverse complementary sequence, wherein an incomplete base pairing may occur between stem1 and stem2.
18. The composition according to any of items 1 to 17, wherein the at least one histone stem-loop is selected from at least one of the following formulae (Ia) or (IIa):
   formula (la) (stem-loop sequence without stem bordering elements): 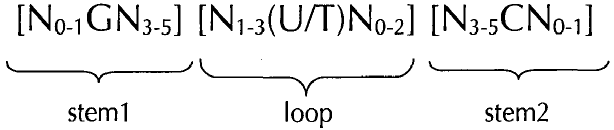
   formula (IIa) (stem-loop sequence with stem bordering elements): 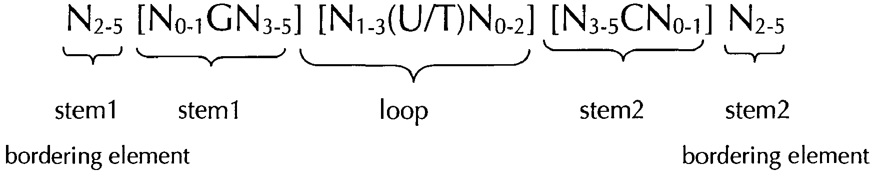
19. The composition according to any of items 1 to 18 comprising any one of the histone stem loop nucleotide sequences according to SEQ ID NOs: 26 to 67, preferably a nucleotide sequence according to SEQ ID NO. 71 and most preferably a RNA sequence according to SEQ ID NO. 72.
20. The composition according to any of items 1 to 19, wherein the at least one mRNA comprises at least one mRNA identical or at least 80% identical to an RNA sequence according to any of the RNA sequences according to SEQ ID NOs: 19 to 24.
21. The composition according to any of items 1 to 20 comprising six mRNAs, wherein each mRNA encodes a different antigen selected from the group consisting of 5T4 (Trophoblast glycoprotein, TPBG), Survivin (Baculoviral IAP repeat-containing protein 5; BIRC5), NY-ESO-1 (New York esophageal squamous cell carcinoma 1, CTAG1B), MAGE-C1 (Melanoma antigen family C1), MAGE-C2 (Melanoma antigen family C2), and MUC1 (Mucin 1) and each mRNA is identical or at least 80% identical to a different RNA sequence selected from the RNA sequences according to SEQ ID NO: 19, 20, 21, 22, 23 or 24.
22. The composition according to any of items 1 to 21 comprising six mRNAs, wherein one mRNA encodes 5T4 and is identical or at least 80% identical to SEQ ID NO: 19, one mRNA encodes Survivin and is identical or at least 80% identical to SEQ ID NO: 20, one mRNA encodes NY-ESO-1 and is identical or at least 80% identical to SEQ ID NO: 21, one mRNA encodes MAGE-C1 and is identical or at least 80% identical to SEQ ID NO: 22, one mRNA encodes MAGE-C2 and is identical or at least 80% identical to SEQ ID NO: 23 and one mRNA encodes MUC1 and is identical or at least 80% identical to SEQ ID NO:24.
23. The composition according to any of items 1 to 22, wherein the at least one mRNA is complexed with one or more polycations, preferably with protamine or oligofectamine, most preferably with protamine.
24. The composition according to item 23, wherein the N/P ratio of the at least one mRNA to the one or more polycations is in the range of about 0.1 to 10, including a range of about 0.3 to 4, of about 0.5 to 2, of about 0.7 to 2 and of about 0.7 to 1.5.
25. The composition according to any of items 1 to 24 comprising at least one RNA, which is complexed with one or more polycations, and at least one free RNA.
26. The composition according to item 25, wherein the complexed RNA is identical to the free RNA.
27. The composition according to item 25 or 26, wherein the molar ratio of the complexed RNA to the free RNA is selected from a molar ratio of about 0.001:1 to about 1:0.001, including a ratio of about 1:1.
28. The composition according to any of items 1 to 27, wherein the composition additionally comprises at least one adjuvant.
29. The composition according to any of items 1 to 28, wherein the at least one adjuvant is selected from the group consisting of:
   cationic or polycationic compounds, comprising cationic or polycationic peptides or proteins, including protamine, nucleoline, spermin or spermidine, poly-L-lysine (PLL), poly-arginine, basic polypeptides, cell penetrating peptides (CPPs), including HIV-binding peptides, Tat, HIV-1 Tat (HIV), Tat-derived peptides, Penetratin, VP22 derived or analog peptides, HSV VP22 (Herpes simplex), MAP, KALA or protein transduction domains (PTDs, PpT620, prolin-rich peptides, arginine-rich peptides, lysine-rich peptides, MPG-peptide(s), Pep-1, L-oligomers, Calcitonin peptide(s), Antennapedia-derived peptides (particularly from Drosophila antennapedia), pAntp, plsl, FGF, Lactoferrin, Transportan, Buforin-2, Bac715-24, SynB, SynB(1), pVEC, hCT-derived peptides, SAP, protamine, spermine, spermidine, or histones, cationic polysaccharides, including chitosan, polybrene, cationic polymers, including polyethyleneimine (PEI), cationic lipids, including DOTMA: 1-(2,3-sioleyloxy)propyl) -N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-(-trimethylammonioacetyl)diethanolamine chloride, CLIP1: rac-(2,3-dioctadecyloxypropyl)(2-hydroxyethyl) -dimethylammonium chloride, CLIP6: rac-2(2,3-dihexadecyloxypropyl-oxymethyloxy)ethyl trimethylammonium, CLIP9: rac-2(2,3-dihexadecyloxypropyl-oxysuccinyloxy)ethyl -trimethylammonium, oligofectamine, or cationic or polycationic polymers, including modified polyaminoacids, including -aminoacid-polymers or reversed polyamides, modified polyethylenes, including PVP (poly(N-ethyl-4-vinylpyridinium bromide)), modified acrylates, including pDMAEMA (poly(dimethylaminoethyl methylacrylate)), modified Amidoamines including pAMAM (poly(amidoamine)), modified polybetaaminoester (PBAE), including diamine end modified 1,4 butanediol diacrylate-co-5-amino-1-pentanol polymers, dendrimers, including polypropylamine dendrimers or pAMAM based dendrimers, polyimine(s), including PEI: poly(ethyleneimine), poly(propyleneimine), polyallylamine, sugar backbone based polymers, including cyclodextrin based polymers, dextran based polymers, Chitosan, etc., silan backbone based polymers , such as PMOXA-PDMS copolymers, etc., Blockpolymers consisting of a combination of one or more cationic blocks selected of a cationic polymer as mentioned before, and of one or more hydrophilic- or hydrophobic blocks (e.g polyethyleneglycole);
      or
   cationic or polycationic proteins or peptides, selected from following proteins or peptides having the following total formula (I): (Arg)l;(Lys)m;(His)n;(Orn)o;(Xaa)x, wherein l + m + n + o + x = 8-15, and l, m, n or o independently of each other may be any number selected from 0, 1, 2, 3, 4, 5, 6, 7, 8, 9, 10, 11, 12, 13, 14 or 15, provided that the overall content of Arg, Lys, His and Orn represents at least 50% of all amino acids of the oligopeptide; and Xaa may be any amino acid selected from native (= naturally occurring) or non-native amino acids except of Arg, Lys, His or Orn; and x may be any number selected from 0, 1, 2, 3 or 4, provided, that the overall content of Xaa does not exceed 50 % of all amino acids of the oligopeptide; or
   nucleic acids having the formula (II): GIXmGn, wherein: G is guanosine, uracil or an analogue of guanosine or uracil; X is guanosine, uracil, adenosine, thymidine, cytosine or an analogue of the above-mentioned nucleotides; l is an integer from 1 to 40, wherein when l = 1 G is guanosine or an analogue thereof, when l > 1 at least 50% of the nucleotides are guanosine or an analogue thereof; m is an integer and is at least 3; wherein when m = 3 X is uracil or an analogue thereof, when m > 3 at least 3 successive uracils or analogues of uracil occur; n is an integer from 1 to 40, wherein when n = 1 G is guanosine or an analogue thereof, when n > 1 at least 50% of the nucleotides are guanosine or an analogue thereof;
      or
   nucleic acids having the formula (III): ClXmCn, wherein: C is cytosine, uracil or an analogue of cytosine or uracil; X is guanosine, uracil, adenosine, thymidine, cytosine or an analogue of the above-mentioned nucleotides; l is an integer from 1 to 40, wherein when l = 1 C is cytosine or an analogue thereof, when l > 1 at least 50% of the nucleotides are cytosine or an analogue thereof; m is an integer and is at least 3; wherein when m = 3 X is uracil or an analogue thereof, when m > 3 at least 3 successive uracils or analogues of uracil occur; n is an integer from 1 to 40, wherein when n = 1 C is cytosine or an analogue thereof, when n > 1 at least 50% of the nucleotides are cytosine or an analogue thereof.
30. A vaccine, comprising a composition according to any of items 1 to 29.
31. The vaccine according to item 30, wherein the composition according to any of items 1 to 29 elicits an adaptive immune response.
32. The vaccine according to item 30 or 31, wherein the vaccine further comprises a pharmaceutically acceptable carrier.
33. The vaccine according to any of items 30 to 32, wherein at least one mRNA of the composition is administered to the subject individually.
34. The composition according to any of items 1 to 29 for use as a vaccine for the treatment of lung cancer, preferably non-small cell lung cancer (NSCLC).
35. Use of a combination of six mRNAs for the treatment of lung cancer, wherein each mRNA encodes one antigen selected from the group consisting of 5T4 (Trophoblast glycoprotein, TPBG), Survivin (Baculoviral IAP repeat-containing protein 5; BIRC5), NY-ESO-1 (New York esophageal squamous cell carcinoma 1, CTAG1B), MAGE-C1 (Melanoma antigen family C1), MAGE-C2 (Melanoma antigen family C2), and MUC1 (Mucin 1).
36. Use according to item 35, wherein
   one mRNA comprises a coding sequence, which encodes 5T4 and contains or consists of an RNA sequence that is identical or at least 80% identical to the RNA sequences of SEQ ID NOs: 2 or 3;
   one mRNA comprises a coding sequence, which encodes Survivin and contains or consists of an RNA sequence that is identical or at least 80% identical to the RNA sequences of SEQ ID NOs: 5 or 6;
   one mRNA comprises a coding sequence, which encodes NY-ESO-1 and contains or consists of an RNA sequence that is identical or at least 80% identical to the RNA sequences of SEQ ID NOs: 8 or 9;
   one mRNA comprises a coding sequence, which encodes MAGE-C1 and contains or consists of an RNA sequence that is identical or at least 80% identical to the RNA sequences of SEQ ID NOs: 11, 12 or 25;
   one mRNA comprises a coding sequence, which encodes MAGE-C2 and contains or consists of an RNA sequence that is identical or at least 80% identical to the RNA sequences of SEQ ID NOs: 14 or 15;
   one mRNA comprises a coding sequence, which encodes MUC1 and contains or consists of an RNA sequence that is identical or at least 80% identical to the RNA sequences of SEQ ID NOs: 17 or 18;
37. Use according to any of items 35 or 36, wherein at least one mRNA comprises a histone stem-loop in the 3' UTR region.
38. Use according to any of items 35 to 37, wherein each mRNA comprises an RNA sequence that is identical or at least 80% identical to a different one of the RNA sequences according to SEQ ID NOs: 19 to 24.
39. Use according to any of items 35 to 38 comprising six mRNAs, wherein one mRNA encodes 5T4 and is identical or at least 80% identical to SEQ ID NO: 19, one mRNA encodes Survivin and is identical or at least 80% identical to SEQ ID NO: 20, one mRNA encodes NY-ESO-1 and is identical or at least 80% identical to SEQ ID NO: 21, one mRNA encodes MAGE-C1 and is identical or at least 80% identical to SEQ ID NO: 22, one mRNA encodes MAGE-C2 and is identical or at least 80% identical to SEQ ID NO: 23 and one mRNA encodes MUC1 and is identical or at least 80% identical to SEQ ID NO:24.
40. Use according to any of items 35 to 39, wherein each of the six mRNAs is administered separately.
41. Use according to any of items 35 to 40, wherein the mRNAs are administered by intradermal injection.
42. Use according to any of items 35 to 41, wherein the treatment comprises the administration of a further active pharmaceutical ingredient.
43. Use according to any of items 35 to 42, wherein the further active pharmaceutical ingredient is a chemotherapeutic agent or a kinase inhibitor.
44. Use according to any of items 35 to 43, wherein the treatment further comprises radiation therapy.
45. A kit, preferably kit of parts, comprising the composition according to any of items 1 to29, and/or a vaccine according to any of items 30 to 33, and optionally a liquid vehicle for solubilising and optionally technical instructions with information on the administration and dosage of the active composition and/or the vaccine.
46. The kit according to item 45, wherein the kit is a kit of parts and each part of the kit contains at least one mRNA preferably encoding a different antigen selected from the antigens defined in item 1, all parts of the kit of parts forming the composition or the vaccine of the preceding items.
47. The kit according to item 45 or 46, wherein the kit contains at least two parts containing six mRNAs.
48. The kit according to any of items 45 to 47, wherein all six mRNAs are provided in lyophilized form in separate parts.
49. The kit according to item 48, wherein the kit contains as a part Ringer-Lactate solution.
50. The kit according to any of items 45 to 49, wherein the kit contains six parts, each part containing one of the six mRNAs.

## Claims

1. Composition comprising six mRNAs, wherein each mRNA encodes a different antigen selected from the group consisting of:
• 5T4 (Trophoblast glycoprotein, TPBG);
• Survivin (Baculoviral IAP repeat-containing protein 5; BIRC5),
• NY-ESO-1 (New York esophageal squamous cell carcinoma 1; CTAG1B),
• MAGE-C1 (Melanoma antigen family C1);
• MAGE-C2 (Melanoma antigen family C2), and
• MUC1 (Mucin 1),
and wherein each mRNA is identical or at least 80% identical to a different RNA sequence selected from the RNA sequences according to SEQ ID NO: 19, 20, 21, 22, 23 or 24.

2. The composition according to claim 1, wherein one mRNA encodes 5T4 and is identical or at least 80% identical to SEQ ID NO: 19, one mRNA encodes Survivin and is identical or at least 80% identical to SEQ ID NO: 20, one mRNA encodes NY-ESO-1 and is identical or at least 80% identical to SEQ ID NO: 21, one mRNA encodes MAGE-C1 and is identical or at least 80% identical to SEQ ID NO: 22, one mRNA encodes MAGE-C2 and is identical or at least 80% identical to SEQ ID NO: 23 and one mRNA encodes MUC1 and is identical or at least 80% identical to SEQ ID NO:24.

3. The composition according to claim 1 or 2, wherein at least one mRNA is complexed with one or more polycations, preferably with protamine or oligofectamine, most preferably with protamine.

4. The composition according to claim 3, wherein the N/P ratio of the at least one mRNA to the one or more polycations is in the range of about 0.1 to 10, including a range of about 0.3 to 4, of about 0.5 to 2, of about 0.7 to 2 and of about 0.7 to 1.5.

5. The composition according to any of claims 1 to 4 comprising at least one RNA, which is complexed with one or more polycations, and at least one free RNA, wherein the complexed RNA is preferably identical to the free RNA.

6. The composition according to any of claims 1 to 5, wherein the composition additionally comprises at least one adjuvant, wherein the at least one adjuvant is preferably selected from the group consisting of:
cationic or polycationic compounds, comprising cationic or polycationic peptides or proteins, including protamine, nucleoline, spermin or spermidine, poly-L-lysine (PLL), poly-arginine, basic polypeptides, cell penetrating peptides (CPPs), including HIV-binding peptides, Tat, HIV-1 Tat (HIV), Tat-derived peptides, Penetratin, VP22 derived or analog peptides, HSV VP22 (Herpes simplex), MAP, KALA or protein transduction domains (PTDs, PpT620, prolin-rich peptides, arginine-rich peptides, lysine-rich peptides, MPG-peptide(s), Pep-1, L-oligomers, Calcitonin peptide(s), Antennapedia-derived peptides (particularly from Drosophila antennapedia), pAntp, plsl, FGF, Lactoferrin, Transportan, Buforin-2, Bac715-24, SynB, SynB(1), pVEC, hCT-derived peptides, SAP, protamine, spermine, spermidine, or histones, cationic polysaccharides, including chitosan, polybrene, cationic polymers, including polyethyleneimine (PEI), cationic lipids, including DOTMA: [1-(2,3-sioleyloxy)propyl)]-N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctaclecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetraclecanoyl-N-(α-trimethylammonioacetyl)diethanolamine chloride, CLIP1: rac-[(2,3-dioctadecyloxypropyl)(2-hydroxyethyl)]-dimethylammonium chloride, CLIP6: rac-[2(2,3-dihexadecyloxypropyl-oxymethyloxy)ethyl]trimethylammonium, CLIP9: rac-[2(2,3-dihexadecyloxypropyl-oxysuccinyloxy)ethyl]-trimethylammonium, oligofectamine, or cationic or polycationic polymers, including modified polyaminoacids, including β-aminoacid-polymers or reversed polyamides, modified polyethylenes, including PVP (poly(N-ethyl-4-vinylpyridinium bromide)), modified acrylates, including pDMAEMA (poly(dimethylaminoethyl methylacrylate)), modified Amidoamines including pAMAM (poly(amidoamine)), modified polybetaaminoester (PBAE), including diamine end modified 1,4 butanediol diacrylate-co-5-amino-1-pentanol polymers, dendrimers, including polypropylamine dendrimers or pAMAM based dendrimers, polyimine(s), including PEI: poly(ethyleneimine), poly(propyleneimine), polyallylamine, sugar backbone based polymers, including cyclodextrin based polymers, dextran based polymers, Chitosan, etc., silan backbone based polymers , such as PMOXA-PDMS copolymers, etc., Blockpolymers consisting of a combination of one or more cationic blocks selected of a cationic polymer as mentioned before, and of one or more hydrophilic- or hydrophobic blocks (e.g polyethyleneglycole);
or
cationic or polycationic proteins or peptides, selected from following proteins or peptides having the following total formula (I): (Arg)ₗ;(Lys)ₘ;(His)ₙ;(Orn)ₒ;(Xaa)ₓ, wherein l + m + n +o + x = 8-15, and l, m, n or o independently of each other may be any number selected from 0, 1, 2, 3, 4, 5, 6, 7, 8, 9, 10, 11, 12, 13, 14 or 15, provided that the overall content of Arg, Lys, His and Orn represents at least 50% of all amino acids of the oligopeptide; and Xaa may be any amino acid selected from native (= naturally occurring) or non-native amino acids except of Arg, Lys, His or Orn; and x may be any number selected from 0, 1, 2, 3 or 4, provided, that the overall content of Xaa does not exceed 50 % of all amino acids of the oligopeptide; or
nucleic acids having the formula (II): GₗXₘGₙ, wherein: G is guanosine, uracil or an analogue of guanosine or uracil; X is guanosine, uracil, adenosine, thymidine, cytosine or an analogue of the above-mentioned nucleotides; l is an integer from 1 to 40, wherein when l = 1 G is guanosine or an analogue thereof, when l > 1 at least 50% of the nucleotides are guanosine or an analogue thereof; m is an integer and is at least 3; wherein when m = 3 X is uracil or an analogue thereof, when m > 3 at least 3 successive uracils or analogues of uracil occur; n is an integer from 1 to 40, wherein when n = 1 G is guanosine or an analogue thereof, when n > 1 at least 50% of the nucleotides are guanosine or an analogue thereof;
or
nucleic acids having the formula (III): CₗXₘCₙ, wherein: C is cytosine, uracil or an analogue of cytosine or uracil; X is guanosine, uracil, adenosine, thymidine, cytosine or an analogue of the above-mentioned nucleotides; l is an integer from 1 to 40, wherein when l = 1 C is cytosine or an analogue thereof, when l > 1 at least 50% of the nucleotides are cytosine or an analogue thereof; m is an integer and is at least 3; wherein when m = 3 X is uracil or an analogue thereof, when m > 3 at least 3 successive uracils or analogues of uracil occur; n is an integer from 1 to 40, wherein when n = 1 C is cytosine or an analogue thereof, when n > 1 at least 50% of the nucleotides are cytosine or an analogue thereof.

7. A vaccine, comprising a composition according to any of claims 1 to 6, wherein the vaccine preferably further comprises a pharmaceutically acceptable carrier.

8. The composition according to any of claims 1 to 6 for use as a vaccine for the treatment of lung cancer, preferably non-small cell lung cancer (NSCLC).

9. The composition for use according to claim 8, wherein the treatment further comprises the administration of a further active pharmaceutical ingredient, wherein the further active pharmaceutical ingredient is preferably a chemotherapeutic agent or a kinase inhibitor, and/or wherein the treatment further comprises radiation therapy.

10. The composition for use according to claim 9, wherein the further active pharmaceutical ingredient is a PD-1 pathway inhibitor.

11. The composition for use according to claim 9, wherein the further active pharmaceutical ingredient is a tyrosine kinase inhibitor, preferably an inhibitor of epidermal growth factor receptor (EGFR) tyrosine kinase, preferably a tyrosine kinase inhibitor selected from the group consisting of erlotinib, gefitinib and afatinib.

12. The composition for use according to claim 9, wherein the further active pharmaceutical ingredient is a platinum-based chemotherapeutic agent.

13. The composition for use according to claim 12, wherein the treatment further comprises radiation therapy.

14. Combination of six mRNAs for use in the treatment of lung cancer, wherein each mRNA encodes one antigen selected from the group consisting of 5T4 (Trophoblast glycoprotein, TPBG), Survivin (Baculoviral IAP repeat-containing protein 5; BIRC5), NY-ESO-1 (New York esophageal squamous cell carcinoma 1, CTAG1B), MAGE-C1 (Melanoma antigen family C1), MAGE-C2 (Melanoma antigen family C2), and MUC1 (Mucin 1) and wherein each mRNA is identical or at least 80% identical to a different RNA sequence selected from the RNA sequences according to SEQ ID NO: 19, 20, 21, 22, 23 or 24.

15. Combination for use according to claim 14, wherein one mRNA encodes 5T4 and is identical or at least 80% identical to SEQ ID NO: 19, one mRNA encodes Survivin and is identical or at least 80% identical to SEQ ID NO: 20, one mRNA encodes NY-ESO-1 and is identical or at least 80% identical to SEQ ID NO: 21, one mRNA encodes MAGE-C1 and is identical or at least 80% identical to SEQ ID NO: 22, one mRNA encodes MAGE-C2 and is identical or at least 80% identical to SEQ ID NO: 23 and one mRNA encodes MUC1 and is identical or at least 80% identical to SEQ ID NO:24.

16. Combination for use according to claim 14 or 15, wherein each of the six mRNAs is administered separately.

17. Combination for use according to any of claims 14 to 16, wherein the mRNAs are administered by intradermal injection.

18. Combination for use according to any of claims 14 to 17, wherein the treatment comprises the administration of a further active pharmaceutical ingredient, wherein the further active pharmaceutical ingredient is preferably a chemotherapeutic agent or a kinase inhibitor, and/or wherein the treatment further comprises radiation therapy.

19. Combination for use according to claim 18, wherein the further active pharmaceutical ingredient is a PD-1 pathway inhibitor.

20. Combination for use according to claim 18, wherein the further active pharmaceutical ingredient is a tyrosine kinase inhibitor, preferably an inhibitor of epidermal growth factor receptor (EGFR) tyrosine kinase, preferably a tyrosine kinase inhibitor selected from the group consisting of erlotinib, gefitinib and afatinib.

21. Combination for use according to claim 18, wherein the further active pharmaceutical ingredient is a platinum-based chemotherapeutic agent.

22. Combination for use according to claim 21, wherein the treatment further comprises radiation therapy.

23. A kit, preferably kit of parts, comprising the composition according to any of claims 1 to 8, and/or a vaccine according to claim 7, and optionally a liquid vehicle for solubilising and optionally technical instructions with information on the administration and dosage of the active composition and/or the vaccine.

24. The kit according to claim 23, wherein the kit is a kit of parts and each part of the kit contains at least one mRNA preferably encoding a different antigen selected from the antigens defined in claim 1, all parts of the kit of parts forming the composition or the vaccine of the preceding claims.

25. The kit according to claim 23 or 24, wherein the kit contains at least two parts containing six mRNAs.

26. The kit according to any of claims 23 to 25, wherein all six mRNAs are provided in lyophilized form in separate parts.

27. The kit according to claim 26, wherein the kit contains as a part Ringer-Lactate solution.

28. The kit according to any of claims 23 to 27, wherein the kit contains six parts, each part containing one of the six mRNAs.
